# EUROPEAN PATENT APPLICATION

(11) **EP 2 703 401 A1**
(43) Date of publication of application: **05.03.2014**
(21) Application number: 12777702.7
(22) Date of filing: 25.04.2012
(51) Int. Cl.: C07D 417/04, A61K 31/541, A61P 25/00, A61P 25/28, A61P 43/00, C07D 417/14, C07D 491/048, C07D 491/056

(54) **PYRIDINE DERIVATIVE AND BACE-1 INHIBITOR CONTAINING SAME**

(30) Priority: 26.04.2011 JP 2011098109; 07.06.2011 JP 2011127204; 22.12.2011 JP 2011280862
(71) Applicant: Shionogi & Co., Ltd., Osaka-shi, Osaka 541-0045 (JP)
(72) Inventor: TADA, Yukio, Toyonaka-shi Osaka 561-0825 (JP); KUSAKABE, Ken-ichi, Toyonaka-shi Osaka 561-0825 (JP); TADANO, Genta, Toyonaka-shi Osaka 561-0825 (JP)
(74) Representative: Vossius & Partner
(86) International application number: PCT/JP2012/061030
(87) International publication number: WO 2012/147762

(57) **Abstract**

The present invention provides a compound of formula (I): 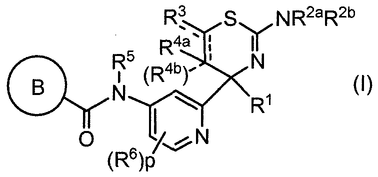 wherein ring B is substituted or unsubstituted carbocycle or heterocycle,
R¹ is substituted or unsubstituted alkyl or the like,
R^{2a} and R^{2b} are each independently hydrogen, substituted or unsubstituted alkyl or the like,
R³, R^{4a} and R^{4b} are each independently hydrogen, halogen, substituted or unsubstituted alkyl or the like, a dashed line represents a presence or absence of a bond,
R⁵ is hydrogen, substituted or unsubstituted alkyl or the like,
R⁶ is halogen, hydroxy, substituted or unsubstituted alkyl or the like,
p is an integer of 0 to 3,
or a pharmaceutically acceptable salt thereof which has an effect of inhibiting amyloid β production, especially an effect of inhibiting BACE1, and which is useful as a therapeutic or prophylactic agent for diseases induced by production, secretion and/or deposition of amyloid β proteins.

## Description

### [Field of the Invention]

The present invention relates to a compound having an effect of inhibiting amyloid β production and is useful as a therapeutic or prophylactic agent for diseases induced by production, secretion and/or deposition of amyloid β proteins.

### [Background Art]

In the brains of patients with Alzheimer's disease, peptides each consisting of approximately 40 amino acids, called amyloid ß proteins, which widely accumulate outside neurons to form insoluble plaques (senile plaques) are observed. These senile plaques are considered to kill neurons and cause the onset of Alzheimer's disease. As therapeutic agents for Alzheimer's disease, agents promoting degradation of amyloid β proteins and amyloid β vaccines have been studied.

Secretases are enzymes which cleave a protein called amyloid precursor protein (APP) within a cell and generate an amyloid β protein. An enzyme which produces N-terminals of amyloid β proteins is called as BACE1 (beta-site APP-cleaving enzyme 1, BACE1). It is considered that production of amyloid β proteins may be suppressed by inhibiting this enzyme, and thus a substance with such an effect can serve as a therapeutic or prophylactic agent for Alzheimer's disease.

Patent Documents 1 to 13 disclose compounds having a structure similar to those of the compounds of the present invention. Each of these documents discloses each of these compounds is useful as a therapeutic agent for Alzheimer's disease, Alzheimer's relating symptoms or diabetes, but each of these substantially disclosed compounds has a structure different from those of the compounds of the present invention.

### [Prior Art]

### [Patent Document]

Patent Document 1: WO2007/049532
Patent Document 2: WO 2008/133273
Patent Document 3: WO 2008/133274
Patent Document 4: WO 2009/151098
Patent Document 5: WO 2010/047372
Patent Document 6: WO 2011/058763
Patent Document 7: WO 2010/128058
Patent Document 8: WO 2009/134617
Patent Document 9: WO 2011/029803
Patent Document 10: WO 2011/071135
Patent Document 11: WO 2011/070781
Patent Document 12: WO 2011/154431
Patent Document 13: WO 2011/044181
Patent Document 14: WO 2011/071057

### [Summary of the invention]

### [Problems to be solved by the Invention]

The present invention provides a compound which has an effect of inhibiting amyloid β production, in particular BACE1 inhibitory effect, and is useful as a therapeutic or prophylactic agent for diseases induced by production, secretion or deposition of amyloid β proteins.

### [Means for Solving the Problem]

The present invention provides:
(1) A compound of formula (I): 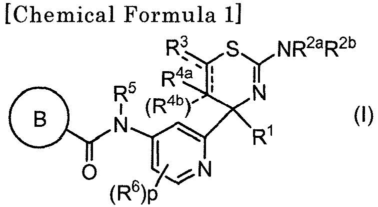 Wherein ring B is a substituted or unsubstituted carbocycle or a substituted or unsubstituted heterocycle, R¹ is substituted or unsubstituted alkyl, substituted or unsubstituted alkenyl, substituted or unsubstituted alkynyl, substituted or unsubstituted acyl, cyano, carboxy, substituted or unsubstituted alkoxycarbonyl, substituted or unsubstituted alkenyloxycarbonyl, substituted or unsubstituted alkynyloxycarbonyl, substituted or unsubstituted carbamoyl, substituted or unsubstituted thiocarbamoyl, a substituted or unsubstituted carbocyclic group or a substituted or unsubstituted heterocyclic group,
   R^{2a} and R^{2b} are each independently hydrogen, substituted or unsubstituted alkyl, substituted or unsubstituted acyl, substituted or unsubstituted alkoxycarbonyl or substituted or unsubstituted carbamoyl, 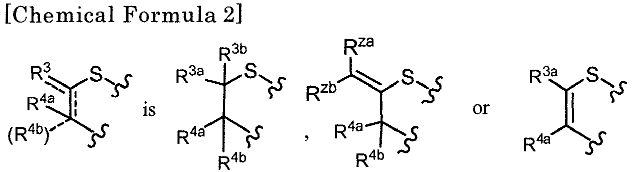 wherein R^{3a}, R^{3b}, R^{4a} and R^{4b} are each independently,
   hydrogen, halogen, hydroxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted alkylthio, substituted or unsubstituted alkenylthio, substituted or unsubstituted alkynylthio, substituted or unsubstituted acyl, substituted or unsubstituted acyloxy, cyano, nitro, carboxy, substituted or unsubstituted alkoxycarbonyl, substituted or unsubstituted alkenyloxycarbonyl, substituted or unsubstituted alkynyloxycarbonyl, substituted or unsubstituted amino, substituted or unsubstituted carbamoyl, substituted or unsubstituted thiocarbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl, substituted or unsubstituted alkynylsulfonyl, substituted or unsubstituted carbocyclyl, substituted or unsubstituted carbocyclyloxy, substituted or unsubstituted carbocyclylthio, substituted or unsubstituted carbocyclylalkyl, substituted or unsubstituted carbocyclylalkoxy, substituted or unsubstituted carbocyclyloxycarbonyl, substituted or unsubstituted carbocyclylsulfinyl, substituted or unsubstituted carbocyclylsulfonyl, substituted or unsubstituted heterocyclyl, substituted or unsubstituted heterocyclyloxy, substituted or unsubstituted heterocyclylthio, substituted or unsubstituted heterocyclylalkyl, substituted or unsubstituted heterocyclylalkoxy, substituted or unsubstituted heterocyclyloxycarbonyl, substituted or unsubstituted heterocyclylsulfinyl or substituted or unsubstituted heterocyclylsulfonyl,
   R^{3a} and R^{3b} together with the carbon atom to which they are attached may form a substituted or unsubstituted carbocycle or a substituted or unsubstituted heterocycle,
   R^{4a} and R^{4b} together with the carbon atom to which they are attached may form a substituted or unsubstituted carbocycle or a substituted or unsubstituted heterocycle,
   R^{Za} and R^{Zb} are each independently hydrogen, halogen, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted alkylthio, substituted or unsubstituted alkenylthio, substituted or unsubstituted alkynylthio, substituted or unsubstituted acyl, carboxy, substituted or unsubstituted alkoxycarbonyl, substituted or unsubstituted alkenyloxycarbonyl, substituted or unsubstituted alkynyloxycarbonyl, substituted or unsubstituted amino, substituted or unsubstituted carbamoyl, substituted or unsubstituted thiocarbamoyl, substituted or unsubstituted carbocyclyl, substituted or unsubstituted carbocyclyloxy, substituted or unsubstituted carbocyclylthio, substituted or unsubstituted carbocyclylalkyl, substituted or unsubstituted carbocyclylalkoxy, substituted or unsubstituted carbocyclyloxycarbonyl, substituted or unsubstituted heterocyclyl, substituted or unsubstituted heterocyclyloxy, substituted or unsubstituted heterocyclylthio, substituted or unsubstituted heterocyclylalkyl, substituted or unsubstituted heterocyclylalkoxy or substituted or unsubstituted heterocyclyloxycarbonyl, or
   R^{Za} and R^{Zb} together with the carbon atom to which they are attached may form a substituted or unsubstituted non-aromatic carbocycle or a substituted or unsubstituted non-aromatic heterocycle,
   R⁵ is hydrogen, substituted or unsubstituted alkyl, substituted or unsubstituted alkenyl, substituted or unsubstituted alkynyl or substituted or unsubstituted acyl,
   R⁶ is each independently halogen, hydroxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted alkylthio, substituted or unsubstituted alkenylthio, substituted or unsubstituted alkynylthio, substituted or unsubstituted acyl, substituted or unsubstituted acyloxy, cyano, nitro, carboxy, substituted or unsubstituted alkoxycarbonyl, substituted or unsubstituted alkenyloxycarbonyl, substituted or unsubstituted alkynyloxycarbonyl, substituted or unsubstituted amino, substituted or unsubstituted carbamoyl, substituted or unsubstituted thiocarbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl or substituted or unsubstituted alkynylsulfonyl,
   p is an integer of 0 to 3,
   provided that the following compounds are excluded: 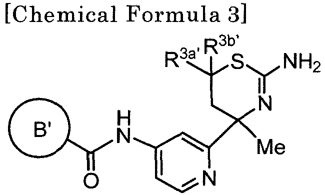 wherein R^{3a'} and R^{3b'} are both hydrogen or both methyl,
   ring B' is 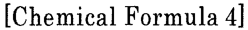 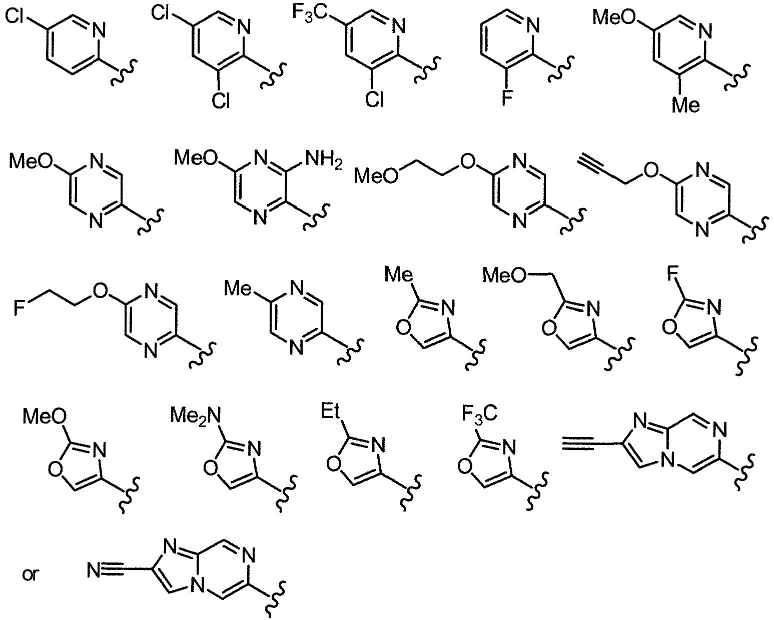 and Me is methyl,
   or a pharmaceutically acceptable salt thereof.
(2) The compound according to item (1) wherein R¹ is C1 to C3 halogenoalkyl, or a pharmaceutically acceptable salt thereof.
(3) The compound according to item (1) wherein R¹ is C1 to C3 unsubstituted alkyl, or a pharmaceutically acceptable salt thereof.
(4) The compound according to any one of items (1) to (3) wherein 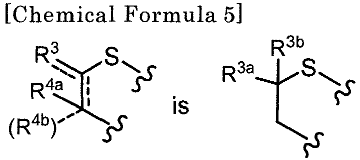 wherein R^{3a} and R^{3b} are each independently hydrogen, substituted or unsubstituted alkyl, or a pharmaceutically acceptable salt thereof.
(5) The compound according to item (4) wherein one of R^{3a} and R^{3b} is hydrogen and the other is substituted or unsubstituted alkyl, or a pharmaceutically acceptable salt thereof.
(6) The compound according to item (4) wherein one of R^{3a} and R^{3b} is hydrogen and the other is halogenoalkyl, or a pharmaceutically acceptable salt thereof.
(7) The compound according to item (4) wherein R^{3a} and R^{3b} are both hydrogen or both alkyl, and ring B is any one of the followings:
   1) pyridine which has at least one substituent selected from the group of dihalogenoalkyl, halogenoalkoxy, alkoxyalkoxy, cyanoalkoxy, alkenyl, alkoxyalkenyl, alkynyl, halogenoalkynyl, alkynyloxy, alkylthio, cyanoalkylthio, cyano, amino and cycloalkyl and which may have additional substituents,
   2) pyrazine optionally substituted with one or more selected from the group of halogen, halogenoalkyl, monohalogenomethoxy, monohalogenopropyloxy, dihalogenoalkoxy, trihalogenoalkoxy, ethoxyethoxy, cyanoalkoxy, alkenyl, alkynyl, halogenoalkynyl, alkylthio, cyanoalkylthio, cyano and amino, or
   3) substituted or unsubstituted benzene,
      or a pharmaceutically acceptable salt thereof.
(7') The compound according to item (4) wherein R^{3a} and R^{3b} are both hydrogen or both alkyl, and ring B is any one of the followings:
   1) pyridine which has at least one substituent selected from the group of dihalogenoalkyl, halogenoalkoxy, alkoxyalkoxy, cyanoalkoxy, alkenyl, alkynyl, halogenoalkynyl, alkynyloxy, alkylthio, cyanoalkylthio, cyano and amino,
   2) pyrazine optionally substituted with one or more selected from the group of halogen, halogenoalkyl, monohalogenomethoxy, monohalogenopropyloxy, dihalogenoalkoxy, trihalogenoalkoxy, ethoxyethoxy, cyanoalkoxy, alkenyl, alkynyl, halogenoalkynyl, alkylthio, cyanoalkylthio, cyano and amino, or
   3) substituted or unsubstituted benzene,
   or a pharmaceutically acceptable salt thereof.
(8) The compound according to any one of items (1) to (3) wherein 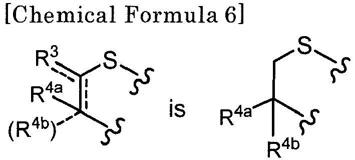 wherein one of R^{4a} and R^{4b} is hydrogen and the other is halogen or substituted or unsubstituted alkoxy, or R^{4a} and R^{4b} are both halogen, or a pharmaceutically acceptable salt thereof.
(9) The compound according to any one of items (1) to (3) wherein 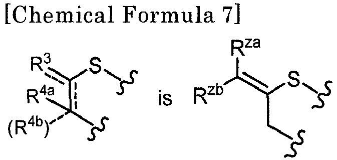 wherein R^{Za} and R^{Zb} are each independently hydrogen, halogen or substituted or unsubstituted alkyl,
   or a pharmaceutically acceptable salt thereof.
(10) The compound according to any one of items (1) to (3) wherein 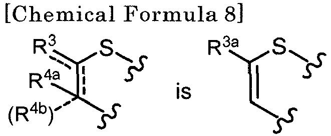 wherein R^{3a} is hydrogen or substituted or unsubstituted alkyl,
   or a pharmaceutically acceptable salt thereof.
(11) The compound according to any one of items (1) to (6) and (8) to (10) wherien ring B is substituted or unsubstituted pyridine, substituted or unsubstituted pyrimidine, substituted or unsubstituted pyrazine, substituted or unsubstituted furan, substituted or unsubstituted oxazole, substituted or unsubstituted thiazole, substituted or unsubstituted pyrazole, substituted or unsubstituted benzene, substituted or unsubstituted benzoxazole, substituted or unsubstituted benzothiazole, substituted or unsubstituted dihydrofuropyridine, substituted or unsubstituted dihydrodioxinopyridine or substituted or unsubstituted furopyridine, or a pharmaceutically acceptable salt thereof.
(11') The compound according to any one of items (1) to (6) and (8) to (10) wherien ring B is substituted or unsubstituted pyridine, substituted or unsubstituted pyrimidine, substituted or unsubstituted pyrazine, substituted or unsubstituted oxazole, substituted or unsubstituted thiazole, substituted or unsubstituted pyrazole, substituted or unsubstituted benzene, substituted or unsubstituted benzoxazole, substituted or unsubstituted benzothiazole, substituted or unsubstituted dihydrofuropyridine, substituted or unsubstituted dihydrodioxinopyridine or substituted or unsubstituted furopyridine, or a pharmaceutically acceptable salt thereof.
(11") The compound according to any one of items (1) to (6) and (8) to (10) wherien ring B is substituted or unsubstituted pyridine, substituted or unsubstituted pyrimidine, substituted or unsubstituted pyrazine, substituted or unsubstituted oxazole or substituted or unsubstituted benzene, or a pharmaceutically acceptable salt thereof.
(12) The compound according to any one of items (1) to (6), (8) to (11), (11') and (11") wherein ring B is optionally substituted with one or more selected from the group of halogen, hydroxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted alkylthio, substituted or unsubstituted alkenylthio, substituted or unsubstituted alkynylthio, cyano, nitro, substituted or unsubstituted amino and a substituted or unsubstituted carbocyclic group,
   or a pharmaceutically acceptable salt thereof.
(12') The compound according to any one of items (1) to (6), (8) to (11), (11') and (11") wherein ring B is optionally substituted with one or more selected from the group of halogen, hydroxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted alkylthio, substituted or unsubstituted alkenylthio, substituted or unsubstituted alkynylthio, cyano, nitro and substituted or unsubstituted amino,
   or a pharmaceutically acceptable salt thereof.
(13) The compound according to any one of items (1) to (7), (7'), (8) to (11), (11'), (11"), (12) and (12') wherein R^{2a} and R^{2b} are both hydrogen, or a pharmaceutically acceptable salt thereof.
(14) A pharmaceutical composition comprising the compound according to any one of items (1) to (7), (7'), (8) to (11), (11'), (11"), (12), (12') and (13), or a pharmaceutically acceptable salt thereof.
(15) A pharmaceutical composition having BACE1 inhibitory activity comprising the compound according to any one of items (1) to (7), (7'), (8) to (11), (11'), (11"), (12), (12') and (13), or a pharmaceutically acceptable salt thereof.
(16) A method for inhibiting BACE1 activity comprising administering the compound according to any one of items (1) to (7), (7'), (8) to (11), (11'), (11"), (12), (12') and (13), or a pharmaceutically acceptable salt thereof.
(17) A compound according to any one of items (1) to (7), (7'), (8) to (11), (11'), (11"), (12), (12') and (13), or a pharmaceutically acceptable salt thereof for use in a method for inhibiting BACE1 activity.
(18) Use of the compound according to any one of items (1) to (7), (7'), (8) to (11), (11'), (11"), (12), (12') and (13), or a pharmaceutically acceptable salt thereof in the manufacture of a medicament for inhibiting BACE1 activity.
(19) A method for treating or preventing diseases induced by production, secretion or deposition of amyloid β proteins comprising administering the compound according to any one of items (1) to (7), (7'), (8) to (11), (11'), (11"), (12), (12') and (13) or a pharmaceutically acceptable salt thereof.
(20) Use of the compound according to any one of items (1) to (7), (7'), (8) to (11), (11'), (11"), (12), (12') and (13) or a pharmaceutically acceptable salt thereof in the manufacture of a medicament for treating or preventing diseases induced by production, secretion or deposition of amyloid β proteins.
(21) A compound according to any one of items (1) to (7), (7'), (8) to (11), (11'), (11"), (12), (12') and (13) or a pharmaceutically acceptable salt thereof for use in a method for treating or preventing diseases induced by production, secretion or deposition of amyloid β proteins.
(22) A method for treating or preventing Alzheimer's disease comprising administering the compound according to any one of items (1) to (7), (7'), (8) to (11), (11'), (11"), (12), (12') and (13) or a pharmaceutically acceptable salt thereof.
(23) Use of the compound according to any one of items (1) to (7), (7'), (8) to (11), (11'), (11"), (12), (12') and (13) or a pharmaceutically acceptable salt thereof in the manufacture of a medicament for treating or preventing Alzheimer's disease.
(24) The compound according to any one of items (1) to (7), (7'), (8) to (11), (11'), (11"), (12), (12') and (13) or a pharmaceutically acceptable salt thereof for use in a method for treating or preventing Alzheimer's disease.
(25) A method, a system, an apparatus, a kit or the like for manufacturing the compound according to any one of items (1) to (7), (7'), (8) to (11), (11'), (11"), (12), (12') and (13) or a pharmaceutically acceptable salt thereof.
(26) A method, a system, an apparatus, a kit or the like for preparing a pharmaceutical composition comprising the compound according to any one of items (1) to (7), (7'), (8) to (11), (11'), (11"), (12), (12') and (13) or a pharmaceutically acceptable salt thereof.
(27) A method, a system, an apparatus, a kit or the like for use the compound according to any one of items (1) to (7), (7'), (8) to (11), (11'), (11"), (12), (12') and (13) or a pharmaceutically acceptable salt thereof.
(28) The pharmaceutical composition according to item (14) or (15) for treating or preventing a disease induced by production, secretion or deposition of amyloid β proteins.
(29) The pharmaceutical composition according to item (14) or (15) for treating or preventing Alzheimer's disease.

### [Effect of the Invention]

The compound of the present invention has BACE1 inhibitory activity and is useful as an agent for treating and/or preventing disease induced by production, secretion or deposition of amyloid β protein such as Alzheimer's disease.

### [Best Mode for Carrying Out the Invention]

Each meaning of terms used herein is described below. In the present specification, each term is used in a unified meaning. Both when used alone and in combination with another word, each term are used in the same meaning.
In the present specification, the term "halogen" includes fluorine, chlorine, bromine, and iodine.

The halogen portions in "halogenoalkyl", "dihalogenoalkyl", "halogenoalkoxy", "monohalogenomethoxy", "monohalogenopropyloxy", "dihalogenoalkoxy", "trihalogenoalkoxy" and "halogenoalkynyl" are the same as the above "halogen".

In the present specification, the term "alkyl" includes linear or branched alkyl of a carbon number of 1 to 15, for example, a carbon number of 1 to 10, for example, a carbon number of 1 to 6, and for example, a carbon number of 1 to 3. Examples include methyl, ethyl, n-propyl, isopropyl, n-butyl, isobutyl, sec-butyl, tert-butyl, n-pentyl, isopentyl, neopentyl, hexyl, isohexyl, n-heptyl, isoheptyl, n-octyl, isooctyl, n-nonyl, and n-decyl.

The alkyl portions in "alkoxy", "halogenoalkyl", "hydroxyalkyl", "halogenoalkoxy", "cyanoalkoxy", "hydroxyalkoxy", "alkoxycarbonyl", "halogenoalkoxycarbonyl", "alkylamino", "aminoalkyl", "alkoxyalkoxy", "alkoxyalkenyl", "alkoxyalkenyloxy", "alkylcarbamoyl", "hydroxyalkylcarbamoyl", "alkoxyimino", "alkylthio", "cyanoalkylthio", "alkylsulfonyl", "alkylsulfonylamino", "alkylsulfonylalkylamino", "alkylsulfonylimino", "alkylsulfinylamino", "alkylsulfinylalkylamino", "alkylsulfinylimino", "alkylsulfamoyl", "alkylsulfinyl", "carbocyclylalkyl", "carbocyclylalkoxy", "carbocyclylalkoxycarbonyl", "carbocyclylalkylamino", "carbocyclylalkylcarbamoyl", "cycloalkylalkyl", "cycloalkylalkoxy", "cycloalkylalkylamino", "cycloalkylalkoxycarbonyl", "cycloalkylalkylcarbamoyl", "arylalkyl", "arylalkoxy", "arylalkylamino", "arylalkoxycarbonyl", "arylalkylcarbamoyl", "heterocyclylalkyl", "heterocyclylalkoxy", "heterocyclylalkylamino", "heterocyclylalkoxycarbonyl" and "heterocyclylalkylcarbamoyl" are the same as the above "alkyl".

"Substituted or unsubstituted alkyl" may be substituted with one or more substituents selected from a substituent group α.

As used herein, the substituent group α is a group consisting of halogen, hydroxy, alkoxy, halogenoalkoxy, hydroxyalkoxy, alkoxyalkoxy, acyl, acyloxy, carboxy, alkoxycarbonyl, amino, acylamino, alkylamino, imino, hydroxyimino, alkoxyimino, alkylthio, carbamoyl, alkylcarbamoyl, hydroxyalkylcarbamoyl, sulfamoyl, alkylsulfamoyl, alkylsulfinyl, alkylsulfonyl, alkylsulfonylamino, alkylsulfonylalkylamino, alkylsulfonylimino, alkylsulfinylamino, alkylsulfinylalkylamino, alkylsulfinylimino, cyano, nitro, a carbocyclic group and a heterocyclic group wherein the carbocycle and heterocycle may be each substituted with one or more substituents selected from halogen, alkyl, hydroxy, and alkoxy.

Examples of the substituent of "substituted or unsubstituted alkoxy", "substituted or unsubstituted alkoxycarbonyl", "substituted or unsubstituted alkylthio", "substituted or unsubstituted alkylsulfonyl" and "substituted or unsubstituted alkylsulfinyl" are one or more substituents selected from the above-mentioned substituent group α.

Examples of "halogenoalkyl" are monofluoromethyl, difluoromethyl, trifluoromethyl, monochloromethyl, dichloromethyl, trichloromethyl, monofluoroethyl, difluoroethyl, trifluoroethyl, monochloroethyl, dichloroethyl and trichloroethyl.

Examples of "dihalogenoalkyl" are difluoromethyl, difluoroethyl, difluoropropyl, dichloromethyl, dichloroethyl and dichloropropyl.

Examples of "halogenoalkoxy" are monofluoromethoxy, difluoromethoxy, trifluoromethoxy, monochloromethoxy, dichloromethoxy, trichloromethoxy, monofluoroethoxy, difluoroethoxy, trifluoroethoxy, monochloroethoxy, dichloroethoxy and trichloroethoxy.

Examples of "dihalogenoalkoxy" are difluoromethoxy, difluoroethoxy, difluoropropyloxy, dichloromethoxy, dichloroethoxy and dichloropropyloxy.

Examples of "trihalogenoalkoxy" are trifluoromethoxy, trifluoroethoxy, trifluoropropyloxy, trichloromethoxy, trichloroethoxy and trichloropropyloxy.

Examples of "monohalogenomethoxy" are monofluoromethoxy and monochloromethoxy.

The term "alkylidene" includes a divalent group of the above "alkyl" and examples include methylidene, ethylidene, propylidene, isopropylidene, butylidene, pentylidene and hexylidene.

The term "alkenyl" includes linear or branched alkenyl of a carbon number of 2 to 15, for example, a carbon number of 2 to 10, for example, a carbon number of 2 to 6, and for example, a carbon number of 2 to 4, having one or more double bonds at any available position. Examples include vinyl, allyl, propenyl, isopropenyl, butenyl, isobutenyl, prenyl, butadienyl, pentenyl, isopentenyl, pentadienyl, hexenyl, isohexenyl, hexadienyl, heptenyl, octenyl, nonenyl, decenyl, undecenyl, dodecenyl, tridecenyl, tetradecenyl and pentadecenyl.

The alkenyl portions in "alkenyloxy", "alkenyloxycarbonyl", "alkoxyalkenyl", "alkoxyalkenyloxy", "alkenylthio", "alkenylamino", "alkenylsulfonyl" and "alkenylsulfinyl" are the same as the above "alkenyl".

The term "alkynyl" includes a linear or branched alkynyl of a carbon number of 2 to 10, for example, a carbon number of 2 to 8, for example, a carbon number 3 to 6, having one or more triple bonds at any available position. Examples include ethynyl, propynyl, butynyl, pentynyl, hexynyl, heptynyl, octynyl, nonynyl and decynyl. These may further a double bond at any available position.

The alkynyl portions in "alkynyloxy", "alkynyloxycarbonyl", "alkoxyalkynyloxy", "alkynylthio", "alkynylsulfinyl", "alkynylsulfonyl", and "alkynylamino" are the same as the above "alkynyl."

Examples of "halogenoalkynyl" are monofluoroethynyl, difluoroethynyl, trifluoroethynyl, monochloroethynyl, dichloroethynyl, trifluoroethynyl, monofluoropropynyl, difluoropropynyl, trifluoropropynyl, monochloropropynyl, dichloropropynyl, trifluoropropynyl, monofuluorobutynyl, difluorobutynyl, trifluorobutynyl, monochlorobutynyl, dichlorobutynyl and trifluorobutynyl.

Examples of the substituent of "substituted or unsubstituted alkenyl", "substituted or unsubstituted alkenyloxy", "substituted or unsubstituted alkenyloxycarbonyl", "substituted or unsubstituted alkenylthio", "substituted or unsubstituted alkenylsulfinyl", "substituted or unsubstituted alkenylsulfonyl", "substituted or unsubstituted alkynyl", "substituted or unsubstituted alkynyloxy", "substituted or unsubstituted alkynylthio", "substituted or unsubstituted alkynyloxycarbonyl", "substituted or unsubstituted alkynylsulfinyl" and "substituted or unsubstituted alkynylsulfonyl" are one or more substituents selected from the above-mentioned substituent group α.

Examples of the substituent of "substituted or unsubstituted amino", "substituted or unsubstituted carbamoyl", "substituted or unsubstituted thiocarbamoyl" and "substituted or unsubstituted sulfamoyl" are one to two substituents selected from alkyl, acyl, hydroxy, alkoxy, alkoxycarbonyl, a carbocyclic group and a heterocyclic group.

The term "acyl" includes formyl, alkylcarbonyl, alkenylcarbonyl, alkynylcarbonyl, carbocyclylcarbonyl, and heterocyclylcarbonyl. Examples are formyl, acetyl, propionyl, butyryl, isobutyryl, valeryl, pivaloyl, hexanoyl, acryloyl, propioloyl, methacryloyl, crotonoyl, benzoyl, cyclohexanecarbonyl, pyridinecarbonyl, furancarbonyl, thiophenecarbonyl, benzothiazolecarbonyl, pyrazinecarbonyl, piperidinecarbonyl and thiomorpholino.

The acyl portions in "acyloxy" and "acylamino" are the same as the above "acyl."

Examples of the substituents of "substituted or unsubstituted acyl" and "substituted or unsubstituted acyloxy" are one or more substituents selected from the substituent group α. The ring portions of carbocyclylcarbonyl and heterocyclylcarbonyl may be substituted with one or more substituents selected from alkyl, substituent group α, and alkyl substituted with one or more substituents selected from substituent group α.

The term "carbocyclic group" includes cycloalkyl, cycloalkenyl, aryl and non-aromatic fused carbocyclyl.

The term "cycloalkyl" includes a carbocyclic group of a carbon number of 3 to 10, for example, a carbon number of 3 to 8, and for example, a carbon number 4 to 8. Examples are cyclopropyl, cyclobutyl, cyclopentyl, cyclohexyl, cycloheptyl, cyclooctyl, cyclononyl and cyclodecyl.
The term "cycloalkane" includes a carbocycle of a carbon number of 3 to 10, for example, a carbon number of 3 to 8, and for example, a carbon number 4 to 8. Examples are cyclopropane, cyclobutane, cyclopentane, cyclohexane, cycloheptane, cyclooctane, cyclononane and cyclodecane.

The cycloalkyl portions in "cycloalkylalkyl", "cycloalkyloxy", "cycloalkylalkoxy", "cycloalkylthio", "cycloalkylamino", "cycloalkylalkylamino", "cycloalkylsulfamoyl", "cycloalkylsulfonyl", "cycloalkylcarbamoyl", "cycloalkylalkylcarbamoyl", "cycloalkylalkoxycarbonyl" and "cycloalkyloxycarbonyl" are the same as that of the above "cycloalkane."

The term "cycloalkenyl" includes a group having one or more double bonds at optionally positions in the ring of the above "cycloalkyl". Examples are cyclopropenyl, cyclobutenyl, cyclopentenyl, cyclohexenyl, cycloheptenyl, cyclooctenyl and cyclohexadienyl.

The term "cycloalkene" includes a group having one or more double bonds at optionally positions in the ring of the above "cycloalkane". Examples are cyclopropene, cyclobutene, cyclopentene, cyclohexene, cycloheptene, cyclooctene and cyclohexadiene.

The term "aryl" includes phenyl, naphthyl, anthryl and phenanthryl. Specific example is phenyl.

The term "aromatic carbocycle" includes benzene, naphthalene, anthracene, and phenanthrene.

The term "non-aromatic fused carbocyclic group" includes non-aromatic groups wherein two or more rings selected from the above "cycloalkane", "cycloalkene" and "aromatic carbocycle", and at least one ring is "cycloalkane" or "cycloalkene" are fused. Examples are indanyl, indenyl, tetrahydronaphthyl and fluorenyl.

The carbocycle portions in "non-aromatic carbocycle" are the same as that of the above "cycloalkane", "cycloalkene" or the ring portions of "non-aromatic fused carbocyclic group." Examples are cyclopropane, cyclobutane, cyclopentane, cyclohexane, cycloheptane, cyclooctane, cyclononane, cyclodecane, cyclopropene, cyclobutene, cyclopentene, cyclohexene, cycloheptene, cyclooctene, indane, indene, tetrahydronaphthalene and fluorene.

The carbocycle portions in "carbocycle", "carbocyclyloxy", "carbocyclylalkyl", "carbocyclylalkoxy", "carbocyclylalkoxycarbonyl", "carbocyclylthio", "carbocyclylamino", "carbocyclylalkylamino", "carbocyclylcarbonyl", "carbocyclylsulfamoyl", "carbocyclylsulfonyl", "carbocyclylcarbamoyl", "carbocyclylalkylcarbamoyl", "carbocyclyloxycarbonyl" and "carbocyclylsulfinyl" are the same as that of the above "carbocyclic group."

The aryl portions in "arylalkyl", "aryloxy", "aryloxycarbonyl", "arylalkoxycarbonyl", "arylthio", "arylamino", "arylalkoxy", "arylalkylamino", "arylsulfonyl", "arylsulfamoyl", "arylcarbamoyl" and "arylalkylcarbamoyl" are the same as that of the above "aryl."

The term "heterocyclyl" includes a heterocyclic group comprising one or more rings and having one or more the same or different hetero atoms arbitrarily selected from O, S, and N in the ring. Specific examples are 5- or 6-membered heteroaryl such as pyrrolyl, imidazolyl, pyrazolyl, pyridyl, pyridazinyl, pyrimidinyl, pyrazinyl, triazolyl, triazinyl, tetrazolyl, furyl, thienyl, isoxazolyl, oxazolyl, oxadiazolyl, isothiazolyl, thiazolyl, and thiadiazolyl;
non-aromatic heterocyclyl such as dioxanyl, thiiranyl, oxiranyl, oxetanyl, oxathiolanyl, azetidinyl, thianyl, thiazolidinyl, pyrrolidinyl, pyrrolinyl, imidazolidinyl, imidazolinyl, pyrazolidinyl, pyrazolinyl, piperidyl, piperazinyl, morpholinyl, morpholino, thiomorpholinyl, thiomorpholino, dihydropyridyl, tetrahydropyridyl, tetrahydrofuryl, tetrahydropyranyl, dihydrothiazolyl, tetrahydrothiazolyl, tetrahydroisothiazolyl, dihydrooxazinyl, hexahydropyrimidinyl, hexahydroazepinyl, tetrahydrodiazepinyl, tetrahydropyridazinyl, dioxolanyl, dioxazinyl, aziridinyl, dioxolinyl, oxepanyl, thiolanyl, thiinyl, and thiazinyl;
fused bicyclic heterocyclyl such as indolyl, isoindolyl, indazolyl, indolizinyl, indolinyl, isoindolinyl, quinolyl, isoquinolyl, cinnolinyl, phthalazinyl, quinazolinyl, naphthyridinyl, quinoxalinyl, purinyl, pteridinyl, benzopyranyl, benzimidazolyl, benzotriazolyl, benzisooxazolyl, benzoxazolyl, benzoxadiazolyl, benzisothiazolyl, benzothiazolyl, benzothiadiazolyl, benzofuryl, isobenzofuryl, benzothienyl, benzotriazolyl, thienopyridyl, thienopyrrolyl, thienopyrazolyl, thienopyrazinyl, furopyrrolyl, furopyridyl, thienothienyl, imidazopyridyl, imidazopyrazolyl, pyrazolopyridyl, pyrazolopyrazinyl, thiazolopyridyl, oxazolopyridyl, pyrazolopyrimidinyl, pyrazolotriazinyl, pyridazolopyridyl, triazolopyridyl, imidazothiazolyl, pyrazinopyridazinyl, dihydrofuropyridyl, dihydrothiazolopyrimidinyl, tetrahydroquinolyl, tetrahydroisoquinolyl, dihydrobenzofuryl, dihydrobenzoxazinyl, dihydrobenzimidazolyl, tetrahydrobenzothienyl, tetrahydrobenzofuryl, benzodioxolyl, benzodioxonyl, chromanyl, chromenyl, octahydrochromenyl, dihydrobenzodioxinyl, dihydrobenzoxezinyl, dihydrobenzodioxepinyl, dihydrothienodioxinyl and dihydrodioxynopyridyl;
fused tricyclic heterocyclyl such as carbazolyl, acridinyl, xanthenyl, phenothiazinyl, phenoxathiinyl, phenoxazinyl, dibenzofuryl, imidazoquinolyl, and tetrahydrocarbazolyl.

Specific examples are 5- or 6-membered heteroaryl and non-aromatic heterocyclyl.

The heterocycle portions in "heterocycle", "heterocyclylalkyl", "heterocyclyloxy", "heterocyclylthio", "heterocyclylcarbonyl", "heterocyclylalkoxy", "heterocyclylamino", "heterocyclylsulfamoyl", "heterocyclylsulfonyl", "heterocyclylcarbamoyl", "heterocyclyloxycarbonyl", "heterocyclylalkylamino", "heterocyclylalkoxycarbonyl", "heterocyclylalkylcarbamoyl" and "heterocyclylsulfinyl" are the same as the above "heterocyclyl."

The heterocycle portions in "non-aromatic heterocycle" are the same as the heterocycle portions in the above "non-aromatic heterocyclyl." Specific examples are dioxane, thiirane, oxirane, oxetane, oxathiolane, azetidine, thiane, thiazolidine, pyrrolidine, pyrroline, imidazolidine, imidazoline, pyrazolidine, pyrazoline, piperidine, piperazine, morpholine, thiomorpholine, dihydropyridine, tetrahydropyridine, tetrahydrofuran, tetrahydropyran, dihydrothiazole, tetrahydrothiazole, tetrahydroisothiazole, dihydrooxazine, hexahydroazepine, tetrahydrodiazepine and tetrahydropyridazine.

A bond of the above "heterocyclyl" may be situated on any ring.

The term "heteroaryl" includes aromatic cyclic groups among the above "heterocyclyl."

Examples of the substituent of "substituted or unsubstituted carbocycle", "substituted or unsubstituted benzene", "substituted or unsubstituted heterocycle", "substituted or unsubstituted pyridine", "substituted or unsubstituted pyrimidine", "substituted or unsubstituted pyrazine", "substituted or unsubstituted oxazole", "substituted or unsubstituted thiazole", "substituted or unsubstituted pyrazole", "substituted or unsubstituted benzoxazole", "substituted or unsubstituted benzothiazole", "substituted or unsubstituted dihydrofuropyridine", "substituted or unsubstituted dihydrodioxynopyridine" and "substituted or unsubstituted furopyridine" in ring B include:
a group selected from the substituent group α such as halogen, hydroxy, alkoxy, acyl, acyloxy, carboxy, alkoxycarbonyl, carbamoyl, amino, cyano, alkylamino and/or alkylthio;
alkyl substituted with one or more groups selected from the substituent group α, hydroxyimino and alkoxyimino, wherein the substituent is, for example, halogen, hydroxy, alkoxy and/or alkoxycarbonyl, or unsubstituted alkyl;
aminoalkyl substituted with one or more groups selected from the substituent group α; wherein the substituent is, for example, acyl, alkyl and/or alkoxy;
alkenyl substituted with one or more substituents selected from the substituent group α, wherein the substituent is, for example, alkoxycarbonyl, halogen, and/or
halogenoalkoxycarbonyl, or unsubstituted alkenyl;
alkynyl substituted with one or more substituents selected from the substituent group α, wherein the substituent is, for example, alkoxycarbonyl, or unsubstituted alkynyl;
alkoxy substituted with one or more substituents selected from the substituent group α, wherein the substituent is, for example, halogen, carbamoyl, alkylcarbamoyl and/or hydroxyalkylcarbamoyl;
alkoxyalkoxy substituted with one or more substituents selected from the substituent group α;
alkenyloxy substituted with one or more substituents selected from the substituent group α, wherein the substituent is, for example, halogen, hydroxy, amino and/or alkylamino, or unsubstituted alkenyloxy;
alkoxyalkenyloxy substituted with one or more substituents selected from the substituent group α;
alkynyloxy substituted with one or more substituents selected from the substituent group α, wherein the substituent is, for example, halogen and/or hydroxy, or unsubstituted alkynyloxy;
alkoxyalkynyloxy substituted with one or more groups selected from the substituent group α;
alkylthio substituted with one or more substituents selected from the substituent group α, or unsubstituted alkylthio;
alkenylthio substituted with one or more substituents selected from the substituent group α, or unsubstituted alkenylthio;
alkynylthio substituted with one or more substituents selected from the substituent group α, or unsubstituted alkynylthio;
alkylamino substituted with one or more substituents selected from the substituent group α;
alkenylamino substituted with one or more substituents selected from the substituent group α;
alkynylamino substituted with one or more substituents selected from the substituent group α;
aminooxy substituted with one or more substituents selected from the substituent group α and alkylidene, or unsubstituted aminooxy;
acyl substituted with one or more substituents selected from the substituent group α; alkylcarbamoyl substituted with one or more substituents selected from the substituent group α;
alkoxycarbonyl substituted with one or more substituents selected from the substituent group α;
alkylsulfonyl substituted with one or more substituents selected from the substituent group α, or unsubstituted alkylsulfonyl;
alkylsulfinyl substituted with one or more substituents selected from the substituent group α, or unsubstituted alkylsulfinyl;
alkylsulfamoyl substituted with one or more substituents selected from the substituent group α;
a carbocyclic group such as cycloalkyl and aryl, substituted with one or more substituents selected from the substituent group α, azide, alkyl and halogenoalkyl;
a heterocyclic group substituted with one or more substituents selected from the substituent group α, azide, alkyl and halogenoalkyl;
carbocyclylalkyl such as cycloalkylalkyl and arylalkyl, substituted with one or more substituents selected from the substituent group α, azide, alkyl and halogenoalkyl, or unsubstituted carbocyclylalkyl;
heterocyclylalkyl substituted with one or more substituents selected from the substituent group α, azide, alkyl and halogenoalkyl, or unsubstituted heterocyclylalkyl;
carbocyclyloxy such as cycloalkyloxy and aryloxy, substituted with one or more substituents selected from the substituent group α, azide, alkyl and halogenoalkyl, or unsubstituted carbocyclyloxy;
heterocyclyloxy substituted with one or more substituents selected from the substituent group α, azide, alkyl and halogenoalkyl, or unsubstituted heterocyclyloxy;
carbocyclylalkoxy such as cycloalkylalkoxy and arylalkoxy, substituted with one or more substituents selected from the substituent group α, azide, alkyl and halogenoalkyl, or unsubstituted carbocyclylalkoxy such as cycloalkylalkoxy and arylalkoxy;
heterocyclylalkoxy substituted with one or more substituents selected from the substituent group α, azide, alkyl and halogenoalkyl, or unsubstituted heterocyclylalkoxy;
carbocyclylalkoxycarbonyl such as cycloalkylalkoxycarbonyl and arylalkoxycarbonyl, substituted with one or more substituents selected from the substituent group α, azide, alkyl and halogenoalkyl, or unsubstituted carbocyclylaloxycarbonyl such as cycloalkylalkoxycarbonyl and arylalkoxycarbonyl;
heterocyclylalkoxycarbonyl substituted with one or more substituents selected from the substituent group α, azide, alkyl and halogenoalkyl, or unsubstituted heterocyclylalkoxycarbonyl;
carbocyclylthio such as cycloalkylthio and arylthio, substituted with one or more substituents selected from the substituent group α, azide, alkyl and halogenoalkyl, or unsubstituted carbocyclylthio cycloalkylthio and arylthio;
heterocyclylthio substituted with one or more substituents selected from the substituent group α, azide, alkyl and halogenoalkyl, or unsubstituted heterocyclylthio;
carbocyclylamino such as cycloalkylamino and arylamino, substituted with one or more substituents selected from the substituent group α, azide, alkyl and halogenoalkyl, or unsubstituted carbocyclylamino such as cycloalkylamino and arylamino;
heterocyclylamino substituted with one or more substituents selected from the substituent group α, azide, alkyl and halogenoalkyl, or unsubstituted heterocyclylamino;
carbocyclylalkylamino such as cycloalkylalkylamino and arylalkylamino, substituted with one or more substituents selected from the substituent group α, azide, alkyl and halogenoalkyl or unsubstituted carbocyclylalkylamino such as cycloalkylalkylamino and arylalkylamino;
heterocyclylalkylamino substituted with one or more substituents selected from the substituent group α, azide, alkyl and halogenoalkyl, or unsubstituted heterocyclylalkylamino;
carbocyclylsulfamoyl such as cycloalkylsulfamoyl and arylsulfamoyl, substituted with one or more substituents selected from the substituent group α, azide, alkyl and halogenoalkyl, or unsubstituted carbocyclylsulfamoyl;
heterocyclylsulfamoyl substituted with one or more substituents selected from the substituent group α, azide, alkyl and halogenoalkyl, or unsubstituted heterocyclylsulfamoyl;
carbocyclylsulfonyl such as cycloalkylsulfonyl and arylsulfonyl, substituted with one or more substituents selected from the substituent group α, azide, alkyl and halogenoalkyl, or unsubstituted carbocyclylsulfonyl such as cycloalkylsulfonyl and arylsulfonyl;
heterocyclylsulfonyl substituted with one or more substituents selected from the substituent group α, azide, alkyl and halogenoalkyl, or unsubstituted heterocyclylsulfonyl;
carbocyclylcarbamoyl such as cycloalkylcarbamoyl and arylcarbamoyl, substituted with one or more substituents selected from the substituent group α, azide, alkyl and halogenoalkyl, or unsubstituted carbocyclylcarbamoyl such as cycloalkylcarbamoyl and arylcarbamoyl;
heterocyclylcarbamoyl substituted with one or more substituents selected from the substituent group α, azide, alkyl and halogenoalkyl, or unsubstituted heterocyclylcarbamoyl;
carbocyclylalkylcarbamoyl such as cycloalkylalkylcarbamoyl and arylalkylcarbamoyl, substituted with one or more substituents selected from the substituent group α, azide, alkyl and halogenoalkyl, or unsubstituted carbocyclylalkylcarbamoyl such as cycloalkylalkylcarbamoyl and arylalkylcarbamoyl;
heterocyclylalkylcarbamoyl substituted with one or more substituents selected from the substituent group α, azide, alkyl and halogenoalkyl, or unsubstituted heterocyclylalkylcarbamoyl;
carbocyclyloxycarbonyl such as cycloalkoxycarbonyl and aryloxycarbonyl, substituted with one or more substituents selected from the substituent group α, azide, alkyl and halogenoalkyl, or unsubstituted carbocyclyloxycarbonyl such as cycloalkoxycarbonyl and aryloxycarbonyl;
heterocyclyloxycarbonyl substituted with one or more substituents selected from the substituent group α, azide, alkyl and halogenoalkyl, or unsubstituted heterocyclyloxycarbonyl;
alkylenedioxy substituted with halogen, or unsubstituted alkylenedioxy; oxo; and
azide. The substituent is optionally substituted with one or more substituents selected from the above substituents.

Examples of the substituents of "substituted or unsubstituted carbocycle", "substituted or unsubstituted benzene", "substituted or unsubstituted heterocycle", "substituted or unsubstituted pyridine", "substituted or unsubstituted pyrimidine", "substituted or unsubstituted pyrazine", "substituted or unsubstituted furan", "substituted or unsubstituted oxazole", "substituted or unsubstituted thiazole", "substituted or unsubstituted pyrazole", "substituted or unsubstituted benzoxazole", "substituted or unsubstituted benzothiazole", "substituted or unsubstituted dihydrofuropyridine", "substituted or unsubstituted dihydrodioxynopyridine" or "substituted or unsubstituted furopyridine" in ring B are one or more substituents selected from:
halogen;
cyano;
hydroxy;
nitro;
carboxy;
alkyl substituted with one or more substituents selected from the substituent group α; unsubstituted alkyl;
alkenyl substituted with one or more substituents selected from the substituent group α; unsubstituted alkenyl;
alkynyl substituted with one or more substituents selected from the substituent group α; unsubstituted alkynyl;
alkoxy substituted with one or more substituents selected from the substituent group α; unsubstituted alkoxy;
alkenyloxy substituted with one or more substituents selected from the substituent group α;
unsubstituted alkenyloxy;
alkynyloxy substituted with one or more substituents selected from the substituent group α;
unsubstituted alkynyloxy;
alkylthio substituted with one or more substituents selected from the substituent group α;
unsubstituted alkylthio;
alkenylthio substituted with one or more substituents selected from the substituent group α;
unsubstituted alkenylthio;
alkynylthio substituted with one or more substituents selected from the substituent group α;
unsubstituted alkynylthio;
amino substituted with one or more substituents selected from the substituent group α; unsubstituted amino;
alkylamino substituted with one or more substituents selected from the substituent group α;
unsubstituted alkylamino;
cycloalkylamino substituted with one or more substituents selected from the substituent group α;
unsubstituted cycloalkylamino;
carbamoyl substituted with one or more substituents selected from the substituent group α;
unsubstituted carbamoyl;
alkylcarbamoyl substituted with one or more substituents selected from the substituent group α;
unsubstituted alkylcarbamoyl;
alkoxycarbonyl substituted with one or more substituents selected from the substituent group α;
unsubstituted alkoxycarbonyl;
a carbocyclic group substituted with one or more substituents selected from (i) alkyl substituted with one or more substituents selected from the substituent group α, (ii) unsubstituted alkyl and (iii) the substituent group α; and
an unsubstituted carbocyclic group;
a heterocyclic group substituted with one or more substituents selected from (i) alkyl substituted with one or more substituents selected from the substituent group α, (ii) unsubstituted alkyl and (iii) the substituent group α; and
an unsubstituted heterocyclic group.

More specifically, examples are one or more substituents selected from halogen, cyano, alkyl, halogenoalkyl, cycloalkylalkyl, benzyl, alkoxy, halogenoalkoxy, alkoxyalkoxy, cyanoalkoxy, alkenyl, halogenoalkenyl, alkoxyalkenyl, alkynyl, halogenoalkynyl, alkenyloxy, alkynyloxy, alkylthio, cyanoalkylthio, alkynylthio, amino, alkylamino, cycloalkyl, phenyl and a heterocyclic group.

In ring B, "pyridine which has at least one substituent selected from the group of dihalogenoalkyl, halogenoalkoxy, alkoxyalkoxy, cyanoalkoxy, alkenyl, alkoxyalkenyl, alkynyl, halogenoalkynyl, alkynyloxy, alkylthio, cyanoalkylthio, cyano, amino and cycloalkyl and which may have additional substituents" comprises pyridine which has at least one substituent selected from the group of dihalogenoalkyl, halogenoalkoxy, alkoxyalkoxy, cyanoalkoxy, alkenyl, alkoxyalkenyl, alkynyl, halogenoalkynyl, alkynyloxy, alkylthio, cyanoalkylthio, cyano, amino and cycloalkyl and which may have one or more substituents selected from the group defined in the above "substituted or unsubstituted pyridine".

In ring B, "pyrazine optionally substituted with one or more selected from the group of halogen, halogenoalkyl, monohalogenomethoxy, monohalogenopropyloxy, dihalogenoalkoxy, trihalogenoalkoxy, ethoxyethoxy, cyanoalkoxy, alkenyl, alkynyl, halogenoalkynyl, alkylthio, cyanoalkylthio, cyano and amino" comprises pyrazine optionally substituted with only one or more selected from the group of halogen, halogenoalkyl, monohalogenomethoxy, monohalogenopropyloxy, dihalogenoalkoxy, trihalogenoalkoxy, ethoxyethoxy, cyanoalkoxy, alkenyl, alkynyl, halogenoalkynyl, alkylthio, cyanoalkylthio, cyano and amino.

In the groups other than ring B, examples of the substituents of "a substituted or unsubstituted carbocyclic group", "substituted or unsubstituted carbocyclylthio", "substituted or unsubstituted carbocyclyloxycarbonyl", "a substituted or unsubstituted heterocyclic group", "substituted or unsubstituted carbocyclyloxy", "substituted or unsubstituted carbocyclylsulfinyl", "substituted or unsubstituted carbocyclylsulfonyl", "substituted or unsubstituted carbocycle", "substituted or unsubstituted carbocyclylalkyl", "substituted or unsubstituted carbocyclylalkoxy", "a substituted or unsubstituted heterocyclic group", "substituted or unsubstituted heterocyclyloxy", "substituted or unsubstituted heterocyclylthio", "substituted or unsubstituted heterocyclyloxycarbonyl", "substituted or unsubstituted heterocyclylsulfinyl", "substituted or unsubstituted heterocyclylsulfonyl", "substituted or unsubstituted heterocycle", "substituted or unsubstituted heterocyclylalkyl" and "substituted or unsubstituted heterocyclylalkoxy" are one or more substituents selected from (i) alkyl substituted with one or more substituents selected from the substituent group α, (ii) unsubstituted alkyl, and (iii) the substituent group α.

The alkylene portion in "alkylenedioxy" includes linear or branched divalent carbon chain of a carbon number 1 to 10, for example, a carbon number of 1 to 6, for example, a carbon number of 1 to 3. Specific examples are methylenedioxy and dimethylenedioxy.

"R^{Za} and R^{Zb} together with the carbon atom to which they are attached may form a substituted or unsubstituted non-aromatic carbocycle or a substituted or unsubstituted non-aromatic heterocycle" includes, for example,

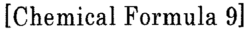

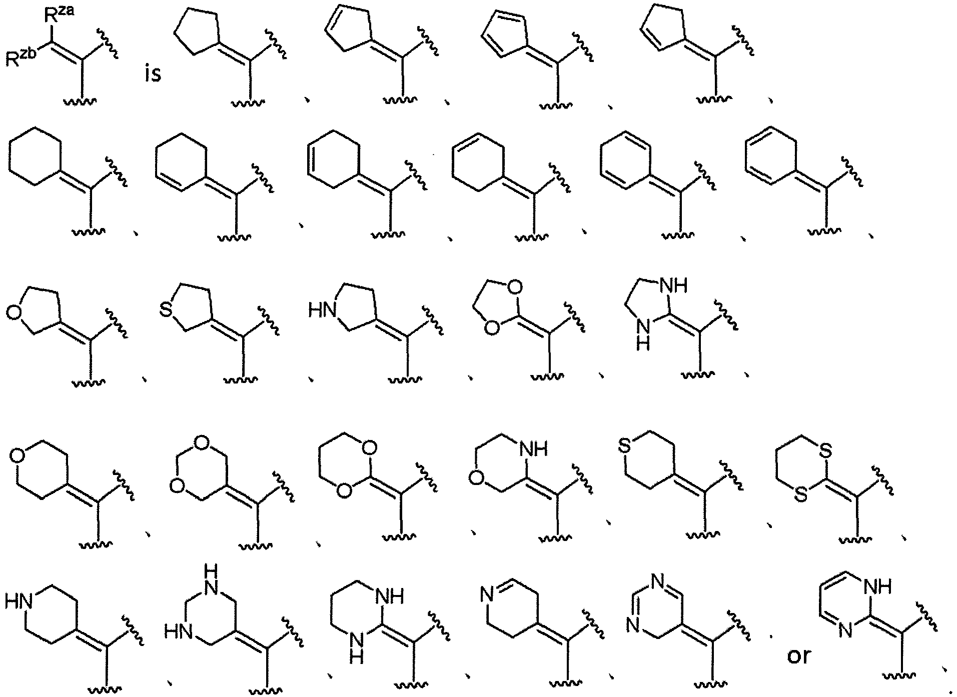

These are optionally substituted with one or more substituents selected from (i) alkyl substituted with one or more substituents selected from the substituent group α, (ii) unsubstituted alkyl, and (iii) the substituent group α at any available position.

"R^{3a} and R^{3b} together with the carbon atom to which they are attached may form a substituted or unsubstituted carbocycle or a substituted or unsubstituted heterocycle" and "R^{4a} and R^{4b} together with the carbon atom to which they are attached may form a substituted or unsubstituted carbocycle or a substituted or unsubstituted heterocycle" include

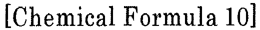

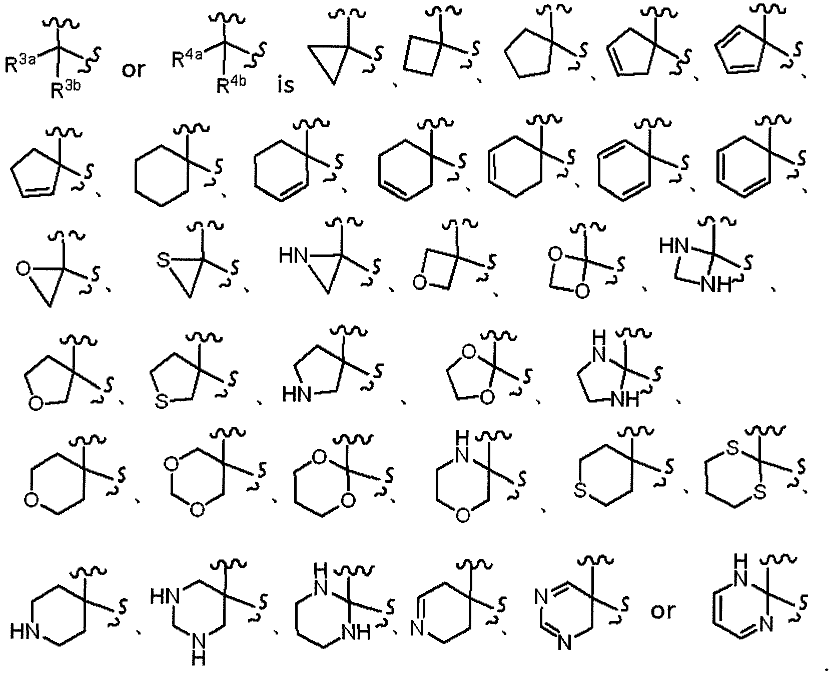

These are optionally substituted with one or more substituents selected from (i) alkyl substituted with one or more substituents selected from the substituent group α, (ii) unsubstituted alkyl, and (iii) the substituent group α at any available position.

The compound of formula (I) is not limited to a specific isomer, and includes all possible isomers such as keto-enol isomers, imine-enamine isomers, diastereoisomers, optical isomers and rotation isomers, racemate and the mixture thereof. For example, the compound of formula (I) in which R^{2a} is hydrogen includes the following tautomers.

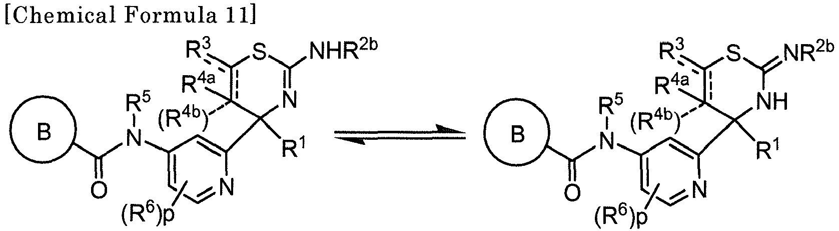

The compound of formula (I) has an asymmetric carbon atom and the compound includes the following optical isomers.

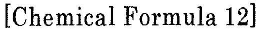

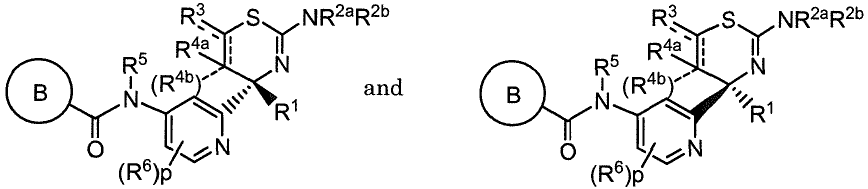

Preferable isomers are as follows.

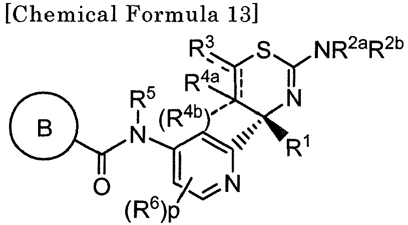

The optical isomer of the compound of formula (I) can be obtained with known methods such as chiral chromatography or diastereomer salt formation using an optical active acid or base.

One or more hydrogen, carbon and/or other atoms of a compound of formula (I) can be replaced by an isotope of the hydrogen, carbon and/or other atoms, respectively. Examples of isotopes include isotopes of hydrogen, carbon, nitrogen, oxygen, phosphorous, sulfur, fluorine, iodine and chlorine, such as ²H, ³H, ¹¹C, ¹³C, ¹⁴C, ¹⁵N, ¹⁸O, ¹⁷O, ³¹P, ³²P, ³⁵S, ¹⁸F, ¹²³I and ³⁶Cl, respectively. The compound of formula (I) also includes the compound replaced with such isotopes. The compound replaced with such isotopes is useful also as a medicament, and includes all the radiolabeled compounds of the compound of formula (I). The invention includes "radiolabelling method" for manufacturing the "radiolabeled compound" and the method is useful as a tool of metabolic pharmacokinetic research, the research in binding assay and/or diagnosis. Radiolabeled compounds of the compound of formula (I) can be prepared by methods known in the art. For example, tritiated compounds of formula (I) can be prepared by introducing tritium into the particular compound of formula (I) such as by catalytic dehalogenation with tritium. This method may include reacting a suitably halogenated precursor of a compound of formula (I) with tritium gas in the presence of a suitable catalyst such as Pd/C, in the presence or absence of a base. Other suitable methods for preparing tritiated compounds can be found in Isotopes in the Physical and Biomedical Sciences, Vol. 1, Labeled Compounds (Part A), Chapter 6 (1987). A ¹⁴C-labeled compound can be prepared by employing starting materials having ¹⁴C carbon.

As pharmaceutically acceptable salt of the compound of formula (I), examples include salts with alkaline metals (e.g. lithium, sodium and potassium), alkaline earth metals (e.g. calcium and barium), magnesium, transition metal (e.g. zinc and iron), ammonia, organic bases (e.g. trimethylamine, triethylamine, dicyclohexylamine, ethanolamine, diethanolamine, triethanolamine, meglumine, diethanolamine, ethylenediamine, pyridine, picoline, quinoline), and amino acids, and salts with inorganic acids (e.g. hydrochloric acid, sulfuric acid, nitric acid, carbonic acid, hydrobromic acid, phosphoric acid and hydroiodic acid) and organic acids (e.g. formic acid, acetic acid, propionic acid, trifluoroacetic acid, citric acid, lactic acid, tartaric acid, oxalic acid, maleic acid, fumaric acid, mandelic acid, glutaric acid, malic acid, benzoic acid, phthalic acid, ascorbic acid, benzenesulfonic acid, p-toluenesulfonic acid, methanesulfonic acid and ethanesulfonic acid). Specific examples are salts with hydrochloric acid, sulfuric acid, phosphoric acid, tartaric acid, or methanesulfonic acid. These salts may be formed by the usual methods.

The compound of formula (I) or its pharmaceutically acceptable salt may form solvate such as hydrate, and/or crystalline polymorphism, and the present invention also includes such various kinds of solvate and crystalline polymorphism. "Solvates" may be those wherein any number of solvent molecules (e.g., water molecules etc.) are coordinated with the compounds of formula (I). When the compounds of formula (I) or pharmaceutically acceptable salts thereof are allowed to stand in the atmosphere, the compounds may absorb water, resulting in attachment of adsorbed water or formation of hydrates. Recrystallization of the compounds of formula (I) or pharmaceutically acceptable salts thereof may produce crystal polymorphs.

The compound of formula (I) of the present invention or its pharmaceutically acceptable salt may form prodrug, and the present invention also includes such various prodrugs. Prodrugs are derivatives of the compounds of the present invention that have chemically or metabolically degradable groups and are compounds that are converted to the pharmaceutically active compounds of the present invention through solvolysis or under physiological conditions in vivo. Prodrugs include compounds that are converted to the compounds of formula (I) through enzymatic oxidation, reduction, hydrolysis and the like under physiological conditions in vivo and compounds that are converted to the compounds of formula (I) through hydrolysis by gastric acid and the like. Methods for selecting and preparing suitable prodrug derivatives are described, for example, in the Design of Prodrugs, Elsevier, Amsterdam 1985. Prodrugs themselves may be active compounds.

When the compounds of formula (I) or pharmaceutically acceptable salts thereof have a hydroxy group, prodrugs include acyloxy derivatives and sulfonyloxy derivatives which can be prepared by reacting a compound having a hydroxy group with a suitable acid halide, suitable acid anhydride, suitable sulfonyl chloride, suitable sulfonylanhydride and mixed anhydride or with a condensing agent. Examples are CH₃COO-, C₂H₅COO-, t-BuCOO-, C₁₅H₃₁COO-, PhCOO-, (m-NaOOCPh)COO-, NaOOCCH₂CH₂COO-, CH₃CH(NH₂) COO-, CH₂N(CH₃)₂COO-, CH₃SO₃-, CH₃CH₂SO₃-, CF₃SO₃-, CH₂FSO₃-, CF₃CH₂SO₃-, p-CH₃-O-PhSO₃-, PhSO₃- and p-CH₃PhSO₃-.

The compound of formula (I) can be prepared, for example, by the general synthetic procedure shown below. The methods for extraction, purification, and the like may be carried out by using the usual method for the experiments of organic chemistry.

The compounds of the present invention can be synthesized in consideration of the condition of the known methods in the art.

In the case that a substituent which inhibits a reaction (e.g. hydroxy, mercapto, amino, formyl, carbonyl and carboxy) exists in any of the following steps, the substituent may be preliminarily protected by, for example, the method described in "Protective Groups in Organic Synthesis, Theodora W Green (John Wiley & Sons)" (hereinafter referred to as Literature A), and the protecting group may be removed at an appropriate step. During all the following steps, the order of the steps to be performed may be appropriately changed. In each step, an intermediate may be isolated and then used in the next step. All of reaction time, reaction temperature, solvents, reagents, protecting groups, etc. are mere exemplification and not limited as long as they do not cause an adverse effect on a reaction.

### (General Synthetic Procedure 1)

The General Synthetic Procedure 1 is a method for preparing compounds of formula (Ia) from material (A) which can be prepared by known methods. 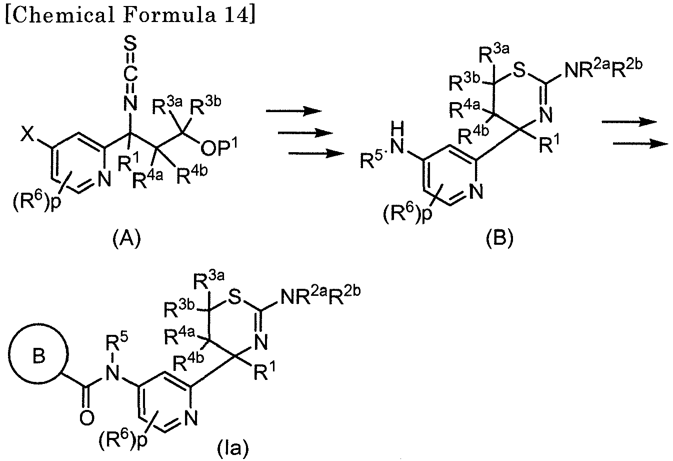 wherein X is a leaving group, P¹ is a hydroxyl protecting group, and other symbols are as defined above.

Synthesis of compound of formula (B) from compound of formula (A) can be conducted in a manner similar to the known methods described in Patent Document 3. For example, to compound (A) in a solvent such as ethyl acetate, dichloromethane, tetrahydrofuran and toluene can be added 15 to 30% aqueous ammonia or a reagent which has a substituent corresponding to the target compound such as tert-butylamine or the like at a temperature between about -30 °C to about 50 °C, preferably between about -10 °C to about 30 °C, then can be reacted at a temperature between about -10 °C to about 30 °C, preferably between about 0 °C to about 30 °C for 0.5 hours to 72 hours. In the case that R^{2a} and/or R^{2b} of the obtained compound is hydrogen, R^{2a} and/or R^{2b} can be introduced by the conventional method as necessary. The protecting group P¹ of the obtained compound is deprotected by the conventional method, and then the hydroxyl group can be converted to the leaving group to afford compound (B).

Examples of the leaving group include, for example, halogen, trifluoromethanesulfonyl or the like. Examples of the hydroxyl protecting group include, for example, tert-butyl, triphenylmethyl, benzyl, trimethylsilyl, tertbutyldimethylsilyl, tert-butyldiphenylsilyl, tribenzylsilyl, methoxymethyl, 1-ethoxyethyl, tetrahydropyranyl, tetrahydrothiopyranyl, benzyloxymethyl, methanesulfonyl, p-toluenesulfonyl, acetyl or the like.

The synthesis of compound of formula (Ia) from compound of formula (B) can be conducted in a manner similar to Patent Document 3 or the like. For example, an amine of formula (B) can be reacted with a carboxylic acid or an acid chloride of the ring B-CO₂H or the ring B-COCl under the known condition. In the case of the condensation reaction with the carboxylic acid of the ring B-CO₂H, preferably, examples of the condensation agent include 1-(3-dimethylaminopropyl)-3-ethylcarbodiimide (EDC), O-(benzotriazol-1-yl)-N,N,N',N'-tetramethyluronium hexafluorophosphate (HATU), diphenyl chlorophosphate or the like. An additive agent such as N,N-dimethyl-4-aminopyridine (DMAP) or the like can be added for accelerating the reaction. The reaction can be conducted at a temperature between about -30°C to room temperature, preferably between about 0°C to room temperature, for about 0.1 hours to about 24 hours, preferably about 30 minutes to about 3 hours.

When R^{2a} and/or R^{2b} of the obtained compound of formula (Ia) is an amino protecting group, for example, the protecting group can be removed suitably in accordance with the method described in Literature A or the like.

### (General Synthetic Procedure 2)

The General Synthetic Procedure 2 is a method for preparing compounds of formula (Ib) from compounds of formula (C). 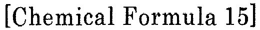 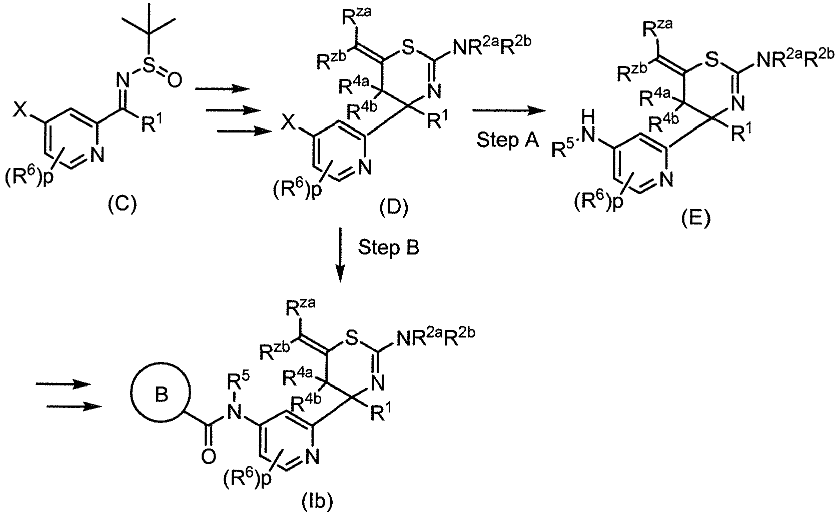 wherein each symbol is as defined above.

The synthesis of compounds of formula (D) from compound of formula (C) can be conducted in accordance with the method described in Patent Document 4 or the like. For example, an enolate obtained from the reaction with a corresponding alkylketone (for example, 3-methyl-2-butanone) can be added to compound (C) which is prepared by the known method in a solvent such as toluene, dichloromethane, tetrahydrofuran, a mixed solvent thereof or the like, in the presence of a base such as lithium diisopropylamide, potassium hexamethyldisilazide or the like, which is reacted at a temperature between about -80 °C to about 30 °C, preferably between about -80 °C to about 0°C, for about 0.1 hours to about 24 hours, preferably about 0.1 hours to about 12 hours. To the obtained compound is added hydrochloric acid, hydrobromic acid, trifluoroacetic acid or the like, which is reacted at a temperature between about 0 °C to about 60 °C, preferably between about 0 °C to about 30°C, for about 0.1 hours to about 24 hours, preferably about 0.5 hours to about 12 hours. The obtained compound in a solvent such as dioxane, tetrahydrofuran, toluene, acetone, a mixed solvent thereof or the like can be added to isothiocyanate with a protecting group (for example, benzoylisothiocyanate) which is commercially available or prepared by the known method and is reacted at a temperature between about -30 °C to about 70 °C, preferably between about -20 °C to about 50°C, for about 0.1 hours to about 12 hours, preferably about 0.1 hours to about 6 hours. After removal of the solvent, concentrated sulfuric acid, concentrated nitric acid or the like is added and reacted at a temperature between about -30 °C to about 70 °C, preferably between about -20 °C to about 50°C, for about 1 hour to about 12 hours, preferably about 1 hour to about 6 hours to afford compound (D).

### Step A:

This process is a step for preparing compounds of formula (E) from compounds of formula (D) by nucleophilic addition reaction with amine or Buchwald amination reaction using a palladium catalyst.

When the amino group is introduced by nucleophilic addition reaction, for example, compounds of formula (D) can be reacted with aqueous ammonia or compounds of formula NH₂R⁵ in an ether solvent such as tetrahydrofuran. This reaction is the conventional method and not limited to the above method, and it can be conducted in accordance with the method described in the various literatures.

When the amino group is introduced by Buchwald amination reaction, this reaction can use Xantphos as a phosphine ligand and cesium carbonate or the like as a base in the presence of a palladium catalyst such as tris(dibenzylideneacetone)dipalladium in a solvent such as dioxane or the like, which is reacted at a temperature between about 0 °C to about 120 °C, preferably between about 80 °C to about 90°C, for about 0.1 hours to about 24 hours, preferably about 2 hours to about 4 hours. This reaction is the conventional method and not limited to the above method, and it can be conducted according to the method described in the various literatures such as Metal-Catalyzed Cross-Coupling Reactions, Armin de Meijere (WILEY-VCH).

The synthesis of compounds of formula (Ib) from compounds of formula (E) can be conducted in accordance with the method described in the General Synthetic Procedure 1, the method described in Patent Document 3, Patent Document 4 or the like.

### Step B:

This process is a step for preparing compounds of formula (Ib) from compounds of formula (D) by Buchwald amidation reaction using a palladium catalyst. This reaction can be conducted in a manner similar to Buchwald amination reaction described in the above step A. This reaction is the conventional method and not limited to the above method, and it can be conducted in accordance with the method described in the various literatures such as Metal-Catalyzed Cross-Coupling Reactions, Armin de Meijere (WILEY-VCH).

When R^{2a} and/or R^{2b} of the obtained compounds of formula (Ia) are an amino protecting group, for example, the protecting group can be removed suitably using the method described in Literature A.

### (General Synthetic Procedure 3)

The General Synthetic Procedure 3 is a method for preparing compounds of formula (Ic) from compounds of formula (C) described in the above. 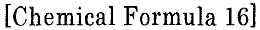 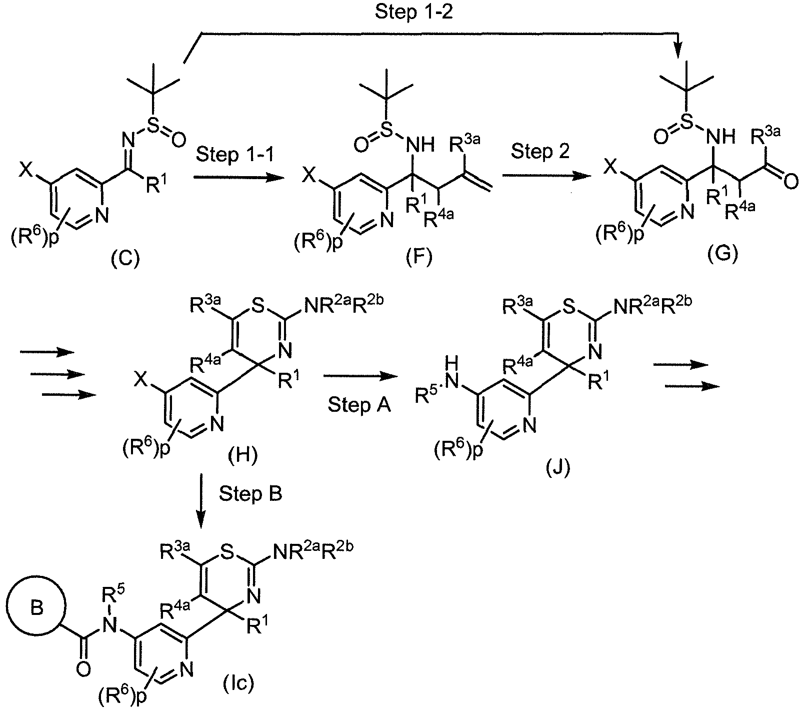 wherein each symbol is as defined above.

### Step 1-1:

This process is a step for preparing compounds of formula (F) from the starting material (C) by reacting with allyl Grignard reagent. As reaction solvents, ethers such as tetrahydrofuran, dioxane and the like are preferable. This reaction can be usually conducted at a temperature between about -80 °C to 0 °C, preferably between about -80 °C to about -45 °C, for about 0.1 hours to about 24 hours, preferably for about 30 minutes to about 3 hours. This reaction can be conducted in a manner similar to the conventional condition of the addition reaction of sulfinylimine with Grignard reagent, which is described in Chem. Rev. 2010, 110, 3600-3740.

### Step 1-2:

This process is a step for preparing compounds of formula (G) by Mannich reaction of compounds of formula (C) with an enolate derived from a ketone. For example, to an enolate obtained from the reaction with a corresponding phenylalkylketone (for example, acetophenone or the like) can be added compound (C) in a solvent such as toluene, dichloromethane, tetrahydrofuran, a mixed solvent thereof or the like, in the presence of a base such as lithium diisopropylamide, potassium hexamethyldisilazide or the like, and reacted at a temperature between about -80 °C to about 30 °C, preferably between about -80 °C to about 0°C, for about 0.1 hours to about 24 hours, preferably about 0.1 hours to about 12 hours to afford compound (G).

This process is the known method as with the above step 1-1, for example, it can be conducted by the method described in Chem. Rev. 2010, 110, 3600-3740.

### Step 2:

This process is a step for preparing compounds of formula (G) from compounds of formula (F) by ozonolysis reaction. This reaction can be usually conducted in an alcohol solvent such as methanol or a halogenated solvent such as dichloromethane at a temperature between about -80 °C to 0 °C, preferably between about -80 °C to about -45 °C. The reaction time is dependent on a blowing amount of ozone or an amount of compounds of formula (F), usually for about 0.1 hours to about 24 hours, preferably for about 30 minutes to about 2 hours.

### Step A:

This process is a step for converting compound of formula (H) to compound of formula (J). This process can be conducted in a manner similar to step A of the General Synthetic Procedure 2.

### Step B:

This process is a step for converting compound of formula (H) to compound of formula(Ic). This process can be conducted in a manner similar to step B of the General Synthetic Procedure 2.

When R^{2a} and R^{2b} in the obtained compounds of formula (Ic) are an amino protecting group, for example, the protecting group can be removed suitably using the method described in Literature A.

The order of above each process to be conducted can be changed suitably, and each intermediate can be used for the next step after isolation.

Optically active compounds of formula (I) can be produced by employing an optically active starting material, by obtaining an optically active intermediate by asymmetry synthesis at a suitable stage, or by performing optical resolution of an intermediate or an objective compound, each of which is a racemate, at a suitable stage. Examples of a method for optical resolution is separation of an optical isomer using an optically active column; kinetic optical resolution utilizing an enzymatic reaction; crystallization resolution of a diastereomer by salt formation using a chiral acid or a chiral base; and preferential crystallization method.

Specific embodiments of the present invention are illustrated below.

A compound of formula (IA): 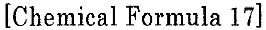 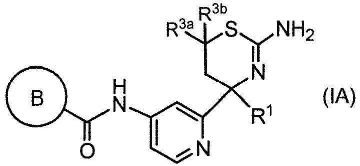 wherein each symbol is as defined above,
or a pharmaceutically acceptable salt thereof.

Specific embodiments of ring B, R¹, R^{3a} and R^{3b} are illustrated below. All combinations of these examples are illustrated as the compound of formula (IA).

Examples of ring B include a substituted or unsubstituted carbocycle or a substituted or unsubstituted heterocycle.

Examples of ring B include a substituted or unsubstituted heterocycle.

Examples of ring B include substituted or unsubstituted pyridine, substituted or unsubstituted pyrimidine, substituted or unsubstituted pyrazine, substituted or unsubstituted furan, substituted or unsubstituted oxazole, substituted or unsubstituted thiazole, substituted or unsubstituted pyrazole, substituted or unsubstituted benzene, substituted or unsubstituted benzoxazole, substituted or unsubstituted benzothiazole, substituted or unsubstituted dihydrofuropyridine, substituted or unsubstituted dihydrodioxynopyridine or substituted or unsubstituted furopyridine.

Examples of ring B include pyridine, pyrimidine, pyrazine, furan, oxazole, thiazole, pyrazole, benzene, benzoxazole, benzothiazole, dihydrofuropyridine, dihydrodioxynopyridine or furopyridine, each of which is optionally substituted with one or more substituents selected from the following groups; halogen, cyano, hydroxy, nitro, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted alkylthio, substituted or unsubstituted alkenylthio, substituted or unsubstituted alkynylthio, substituted or unsubstituted amino, a substituted or unsubstituted carbocyclic group and substituted or a unsubstituted heterocyclic group.

Examples of ring B include pyridine, pyrimidine, pyrazine, furan, oxazole, thiazole, pyrazole, benzene, benzoxazole, benzothiazole, dihydrofuropyridine, dihydrodioxynopyridine or furopyridine, each of which is optionally substituted with one or more substituents selected from the following groups; halogen, cyano, alkyl, halogenoalkyl, cycloalkylalkyl, benzyl, alkoxy, halogenoalkoxy, alkoxyalkoxy, cyanoalkoxy, alkenyl, halogenoalkenyl, alkoxyalkenyl, alkynyl, halogenoalkynyl, alkenyloxy, alkynyloxy, alkylthio, cyanoalkylthio, alkynylthio, amino, alkylamino, cycloalkyl, phenyl and a heterocyclic group.

Examples of ring B include pyridine or pyrazine, each of which is optionally substituted with one or more substituents selected from the following groups; halogen, cyano, alkyl, halogenoalkyl, cycloalkylalkyl, benzyl, alkoxy, halogenoalkoxy, alkoxyalkoxy, cyanoalkoxy, alkenyl, halogenoalkenyl, alkoxyalkenyl, alkynyl, halogenoalkynyl, alkenyloxy, alkynyloxy, alkylthio, cyanoalkylthio, alkynylthio, amino, alkylamino, cycloalkyl, phenyl and a heterocyclic group.

Examples of R^{3a} and R^{3b} include each independently hydrogen, substituted or unsubstituted alkyl, substituted or unsubstituted acyl, substituted or unsubstituted alkoxycarbonyl or substituted or unsubstituted carbamoyl.

Examples of R^{3a} and R^{3b} include each hydrogen.

Examples of R^{3a} and R^{3b} include one is hydrogen and the other is substituted or unsubstituted alkyl.

Examples of R^{3a} and R^{3b} include one is hydrogen and the other is halogenoalkyl. Examples of R^{3a} and R^{3b} include one is hydrogen and the other is alkoxyalkyl.

Examples of R^{3a} and R^{3b} include one is hydrogen and the other is halogenoalkoxyalkyl.

Examples of R^{3a} and R^{3b} include each alkyl.

Examples of R^{3a} and R^{3b} include each methyl.

Examples of R¹ include methyl.

Examples of R¹ include ethyl.

Examples of R¹ include halogenomethyl.

A compound of formula (IA'):

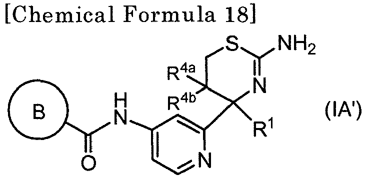

wherein each symbol is as defined above,
or a pharmaceutically acceptable salt thereof.

Specific embodiments of ring B, R¹, R^{4a} and R^{4b} are illustrated below. All combinations of these examples are illustrated as the compound of formula (IA').

Examples of ring B include a substituted or unsubstituted carbocycle or a substituted or unsubstituted heterocycle.

Examples of ring B include a substituted or unsubstituted heterocycle.

Examples of ring B include substituted or unsubstituted pyridine, substituted or unsubstituted pyrimidine, substituted or unsubstituted pyrazine, substituted or unsubstituted furan, substituted or unsubstituted oxazole, substituted or unsubstituted thiazole, substituted or unsubstituted pyrazole, substituted or unsubstituted benzene, substituted or unsubstituted benzoxazole, substituted or unsubstituted benzothiazole, substituted or unsubstituted dihydrofuropyridine, substituted or unsubstituted dihydrodioxynopyridine or substituted or unsubstituted furopyridine.

Examples of ring B include pyridine, pyrimidine, pyrazine, furan, oxazole, thiazole, pyrazole, benzene, benzoxazole, benzothiazole, dihydrofuropyridine, dihydrodioxynopyridine or furopyridine, each of which is optionally substituted with one or more substituents selected from the following groups; halogen, cyano, hydroxy, nitro, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted alkylthio, substituted or unsubstituted alkenylthio, substituted or unsubstituted alkynylthio, substituted or unsubstituted amino, a substituted or unsubstituted carbocyclic group and a substituted or unsubstituted heterocyclic group.

Examples of ring B include pyridine, pyrimidine, pyrazine, oxazole, thiazole, pyrazole, benzene, benzoxazole, benzothiazole, dihydrofuropyridine, dihydrodioxynopyridine or furopyridine, each of which is optionally substituted with one or more substituents selected from the following groups; halogen, cyano, alkyl, halogenoalkyl, cycloalkylalkyl, benzyl, alkoxy, halogenoalkoxy, alkoxyalkoxy, cyanoalkoxy, alkenyl, halogenoalkenyl, alkoxyalkenyl, alkynyl, halogenoalkynyl, alkenyloxy, alkynyloxy, alkylthio, cyanoalkylthio, alkynylthio, amino, alkylamino, cycloalkyl, phenyl and a heterocyclic group.

Examples of ring B include pyridine or pyrazine, each of which is optionally substituted with one or more substituents selected from the following groups; halogen, cyano, alkyl, halogenoalkyl, cycloalkylalkyl, benzyl, alkoxy, halogenoalkoxy, alkoxyalkoxy, cyanoalkoxy, alkenyl, halogenoalkenyl, alkoxyalkenyl, alkynyl, halogenoalkynyl, alkenyloxy, alkynyloxy, alkylthio, cyanoalkylthio, alkynylthio, amino, alkylamino, cycloalkyl, phenyl and a heterocyclic group.

Examples of R^{4a} and R^{4b} include each independently hydrogen, halogen, substituted or unsubstituted alkyl or substituted or unsubstituted alkoxy.

Examples of R^{4a} and R^{4b} include one is hydrogen and the other is substituted or unsubstituted alkoxy.

Examples of R^{4a} and R^{4b} include one is hydrogen and the other is halogen.

Examples of R^{4a} and R^{4b} include each halogen.

Examples of R¹ include methyl.

Examples of R¹ include ethyl.

Examples of R¹ include halogenomethyl.

A compound of formula (IB):

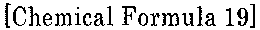

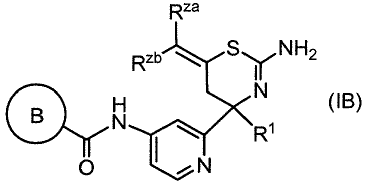

wherein each symbol is as defined above,
or a pharmaceutically acceptable salt thereof.

Specific embodiments of ring B, R¹, R^{Za} and R^{Zb} are illustrated below. All combinations of these examples are illustrated as the compound of formula (IB).

Examples of ring B include a substituted or unsubstituted carbocycle or a substituted or unsubstituted heterocycle.

Examples of ring B include a substituted or unsubstituted heterocycle.

Examples of ring B include substituted or unsubstituted pyridine, substituted or unsubstituted pyrimidine, substituted or unsubstituted pyrazine, substituted or unsubstituted furan, substituted or unsubstituted oxazole, substituted or unsubstituted thiazole, substituted or unsubstituted pyrazole, substituted or unsubstituted benzene, substituted or unsubstituted benzoxazole, substituted or unsubstituted benzothiazole, substituted or unsubstituted dihydrofuropyridine, substituted or unsubstituted dihydrodioxynopyridine or substituted or unsubstituted furopyridine.

Examples of ring B include pyridine, pyrimidine, pyrazine, furan, oxazole, thiazole, pyrazole, benzene, benzoxazole, benzothiazole, dihydrofuropyridine, dihydrodioxynopyridine or furopyridine, each of which is optionally substituted with one or more substituents selected from the following groups; halogen, cyano, hydroxy, nitro, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted alkylthio, substituted or unsubstituted alkenylthio, substituted or unsubstituted alkynylthio, substituted or unsubstituted amino, a substituted or unsubstituted carbocyclic group and a substituted or unsubstituted heterocyclic group.

Examples of ring B include pyridine, pyrimidine, pyrazine, furan, oxazole, thiazole, pyrazole, benzene, benzoxazole, benzothiazole, dihydrofuropyridine, dihydrodioxynopyridine or furopyridine, each of which is optionally substituted with one or more substituents selected from the following groups; halogen, cyano, alkyl, halogenoalkyl, cycloalkylalkyl, benzyl, alkoxy, halogenoalkoxy, alkoxyalkoxy, cyanoalkoxy, alkenyl, halogenoalkenyl, alkoxyalkenyl, alkynyl, halogenoalkynyl, alkenyloxy, alkynyloxy, alkylthio, cyanoalkylthio, alkynylthio, amino, alkylamino, cycloalkyl, phenyl and a heterocyclic group.

Examples of ring B include pyridine or pyrazine, each of which is optionally substituted with one or more substituents selected from the following groups; halogen, cyano, alkyl, halogenoalkyl, cycloalkylalkyl, benzyl, alkoxy, halogenoalkoxy, alkoxyalkoxy, cyanoalkoxy, alkenyl, halogenoalkenyl, alkoxyalkenyl, alkynyl, halogenoalkynyl, alkenyloxy, alkynyloxy, alkylthio, cyanoalkylthio, alkynylthio, amino, alkylamino, cycloalkyl, phenyl and a heterocyclic group.

Examples of R¹ include methyl.

Examples of R¹ include ethyl.

Examples of R¹ include halogenomethyl.

Examples of R^{Za} and R^{Zb} include each hydrogen.

Examples of R^{Za} and R^{Zb} include each methyl.

A compound of formula (IC): 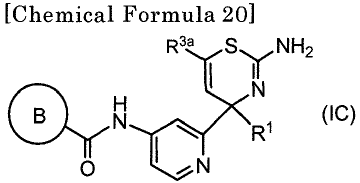 wherein each symbol is as defined above,
or a pharmaceutically acceptable salt thereof.

Specific embodiments of ring B, R¹ and R^{3a} are illustrated below. All combinations of these examples are illustrated as the compound of formula (IC).

Examples of ring B include a substituted or unsubstituted carbocycle or a substituted or unsubstituted heterocycle.

Examples of ring B include a substituted or unsubstituted heterocycle.

Examples of ring B include substituted or unsubstituted pyridine, substituted or unsubstituted pyrimidine, substituted or unsubstituted pyrazine, substituted or unsubstituted furan, substituted or unsubstituted oxazole, substituted or unsubstituted thiazole, substituted or unsubstituted pyrazole, substituted or unsubstituted benzene, substituted or unsubstituted benzoxazole, substituted or unsubstituted benzothiazole, substituted or unsubstituted dihydrofuropyridine, substituted or unsubstituted dihydrodioxynopyridine or substituted or unsubstituted furopyridine.

Examples of ring B include pyridine, pyrimidine, pyrazine, furan, oxazole, thiazole, pyrazole, benzene, benzoxazole, benzothiazole, dihydrofuropyridine, dihydrodioxynopyridine or furopyridine, each of which is optionally substituted with one or more substituents selected from the following groups; halogen, cyano, hydroxy, nitro, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted alkylthio, substituted or unsubstituted alkenylthio, substituted or unsubstituted alkynylthio, substituted or unsubstituted amino, a substituted or unsubstituted carbocyclic group and a substituted or unsubstituted heterocyclic group.

Examples of ring B include pyridine, pyrimidine, pyrazine, furan, oxazole, thiazole, pyrazole, benzene, benzoxazole, benzothiazole, dihydrofuropyridine, dihydrodioxynopyridine or furopyridine, each of which is optionally substituted with one or more substituents selected from the following groups; halogen, cyano, alkyl, halogenoalkyl, cycloalkylalkyl, benzyl, alkoxy, halogenoalkoxy, alkoxyalkoxy, cyanoalkoxy, alkenyl, halogenoalkenyl, alkoxyalkenyl, alkynyl, halogenoalkynyl, alkenyloxy, alkynyloxy, alkylthio, cyanoalkylthio, alkynylthio, amino, alkylamino, cycloalkyl, phenyl and a heterocyclic group.

Examples of ring B include pyridine or pyrazine, each of which is optionally substituted with one or more substituents selected from the following groups; halogen, cyano, alkyl, halogenoalkyl, cycloalkylalkyl, benzyl, alkoxy, halogenoalkoxy, alkoxyalkoxy, cyanoalkoxy, alkenyl, halogenoalkenyl, alkoxyalkenyl, alkynyl, halogenoalkynyl, alkenyloxy, alkynyloxy, alkylthio, cyanoalkylthio, alkynylthio, amino, alkylamino, cycloalkyl, phenyl and a heterocyclic group.

Examples of R^{3a} include hydrogen, substituted or unsubstituted alkyl, substituted or unsubstituted acyl, substituted or unsubstituted alkoxycarbonyl or substituted or unsubstituted carbamoyl.

Examples of R^{3a} include hydrogen.

Examples of R^{3a} include alkyl.

Examples of R^{3a} include halogenoalkyl.

Examples of R^{3a} include alkoxyalkyl.

Examples of R^{3a} include halogenoalkoxyalkyl.

Examples of R¹ include methyl.

Examples of R¹ include ethyl.

Examples of R¹ include halogenomethyl.

Examples of preferable combination of the substituents of the compounds of formula (IA) are as follows:
1) Compound wherein ring B is pyridine, pyrimidine, pyrazine, furan, oxazole, thiazole, pyrazole, benzene, benzoxazole, benzothiazole, dihydrofuropyridine, dihydrodioxynopyridine or furopyridine, each of which is optionally substituted with one or more substituents selected from the following groups; halogen, cyano, alkyl, halogenoalkyl, cycloalkylalkyl, benzyl, alkoxy, halogenoalkoxy, alkoxyalkoxy, cyanoalkoxy, alkenyl, halogenoalkenyl, alkoxyalkenyl, alkynyl, halogenoalkynyl, alkenyloxy, alkynyloxy, alkylthio, cyanoalkylthio, alkynylthio, amino, alkylamino, cycloalkyl, phenyl and a heterocyclic group, each of R^{3a} and R^{3b} is hydrogen, and R¹ is methyl.
2) Compound wherein ring B is pyridine or pyrazine, each of which is optionally substituted with one or more substituents selected from the following groups; halogen, cyano, alkyl, halogenoalkyl, cycloalkylalkyl, benzyl, alkoxy, halogenoalkoxy, alkoxyalkoxy, cyanoalkoxy, alkenyl, halogenoalkenyl, alkoxyalkenyl, alkynyl, halogenoalkynyl, alkenyloxy, alkynyloxy, alkylthio, cyanoalkylthio, alkynylthio, amino, alkylamino, cycloalkyl, phenyl and a heterocyclic group, each of R^{3a} and R^{3b} is hydrogen, and R¹ is methyl.
3) Compound wherein ring B is pyridine or pyrazine, each of which is optionally substituted with one or more substituents selected from the following groups; halogen, cyano, alkyl, halogenoalkyl, cycloalkylalkyl, benzyl, alkoxy, halogenoalkoxy, alkoxyalkoxy, cyanoalkoxy, alkenyl, halogenoalkenyl, alkoxyalkenyl, alkynyl, halogenoalkynyl, alkenyloxy, alkynyloxy, alkylthio, cyanoalkylthio, alkynylthio, amino, alkylamino, cycloalkyl, phenyl and a heterocyclic group, each of R^{3a} and R^{3b} is alkyl, and R¹ is methyl.
4) Compound wherein ring B is pyridine, pyrimidine, pyrazine, furan, oxazole, thiazole, pyrazole, benzene, benzoxazole, benzothiazole, dihydrofuropyridine, dihydrodioxynopyridine or furopyridine, each of which is optionally substituted with one or more substituents selected from the following groups; halogen, cyano, alkyl, halogenoalkyl, cycloalkylalkyl, benzyl, alkoxy, halogenoalkoxy, alkoxyalkoxy, cyanoalkoxy, alkenyl, halogenoalkenyl, alkoxyalkenyl, alkynyl, halogenoalkynyl, alkenyloxy, alkynyloxy, alkylthio, cyanoalkylthio, alkynylthio, amino, alkylamino, cycloalkyl, phenyl and a heterocyclic group, one of R^{3a} and R^{3b} is hydrogen and the other is alkyl, and R¹ is methyl.
5) Compound wherein ring B is pyridine or pyrazine, each of which is optionally substituted with one or more substituents selected from the following groups; halogen, cyano, alkyl, halogenoalkyl, cycloalkylalkyl, benzyl, alkoxy, halogenoalkoxy, alkoxyalkoxy, cyanoalkoxy, alkenyl, halogenoalkenyl, alkoxyalkenyl, alkynyl, halogenoalkynyl, alkenyloxy, alkynyloxy, alkylthio, cyanoalkylthio, alkynylthio, amino, alkylamino, cycloalkyl, phenyl and a heterocyclic group, one of R^{3a} and R^{3b} is hydrogen and the other is alkyl, and R¹ is methyl.
6) Compound wherein ring B is pyridine, pyrimidine, pyrazine, furan, oxazole, thiazole, pyrazole, benzene, benzoxazole, benzothiazole, dihydrofuropyridine, dihydrodioxynopyridine or furopyridine, each of which is optionally substituted with one or more substituents selected from the following groups; halogen, cyano, alkyl, halogenoalkyl, cycloalkylalkyl, benzyl, alkoxy, halogenoalkoxy, alkoxyalkoxy, cyanoalkoxy, alkenyl, halogenoalkenyl, alkoxyalkenyl, alkynyl, halogenoalkynyl, alkenyloxy, alkynyloxy, alkylthio, cyanoalkylthio, alkynylthio, amino, alkylamino, cycloalkyl, phenyl and a heterocyclic group, one of R^{3a} and R^{3b} is hydrogen and the other is halogenoalkyl, and R¹ is methyl.
7) Compound wherein ring B is pyridine or pyrazine, each of which is optionally substituted with one or more substituents selected from the following groups; halogen, cyano, alkyl, halogenoalkyl, cycloalkylalkyl, benzyl, alkoxy, halogenoalkoxy, alkoxyalkoxy, cyanoalkoxy, alkenyl, halogenoalkenyl, alkoxyalkenyl, alkynyl, halogenoalkynyl, alkenyloxy, alkynyloxy, alkylthio, cyanoalkylthio, alkynylthio, amino, alkylamino, cycloalkyl, phenyl and a heterocyclic group, one of R^{3a} and R^{3b} is hydrogen and the other is halogenoalkyl, and R¹ is methyl.
8) Compound wherein ring B is pyridine or pyrazine, each of which is optionally substituted with one or more substituents selected from the following groups; halogen, cyano, alkyl, halogenoalkyl, cycloalkylalkyl, benzyl, alkoxy, halogenoalkoxy, alkoxyalkoxy, cyanoalkoxy, alkenyl, halogenoalkenyl, alkoxyalkenyl, alkynyl, halogenoalkynyl, alkenyloxy, alkynyloxy, alkylthio, cyanoalkylthio, alkynylthio, amino, alkylamino, cycloalkyl, phenyl and a heterocyclic group, one of R^{3a} and R^{3b} is hydrogen and the other is alkoxyalkyl, and R¹ is methyl.
9) Compound wherein ring B is pyridine or pyrazine, each of which is optionally substituted with one or more substituents selected from the following groups; halogen, cyano, alkyl, halogenoalkyl, cycloalkylalkyl, benzyl, alkoxy, halogenoalkoxy, alkoxyalkoxy, cyanoalkoxy, alkenyl, halogenoalkenyl, alkoxyalkenyl, alkynyl, halogenoalkynyl, alkenyloxy, alkynyloxy, alkylthio, cyanoalkylthio, alkynylthio, amino, alkylamino, cycloalkyl, phenyl and a heterocyclic group, one of R^{3a} and R^{3b} is hydrogen and the other is halogenoalkoxyalkyl, and R¹ is methyl.
10) Compound wherein ring B is pyridine, pyrimidine, pyrazine, furan, oxazole, thiazole, pyrazole, benzene, benzoxazole, benzothiazole, dihydrofuropyridine, dihydrodioxynopyridine or furopyridine, each of which is optionally substituted with one or more substituents selected from the following groups; halogen, cyano, alkyl, halogenoalkyl, cycloalkylalkyl, benzyl, alkoxy, halogenoalkoxy, alkoxyalkoxy, cyanoalkoxy, alkenyl, halogenoalkenyl, alkoxyalkenyl, alkynyl, halogenoalkynyl, alkenyloxy, alkynyloxy, alkylthio, cyanoalkylthio, alkynylthio, amino, alkylamino, cycloalkyl, phenyl and a heterocyclic group, each of R^{3a} and R^{3b} is hydrogen, and R¹ is halogenomethyl.
11) Compound wherein ring B is pyridine or pyrazine, each of which is optionally substituted with one or more substituents selected from the following groups; halogen, cyano, alkyl, halogenoalkyl, cycloalkylalkyl, benzyl, alkoxy, halogenoalkoxy, alkoxyalkoxy, cyanoalkoxy, alkenyl, halogenoalkenyl, alkoxyalkenyl, alkynyl, halogenoalkynyl, alkenyloxy, alkynyloxy, alkylthio, cyanoalkylthio, alkynylthio, amino, alkylamino, cycloalkyl, phenyl and a heterocyclic group, each of R^{3a} and R^{3b} is hydrogen, and R¹ is halogenomethyl.

Examples of preferable combination of the substituents of the compounds of formula (IA') are as follows:
1) Compound wherein ring B is pyridine, pyrimidine, pyrazine, furan, oxazole, thiazole, pyrazole, benzene, benzoxazole, benzothiazole, dihydrofuropyridine, dihydrodioxynopyridine or furopyridine, each of which is optionally substituted with one or more substituents selected from the following groups; halogen, cyano, alkyl, halogenoalkyl, cycloalkylalkyl, benzyl, alkoxy, halogenoalkoxy, alkoxyalkoxy, cyanoalkoxy, alkenyl, halogenoalkenyl, alkoxyalkenyl, alkynyl, halogenoalkynyl, alkenyloxy, alkynyloxy, alkylthio, cyanoalkylthio, alkynylthio, amino, alkylamino, cycloalkyl, phenyl and a heterocyclic group, one of R^{4a} and R^{4b} is hydrogen and the other is alkoxy, and R¹ is methyl.
2) Compound wherein ring B is pyridine or pyrazine, each of which is optionally substituted with one or more substituents selected from the following groups; halogen, cyano, alkyl, halogenoalkyl, cycloalkylalkyl, benzyl, alkoxy, halogenoalkoxy, alkoxyalkoxy, cyanoalkoxy, alkenyl, halogenoalkenyl, alkoxyalkenyl, alkynyl, halogenoalkynyl, alkenyloxy, alkynyloxy, alkylthio, cyanoalkylthio, alkynylthio, amino, alkylamino, cycloalkyl, phenyl and a heterocyclic group, one of R^{4a} and R^{4b} is hydrogen and the other is alkoxy, and R¹ is methyl.
3) Compound wherein ring B is pyridine, pyrimidine, pyrazine, furan, oxazole, thiazole, pyrazole, benzene, benzoxazole, benzothiazole, dihydrofuropyridine, dihydrodioxynopyridine or furopyridine, each of which is optionally substituted with one or more substituents selected from the following groups; halogen, cyano, alkyl, halogeno alkyl, cycloalkylalkyl, benzyl, alkoxy, halogenoalkoxy, alkoxyalkoxy, cyanoalkoxy, alkenyl, halogenoalkenyl, alkoxyalkenyl, alkynyl, halogenoalkynyl, alkenyloxy, alkynyloxy, alkylthio, cyanoalkylthio, alkynylthio, amino, alkylamino, cycloalkyl, phenyl and a heterocyclic group, each of R^{4a} and R^{4b} is halogen, and R¹ is methyl.
4) Compound wherein ring B is pyridine or pyrazine, each of which is optionally substituted with one or more substituents selected from the following groups; halogen, cyano, alkyl, halogenoalkyl, cycloalkylalkyl, benzyl, alkoxy, halogenoalkoxy, alkoxyalkoxy, cyanoalkoxy, alkenyl, halogenoalkenyl, alkoxyalkenyl, alkynyl, halogenoalkynyl, alkenyloxy, alkynyloxy, alkylthio, cyanoalkylthio, alkynylthio, amino, alkylamino, cycloalkyl, phenyl and a heterocyclic group, each of R^{4a} and R^{4b} is halogen, and R¹ is methyl.

Examples of preferable combination of the substituents of the compounds of formula (IB) are as follows:
1) Compound wherein ring B is pyridine, pyrimidine, pyrazine, furan, oxazole, thiazole, pyrazole, benzene, benzoxazole, benzothiazole, dihydrofuropyridine, dihydrodioxynopyridine or furopyridine, each of which is optionally substituted with one or more substituents selected from the following groups; halogen, cyano, alkyl, halogenoalkyl, cycloalkylalkyl, benzyl, alkoxy, halogenoalkoxy, alkoxyalkoxy, cyanoalkoxy, alkenyl, halogenoalkenyl, alkoxyalkenyl, alkynyl, halogenoalkynyl, alkenyloxy, alkynyloxy, alkylthio, cyanoalkylthio, alkynylthio, amino, alkylamino, cycloalkyl, phenyl and a heterocyclic group, each of R^{Za} and R^{Zb} is hydrogen, and R¹ is methyl.
2) Compound wherein ring B is pyridine or pyrazine, each of which is optionally substituted with one or more substituents selected from the following groups; halogen, cyano, alkyl, halogenoalkyl, cycloalkylalkyl, benzyl, alkoxy, halogenoalkoxy, alkoxyalkoxy, cyanoalkoxy, alkenyl, halogenoalkenyl, alkoxyalkenyl, alkynyl, halogenoalkynyl, alkenyloxy, alkynyloxy, alkylthio, cyanoalkylthio, alkynylthio, amino, alkylamino, cycloalkyl, phenyl and a heterocyclic group, each of R^{Za} and R^{Zb} is hydrogen, and R¹ is methyl.
3) Compound wherein ring B is pyridine, pyrimidine, pyrazine, furan, oxazole, thiazole, pyrazole, benzene, benzoxazole, benzothiazole, dihydrofuropyridine, dihydrodioxynopyridine or furopyridine, each of which is optionally substituted with one or more substituents selected from the following groups; halogen, cyano, alkyl, halogenoalkyl, cycloalkylalkyl, benzyl, alkoxy, halogenoalkoxy, alkoxyalkoxy, cyanoalkoxy, alkenyl, halogenoalkenyl, alkoxyalkenyl, alkynyl, halogenoalkynyl, alkenyloxy, alkynyloxy, alkylthio, cyanoalkylthio, alkynylthio, amino, alkylamino, cycloalkyl, phenyl and a heterocyclic group, each of R^{Za} and R^{Zb} is methyl, and R¹ is methyl.
4) Compound wherein ring B is pyridine or pyrazine, each of which is optionally substituted with one or more substituents selected from the following groups; halogen, cyano, alkyl, halogenoalkyl, cycloalkylalkyl, benzyl, alkoxy, halogenoalkoxy, alkoxyalkoxy, cyanoalkoxy, alkenyl, halogenoalkenyl, alkoxyalkenyl, alkynyl, halogenoalkynyl, alkenyloxy, alkynyloxy, alkylthio, cyanoalkylthio, alkynylthio, amino, alkylamino, cycloalkyl, phenyl and a heterocyclic group, each of R^{Za} and R^{Zb} is methyl, and R¹ is methyl.

Examples of preferable combination of the substituents of the compounds of formula (IC) are as follows:
1) Compound wherein ring B is pyridine, pyrimidine, pyrazine, furan, oxazole, thiazole, pyrazole, benzene, benzoxazole, benzothiazole, dihydrofuropyridine, dihydrodioxynopyridine or furopyridine, each of which is optionally substituted with one or more substituents selected from the following groups; halogen, cyano, alkyl, halogenoalkyl, cycloalkylalkyl, benzyl, alkoxy, halogenoalkoxy, alkoxyalkoxy, cyanoalkoxy, alkenyl, halogenoalkenyl, alkoxyalkenyl, alkynyl, halogenoalkynyl, alkenyloxy, alkynyloxy, alkylthio, cyanoalkylthio, alkynylthio, amino, alkylamino, cycloalkyl, phenyl and a heterocyclic group, R^{3a} is hydrogen, and R¹ is methyl
2) Compound wherein ring B is pyridine or pyrazine, each of which is optionally substituted with one or more substituents selected from the following groups; halogen, cyano, alkyl, halogenoalkyl, cycloalkylalkyl, benzyl, alkoxy, halogenoalkoxy, alkoxyalkoxy, cyanoalkoxy, alkenyl, halogenoalkenyl, alkoxyalkenyl, alkynyl, halogenoalkynyl, alkenyloxy, alkynyloxy, alkylthio, cyanoalkylthio, alkynylthio, amino, alkylamino, cycloalkyl, phenyl and a heterocyclic group, R^{3a} is hydrogen, and R¹ is methyl.
3) Compound wherein ring B is pyridine, pyrimidine, pyrazine, furan, oxazole, thiazole, pyrazole, benzene, benzoxazole, benzothiazole, dihydrofuropyridine, dihydrodioxynopyridine or furopyridine, each of which is optionally substituted with one or more substituents selected from the following groups; halogen, cyano, alkyl, halogenoalkyl, cycloalkylalkyl, benzyl, alkoxy, halogenoalkoxy, alkoxyalkoxy, cyanoalkoxy, alkenyl, halogenoalkenyl, alkoxyalkenyl, alkynyl, halogenoalkynyl, alkenyloxy, alkynyloxy, alkylthio, cyanoalkylthio, alkynylthio, amino, alkylamino, cycloalkyl, phenyl and a heterocyclic group, R^{3a} is alkyl, and R¹ is methyl.
4) Compound wherein ring B is pyridine or pyrazine, each of which is optionally substituted with one or more substituents selected from the following groups; halogen, cyano, alkyl, halogenoalkyl, cycloalkylalkyl, benzyl, alkoxy, halogenoalkoxy, alkoxyalkoxy, cyanoalkoxy, alkenyl, halogenoalkenyl, alkoxyalkenyl, alkynyl, halogenoalkynyl, alkenyloxy, alkynyloxy, alkylthio, cyanoalkylthio, alkynylthio, amino, alkylamino, cycloalkyl, phenyl and a heterocyclic group, R^{3a} is alkyl, and R¹ is methyl.

The compounds of the present invention have BACE1 inhibitory activity, and therefore, are useful as a medicament for treatment, prevention, and/or symptom improvement of the diseases induced by the generation, secretion or deposition of amyloid β protein such as dementia of the Alzheimer's type (Alzheimer's disease, senile dementia of Alzheimer type), Down's syndrome, memory impairment, prion disease (Creutzfeldt-Jakob disease), mild cognitive impairment (MCI), Dutch type of hereditary cerebral hemorrhage with amyloidosis, cerebral amyloid angiopathy, other type of degenerative dementia, mixed dementia such as coexist Alzheimer's disease with vascular type dementia, dementia with Parkinson's Disease, dementia with progressive supranuclear palsy, dementia with Cortico-basal degeneration, Alzheimer's disease with diffuse Lewy body disease, age-related macular degeneration, Parkinson's Disease and amyloid angiopathy.

The term "treating Alzheimer's disease" includes prevention of progression of MCI and prevention of onset of familial Alzheimer's disease. The term "a pharmaceutical composition for treating Alzheimer's disease" includes a pharmaceutical composition for preventing progression of MCI, and a pharmaceutical composition for preventing onset of the familial Alzheimer's disease.

The compound of the present invention has not only BACE1 inhibitory activity but the beneficialness as a medicament. The compound has any or all of the following superior properties.
a) The compound has weak inhibitory activity for CYP enzymes such as CYP1A2, CYP2C9, CYP2C19, CYP2D6, CYP3A4.
b) The compound show excellent pharmacokinetics such as high bioavailability or moderate clearance.
c) The compound has high metabolic stability.
d) The compound does not show irreversible inhibition to CYP enzyme such as CYP3A4 in the range of the concentration of the measurement conditions described in this description.
e) The compound does not show mutagenesis.
f) The compound has low risk of cardiovascular systems.
g) The compound show high solubility.
h) The compound show high brain distribution.
i) The compound has high oral absorption.
j) The compound has long half-life period.
k) The compound has high protein unbinding ratio.
l) The compound show negative in the Ames test.

Since the compound of the present invention has high inhibitory activity on BACE1 and/or high selectivity on other enzymes, it can be a medicament with reduced side effect. Further, since the compound has high effect of reducing amyloid β production in a cell system, particularly, has high effect of reducing amyloid β production in brain, it can be an excellent medicament. In addition, by converting the compound into an optically active compound having suitable stereochemistry, the compound can be a medicament having a wider safety margin on the side effect.

When a pharmaceutical composition of the present invention is administered, it can be administered orally or parenterally. The composition for oral administration can be administered in usual dosage forms such as tablets, granules, powders, capsules which can be prepared according to the conventional manners. The composition for parenteral administration can be administered suitably in usual parenteral dosage forms such as injections. Since the compounds of the present invention have high oral absorption, they can be preferably administered in an oral dosage form.

A pharmaceutical composition can be formulated by mixing various additive agents for medicaments, if needed, such as excipients, binders, disintegrating agents, and lubricants which are suitable for the formulations with an effective amount of the compound of the present invention.

Although the dosage of a pharmaceutical composition of the present invention should be determined in consideration of the patient's age and body weight, the type and degree of diseases, the administration route and the like, a usual oral dosage for an adult is 0.05 to 100 mg/kg/day and preferable is 0.1 to 10 mg/kg/day. For parenteral administration, although the dosage highly varies with administration routes, a usual dosage is 0.005 to 10 mg/kg/day and preferably 0.01 to 1 mg/kg/day. The dosage may be administered once or several times per day.

The compound of the present invention can be used combining other medicaments for treating Alzheimer's disease such as acetylcholinesterase inhibitor (hereinafter referred to as a concomitant medicament) for the purpose of enforcement of the activity of the compound or reduction of the amount of medication of the compound or the like. In this case, timing of administration of the compound of the present invention and the concomitant medicament is not limited and these may be administered to the subject simultaneously or at regular intervals. Furthermore, the compound of the present invention and concomitant medicament may be administered as two different compositions containing each active ingredient or as a single composition containing both active ingredients.

The dose of the concomitant medicament can be suitably selected on the basis of the dose used on clinical. Moreover, the mix ratio of the compound of the present invention and a concomitant medicament can be suitably selected in consideration of the subject of administration, administration route, target diseases, symptoms, combinations, etc. For example, when the subject of administration is human, the concomitant medicament can be used in the range of 0.01 to 100 parts by weight relative to 1 part by weight of the compounds of the present invention.

Examples of a concomitant medicament are Donepezil hydrochloride, Tacrine, Galanthamine, Rivastigmine, Zanapezil, Memantine and Vinpocetine.

### [Example]

The present invention will be described in more detail with reference to, but not limited to, the following examples and test examples.

In this description, meaning of each abbreviation is as follows:

| | |
|---|---|
| Me | methyl |
| t-Bu | tert-butyl |
| Bz | benzoyl |
| Bn | benzyl |
| Boc | tert-butoxy carbonyl |
| DMSO | dimethyl sulfoxide |
| TFA | trifluoroacetic acid |

NMR analysis of each Example was performed by 300 MHz using DMSO-d₆ and CDCl₃.

"RT" in tables means retention time in LC/MS: liquid column chromatography/mass analysis, and these are measured under the conditions as mentioned below:
Conditions A
   Column: XBridge (registered trademark) C18 (5 µm, i.d.4.6 x 50 mm) (Waters)
   Flow rate: 3 mL/min
   UV detection wavelength: 254 nm
   Mobile phases:[A] is 0.1% formic acid solution, and [B] is 0.1% formic acid in acetonitrile solvent.
   Gradient: linear gradient of 10% to 100% solvent [B] for 3 minutes was performed, and 100% solvent [B] was maintained for 1 minute.
Conditions B
   Column: Shim-pack XR-ODS (2.2 µm, i.d.50 x 3.0 mm) (Shimadzu)
   Flow rate: 1.6 mL/min
   Columns oven: 50 °C
   UV detection wavelength: 254 nm
   Mobile phases: [A] is 0.1% formic acid solution, and[B] is 0.1% formic acid in acetonitrile solvent.
   Gradient: linear gradient of 10% to 100% solvent[B] for 3 minutes was performed, and 100 % solvent[B] was maintained for 1 minute.

### Example 1 Synthesis of compound (I-19)

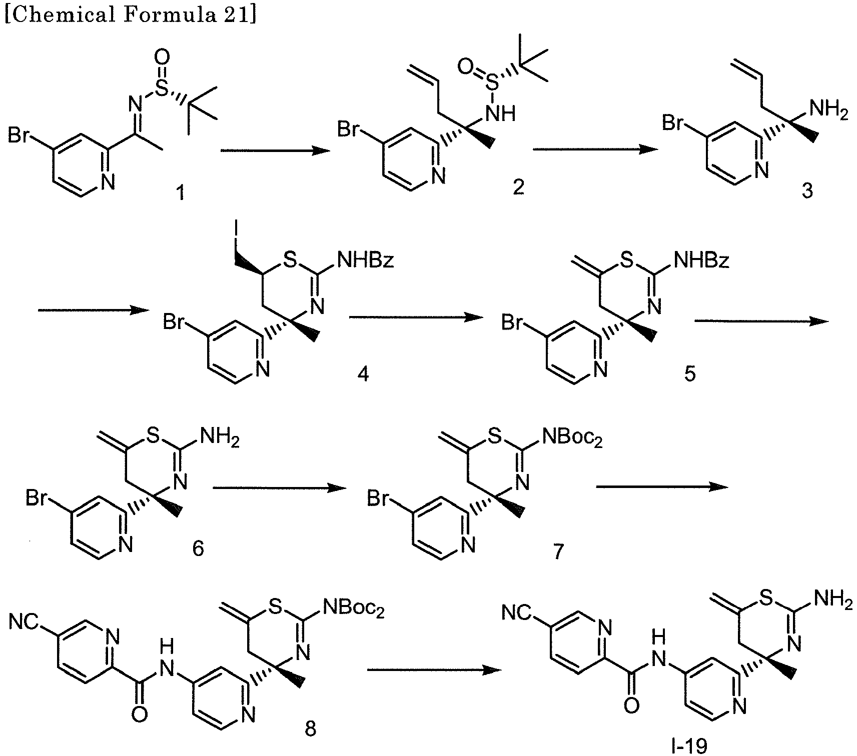

### Step 1

Allylmagnesium bromide (64.6 ml, 1 M tetrahydrofuran solution) was dissolved in tetrahydrofuran (120 ml) and diethyl ether (180 ml). To the solution was added compound (1) (9.80 g) dissolved in tetrahydrofuran (60 ml) at -70 °C, and the mixture was stirred for 30 minutes. To the reaction mixture were added an aqueous ammonium chloride solution and ethyl acetate, and the mixture was washed with water and brine. The organic layer was dried over sodium sulfate, and the solvent was evaporated under reduced pressure. The resulting residue was purified by silica gel column chromatography to afford compound (2) (7.01 g).
¹H-NMR (DMSO-d₆) δ: 1.14 (9H, s), 1.59 (3H, s), 2.63-2.81 (2H, m), 4.93-4.97 (1H, m), 4.99 (1H, s), 5.44 (1H, s), 5.55-5.70 (1H, m), 7.55 (1H, d, J = 5.1 Hz), 7.84 (1H, s), 8.43 (1H, d, J = 5.1 Hz).

### Step 2

Compound (2) (7.01 g) was dissolved in methanol (15 ml). To the solution was added hydrogen chloride (70.0 ml, 1.16 M methanol solution) at room temperature, and the mixture was stirred for 20 minutes. To the reaction mixture were added aqueous potassium carbonate solution and ethyl acetate, and the mixture was washed with water and brine. The organic layer was dried over sodium sulfate, and the solvent was evaporated under reduced pressure to afford compound (3) (3.72 g).
¹H-NMR (DMSO-d₆) δ: 1.34 (3H, s), 2.33-2.43 (1H, m), 2.49-2.59 (1H, m), 3.32 (2H, brs), 4.93 (1H, d, J = 5.1 Hz), 4.96 (1H, s), 5.66-5.53 (1H, m), 7.47 (1H, d, J = 5.1 Hz), 7.88 (1H, s), 8.41 (1H, d, J = 5.1 Hz).

### Step 3

Compound (3) (3.90 g) was dissolved in dichloromethane (40 ml). To the solution was added benzoyl isothiocyanate (2.22 ml) at 0 °C, and the mixture was stirred for 20 minutes. After the solvent was evaporated under reduced pressure, the residue was dissolved in acetonitrile (20 ml), and p-toluenesulfonic acid monohydrate (3.69 g) was added. Iodine (8.21 g) was dissolved in acetonitrile (140 ml) in another flask, and the above solution under ice bath was added. The mixture was stirred at room temperature for 2 hours. To the mixture were added sodium hydrogen sulfite and ethyl acetate, and the mixture was washed with water and brine. The organic layer was dried over sodium sulfate, and the solvent was evaporated under reduced pressure. The resulting residue was purified by silica gel column chromatography to afford compound (4) (5.82 g).
¹H-NMR (DMSO-d₆) δ: 1.65 (3H, s), 1.83 (1H, brs), 2.70-2.82 (1H, m), 3.06 (1H, d, J = 11.6 Hz), 3.42 (2H, d, J = 6.1 Hz), 7.44-7.66 (5H, m), 8.09 (2H, br s), 8.53 (1H, d, J = 5.1 Hz), 10.59 (1H, br s).

### Step 4

Potassium tert-butoxide (25.9 ml, 1 M tert-butanol solution) was dissolved in dimethoxyethane (85 ml). To the solution was added the solution of compound (4) (5.50 g) dissolved in dimethoxyethane (80 ml) at room temperature, and the mixture was stirred for 10 minutes. To the mixture were added an aqueous citric acid solution and ethyl acetate, and the mixture was washed with water and brine. The organic layer was dried over sodium sulfate, and the solvent was evaporated under reduced pressure. The obtained residue was purified by silica gel column chromatography to afford compound (5) (3.60 g).
¹H-NMR (DMSO-d₆) δ: 1.54 (3H, s), 2.43-2.55 (1H, m), 3.01 (1H, d, J = 12.1 Hz), 5.24 (2H, s), 7.45-7.64 (4H, m), 7.86 (1H, brs), 8.06 (2H, d, J = 6.6 Hz), 8.50 (1H, d, J = 4.5 Hz), 11.12 (1H, br s).

### Step 5

Compound (5) (3.60 g) was dissolved in ethanol (22 ml). To the solution was added hydrazine monohydrate (2.17 ml) at room temperature, and the solution was stirred for 3 hours. After the solvent was evaporated under reduced pressure, the resulting residue was purified by silica gel column chromatography to afford compound (6) (2.08 g).
¹H-NMR (DMSO-d₆) δ: 1.31 (3H, s), 2.40 (1H, d, J = 13.6 Hz), 2.67 (1H, d, J = 13.6 Hz), 5.03 (1H, s), 5.11 (1H, s), 6.26 (2H, s), 7.49 (1H, d, J = 4.5 Hz), 7.81 (1H, s), 8.42 (1H, d, J = 4.5 Hz).

### Step 6

Compound (6) (2.08 g) was dissolved in dichloromethane (20 ml). To the solution were added di-tert-butyl dicarbonate (4.05 ml)and N,N-dimethylaminopyridine (43.0 mg) at room temperature, and the mixture was stirred for 30 minutes. After the solvent was evaporated under reduced pressure, the resulting residue was purified by silica gel column chromatography to afford compound (7) (3.19 g).
¹H-NMR (DMSO-d₆) δ: 1.39 (3H, s), 1.45 (18H, s), 2.30 (1H, d, J = 13.6 Hz), 2.87 (1H, d, J = 13.6 Hz), 5.37 (1H, s), 5.46 (1H, s), 7.61 (1H, d, J = 5.1 Hz), 7.66 (1H, s), 8.49 (1H, d, J = 5.1 Hz).

### Step 7

4,5-bis(diphenylphosphino)-9,9-dimethylxanthene (711 mg) and tris(dibenzylideneaceton)dipalladium(0) (375 mg) were dissolved in 1,4-dioxane (20 ml) that was degassed and replaced with nitrogen gas. The solution was stirred at room temperature for 10 minutes. To the solution were added cesium carbonate (1.55 g), compound (7) (1.98 g) dissolved in 1,4-dioxane (50 ml) and 5-cyano picolinamide (701 mg), and the mixture was stirred at 80 °C for 1 hour, at 90 °C for 6 hours and 15 minutes and at 100 °C for 1 hour. To the mixture were added an aqueous citric acid solution and ethyl acetate, and the mixture was washed with water and brine. The organic layer was dried over sodium sulfate, and the solvent was evaporated under reduced pressure. The resulting residue was purified by silica gel column chromatography to afford compound (8) (1.12 g).
¹H-NMR (DMSO-d₆) δ: 1.36 (3H, s), 1.42 (18H, s), 2.24 (1H, d, J = 13.6 Hz), 3.00 (1H, d, J = 13.6 Hz), 5.35 (1H, s), 5.48 (1H, s), 7.78 (1H, d, J = 5.0 Hz), 8.23 (1H, s), 8.27 (1H, d, J = 7.6 Hz), 8.50 (1H, d, J = 5.0 Hz), 8.59 (1H, d, J = 7.6 Hz), 9.20 (1H, s), 11.15 (1H, s).

### Step 8

Compound (8) (1.26 g) was dissolved in formic acid (1.37 ml). The solution was stirred at room temperature for 3.5 hours. The solution was added into aqueous potassium carbonate solution, and the obtained solid was collected by filtration and triturated from hexane-ethyl acetate to afford compound (I-19) (634 mg).
1H-NMR (DMSO-d₆) δ: 1.36 (3H, s), 2.53-2.62 (1H, m), 3.31-3.42 (1H, m), 4.98 (1H, s), 5.08 (1H, s), 6.13 (2H, s), 7.73 (1H, dd, J = 5.3, 2.3 Hz), 8.11 (1H, d, J = 2.3 Hz), 8.29 (1H, d, J = 8.1 Hz), 8.45 (1H, d, J = 5.3 Hz), 8.60 (1H, dd, J = 2.3, 8.1 Hz), 9.22 (1H, d, J = 2.3 Hz), 11.04 (1H, s).

### Example 2 Synthesis of compound (I-21)

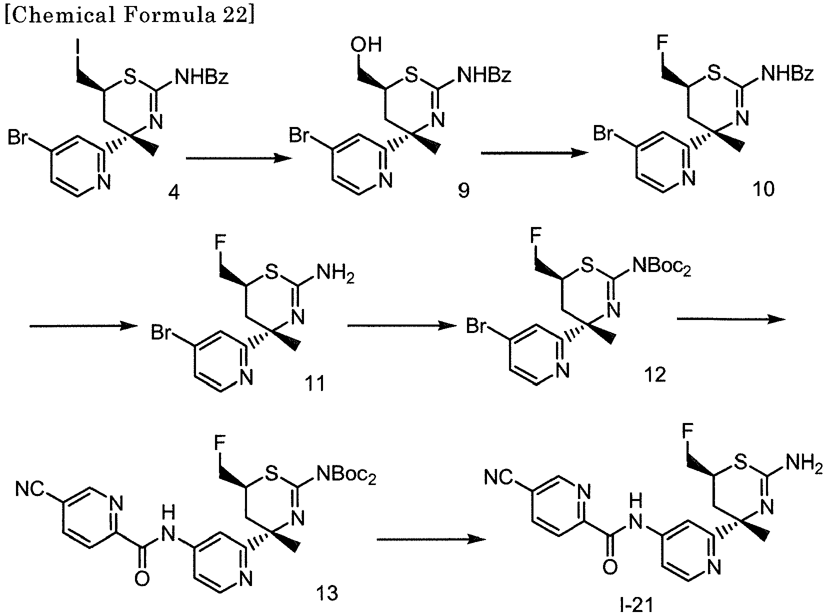

### Step 1

Potassium peroxide (1.07 g) and 18-crown-6 (3.99 g) were dissolved in dimethylsulfoxide (10 ml). To the solution was added compound (4) (2.00 g) dissolved in dimethylsulfoxide (10 ml) at room temperature, and the mixture was stirred for 10 minutes. To the mixture were added an aqueous citric acid solution and ethyl acetate, and the mixture was washed with water and brine. The organic layer was dried over sodium sulfate, and the solvent was evaporated under reduced pressure. The resulting residue was purified by silica gel column chromatography to afford compound (9) (1.05 g). ¹H-NMR (DMSO-d₆) δ: 1.65 (3H, s), 1.72-1.83 (1H, m), 2.72-2.82 (1H, m), 2.88 (1H, d, J = 13.6 Hz), 3.37-3.45 (1H, m), 3.54-3.63 (1H, m), 5.09 (1H, s), 7.42-7.57 (3H, m), 7.60-7.67 (2H, m), 8.10 (2H, d, J = 6.6 Hz), 8.53 (1H, d, J = 4.0 Hz), 10.87 (1H, s)..

### Step 2

Compound (9) (968 mg) was dissolved in dichloromethane (10 ml). To the solution was added N,N- diethylaminosulfur trifluoride (1.83 ml) at -78 °C, and the mixture was stirred for 1.5 hours under ice bath. To the mixture were added aqueous potassium carbonate solution and ethyl acetate, and the mixture was washed with water and brine. The organic layer was dried over sodium sulfate, and the solvent was evaporated under reduced pressure. The resulting residue was purified by silica gel column chromatography to afford compound (10) (139 mg).
¹H-NMR (DMSO-d₆) δ: 1.65 (3H, s), 1.79-1.96 (1H, m), 2.87-2.98 (1H, m), 3.00-3.14 (1H, m), 4.40-4.64 (2H, m), 7.43-7.58 (3H, m), 7.66 (2H, s), 8.09 (2H, br s), 8.52 (1H, d, J = 4.5 Hz), 10.69 (1H, br s).

### Step 3

Compound (10) (150 mg) was dissolved in methanol (3.0 ml). To the solution was added hydrazine monohydrate (0.0860 ml) at room temperature, and the mixture was stirred for 2 hours. After the solvent was evaporated under reduced pressure, the resulting residue was purified by silica gel column chromatography to afford compound (11) (100 mg).
¹H-NMR (DMSO-d₆) δ: 1.25 (1H, t, J = 13.1 Hz), 1.43 (3H, s), 2.68 (1H, d, J = 13.1 Hz), 2.90-3.03 (1H, m), 4.26-4.55 (2H, m), 6.18 (2H, br s), 7.44 (1H, s), 7.52 (1H, d, J = 3.5 Hz), 8.46 (1H, d, J = 3.5 Hz).

### Step 4

Compound (11) (100 mg) was dissolved in dichloromethane (1 ml). To the solution were added di-tert-butyl dicarbonate (0.182 ml) and N,N-dimethylaminopyridine (1.92 mg) at room temperature, and the mixture was stirred for 30 minutes. After the solvent was evaporated under reduced pressure, the resulting residue was purified by silica gel column chromatography to afford compound (12) (154 mg).
¹H-NMR (DMSO-d₆) δ: 1.38-1.44 (1H, m), 1.46 (18H, s), 1.55 (3H, s), 2.82-2.89 (1H, m), 3.17-3.35 (1H, m), 4.41-4.66 (2H, m), 7.50 (1H, s), 7.61 (1H, d, J = 4.5 Hz), 8.51 (1H, d, J = 4.5 Hz).

### Step 5

4,5-bis(diphenylphosphino)-9,9-dimethylxanthene (53.2 mg) and tris(dibenzylideneaceton)dipalladium(0) (28.0 mg) were dissolved in 1,4-dioxane (1.5 ml) that was degassed and replaced with nitrogen gas. The solution was stirred at room temperature for 10 minutes. To the solution were added cesium carbonate (116 mg), compound (12) (154 mg) dissolved in 1,4-dioxane (4 ml) and 5-cyano picolinamide (52.4 mg), and the mixture was stirred at 80 °C for 1 hour and at 100 °C for 7.5 hours. 4,5-Bis(diphenylphosphino)-9,9-dimethylxanthene (53.2 mg) and
tris(dibenzylideneaceton)dipalladium(0) (28.0 mg) were dissolved in 1,4-dioxane (1 ml). The solution was stirred at room temperature for 10 minutes and added into the reaction mixture. The whole mixture was stirred for 1.5 hours. To the mixture were added an aqueous citric acid solution and ethyl acetate, and the mixture was washed with water and brine. The organic layer was dried over sodium sulfate, and the solvent was evaporated under reduced pressure. The resulting residue was purified by silica gel column chromatography to afford compound (13) (40.0 mg).
¹H-NMR (DMSO-d₆) δ: 1.36-1.41 (19H, m), 1.56 (3H, s), 2.94-3.01 (1H, m), 3.31-3.38 (1H, m), 4.44-4.69 (2H, m), 7.76-7.80 (1H, m), 7.83-7.86 (1H, m), 8.26-8.31 (1H, m), 8.50-8.54 (1H, m), 8.58-8.64 (1H, m), 9.19 (1H, s), 11.05 (1H, s).

### Step 6

Compound (13) (40.0 mg) was dissolved in formic acid (0.420 ml). The solution was stirred at room temperature for 3 hours. To the mixture were added an aqueous potassium carbonate solution and ethyl acetate, and the mixture was washed with water and brine. The organic layer was dried over sodium sulfate, and the solvent was evaporated under reduced pressure. The resulting residue was triturated from hexane-ethyl acetate to afford compound (I-21) (18.5 mg).
1H-NMR (DMSO-d₆) δ: 1.16-1.27 (1H, m), 1.46 (3H, s), 2.66-2.74 (1H, m), 2.96-3.07 (1H, m), 4.25-4.57 (2H, m), 6.06 (2H, brs), 7.73-7.78 (1H, m), 7.91 (1H, s), 8.27-8.32 (1H, m), 8.46-8.50 (1H, m), 8.57-8.62 (1H, m), 9.21 (1H, s), 11.13 (1H, s).

### Example 3 Synthesis of compound (I-12)

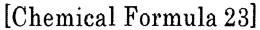

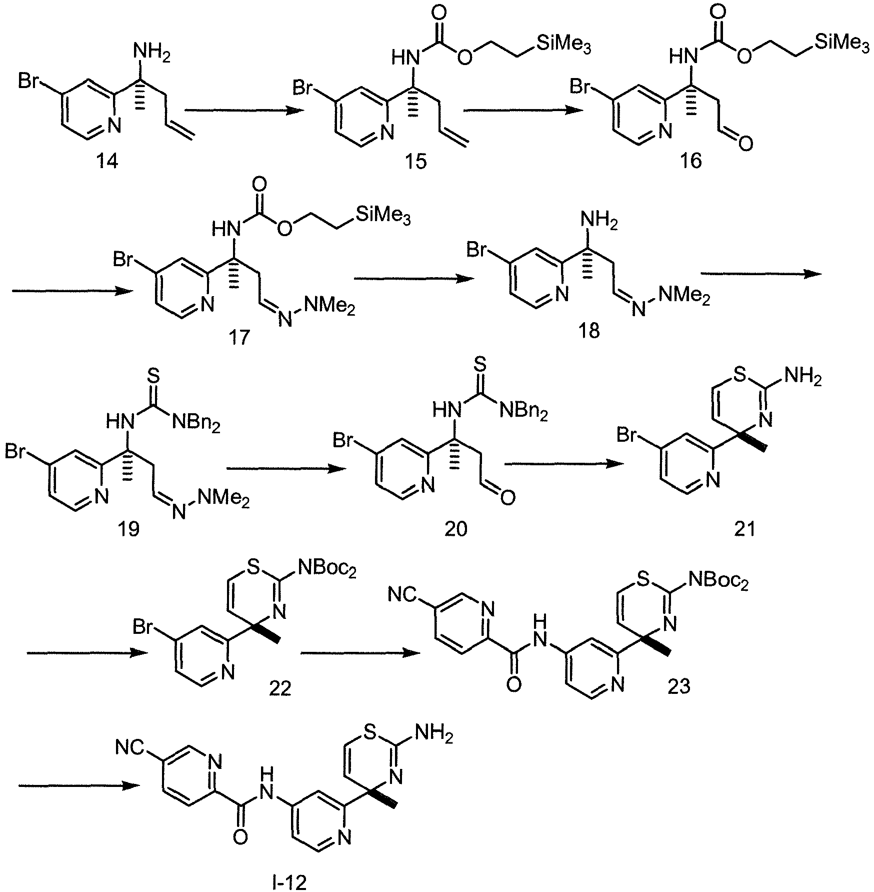

### Step 1

Compound (14) (23.8 g) was dissolved in 1,4-dioxane (500 ml). To the solution were added triethylamine (20.66 ml) and N-[2-(trimethylsilyl)ethoxycarbonyloxy]succinimide (38.5 g), and the mixture was stirred at room temperature for 67.5 hours. After the reaction mixture was concentrated, to the mixture was added a saturated sodium bicarbonate solution, and this was extracted with ethyl acetate. The organic layer was washed with water and dried over sodium sulfate. The solvent was evaporated under reduced pressure to afford compound (15) (47.7g, crude yield).
¹H-NMR (CDCl₃) δ: 0.04 (9H, s), 0.99 (2H, m), 1.74 (3H, s), 2.65 (1H, dd, J = 13.9, 7.0 Hz), 2.98 (1H, m), 4.12 (2H, m), 5.02 (1H, s), 5.07 (1H, m), 5.47 (1H, m), 6.22 (1H, s), 7.34 (1H, dd, J = 5.2, 1.7 Hz), 7.54 (1H, d, J = 1.7 Hz), 8.35 (1H, d, J = 5.2 Hz).

### Step 2

Compound (15) (19.27 g) was dissolved in tetrahydrofuran (500 ml) and water (500 ml). To the solution were added potassium osmium(VI) oxide dihydrate (921 mg) and sodium periodate (32.1 g) under ice bath, and the mixture was stirred at room temperature for 2 hours. To the mixture was added water, and this was extracted with ethyl acetate. The organic layer was washed with water and dried over sodium sulfate. The solvent was evaporated under reduced pressure to afford a residue. An insoluble material was removed by filtration, and the solvent was evaporated under reduced pressure to afford compound (16) (19.37 g).
¹H-NMR (CDCl₃) δ: 0.04 (9H, s), 0.99 (2H, m), 1.73 (3H, s), 3.02 (1H, brd, J = 15.6 Hz), 3.44 (1H, brd, J = 15.6 Hz), 4.12 (2H, m), 6.26 (1H, s), 7.48 (1H, m), 7.62 (1H, brs), 8.50 (1H, brs), 9.29 (1H, brs).

### Step 3

Compound (16) (19.37 g) was dissolved in dichloromethane (200 ml). To the solution was added dropwise 1,1-dimethylhydrazine (4.18 ml) dissolved in dichloromethane (50 ml) at -30 °C, and the mixture was stirred for 1 hour. To the mixture was added a saturated ammonium chloride solution, and this was extracted with ethyl acetate. The organic layer was dried over sodium sulfate. The solvent was evaporated under reduced pressure, and the resulting residue was purified by silica gel column chromatography to afford compound (17) (14.0 g).
¹H-NMR (CDCl₃) δ: 0.04 (9H, s), 0.99 (2H, m), 1.79 (3H, s), 2.67 (6H, s), 2.75 (1H, dd, J = 14.0, 6.0 Hz), 2.98 (1H, dd, J = 14.0, 5.3 Hz), 4.13 (2H, m), 6.30 (1H, dd, J = 6.0, 5.3 Hz), 6.31 (1H, brs), 7.32 (1H, dd, J = 5.3, 1.7 Hz), 7.54 (1H, dd, J = 1.7, 0.5 Hz), 8.34 (1H, dd, J = 5.3, 0.5 Hz).

### Step 4

Compound (17) (23.7 g) was dissolved in tetrahydrofuran (237 ml). To the solution was added tetra-n-butylammonium fluoride in a tetrahydrofuran solution (193.1 ml, 1 M), and the mixture was stirred at room temperature for 6 hours. To the mixture was added water, and the mixture was extracted with chloroform-methanol (9:1) and dried over sodium sulfate. The solvent was evaporated under reduced pressure, and the resulting residue was purified by silica gel column chromatography to afford compound (18) (14.9 g).
¹H-NMR (CDCl₃) δ: 1.50 (3H, s), 2.03 (2H, brs), 2.61 (1H, dd, J = 14.0, 6.2 Hz), 2.67 (6H, s), 2.84 (1H, dd, J = 14.0, 5.2 Hz), 6.41 (1H, dd, J = 6.2, 5.2 Hz), 7.30 (1H, dd, J = 5.2, 1.9 Hz), 7.74 (1H, d, J = 1.9 Hz), 8.37 (1H, d, J = 5.2 Hz).

### Step 5

Compound (18) (11.9 g) was dissolved in dichloromethane (120 ml). To the solution was added dropwise thiocarbonyldiimidazole (8.18 g) in dichloromethane (120 ml) at 0 °C over 20 minutes. The mixture was stirred at 0 °C for 4 hours, and dibenzylamine (10.43 ml) in dichloromethane (120 ml) was added dropwise to the mixture, and the whole mixture was stirred at 0 °C for 17 hours. The solvent was evaporated under reduced pressure, and the residue was purified by silica gel column chromatography to afford compound (19) (9.77 g).
¹H-NMR (CDCl₃) δ: 2.06 (3H, s), 2.51 (6H, s), 2.65 (1H, dd, J = 14.1, 6.6 Hz), 3.43 (1H, dd, J = 14.1, 5.0 Hz), 4.92 (2H, d, J = 16.5 Hz), 5.07 (2H, d, J = 16.5 Hz), 6.20 (1H, dd, J = 6.6, 5.0 Hz), 7.24-7.41 (12H, m), 7.78 (1H, s), 8.17 (1H, d, J = 5.4 Hz).

### Step 6

Compound (19) (4.76 g) was dissolved in tetrahydrofuran (95 ml). To the solution was added hydrochloric acid (18.15 ml, 5 M) at 0 °C, and the mixture was stirred at room temperature for 18 hours. To an aqueous sodium hydroxide solution (45.5 ml, 2 M) and ice (140 g) in another flask was added dropwise the reaction mixture over 1 hour. When the reaction temperature was increased, ice (140 g, twice) was added. After dropping, to the mixture was added sodium bicarbonate, and this was extracted with ethyl acetate. The organic layer was washed with water and brine and dried over sodium sulfate. The solvent was evaporated under reduced pressure to afford compound (20) (4.48 g).
¹H-NMR (CDCl₃) δ: 1.81 (3H, s), 3.28 (1H, dd, J = 17.0, 2.4 Hz), 4.63 (1H, d, J = 17.0 Hz), 5.04 (4H, s), 7.26-7.40 (12H, m), 8.01 (1H, s), 8.09 (1H, d, J = 5.2 Hz), 9.46 (1H, brs).

### Step 7

Compound (20) (1.22 g) was dissolved in sulfuric acid (12.2 ml, 99.999%). The solution was stirred at room temperature for 15 minutes and then at 80 °C for 45 minutes. The solution was cooled in ice bath and added dropwise into aqueous sodium hydroxide solution (222 ml, 2 M) and ice (230 g). The mixture was extracted with ethyl acetate, and the organic layer was washed with water and brine and dried over sodium sulfate. The solvent was evaporated under reduced pressure to afford compound (21) (580 mg, purity 63%)

### Step 8

Compound (21) (2.24 g, purity 61%) was dissolved in dichloromethane (34 ml). To the solution were added di-t-butyl dicarbonate (14.64 ml) and dimethylaminopyridine (192 mg) under ice bath, and the mixture was stirred at room temperature for 1 hour. To the mixture was added a saturated sodium bicarbonate solution at 0 °C, and the mixture was extracted with ethyl acetate and dried over sodium sulfate. The solvent was evaporated under reduced pressure, and the resulting residue was purified by silica gel column chromatography to afford compound (22) (796 mg).
¹H-NMR (CDCl₃) δ: 1.51 (18H, s), 1.65 (3H, s), 6.05 (1H, d, J = 9.7 Hz), 6.27 (1H, d, J = 9.7 Hz), 7.34 (1H, dd, J = 5.2, 1.9 Hz), 7.73 (1H, d, J = 1.9 Hz), 8.40 (1H, d, J = 5.2 Hz).

### Step 9

4,5- bis(diphenylphosphino)-9,9-dimethylxanthene (71.0 mg) and tris(dibenzylideneaceton)dipalladium(0) (37.4 mg) were dissolved in 1,4-dioxane (4 ml). The solution was degassed and replaced with nitrogen gas and stirred at room temperature for 15 minutes, and then cesium carbonate (160 mg) was added. The mixture was added dropwise into compound (22) (198 mg) and 5-cyano picolinamide (75 mg) in 1,4-dioxane (4 ml) that was degassed and replaced with nitrogen gas, and washed with 1,4-dioxane (2 ml). The mixture was degassed and replaced with nitrogen gas and stirred at 80 °C for 2 hours and then 90 °C for 3 hours. After the mixture was cooled to 0 °C, water and ethyl acetate were added. The mixture was extracted, and the organic layer was dried over sodium sulfate, and the solvent was evaporated under reduced pressure. The resulting residue was purified by silica gel column chromatography to afford compound (23) (122.6 mg).
¹ H-NMR (CDCl₃) δ: 1.53 (18H, s), 1.69 (3H, s), 6.19 (1H, d, J = 9.6 Hz), 6.31 (1H, d, J = 9.6 Hz), 7.55 (1H, d, J = 2.2 Hz), 8.08 (1H, dd, J = 5.3, 2.2 Hz), 8.24 (1H, dd, J = 8.2, 1.9 Hz), 8.43 (1H, dd, J = 8.2, 0.8 Hz), 8.61 (1H, d, J = 5.3 Hz), 8.89 (1H, dd, J = 1.9, 0.8 Hz), 10.03 (1H, s).

### Step 10

Formic acid (2.25 ml) was added to compound (23) (162 mg), and the mixture was stirred at room temperature for 3 hours. After the mixture was cooled to 0 °C, a saturated sodium bicarbonate solution was added to the mixture, which was then extracted with ethyl acetate. The organic layer was dried over sodium sulfate, and the solvent was evaporated under reduced pressure. The resulting residue was purified by silica gel column chromatography to afford compound (I-12) (57.0 mg)
¹ H-NMR (CDCl₃) δ: 1.65 (3H, s), 4.56 (2H, brs), 6.31 (1H, d, J = 9.6 Hz), 6.37 (1H, d, J = 9.6 Hz), 7.71 (1H, d, J = 2.1 Hz), 7.83 (1H, dd, J = 5.5, 2.1 Hz), 8.22 (1H, dd, J = 8.1, 2.0 Hz), 8.43 (1H, d, J = 8.1 Hz), 8.58 (1H, d, J = 5.5 Hz), 8.91 (1H, m), 9.96 (1H, s).

### Example 4 Synthesis of compound (I-7)

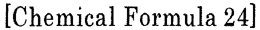

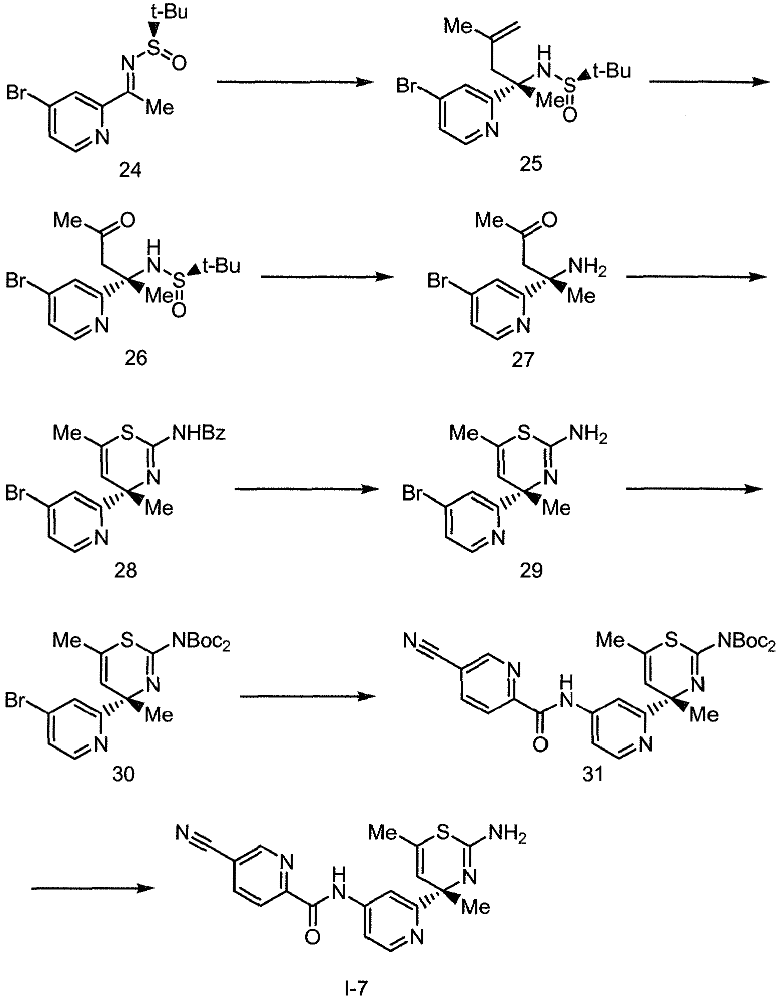

### Step 1

To a solution of compound (24) (3.0 g) in tetrahydrofuran (10 mL) was added dropwise 2-methyl allylmagnesium chloride (59 mL, 0.5 M in tetrahydrofuran) at -45 °C over 15 minutes. After dropping, the mixture was poured into an aqueous ammonium chloride solution and diluted with ethyl acetate, and the organic layer was separated. The aqueous layer was extracted with ethyl acetate three times, and the combined organic layer was washed with water and brine, and dried over magnesium sulfate. After filtration of the organic layer, the organic layer was concentrated, and the residue was purified by silica gel column chromatography eluting with hexane: ethyl acetate=1:1 to afford compound (25) (1.6 g) as a dark green oil.
¹H-NMR (CDCl3) δ: 1.26 (12H, s), 1.79 (3H, s), 2.73 (1H, d, J = 13.1 Hz), 2.92 (1H, d, J = 13.1 Hz), 4.68 (2H, s), 4.87 (1H, t, J = 1.8 Hz), 7.33-7.35 (1H, m), 7.57 (1H, d, J = 1.8 Hz), 8.40 (1H, d, J = 5.2 Hz).

### Step 2

Ozone was bubbled into a dichloromethane (30 mL) solution of compound (25) (1.7 g) at -78 °C for 1 hour, and then triethylamine (1.9 mL) was added to the solution. After the mixture was allowed to warm to room temperature, the mixture was concentrated to afford compound (26) (2.7 g) as a brown amorphous.
MS (ESI) m/z = 361 (M+H)⁺

### Step 3

To a solution of compound (26) (2.7 g) in methanol (15 mL) was added a hydrochloric acid/dioxane solution (7.5 mL, 4 M in dioxane) at room temperature, and the mixture was stirred for 10 minutes. To the mixture was added water, this was washed with ether, and then made alkaline with a saturated sodium bicarbonate solution. The aqueous layer was extracted with ethyl acetate, and the combined organic layer was washed with brine and dried over magnesium sulfate. After filtration of the organic layer, the solvent was concentrated to afford compound (27) (426 mg) as a brown oil.
¹H-NMR (CDCl3) δ: 1.40 (3H, s), 2.08 (3H, s), 2.80 (1H, d, J = 17.2 Hz), 3.50 (1H, d, J = 17.2 Hz), 7.27 (1H, dd, J = 5.3, 1.7 Hz), 7.84 (1H, d, J = 1.7 Hz), 8.27 (1H, d, J = 5.3 Hz).

### Step 4

To a solution of compound (27) (408 mg) in tetrahydrofuran (4 mL) was added benzoyl thioisocyanate (285 mg) at 0 °C, and the mixture was stirred for 20 minutes. After the mixture was concentrated under reduced pressure, the mixture was added to sulfuric acid (3 mL) at 0 °C and then allowed to warm to room temperature through overnight. The mixture was made alkaline with an aqueous ammonia solution, and then ethyl acetate was added. The organic layer was separated, and the aqueous layer was extracted with ethyl acetate. The combined organic layer was washed with brine and dried over magnesium sulfate. After filtration of the organic layer, the solvent was concentrated under reduced pressure, and the resulting residue was purified by silica gel column chromatography eluting with hexane/ ethyl acetate (10 to 40% ethyl acetate) to afford compound (28) (29 mg).
(ESI) m/z = 402 (M+H)⁺.

### Step 5

To a solution of compound (28) (29 mg) in ethanol (0.3 mL) was added hydrazine monohydrate (35 uL) at room temperature, and the mixture was stirred for 2 hours. The mixture was concentrated under reduced pressure to afford compound (29) (25 mg) as a yellow oil.

### Step 6

To a solution of compound (29) (25 mg) in dichloromethane (0.5 mL) were added Boc₂ O (110 mg) and DMAP (12 mg), and the mixture was stirred at 50 °C overnight. The mixture was concentrated, and the resulting residue was purified by silica gel column chromatography eluting with hexane:ethyl acetate= 2:1 to afford compound (30) (30 mg) as a colorless oil.
MS (ESI) m/z = 498 (M+H)⁺

### Step 7

A dioxane solution (1.0 mL) of tris(dibenzylideneaceton)dipalladium (11 mg) and Xantphos (20 mg) was stirred for 5 minutes under nitrogen atmosphere. To the mixture were added compound (30) (29 mg), 5-cyano picolinamide (11 mg) and cesium carbonate (25 mg), and the mixture was stirred at 90 °C for 3 hours. After being warmed to room temperature, the mixture was diluted with a saturated ammonium chloride solution and ethyl acetate. The organic layer was separated, and the aqueous layer was extracted with ethyl acetate. The combined organic layer was washed with water and brine and dried over magnesium sulfate. After filtration of the organic layer, the solvent was concentrated under reduced pressure, and the residue was purified by reversed phase high performance liquid chromatography (ODS C18; acetonitrile/water/0.3% formic acid: 10 to 100% acetonitrile) to afford compound (31) (6.2 mg) as a brown oil.
MS (ESI) m/z = 565 (M+H)⁺.

### Step 8

Formic acid (550 uL) was added to compound (31) (5.4 mg), and the mixture was stirred at room temperature for 50 minutes. To the mixture was added a 2 M aqueous sodium hydroxide solution (7.2 mL) and diluted with ethyl acetate. The organic layer was separated, and the aqueous layer was washed with ethyl acetate. The combined organic layer was washed with water and brine and dried over magnesium sulfate. After filtration of the organic layer, the solvent was concentrated under reduced pressure, and the resulting residue was purified by silica gel column chromatography eluting with chloroform:methanol= 9:1 to afford compound (I-7) (0.8 mg) as a pale yellow oil.
¹H-NMR (CDCl₃) δ: 1.94 (3H, s), 2.08 (3H, d, J = 1.5 Hz), 6.24 (1H, d, J = 1.5 Hz), 7.70-7.71 (1H, m), 7.98 (1H, dd, J = 5.3, 1.9 Hz), 8.22 (1H, dd, J = 8.1, 2.1 Hz), 8.41 (1H, dd, J = 8.1, 0.8 Hz), 8.55 (1H, d, J = 5.4 Hz), 8.94 (1H, br s), 10.17 (1H, s).
MS (ESI) m/z = 365 (M+H)⁺

### Example 5 Synthesis of compound (I-198)

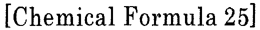

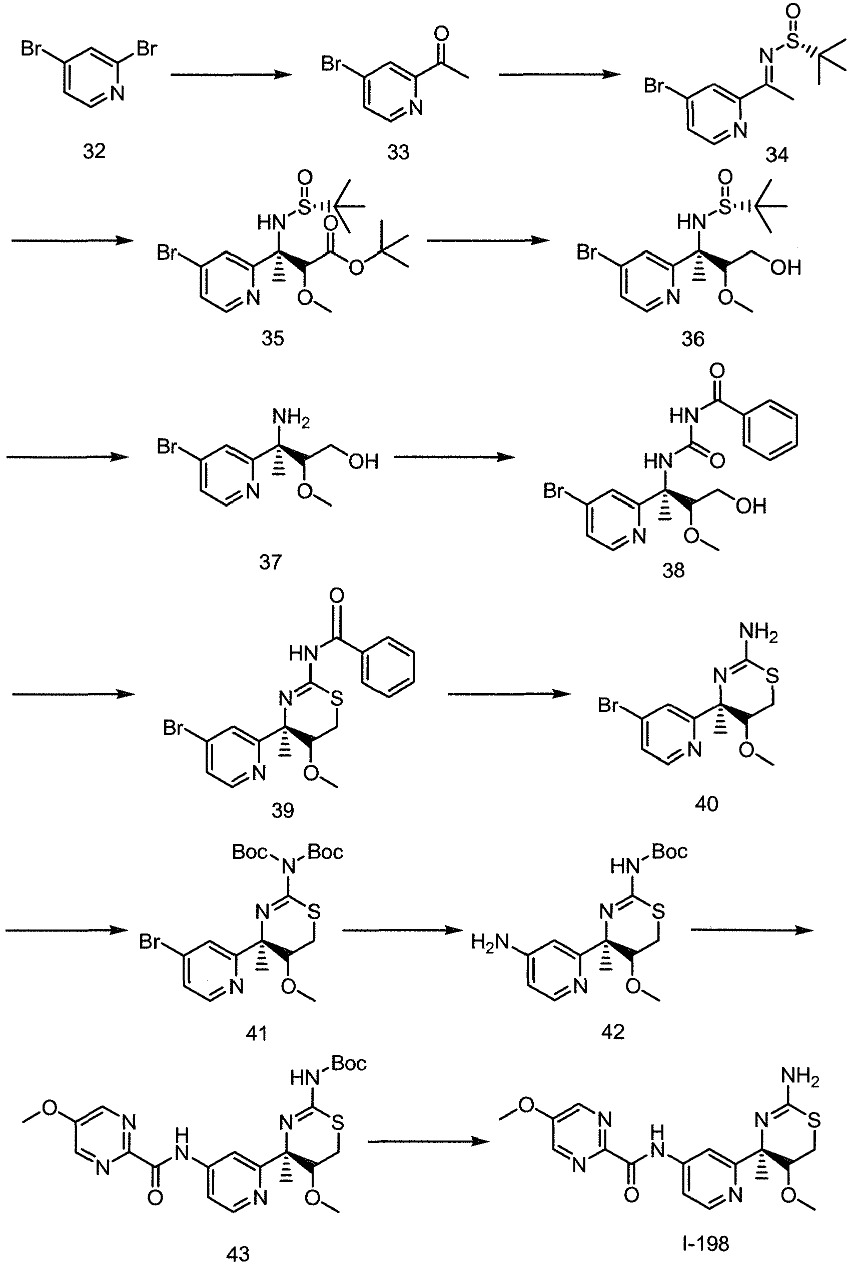

### Step 1 Synthesis of compound 33

Compound 32 (60.3 g, 255 mmol) was dissolved in toluene (1800 mL) under nitrogen atmosphere, and the solution was cooled to -66 °C. To the solution was added dropwise 2.67 mol/L of n-butyllithium in hexane (100 mL, 267 mmol), and the mixture was stirred for 40 minutes. To the mixture was added dropwise N-methoxy-N-methylacetamide (79 g, 764 mmol), and the mixture was stirred at the same temperature for 40 minutes. The mixture was allowed to warm to 0 °C by removing the cooling bath, and then cooled to -55 °C. To the mixture was added a saturated ammonium chloride solution (300 mL), which was allowed to warm to room temperature. To the mixture was added water (200 mL) and extracted. The organic layer was washed with water (500 mL) and dried over anhydrous magnesium sulfate. The solvent was evaporated under reduced pressure, and the resulting residue was purified by silica gel column chromatography eluting with hexane-ethyl acetate to afford compound 33 (34.2 g, Yield 67 %).
¹ H-NMR (CDCl₃) δ: 2.72 (3H, s), 7.64 (1H, dd, J = 5.1, 1.8 Hz), 8.20 (1H, dd, J = 1.8, 0.6 Hz), 8.50 (1H, dd, J = 5.1, 0.6 Hz)

### Step 2 Synthesis of compound 34

Compound 33 (62.11 g, 310 mmol) was dissolved in tetrahydrofuran (1240 mL) under nitrogen atmosphere. To the solution were added (R)-2-methylpropane-2-sulfinamide (48.9 g, 404 mmol) and tetraethyl titanate (13.75 mL), and the mixture was refluxed for 3 hours. After the mixture was cooled to room temperature, the mixture was added into a mixture of a saturated sodium bicarbonate solution (1100 mL) and ethyl acetate (500 mL) under ice bath and was filtered through Celite. The mixture was washed with ethyl acetate (400 mL) four times, and the filtrate was washed with brine (100 mL) and water (250 mL) and dried over anhydrous magnesium sulfate. The solvent was evaporated under reduced pressure, and the resulting residue was purified by silica gel column chromatography eluting with hexane-ethyl acetate to afford compound 34 (74.3 g, Yield 79%).
¹H-NMR (CDCl₃) δ: 1.34 (9H, s), 2.84 (3H, s), 7.55 (1H, dd, J = 5.1, 1.8 Hz), 8.25 (1H, d, J = 1.8 Hz), 8.47 (1H, d, J = 5.1 Hz)

### Step 3 Synthesis of compound 35

A solution of diisopropylamine (19.74 mL, 139 mmol) in diethyl ether (140 mL) was cooled to -78 °C under dry ice-acetone bath under nitrogen atmosphere. A solution of 1.62 mol/L of n-butyllithium in hexane (83 mL, 134 mmol) was added dropwise to the solution, and the mixture was stirred for 20 minutes. A solution of tert-butyl-2-methoxy acetate in diethyl ether (100 mL) was added dropwise to the mixture, and the mixture was stirred at -70 °C for 50 minutes. At the same temperature, a solution of chlorotitanium triisopropoxide (36.1 g, 139 mmol) in diethyl ether (100 mL) was added dropwise to the mixture, and the mixture was stirred for 30 minutes. A solution of compound 34 (14.0 g, 46.2 mmol) in diethyl ether (100 mL) was added dropwise to the mixture, and the mixture was stirred for 40 minutes. After adding a saturated ammonium chloride solution (90 mL), the mixture was allowed to warm to room temperature and filtered through Celite. The mixture was washed with diethyl ether (200 mL), and the filtrate was washed with water (100 mL) twice and dried over anhydrous magnesium sulfate. The solvent was evaporated under reduced pressure, and the resulting residue was purified by silica gel column chromatography eluting with hexane-ethyl acetate to afford compound 35 (15.4 g, Yield 74%).
¹ H-NMR (CDCl₃) δ: 1.29 (9H, s), 1.43 (9H, s), 1.90 (3H, s), 3.39 (3H, s), 4.06 (1H, s), 4.97 (1H, brs), 7.34 (1H, dd, J = 5.1, 1.8 Hz), 7.60 (1H, d, J = 1.8 Hz), 8.34 (1H, d, J = 5.1 Hz)

### Step 4 Synthesis of compound 36

To a solution of compound 35 (14.28 g, 31.8 mmol) in tetrahydrofuran (140 mL) was added distillated water (14 mL) under nitrogen atmosphere, and the solution was stirred under ice bath. A solution of 2 mol/L of lithium borohydride in tetrahydrofuran (79 mL, 159 mmol) was added dropwise to the solution, and the mixture was stirred at room temperature for 50 minutes. To the mixture was added water (500 mL), and this was extracted with ethyl acetate (200 mL) twice. The organic layer was washed with water (100 mL) and dried over anhydrous magnesium sulfate. The solvent was evaporated under reduced pressure, and the resulting residue was dissolved in ethyl acetate. Hexane was added to the solution to afford a solid, and which was collected by filtration to afford compound 36 (10.64 g, Yield 88%).
¹ H-NMR (CDCl₃) δ: 1.26 (9H, s), 1.73 (3H, s), 3.36 (3H, s), 3.55-3.64 (2H, m), 3.88-3.96 (1H, m), 4.55-4.57 (1H, m), 5.08 (1H, s), 7.39 (1H, dd, J = 5.1, 1.5 Hz), 7.66 (1H, d, J = 1.5 Hz), 8.37 (1H, d, J = 5.1 Hz)

### Step 5 Synthesis of compound 37

Compound 36 (10.61 g, 28.0 mmol) was dissolved in methanol (106 mL). To the solution was added 2 mol/L of hydrochloric acid in methanol (140 mL, 280 mmol), and the mixture was stirred at room temperature for 35 minutes. The mixture was added to a mixture of sodium bicarbonate (25 g) and water (150 mL), and methanol was evaporated under reduced pressure. The mixture was extracted with chloroform (100 mL) three times, and the combined organic layer was dried over anhydrous magnesium sulfate. The solvent was evaporated under reduced pressure to afford crude compound 37 (9.38 g, Yield 122%). The obtained product was used for the next step without further purification.
¹H-NMR (CDCl₃) δ: 1.56 (3H, s), 3.24 (3H, s), 3.41 (1H, dd, J = 6.6, 3.3 Hz), 3.55 (1H, dd, J = 12.0, 3.3 Hz), 3.78 (1H, dd, J = 12.0, 6.6 Hz), 7.36 (1H, dd, J = 5.1, 1.8 Hz), 7.67 (1H, d, J = 1.8 Hz), 8.39 (1H, d, J = 5.1 Hz)

### Step 6 Synthesis of compound 38

Compound 37 (9.38 g) was dissolved in dichloromethane (150 mL) under nitrogen atmosphere. To the solution was added benzoyl isothiocyanate (4.60 g, 28.2 mmol), and the mixture was stirred at room temperature for 1 hour and 40 minutes. The solvent was evaporated under reduced pressure, and the resulting residue was purified by silica gel column chromatography eluting with hexane-ethyl acetate to afford compound 38 (7.26 g, Yield 80%)
¹H-NMR (CDCl₃) δ: 2.24 (3H, s), 2.78 (1H, Br), 3.40-3.49 (1H, brd), 3.54 (1H, t, J = 3.9 Hz), 3.60 (3H, s), 3.70-3.80 (1H, brd), 7.37 (1H, dd, J = 5.1, 1.8 Hz), 7.47-7.53 (2H, m), 7.59-7.63 (2H, m), 7.85-7.88 (2H, m), 8.38 (1H, d, J = 5.1 Hz), 8.82 (1H, brs), 11.81 (1H, brs)

### Step 7 Synthesis of compound 39

Compound 38 (9.42 g, 21.5 mmol) was dissolved in dichloromethane (160 mL) under nitrogen atmosphere. To the solution was added dropwise 1-chloro-N,N-2-trimethylpropenylamine (7.18 g, 53.7 mmol) in dichloromethane (18 mL), and the mixture was stirred at room temperature for 20 minutes. The mixture was washed with a saturated sodium bicarbonate solution (50 mL) and water (100 mL). The aqueous layer was extracted with dichloromethane (50 mL) twice, and the combined organic layer was dried over anhydrous magnesium sulfate. The solvent was evaporated under reduced pressure, and the resulting residue was purified by silica gel column chromatography eluting with hexane-ethyl acetate to afford compound 39 (7.26 g, Yield 80%).
¹ H-NMR (CDCl₃) δ: 1.74 (3H, s), 2.71 (1H, dd, J = 13.5, 2.1 Hz), 3.09 (1H, dd, J = 13.5, 4.2 Hz), 3.53 (3H, s), 4.37 (1H, dd, J = 4.2, 2.1 Hz), 7.38-7.52 (4H, m), 7.64 (1H, d, J = 1.2 Hz), 8.22 (2H, d, J = 6.9 Hz), 8.41 (1H, d, J = 5.1 Hz)

### Step 8 Synthesis of compound 40

Compound 39 (7.67 g, 18.25 mmol) was dissolved in ethanol (38 mL) under nitrogen atmosphere. To the solution was added hydrazine monohydrate (4.43 mL, 91 mmol), and the mixture was stirred at room temperature for 3.5 hours. The solvent was evaporated under reduced pressure, and the resulting residue was purified by silica gel column chromatography eluting with hexane-ethyl acetate. To the residue was added hexane (20 mL) to afford a solid, which was washed and collected by filtration to afford compound 40 (4.27 g, Yield 74%).
¹H-NMR (CDCl₃) δ: 1.62 (3H, s), 2.72 (1H, dd, J = 12.9, 2.4 Hz), 3.06 (1H, dd, J = 12.9, 4.5 Hz), 3.48 (3H, s), 4.15 (1H, dd, J = 4.8, 2.4 Hz), 4.45 (2H, br), 7.31 (1H, dd J = 5.1, 1.5 Hz), 7.57 (1H, d, J = 1.5 Hz), 8.36 (1H, d, J = 5.1 Hz)

### Step 9 Synthesis of compound 41

Compound 40 (4.31 g, 13.63 mmol) was dissolved in dichloromethane (43 mL). To the solution was added di-tert-butyl dicarbonate (10.41 g, 47.7 mmol), and the mixture was stirred at room temperature for 15 minutes. To the mixture was added 4-dimethylaminopyridine (167 mg, 1.363 mmol), and the mixture was stirred for 1 hour and 45 minutes. The solvent was evaporated under reduced pressure, and the resulting residue was purified by silica gel column chromatography eluting with hexane-ethyl acetate. To the residue was added hexane (20 mL) to afford a solid, which was collected by filtration to afford compound 41 (6.00 g, Yield 85%).
¹ H-NMR (CDCl₃) δ: 1.54 (18H, s), 1.65 (3H, s), 2.85 (1H, dd, J = 13.2, 2.4 Hz), 3.10 (1H, dd, J = 13.2, 6.0 Hz), 3.40 (3H, s), 4.18 (1H, dd, J = 6.0, 2.4 Hz), 7.34 (1H, dd J = 5.1, 1.5 Hz), 7.69 (1H, d, J = 1.5 Hz), 8.37 (1H, d, J = 5.1 Hz)

### Step 10 Synthesis of compound 42

To Pd₂(dba)₃ (133 mg, 0.145 mmol) and butylbis(1-adamantyl)phosphine (104 mg, 0.290 mmol) was added toluene (10 mL). The mixture was degassed and replaced with nitrogen gas and stirred at room temperature for 30 minutes. To the mixture was added 1.3 mol/L of LHMDS in tetrahydrofuran (2.3 mL, 2.99 mmol). The mixture was degassed and replaced with nitrogen gas and stirred at room temperature for 10 minutes. To the mixture was added compound 10 (500 mg, 0.968 mmol), and the mixture was degassed and replaced with nitrogen gas and stirred at 80 °C for 45 minutes. After the mixture was cooled to room temperature, was added 2 mol/L hydrochloric acid (2.42 mL), and the mixture was stirred at room temperature for 30 minutes. To the mixture was added a 2 mol/L aqueous potassium carbonate solution (2.42 mL), and this was extracted with ethyl acetate. The organic layer was washed with brine and dried over anhydrous magnesium sulfate. The solvent was evaporated under reduced pressure, and the residue was purified by silica gel column chromatography eluting with chloroform-methanol. The resulting residue was washed with diisopropylether (2 mL) and collected by filtration to afford compound 42 (249 mg, Yield 73%).
¹ H-NMR (CDCl₃) δ: 1.51 (9H, s), 1.67 (3H, s), 2.67 (1H, dd, J = 13.5, 2.4 Hz), 2.96 (1H, dd, J = 13.5, 3.9 Hz), 3.51 (3H, s), 4.26 (2H, br), 4.30 (1H, dd, J = 4.2, 2.4 Hz), 6.40 (1H, dd J = 5.4, 2.1 Hz), 6.64 (1H, d, J = 2.1 Hz), 8.15 (1H, d, J = 5.4 Hz), 10.01 (1H, br)

### Step 11 Synthesis of compound 43

5-Methoxypyrazine-2-carboxylic acid (59.5 mmol) was dissolved in tetrahydrofuran (1 mL) under nitrogen atmosphere. To the solution were added oxalyl chloride (60 µL, 0.685 mmmol) and N,N-dimethylformamide (5 µL, 0.064 mmmol), and the mixture was stirred at room temperature for 15 minutes. The solvent was evaporated under reduced pressure, and the resulting residue was dissolved in tetrahydrofuran (1 mL). To the mixture were added dropwise compound 42 (103.1 mg, 0.293 mmmol), N, N-diisopropylethylamine (102 µL, 0.585 mmmol) in tetrahydrofuran (2 mL) under ice bath. After dropping, the mixture was allowed to warm to room temperature and stirred for 2.5 hours. To the mixture was added water, and this was with ethyl acetate. The organic layer was washed with a saturated sodium bicarbonate solution and water and dried over anhydrous magnesium sulfate. The solvent was evaporated under reduced pressure, and the residue was purified by silica gel column chromatography eluting with hexane-ethyl acetate to afford compound 43 (89 mg, Yield 62%).
¹H-NMR (CDCl₃) δ: 1.54 (9H, s), 1.72 (3H, s), 2.64 (1H, dd, J = 13.5, 2.1 Hz), 3.00 (1H, dd, J = 13.5, 4.2 Hz), 3.53 (3H, s), 4.09 (2H, br), 4.33 (1H, dd, J = 4.2, 2.1 Hz), 7.24 (1H, d, J = 1.8 Hz), 8.17 (1H, dd J = 5.4, 1.8 Hz), 8.22 (1H, d, J = 1.5 Hz), 8.54 (1H, d, J = 5.4 Hz), 9.01 (1H, d, J = 1.5 Hz), 9.74 (1H, brs)

### Step 12 Synthesis of compound I-198

To a solution of compound 43 (83 mg, 0.170 mmmol) in dichloromethane (1.5 mL) was added trifluoroacetic acid (0.8 mL, 10.38 mmmol) under nitrogen atmosphere, and the mixture was stirred at room temperature for 3 hours. The mixture was added into a 2 mol/L aqueous potassium carbonate solution (7 mL) and extracted with ethyl acetate (15 mL). The organic layer was washed with water (10 mL) and dried over anhydrous magnesium sulfate. The solvent was evaporated under reduced pressure, and the resulting residue was purified by silica gel column chromatography eluting with hexane-ethyl acetate to afford compound I-198 (89 mg, Yield 62%).
¹ H-NMR (CDCl₃) δ: 1.66 (3H, s), 2.76 (1H, dd, J = 12.9, 2.4 Hz), 3.07 (1H, dd, J = 12.9, 4.2 Hz), 3.51 (3H, s), 4.08 (2H, br), 4.22 (1H, dd, J = 4.2, 2.4 Hz), 7.33 (1H, d, J = 2.1 Hz), 7.96 (1H, dd J = 5.4, 2.1 Hz), 8.16 (1H, d, J = 1.2 Hz), 8.52 (1H, d, J = 5.4 Hz), 9.01 (1H, d, J = 1.2 Hz), 9.63 (1H, br)

### Example 6 Synthesis of compound (I-275)

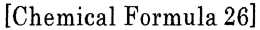

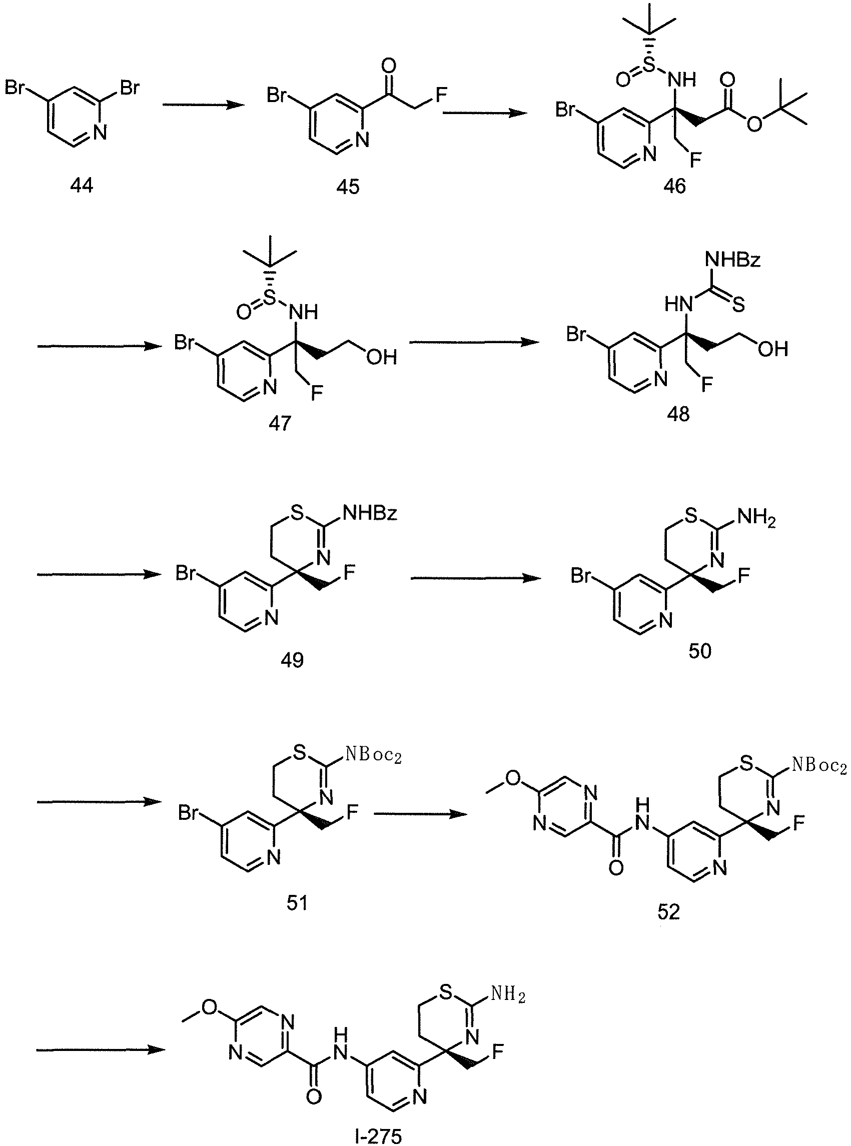

### Step 1 Synthesis of compound 45

Compound 44 (100 mg) was dissolved in toluene (4 mL) under nitrogen atmosphere, and the solution was stirred at -78 °C for 15 minutes. To the solution was added dropwise n-butyllithium in hexane (2.64 mol/L, 168 µL) at the same temperature, and the mixture was stirred for 5 minutes. The mixture was warmed to -50 °C and stirred for 40 minutes. The mixture was quenched with saturated ammonium chloride solution (4 mL) and extracted with ethyl acetate. The organic layer was washed with water and brine and dried over magnesium sulfate. After filtration of the extract, the solvent was evaporated under reduced pressure, and the resulting residue was purified by silica gel column chromatography to afford compound 45 (44.5 mg).
¹ H-NMR (CDCl₃) δ: 5.75 (1H, s), 5.91 (1H, s), 7.69 (1H, dd, J = 5.2, 2.0 Hz), 8.22 (1H, d, J = 2.0 Hz), 8.44 (1H, d, J = 5.2 Hz).

### Step 2 Synthesis of compound 46

Diisopropylamine (1.01 mL) was dissolved in tetrahydrofuran (5 mL) under nitrogen atmosphere, and the solution was stirred at -78 °C for 10 minutes. To the solution was added dropwise n-butyllithium in hexane (2.69 mol/L, 2.56 mL) at the same temperature. The mixture was stirred for 30 minutes under ice bath and stirred at -78 °C for 15 minutes. To the mixture was added dropwise t-butylacetate (799 mg) in tetrahydrofuran (3 mL) at the same temperature, and the mixture was stirred for 20 minutes. To the mixture was added dropwise chlorotitanium triisopropoxide (2.39 g) in tetrahydrofuran (5 mL) at the same temperature, and the mixture was stirred for 75 minutes. A mixture of compound 45 (500 mg), (R)-2-methylpropane-2-sulfonamide (306 mg) and tetraethyl titanate (889 mg) in tetrahydrofuran (4.5 mL) was stirred at reflux for 165 minutes, stirred at room temperature for 15 minutes, and diluted with tetrahydrofuran (2.5 mL), and then the mixture was added dropwise into the reaction mixture at the same temperature and stirred for 30 minutes. The mixture was quenched with an aqueous ammonium chloride solution and extracted with ethyl acetate. The organic layer was washed with water and brine and dried over magnesium sulfate. After filtration of the extract, the solvent was evaporated under reduced pressure, and the resulting residue was purified by silica gel column chromatography to afford Compound46 (295 mg).
¹H-NMR (CDCl₃) δ: 1.31 (9H, s), 1.33 (9H, s), 3.14 (2H, dd, J = 66.2, 16.3 Hz), 4.84 (2H, ddd, J = 79.8, 46.7, 9.8 Hz), 5.34 (1H, s), 7.37 (1H, dd, J = 5.2, 1.8 Hz), 7.74 (1H, d, J = 1.8 Hz), 8.35 (1H, d, J = 5.2 Hz).

### Step 3 Synthesis of compound 47

To a solution of compound 46 (9.03 g) in tetrahydrofuran (90 mL) was added distilled water (9 mL). To the mixture was added lithium borohydride in tetrahydrofuran (2 mol/L, 51.6 mL) under ice bath. The mixture was stirred at room temperature for 2.5 hours. To the mixture was added distilled water and extracted with ethyl acetate. The organic layer was washed with water and brine and dried over magnesium sulfate. After filtration of the extract, the solvent was evaporated under reduced pressure, and the resulting residue was purified by silica gel column chromatography to afford compound 47 (5.68 g).
¹ H-NMR (CDCl₃)δ: 1.32 (9H, s), 2.30-2.47 (2H, m), 3.15 (1H, dd, J = 6.6, 4.7 Hz), 3.43-3.54 (1H, m), 3.66-3.75 (1H, m), 4.73 (2H, ddd, J = 47.1, 17.5, 9.8 Hz), 5.75 (1H, s), 7.42 (1H, dd, J = 5.3, 1.0 Hz), 7.69 (1H, d, J = 1.0 Hz), 8.40 (1H, d, J = 5.3 Hz).

### Step 4 Synthesis of compound 48

To a solution of compound 47 (5.68 g) in methanol (50 mL) was added hydrochloric acid in methanol (2 mol/L, 50 mL) at room temperature. The mixture was stirred at the same temperature for 45 minutes, concentrated and dissolved in water. The mixture was washed with diethyl ether, neutralized with an aqueous sodium carbonate solution and extracted with ethyl acetate. The organic layer was washed with brine and dried over magnesium sulfate. After filtration of the extract, the solvent was evaporated under reduced pressure. The obtained crude product (4.23 g) was dissolved in dichloromethane (50 mL), and benzoyl isothiocyanate (2.67 g) was added under ice bath. The mixture was stirred at room temperature for 2 hours. The solvent was evaporated under reduced pressure, and the resulting residue was purified by silica gel column chromatography to afford compound 48 (6.59 g).
¹ H-NMR (CDCl₃) δ: 2.24 (1H, t, J = 5.8 Hz), 2.40 (1H, dt, J = 14.5, 5.8 Hz), 3.03 (1H, dt, J = 14.5, 5.8 Hz), 3.73 (2H, q, J = 5.8 Hz), 5.38 (2H, ddd, J = 57.6, 47.3, 9.8 Hz), 7.43 (1H, dd, J = 5.3, 1.3 Hz), 7.53 (2H, t, J = 7.7 Hz), 7.61-7.66 (2H, m), 7.87-7.90 (2H, m), 8.43 (1H, d, J = 5.3 Hz), 8.92 (1H, s), 11.95 (1H, s).

### Step 5 Synthesis of compound 49

Compound 48 (6.59 g) was dissolved in dichloromethane (130 mL) under nitrogen atmosphere and was added 1-chloro-N,N 2-trimethylpropenylamine (3.96 mL) under ice bath. The mixture was stirred at the same temperature for 1 hour, stirred at room temperature for 10 minutes and quenched with an aqueous sodium bicarbonate solution. The aqueous layer was extracted with ethyl acetate, and the organic layer was washed with brine and dried over magnesium sulfate. After filtration of the extract, the solvent was evaporated under reduced pressure, and the resulting residue was purified by silica gel column chromatography to afford compound 49 (3.09 g).
¹ H-NMR (CDCl₃) δ: 2.16-2.25 (1H, m), 2.67-2.94 (4H, m), 4.74 (2H, ddd, J = 59.6, 47.0, 8.8 Hz), 7.41-7.53 (4H, m), 7.65 (1H, s), 8.18 (2H, d, J = 7.0 Hz), 8.45 (1H, d, J = 5.2 Hz).

### Step 6 Synthesis of compound 50

Compound 49 (3.09 g) was dissolved in methanol (50 mL). To the solution was added hydrazine monohydrate (1.89 g) at room temperature. The mixture was stirred at the same temperature for 4 hours, and the solvent was evaporated under reduced pressure. The resulting residue was purified by silica gel column chromatography. The resulting crude product was triturated from hexane/ethyl acetate to afford a solid, which was collected by filtration. The obtained colorless solid was washed with hexane/ethyl acetate, and air-dried and dried in vacuo to afford compound 50 (2.02 g).
¹ H-NMR (CDCl₃) δ: 1.95 (1H, ddd, J = 13.2, 11.7, 3.6 Hz), 2.54-2.74 (2H, m), 2.88-2.95 (1H, m), 4.58 (2H, ddd, J = 46.8, 11.1, 8.7 Hz), 7.33-7.36 (1H, m), 7.66-7.67 (1H, m), 8.37 (1H, d, J = 5.0 Hz).

### Step 7 Synthesis of compound 51

Compound 50 (2.01 g) was dissolved in dichloromethane (20 mL). To the solution were added Boc₂O (3.61 g) and DMAP (40 mg) at room temperature, and the mixture was stirred at the same temperature for 1 hour. The solvent was evaporated under reduced pressure, and the residue was purified by silica gel column chromatography. The obtained crude product was triturated from hexane to afford a solid, which was collected by filtration. The resulting colorless solid was washed with hexane, and air-dried and dried in vacuo to afford compound 51 (2.82 g).
¹ H-NMR (CDCl₃) δ: 1.52 (9H, s), 1.55 (9H, s), 2.08-2.17 (1H, m), 2.62-2.70 (1H, m), 2.76-2.85 (1H, m), 2.98-3.05 (1H, m), 4.66 (2H, ddd, J = 47.7, 23.4, 8.7 Hz), 7.38 (1H, ddd, J = 5.3, 1.8, 0.7 Hz), 7.74 (1H, d, J = 1.8 Hz), 8.38 (1H, d, J = 5.3 Hz).

### Step 8 Synthesis of compound 52

Xantphos (42 mg) and Pd₂(dba)₃ (22 mg) were dissolved in degased 1,4-dioxane (1.5 mL) under nitrogen atmosphere. The mixture was stirred at room temperature for 20 minutes. To the mixture were added cesium carbonate (94 mg), compound 51 (121 mg) in 1,4-dioxane (3.8 mL) and 5-methoxypyrazine-2-carboxamide (44 mg). The mixture was stirred at 80 °C for 1 hour, then at 90 °C for 7 hours and left at room temperature overnight. The mixture was quenched with an aqueous citric acid solution and extracted with ethyl acetate. The organic layer was washed with water and brine and dried over sodium sulfate, and the solvent was evaporated under reduced pressure. The resulting residue was purified by silica gel column chromatography to afford compound 52 (131 mg).
¹ H-NMR (CDCl₃) δ: 1.55 (9H, s), 1.56 (9H, s), 2.04-2.13 (1H, m), 2.70-2.83 (2H, m), 2.93-3.01 (1H, m), 4.07 (3H, s), 4.75 (2H, ddd, J = 47.6, 31.6, 8.6 Hz), 7.42 (1H, d, J = 2.0 Hz), 8.07 (1H, dd, J = 1.4, 0.5 Hz), 8.25 (1H, dd, J = 5.5, 2.1 Hz), 8.54 (1H, d, J = 5.5 Hz), 9.00 (1H, dd, J = 1.4, 0.5 Hz), 9.75 (1H, s).

### Step 9 Synthesis of compound I-275

Compound 52 (130 mg) was dissolved in formic acid (863 µL), and this was stirred at room temperature for 3.5 hours. To the solution were added water and diethyl ether, and separated. To the aqueous layer was added an aqueous sodium carbonate solution. The mixture was extracted with ethyl acetate, washed with water and brine, and dried over magnesium sulfate. After filtration of the extract, the solvent was evaporated under reduced pressure, and the residue was purified by silica gel column chromatography. The resulting crude product was triturated from hexane-ethyl acetate to afford a solid, which was collected by filtration. The resulting colorless solid was washed with hexane-ethyl acetate, air-dried and dried in vacuo to afford compound (I-275) (68 mg).
¹H-NMR (CDCl₃) δ: 1.96 (1H, ddd, J = 12.6, 11.7, 4.2 Hz), 2.62-2.79 (2H, m), 2.88-2.95 (1H, m), 4.07 (3H, s), 4.64 (2H, ddd, J = 47.5, 16.1, 8.6 Hz), 7.44 (1H, d, J = 2.1 Hz), 7.96 (1H, dd, J = 5.5, 2.1 Hz), 8.15 (1H, dd, J = 1.2, 0.6 Hz), 8.53 (1H, d, J = 5.5 Hz), 9.01 (1H, dd, J = 1.2, 0.6 Hz), 9.63 (1H, s).

### Example 7 Synthesis of compound (I-281)

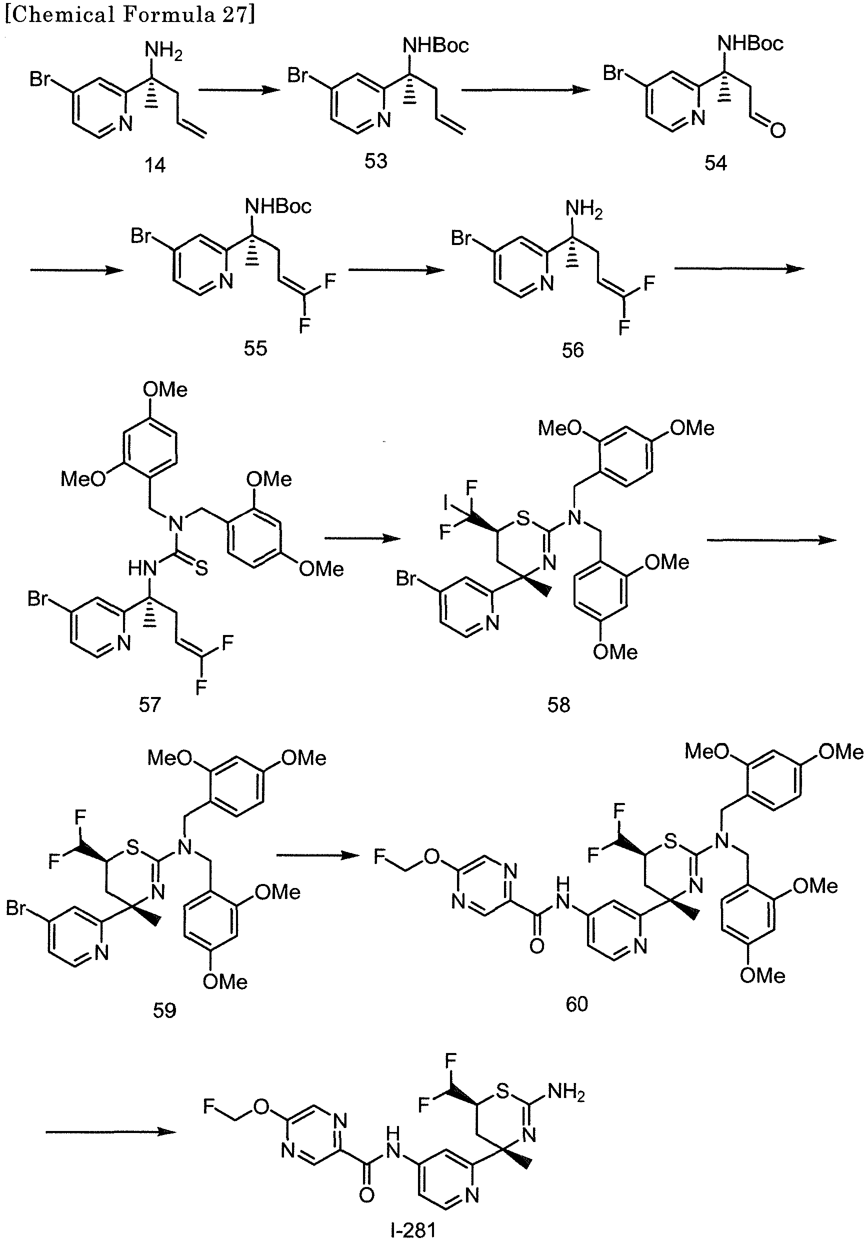

### Step 1 Synthesis of compound 53

Compound 14 (5.4 g) was dissolved in dichloromethane (70 mL). To the solution was added Boc₂O (5.2 mL) at room temperature, and the mixture was stirred at room temperature for 19 hours. The solvent was evaporated under reduced pressure, and the resulting residue was purified by silica gel column chromatography to afford compound 53 (7.5 g).
¹ H-NMR (CDCl₃) δ: 1.41 (9H, s), 1.69 (3H, s), 2.64 (1H, dd, J = 13.9, 6.9 Hz), 2.94 (1H, brs), 5.06 (1H, d, J = 13.9 Hz), 5.50 (1H, m), 5.98 (1H, brs), 7.32 (1H, dd, J = 5.3, 1.7 Hz), 7.54 (1H, d, J = 1.7 Hz), 8.34 (1H, d, J = 5.3 Hz).

### Step 2 Synthesis of compound 54

Compound 53 (7.5 g) was dissolved in tetrahydrofuran (150 mL) and was added water (150 mL). To the mixture were added potassium osmium(VI) oxide dihydrate (405 mg) and sodium periodate (14.1 g) under ice bath, and the mixture was stirred at room temperature for 2 hours. To the mixture was added water and extracted with ethyl acetate. The organic layer was dried over sodium sulfate, and the solvent was evaporated under reduced pressure. The resulting residue was purified by silica gel column chromatography to afford compound 54 (3.1 g).
¹ H-NMR (CDCl3) δ: 1.41 (9H, s), 1.68 (3H, s), 3.10 (1H, dd, J = 16.2, 2.2 Hz), 3.44 (1H, d, J = 16.2 Hz), 6.04 (1H, brs), 7.37 (1H, dd, J = 5.4, 1.7 Hz), 7.58 (1H, d, J = 1.7 Hz), 8.35 (1H, d, J = 5.4 Hz), 9.69 (1H, s).

### Step 3 Synthesis of compound 55

Compound 54 (3.1 g) was dissolved in DMF (31 mL). To the solution were added triphenylphosphine (9.48 g) and sodium 2-chloro-2,2-difluoroacetate (6.89 g) at room temperature, and the mixture was stirred at 80 °C for 1.5 hours. After the mixture was cooled under ice bath, water was added, and this was extracted with ethyl acetate. The organic layer was dried over sodium sulfate, and the solvent was evaporated under reduced pressure. The resulting residue was purified by silica gel column chromatography to afford compound 55 (1.81 g).
¹ H-NMR (CDCl₃)δ: 1.42 (9H, s), 1.65 (3H, s), 2.61 (1H, dd, J = 14.4, 7.9 Hz), 3.06 (1H, brs), 3.94 (1H, m), 6.13 (1H, brs), 7.35 (1H, d, J = 5.2 Hz), 7.54 (1H, brs), 8.34 (1H, d, J = 5.2 Hz).

### Step 4 Synthesis of compound 56

To a solution of compound 55 (1.81 g) in dichloromethane (36 mL) was added TFA (3.7 mL) under ice bath and stirred at room temperature for 5 hours. To the mixture was added saturated sodium bicarbonate solution under ice bath, and this was extracted with ethyl acetate. The organic layer was dried over sodium sulfate, and the solvent was evaporated under reduced pressure. The resulting residue was purified by silica gel column chromatography to afford compound 56 (1.18 g).
¹ H-NMR (CDCl₃) δ: 1.47 (3H, s), 2.42 (1H, dddd, J = 14.3, 8.6, 1.7, 0.8 Hz), 2.54 (1H, ddt, J = 14.3, 7.7, 2.0 Hz), 4.02 (1H, dddd, J = 25.2, 8.6, 7.7, 2.5 Hz), 7.33 (1H, dd, J = 5.2, 1.9 Hz), 7.69 (1H, d, J = 1.9 Hz), 8.38 (1H, d, J = 5.2 Hz).

### Step 5 Synthesis of compound 57

Compound 56 (1.18 g) was dissolved in dichloromethane (20 mL). To the solution was added thiocarbonyldiimidazole (1.14 g) under ice bath, and the mixture was stirred at room temperature for 3 hours. To the mixture was added bis(2,4-dimethoxybenyl)amine (2.03 g), and the mixture was stirred at room temperature for 2 hours and purified by silica gel column chromatography to afford compound 57 (1.69 g).
¹ H-NMR (CDCl₃) δ: 1.78 (3H, s), 2.92 (1H, dd, J = 14.5, 8.0 Hz), 3.57 (1H, ddt, J = 14.5, 8.0, 1.9 Hz), 3.71 (1H, td, J = 8.0, 2.7 Hz), 3.79 (6H, s), 3.79 (6H, s), 4.93 (4H, brs), 6.44-6.51 (4H, m), 7.18 (2H, d, J = 8.2 Hz), 7.26 (1H, dd, J = 5.3, 1.7 Hz), 7.38 (1H, dd, J = 1.7, 0.5 Hz), 7.43 (1H, s), 8.10 (1H, dd, J = 5.3, 0.5 Hz).

### Step 6 Synthesis of compound 58

Iodine (134 mg) was dissolved in acetonitrile (20 mL). To the solution were added compound 57 (280 mg) and p-toluenesulfonic acid monohydrate (100 mg) in acetonitrile (15 mL) under ice bath, and additional acetonitrile (5 mL) was added to wash the reactor wall. The mixture was stirred at 0 °C for 2 hours, 20 mL of an aqueous solution of sodium hydrogen sulfite (92 mg) and sodium bicarbonate (185 mg) were added, and this was extracted with ethyl acetate. The organic layer was dried over sodium sulfate, and the solvent was evaporated under reduced pressure. The resulting residue was purified by silica gel column chromatography to afford compound 58 (47.3 mg).
¹ H-NMR (CDCl₃) δ: 1.39 (1H, dd, J = 12.5, 11.8 Hz), 1.55 (3H, s), 3.17 (1H, dd, J = 12.5, 5.4 Hz), 3.30 (1H, ddt, J = 11.8, 7.7, 5.4 Hz), 3.75 (6H, s), 3.80 (6H, s), 4.62 (2H, d, J = 16.3 Hz), 4.71 (2H, d, J = 16.3 Hz), 6.43 (2H, d, J = 2.4 Hz), 6.48 (2H, dd, J = 8.4, 2.4 Hz), 7.15 (2H, d, J = 8.4 Hz), 7.26 (1H, dd, J = 5.2, 1.7 Hz), 7.34 (1H, d, J = 1.7 Hz), 8.32 (1H, d, J = 5.2 Hz).

### Step 7 Synthesis of compound 59

Compound 58 (54.9 mg) was dissolved in toluene (5 mL). To the solution were added tributyltin (114 µL) and azobisisobutyronitrile (4.7 mg) at room temperature. The mixture was stirred at 60 °C for 3 hours, and the solvent was evaporated under reduced pressure. The resulting residue was purified by silica gel column chromatography to afford compound 59 (15.6 mg).
¹ H-NMR (CDCl₃) δ: 1.40 (1H, m), 1.54 (3H, s), 2.98 (1H, dd, J = 12.9, 4.5 Hz), 3.25 (1H, m), 3.75 (6H, s), 3.81 (6H, s), 4.58 (2H, d, J = 16.5 Hz), 4.75 (2H, d, J = 16.5 Hz), 5.58 (1H, td, J = 56.3, 5.2 Hz), 6.44 (2H, d, J = 2.3 Hz), 6.49 (2H, dd, J = 8.2, 2.3 Hz), 7.15 (2H, d, J = 8.2 Hz), 7.26 (1H, dd, J = 5.2, 1.8 Hz), 7.39 (1H, brs), 8.32 (1H, d, J = 5.2 Hz).

### Step 8 Synthesis of compound 60

A solution of Pd₂(dba)₃ (5.0 mg) and Xantphos (9.4 mg) in 1,4-dioxane (1 mL) was degassed and replaced with nitrogen gas. The solution was stirred at room temperature for 20 minutes, and cesium carbonate (26.4 mg) was then added. The mixture was added dropwise into a solution of compound 59 (17.2 mg) and 5-fluoromethoxypyrazine-2-carboxamide (5.6 mg) in 1,4-dioxane (1 mL) that was degassed and replaced with nitrogen gas, and additional 1,4-dioxane (1 mL) was added to wash the reactor wall. The mixture was degassed and replaced with nitrogen gas and stirred at 80 °C for 3 hours then at 90 °C for 4.5 hours. To the mixture was added water under ice bath and extracted with ethyl acetate. The organic layer was dried over sodium sulfate, and the solvent was evaporated under reduced pressure. The resulting residue was purified by silica gel column chromatography to afford compound 60 (6.2 mg).
¹ H-NMR (CDCl₃)δ: 1.36 (1H, m), 1.60 (3H, s), 3.02 (1H, dd, J = 12.8, 4.4 Hz), 3.30 (1H, m), 3.70 (6H, s), 3.78 (6H, s), 4.53 (2H, d, J = 16.5 Hz), 4.86 (2H, d, J = 16.5 Hz), 5.60 (1H, td, J = 56.3, 5.3 Hz), 6.18 (2H, d, J = 51.0 Hz), 6.43-6.51 (4H, m), 6.97 (1H, d, J = 2.2 Hz), 7.23 (2H, d, J = 7.9 Hz), 8.01 (1H, dd, J = 5.5, 2.2 Hz), 8.42 (1H, d, J = 1.3 Hz), 8.52 (1H, d, J = 5.5 Hz), 9.09 (1H, d, J = 1.3 Hz) 9.28 (1H, s).

### Step 9 Synthesis of compound (I-281)

Compound 60 (6.7 mg) was dissolved in TFA (657 µL). To the solution was added anisole (9.3 µL), and the mixture was stirred at 80 °C for 13.5 hours. To the mixture was added a saturated sodium bicarbonate solution under ice bath, and this was extracted with ethyl acetate. The organic layer was dried over sodium sulfate, and the solvent was evaporated under reduced pressure. The resulting residue was purified by silica gel column chromatography. The resulting crude product was triturated from diisopropylether/hexane to afford a solid, which was collected by filtration. The solid was air-dried and dried in vacuo to afford compound (I-281) (1.5 mg).
¹ H-NMR (CDCl₃) δ: 1.57 (1H, dd, J = 13.0, 13.0 Hz), 1.67 (3H, s), 3.14 (1H, dd, J = 13.0, 4.6 Hz), 3.49 (1H, m), 3.85 (2H, s), 5.75 (1H, td, J = 55.7, 4.9 Hz), 6.16 (2H, d, J = 50.9 Hz), 7.36 (1H, d, J = 2.0 Hz), 7.98 (1H, dd, J = 5.5, 2.0 Hz), 8.32 (1H, d, J = 1.3 Hz), 8.54 (1H, d, J = 5.5 Hz), 9.08 (1H, d, J = 1.3 Hz), 9.68 (1H, s).

### Reference Example 1 Synthesis of compound 64

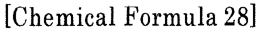

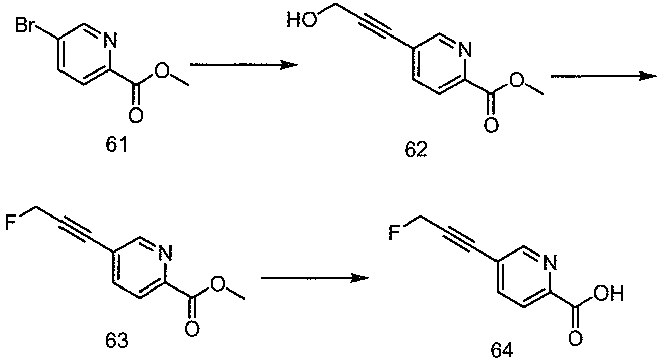

### Step 1

Methyl 5-bromopicolinate (61) (2.5 g) was dissolved in tetrahydrofuran (50 ml). To the solution were added propargylalcohol (828 µl), triethylamine (4.81 ml) and dichlorobis(triphenylphosphine) palladium(II) (406 mg), and the mixture was stirred at 70 °C for 3 hours. The mixture was filtered, and the solvent was evaporated under reduced pressure. The resulting residue was purified by silica gel column chromatography to afford compound (62) (1.32 g).
¹ H-NMR (CDCl₃) δ: 2.45 (1H, t, J = 6.1 Hz), 4.01 (3H, s), 4.55 (2H, d, J = 6.1 Hz), 7.86 (1H, d, J = 8.1 Hz), 8.09 (1H, d, J = 8.1 Hz), 8.80 (1H, s).

### Step 2

Compound (62) (1.30 g) was dissolved in dichloromethane (40 ml). To the solution was added bis(2-methoxyethyl) aminosulfur trifluoride (1.88 ml) at 0 °C, and the mixture was stirred for 40 minutes. To the mixture were added a saturated sodium bicarbonate solution and chloroform, which was then filtered. The filtrate was washed with brine and dried over sodium sulfate, and the solvent was evaporated under reduced pressure. The resulting residue was purified by silica gel column chromatography to afford compound (63) (202 mg).
¹H-NMR (CDCl₃) δ: 4.02 (3H, s), 5.22 (2H, d, J = 47.2 Hz), 7.90 (1H, dd, J = 8.1, 2.0 Hz), 8.12 (1H, d, J = 8.1 Hz), 8.80 (1H, d, J = 2.0 Hz).

### Step 3

Compound (63) (238 mg) was dissolved in tetrahydrofuran (2.4 ml) and methanol (2.4 ml). To the solution was added a 2.0 M sodium hydroxide solution (922 µl) at room temperature, and the mixture was stirred for 25 minutes. To the mixture was added ether, and this was extracted with water. A 2.0 M hydrochloric acid solution was added to the mixture at 0 °C, and the resulting solid was collected to afford compound (64) (197 mg).
¹ H-NMR (DMSO-d₆) δ: 5.42 (2H, d, J = 46.8 Hz), 8.05 (1H, d, J = 8.1 Hz), 8.11 (1H, dd, J = 8.2, 2.0 Hz), 8.81 (1H, d, J = 2.0 Hz), 13.43 (1H, brs).

### Reference Example 2 Synthesis of compound (II-1)

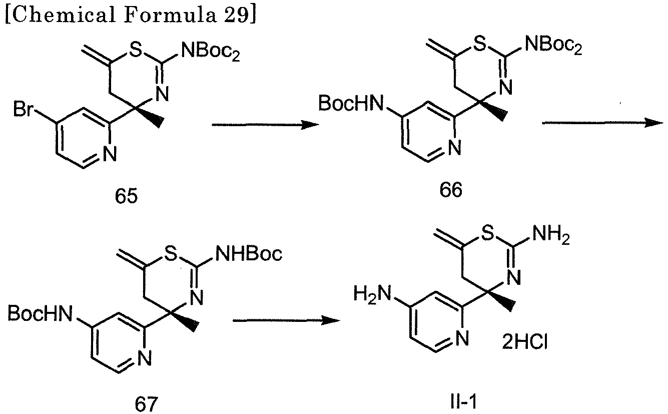

### Step 1

4,5-Bis(diphenylphosphino)-9,9-dimethylxanthene (1.045 g), tris(dibenzylideneacetone) dipalladium(0) (551 mg) was dissolved in 1,4-dioxane (20 ml), and the mixture was degassed and replaced with nitrogen gas. The solution was stirred at room temperature for 20 minutes and was added cesium carbonate (5.88 g). The mixture was added dropwise into a solution of compound (65) (3.0 g) and t- butyl carbamate (776 mg) in 1,4-dioxane (20 ml), which was then degassed and replaced with nitrogen gas following addition of 1,4-dioxane (20 ml) to wash the reactor wall. The mixture was degassed and replaced with nitrogen gas and stirred at 80 °C for 1 hour then at 90 °C for 5.5 hours. After the mixture was cooled to 0 °C, water and ethyl acetate were added. The mixture was filtered through Celite, and the filtrate was extracted with ethyl acetate. The organic layer was dried over sodium sulfate, and the solvent was evaporated under reduced pressure. The resulting residue was purified by silica gel column chromatography to afford compound (66) (1.78 g, a mixture with t-butyl carbamate).
¹H-NMR (CDCl₃) δ: 1.45 (3H, s), 1.50 (18H, s), 1.51 (9H, s), 2.32 (1H, brd, J = 14.0 Hz), 2.95 (1H, d, J = 14.0 Hz), 5.22 (1H, brs), 5.30 (1H, brs), 6.78 (1H, s), 7.43 (1H, dd, J = 5.5, 2.1 Hz), 7.49 (1H, d, J = 2.1 Hz), 8.40 (1H, d, J = 5.5 Hz).

### Step 2

Compound (66) (1.25 g, a mixture with t-butyl carbamate) was dissolved in tetrahydrofuran (5 ml). To the solution was added 4 M hydrochloric acid in 1,4-dioxane (5 ml), and the mixture was stirred at room temperature for 6 hours. To the mixture was added a saturated sodium bicarbonate solution and extracted with ethyl acetate. The organic layer was dried over sodium sulfate, and the solvent was evaporated under reduced pressure. The resulting residue was purified by silica gel column chromatography. The resulting crude product was triturated from ethyl acetate and hexane to afford compound (67) (567 mg).
¹ H-NMR (CDCl₃) δ: 1.53 (18H, s), 1.62 (3H, s), 2.80 (1H, d, J = 13.6 Hz), 3.09 (1H, brd, J = 13.6 Hz), 5.09 (2H, s), 6.72 (1H, s), 7.20 (1H, d, J = 2.0 Hz), 7.38 (1H, dd, J = 5.5, 2.0 Hz), 8.42 (1H, d, J = 5.5 Hz).

### Step 3

Compound (67) (335 mg) was dissolved in 4 M hydrochloric acid in ethyl acetate (9.64 ml). The solution was stirred at room temperature for 28 hours. The precipitated solid was collected by filtration and washed with ethyl acetate to afford compound (II-1) (191 mg).

The following compounds are prepared in a manner similar to the above. In tables, RT means a retention time (min).

**[Table 1-1]**

| No. | Structure | NMR(solvent:shift value;ascending order) | MS [M+1] | LC/MS RT | LC/MS Method |
|---|---|---|---|---|---|
| I-1 | 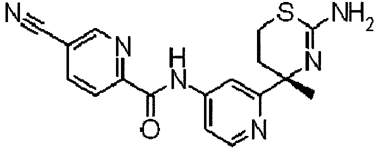 | 1H-NMR (DMSO-d6) d: 1.41 (3H, s), 1.67-1.76(1H, m), 1.99-2.28(1H, m), 2.54-2 60(1H, m), 2 78-2.93(1H, m), 1.79(2H, bs), 7.74(1H, dd, J= 3.0, 60Hz), 7.99 (1H, d, J=1.8Hz), 8.28(1H, dd, J = 0.9, 8.1Hz). 8.44(1H, d, J = 5.7Hz), 8.59(1H, dd, J = 2.1, 8.1Hz), 9.21(1H, dd, J= 0.9, 2.1Hz), 11.07(1H, bs). | 353 | 0.77 | A |
| I-2 | 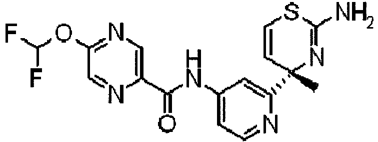 | 1H-NMR (CDCl3) δ: 1.65 (3H, s), 4.55 (2H, br), 6.30 (1H, d, J = 9.60 Hz) 6.37(1H, d, J = 9.60 Hz), 7.51 (1H, t, J = 71.1Hz), 7.69(1H, d, J= 2.1 Hz), 781(1H, dd, J = 2.1, 5.4 Hz), 8.34 (1H, d, J = 1.2Hz), 8.57 (1H, d, J = 5.4 Hz), 9.07 (1H, d, J = 1.2Hz), 9.58(1H, brs) | 393 | 1.1 | B |
| I-3 | 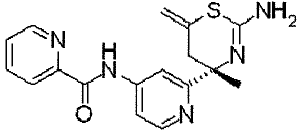 | 1H-NMR (CDCl3) δ: 1.55 (3H, s), 2.76 (2H, s), 5.08 (1H, s), 5.19 (1H, s), 7.50-7.54 (1H, m), 7 67 (1H, s), 7.90-7.95 (2H, m), 8 28 (1H, d, J= 8.2Hz), 8.53 (1H, d, J = 4.4Hz), 8.63 (1H, d, J = 4.6Hz), 10.18 (1H, s). | 340 | 1 14 | A |
| I-4 | 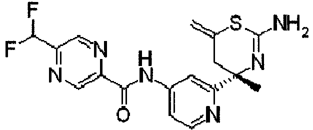 | 1H-NMR (CDCl3) δ: 1.67 (3H, s), 1.84 (3H, s), 2.69-2.74 (2H, m), 5.18 (1H, s), 5.32 (1H, s) 6 80 (1H, t, J = 53.9Hz), 7.51(1H, s), 8.05 (1H, br s), 8.56(1H, d, J = 5.0Hz) ), 8.96 (1H, s), 9.50 (1H, s), 9.95 (1H, s). | 391 | 1 24 | A |
| I-5 | 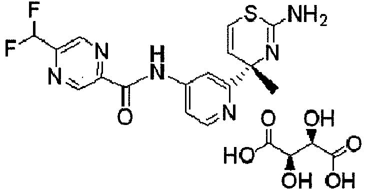 | 1H-NMR (CD3OD) δ: 1.87 (3H, s), 4.45 (2H, s), 6.53 (2H, s), 7.88-7.93 (1H, m), 8.03 (1H, dd, J = 13.2, 1.6 Hz), 8.34-8 43 (2H, m) 8.48-8.51 (1H m), 9.06-9.07 (1H, m). | 351 | 0.85 | A |
| I-6 | 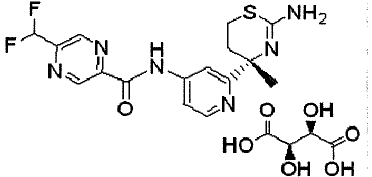 | 1H-NMR (CD3OD) δ: 1.75 (3H, s), 2.15-2.25 (1H, m), 2.76-2.94 (2H, m), 3.10-3.7 (1H, m), 4.45 (2H, s), 6.95 (1H, t, J = 54.2 Hz), 7.95-7.97 (2H, m), 8.54 (1H, d, J = 6.1 Hz), 9.04 (1H, s), 9.40 (1H, s). | 379 | 087 | A |
| I-7 | 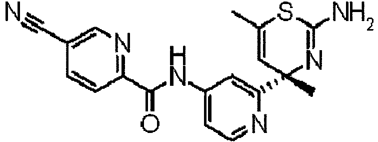 | 1H-NMR (CDCl3) δ: 1.94 (3H, s), 2.08 (3H, d, J = 1.5Hz), 6.24 (1H, d, J = 1.5 Hz), 7.70-7.71 (1H, m), 7.98 (1H, dd, J = 5.3, 1 9 Hz), 822 (1H, dd, J = 8.1, 2.1 Hz), 841 (1H dd, J = 8.1, 0.8 Hz), 8.55 (1H, d, J = 5.4 Hz), 894 (1H, br s), 10.17 (1H, s). | 365 | 092 | A |

**[Table 1-2]**

| No. | Structure | NMR(solvent:shift value;ascending order) | MS [M+1] | LC/MS RT | LC/MS Method |
|---|---|---|---|---|---|
| I-8 | 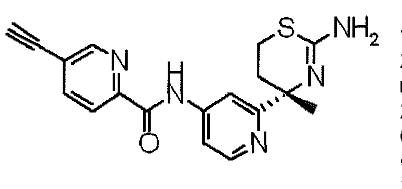 | 1H-NMR (CDCl3) δ: 1.25 (2H, s), 1.65 (3H, s), 1.89-1.98 (1H, m), 2.71-2.83 (2H, m), 2.92-2.99 (1H, m), 3.41 (1H, s), 7.45 (1H, d, J = 2.0 Hz), 7.98-8.02 (2H, m), 8.25 (1H, dd, J = 8.1, 0.8Hz), 8.54(1H, d, J = 5.5 Hz), 8.71-8.72 (1H, m), 10.10 (1H, s). | 352 | 0.98 | A |
| I-9 | 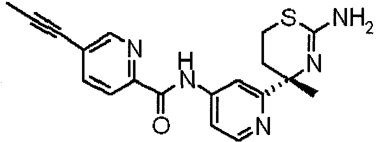 | 1H-NMR (CDCl3) δ: 1.82 (3H, s), 2.13 (3H, s), 2.28-2.35 (1H, m), 2.82-3.02 (2H, m), 3.20-3.28 (1H, m), 7.43 (1H, s), 7.86-7.88 (2H, m), 8.13 (1H, d, J = 4.4 Hz), 8.18 (2H, d, J = 8.1 Hz), 8.53 (2H, d, J = 5.4 Hz), 8.63 (2H, s), 10.20 (1H, s). | 366 | 1.15 | A |
| I-10 | 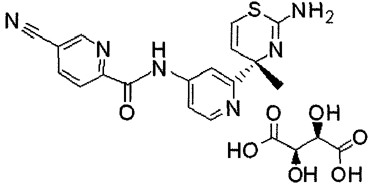 | | 351 | 0.85 | A |
| I-11 | 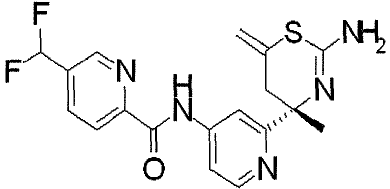 | 1H-NMR (CDCl3) δ: 1.57 (9H, s), 2.76 (3H, d. J = 13.9 Hz), 2.83 (3H, d, J =13.9 Hz), 5.09 (3H, s), 5.20 (3H, s), 6.81 (4H, t, J = 55.3 Hz), 7.68 (3H, d, J = 2.0 Hz), 7.91 (3H, dd, J = 5.6. 2.0 Hz), 8.08 (3H, d, J = 8.2 Hz), 8.39 (3H, d, J = 8.2 Hz), 8.55 (3H, d, J = 5.6 Hz), 8.78 (2H, s), 10.11 (2H, s). | 390 | 1.21 | A |
| I-12 | 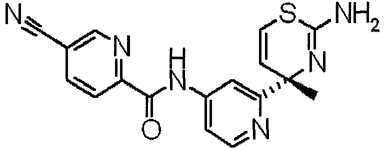 | 1H-N MR (CDCl3) δ: 1.65 (3H, s). 4.56 (2H, brs), 6.31 (1H, d, J = 9.6 Hz), 6.37 (1H, d, J = 9.6 Hz), 7.71 (1H, d. J = 2.1 Hz), 7.83 (1H, dd, J = 5.5, 2.1 Hz), 8.22 (1H, dd, J = 8.1, 2.0 Hz), 8.43 (1H, d, J = 8.1 Hz), 8.58 (1H, d, J = 5.5 Hz), 8.91 (1H, m), 9.96 (1H, s) | 351 | 0.82 | A |
| I-13 | 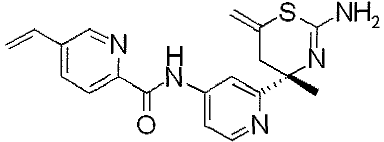 | | 366 | 1.44 | A |

**[Table 1-3]**

| No. | Structure | NMR(solvent:shift value;ascending order) | MS [M+1] | LC/MS RT | LC/MS method |
|---|---|---|---|---|---|
| I-14 | 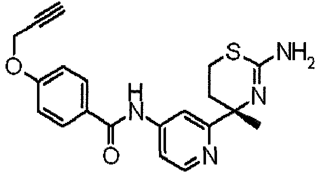 | 1H-NMR (DMSO-d6) δ: 1.40 (3H, s), 1.65-1.74 (1H, m), 2.22-2.29 (1H, m), 2.53-2.61 (1H, m), 2.86-2.95 (1H, m), 3.60 (1H, s), 4.91 (2H, s). 5.79 (2H, br s). 7.12 (2H, d, J = 8.1 Hz), 7.69 (1H, d, J = 5.6 Hz), 7.77 (1H, s), 7.97 (2H, d, J = 8.1 Hz), 8.40 (1H, d, J = 5.6 Hz), 10.48 (1H, s). | 381 | 1.11 | B |
| I-15 | 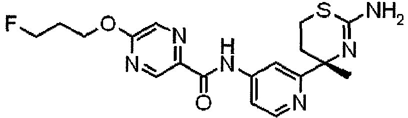 | 1H-NMR (DMSO-d6) δ: 1.40 (3H. s), 1.65-1.76 (1H, m), 2.10-2.28 (3H, m), 2.53-2.61 (1H, m), 2.85-2.94 (1H, m). 4.48-4.60 (3H, m), 4.66-4.72 (1H, m), 5.78 (2H, brs), 7.71 (1H, d, J = 4.5 Hz), 7.96 (1H, s), 8.38-8.45 (2H, m), 8.89 (1H, s), 10.73 (1H, brs). | 405 | 1.08 | B |
| I-16 | 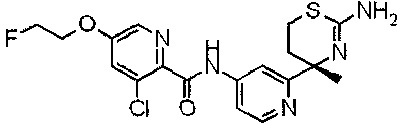 | 1H-NMR (DMSO-d6) δ: 1.40 (3H. s), 1.65-1.74 (1H, m), 2.21-2.29 (1H, m), 2.53-2.60 (1H, m), 2.85-2.94 (1H, m), 4.41-4.45 (1H, m), 4.48-4.53 (1H, m), 4.71-4.75 (1H, m), 4.82-4.87 (1H, m), 5.82 (2H, br s), 7.66-7.74 (2H, m), 7.79 (1H, s), 8.37-8.45 (2H, m), 10.87 (1H, br s). | 424 | 1.05 | B |
| I-17 | 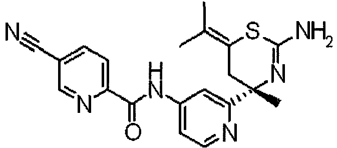 | 1H-NMR (DMSO-d6) δ: 1.35 (3H, s), 1.63 (3H, s), 1.69 (3H, s), 2.62 (2H, s), 6.02 (2H, brs). 7.72 (1H, d. J = 5.6 Hz), 8.06 (1H, s), 8.29 (1H, d, J = 8.1 Hz), 8.44 (1H, d, J = 5.6 Hz), 8.59 (1H, d, J = 8.1 Hz), 9.21 (1H, s), 11.03 (1H, brs). | 393 | 1.16 | B |
| I-18 | 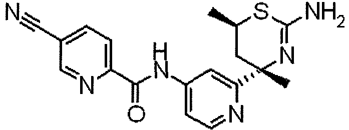 | 1H-NMR (DMSO-d6) δ: 1.12 (3H, d, J = 6.6 Hz), 1.21-1.30 (1H, m), 1.48 (3H, s), 2.57-2.73 (2H, m), 7.74-7.79 (1H, m), 7.89 (1H, s), 8.29 (1H, d, J = 8.1 Hz), 8.48 (1H, d, J = 5.6 Hz), 8.59 (1H, d, J = 8.1 Hz), 9.21 (1H, s), 11.13 (1H, br s). | 367 | 1.05 | B |
| I-19 | 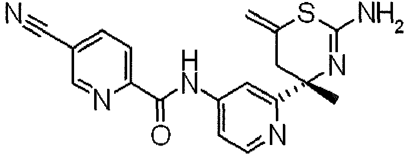 | 1H-NMR (DMSO-d6) δ: 1.36 (3H, s), 2.53-2.62 (1H, m), 3.31-3.42 (1H, m), 4.98 (1H, s), 5.08 (1H, s), 6.13 (2H, s), 7.73 (1H, dd, J = 5.3, 2.3 Hz). 8.11 (1H, d, J = 2.3 Hz), 8.29 (1H, d, J = 8.1 Hz), 8.45 (1H, d, J = 5.3 Hz), 8.60 (1H, dd, J = 2.3, 8.1 Hz), 9.22 (1H, d, J = 2.3 Hz), 11.04 (1H, s). | 365 | 1 | B |

**[Table 1-4]**

| No. | Structure | NMR(solvent:shift value;asceding order) | MS [M+1] | LC/MS RT | LC/MS Method |
|---|---|---|---|---|---|
| I-20 | 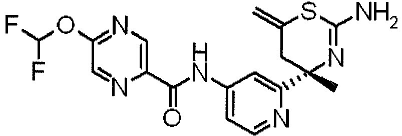 | 1H-NMR (DMSO-d6) δ: 1.36 (3H. s), 2.54-2.58 (1H, m), 3.29-3.34 (1H, m), 4.97 (1H, s), 5.07 (1H, s). 6.11 (2H, br s), 7.71 (1H, dd, J = 2.0, 5.6 Hz). 7.81 (1H, t, J = 71.5 Hz), 8.10 (1H, d, J = 2.0 Hz), 8.44 (1H, d, J = 5.6 Hz), 8.70 (1H, d, J = 1.0 Hz), 8.99 (1H, d, J = 1.0 Hz), 10.90 (1H, s). | 407 | 1.21 | B |
| I-21 | 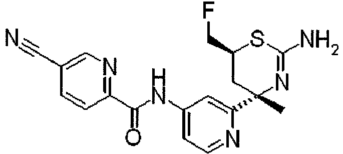 | 1H-NMR (DMSO-d6) δ: 1.16-1.27 (1H, m), 1.46 (3H, s), 2.6-2.74 (1H, m), 2.96-3.07 (1H, m), 4.25-4.57 (2H, m), 6.06 (2H, br s), 7.73-7.78 (1H, m), 7.91 (1H, s), 8.27-8.32 (1H, m), 8.46-8.50 (1H, m), 8.57-8.62 (1H. m). 9.21 (1H, s), 11.13 (1H,s). | 385 | 1.04 | B |
| I-22 | 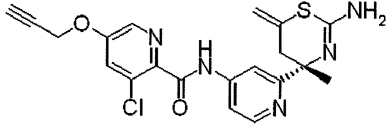 | 1H-NMR (DMSO-d6) δ: 1.38 (3H, s). 2.60 (2H, s), 3.73 (1H, s), 5.01 (1H, s), 5.06 (2H, s), 5.09 (1H, s), 7.61-7.68 (1H, m), 7.81 (1H, s), 7.86 (1H, s), 8.38-8.45 (2H, m), 10.89 (1H, s), | 428 | 1.26 | B |
| I-23 | 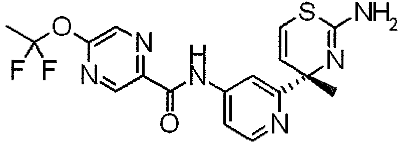 | 1H-NMR (CDCl3) δ: 1.65 (3H, s). 2.14 (3H, t, J = 13.9 Hz), 4.54 (2H, br s), 6.30 (1H, d, J = 9.6 Hz). 6.38 (1H, d, J = 9.6 Hz), 7.70 (1H, d, J = 2.0 Hz), 7.81 (1H, dd, J = 5.6, 2.0 Hz), 8.40 (1H, s), 8.57 (1H, d, J = 5.6 Hz), 9.17 (1H, s), 9.60 (1H, s). | 407 | 1.09 | A |
| I-24 | 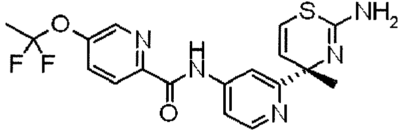 | 1H-NMR (CDCl₃) δ: 1.66 (3H, s). 2.02 (3H, t, J = 13.4 Hz). 4.55 (2H. br s), 6.30 (1H, d, J = 9.6 Hz), 6.38 (1H, d, J = 9.6 Hz), 7.70-7.75 (2H. m), 7.82 (1H, dd, J = 5.6, 2.0 Hz), 8.29 (1H, d, J = 8.6 Hz), 8.47 (1H, d, J = 2.5 Hz), 8.56 (1H, d, J = 5.6 Hz), 9.99 1H, s). | 406 | 1.2 | A |
| I-25 | 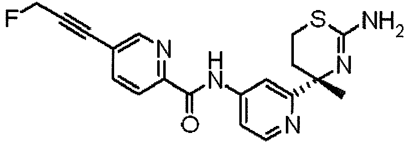 | 1H-NMR (DMSO-d6) δ: 1.40 (3H, s), 1.68-1.75 (1H, m), 2.22-2.25 (1H, m), 2.55-2.61 (1H, m), 2.88-2.93 (1H, m), 5.44 (2H, d, J = 46.6 Hz), 5.81 (2H, brs), 7.75 (1H, dd, J = 5.6, 2.3 Hz). 7.99 (1H, d, J = 1.5 Hz), 8.18 (1H, d, J = 8.1 Hz). 8.22 (1H, dd, J = 8.1, 2.3 Hz). 8.44 (1H, d, J = 5.6 Hz), 8.87 (1H, d, J = 1.5 Hz), 10.96 (1H, s). | 384 | 1.03 | A |

**[Table 1-5]**

| No. | Structure | NMR(solvent:shift value;ascending order) | MS [M+1] | LC/MS RT | LC/MS Method |
|---|---|---|---|---|---|
| I-26 | 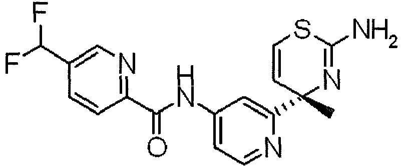 | 1H-NMR (CDCl₃) δ: 1.66 (3H. s). 4.56 (2H. br s). 6.30 (1H. d. J = 9.6 Hz). 6.39 (1H, d. J = 9.6 Hz). 6.82 (1H. t. J = 55.5 Hz). 7.73 (1H. d. J = 2.0 Hz). 7.85 (1H. dd. J = 5.6, 2.0 Hz). 8.08 (1H. d. J = 8.1 Hz). 8.40 (1H. d. J = 8.1 Hz), 8.58 (1H, d, J = 5.6 Hz). 8.78 (1H. s). 10.08 (1H. s). | 376 | 1.01 | A |
| I-27 | 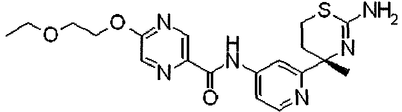 | 1H-NMR (CDCl3) δ: 1.74 (3H, s), 1.96 (1H, ddd, J = 13.5, 12.3, 3.9 Hz). 2.82 (1H, dt, J = 12.3, 3.6 Hz), 2.94 (1H, dt, J = 12.6, 3.9 Hz), 3.06 (1H, dt, 13.5, 3.6 Hz), 3.60 (1H, q, J = 7.2 Hz). 3.83 (2H, m), 4.60 (2H. m). 7.41 (1H, d, J = 1.8 Hz), 8.06 (1H, dd, J = 5.7, 1.5 Hz), 8.24 (1H, dd, J = 1.2 0.6 Hz), 8.52 (1H, d, J = 5.7 Hz), 8.98 (1H, dd. J =1.2, 0.6 Hz), 9.78 (1H. br s) | 417 | 1.08 | B |
| I-28 | 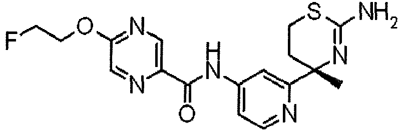 | 1H-NMR (DMSO-d6) δ: 1.49 (3H. s), 1.81 (1H, m), 2.39 (1H, m), 2.54 (1H, m), 2.96 (1H, m), 4.67 (1H, dt, J = 31.5, 3.0 Hz), 4.81 (1H, dt, J = 43.8, 3.9 Hz), 7.79 (1H, dd, J = 5.4, 1.8 Hz), 7.98 (1H, d, J = 1.8 Hz), 8.45 (1H, d, J = 5.7Hz), 8.49 (1H, dd, J = 1.5Hz), 8.90 (1H, d, J = 1.2 Hz), 10.9 (1H, brs) | 391 | 1.02 | B |
| I-29 | 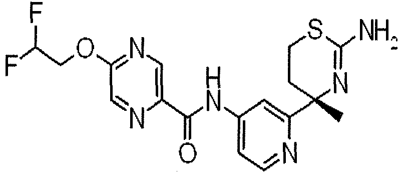 | 1H-NMR (DMSO-d6) δ: 1.40 (3H. s), 1.70 (1H, m), 2.23 (1H, m), 2.56 (1H, m), 2.89 (1H, m), 4.74 (1H, td, J = 15.0. 3.3 Hz). 5.83 (1H, br s), 6.47 (1H, tt, J = 54.0, 3.3 Hz), 7.71 (1H, dd, J = 5.4, 1.8 Hz). 7.98 (1H, d. J = 2.1 Hz), 8.42 (1H, d, J = 5.7 Hz). 8.54 (1H, d, J = 1.2 Hz), 8.91 (1H, d, J = 1.2 Hz), 10.8 (1H, br s) | 409 | 1.1 | B |
| I-30 | 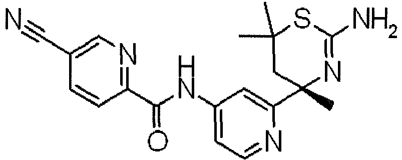 | 1H-NMR (CDCl3) δ: 0.81 (3H, s), 1.38 (3H, s), 1.56 (3H, s), 1.75 (1H, d, J = 14.1 Hz), 3.01 (1H, d, J = 14.1 Hz), 7.56 (1H, d, J = 1.8 Hz), 7.95 (1H, dd, J = 5.4, 2.1 Hz). 8.22 (1H, dd, J = 8.1, 2.1 Hz), 8.42 (1H, dd, J = 8.1, 0.6Hz), 8.54 (1H, d, J = 5.4 Hz), 8.91 (1H, dd, J = 2.1, 0.9 Hz) 9.96 (1H, br s) | 381 | 1.06 | B |

**[Table 1-6]**

| No. | Structure | NMR(solvent:shift value;ascending order) | MS [M+1] | LC/MS RT | LC/MS Method |
|---|---|---|---|---|---|
| I-31 | 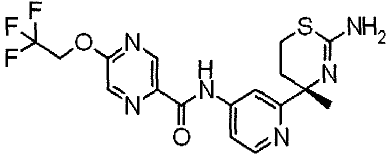 | 1H-NMR (DMSO-d₆) δ: 1.40 (3H, s), 1.69 (1H, ddd, J = 12.6, 10.5. 3.6 Hz), 2.24 (1H, ddd, J = 12.6, 6.3, 3.3 Hz), 2.55 (1H, m), 2.90 (1H. ddd. J = 12.0, 6.3. 3.9 Hz), 5.16 (q. J = 9.0 Hz), 5.80 (2H, br s), 7.71 (1H, dd, J = 5.4, 2.1 Hz), 7.98 (1H, d, J = 1.5 Hz), 8.43 (1H, dd, J = 5.7. 0.6 Hz), 8.62 (1H, d, J = 1.5 Hz), 8.93 (1H, d, J = 1.5 Hz). 10.9 (1H, br s) | 427 | 1.24 | B |
| I-32 | 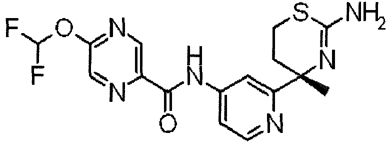 | 1H-NMR (DMSO-d6) δ: 1.85 (3H, s), 2.35, (1H, m), 2.62 (1H, m). 2.97 (1H, m), 7.78 (1H, m), 7.80 (1H, t, J = 70.5 Hz). 8.00 (1H, d, J = 1.8 Hz), 8.45 (1H, dd, J = 3.6 Hz), 8.69 (1H, d, J = 1.5 Hz), 8.98 (1H, d, J = 1.2 Hz), 11.0 (1H, br s) | 395 | 1.11 | A |
| I-33 | 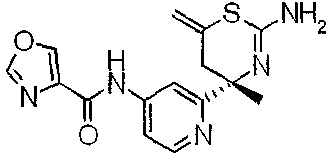 | 1H-NMR (DMSO-d6) δ: 1.35 (3H, s), 2.50-2.60 (2H, m), 4.97 (1H, s), 5.06 (1H, s), 6.10 (2H, brs), 7.65 (1H, d J = 6.1 Hz), 7.99 (1H, s), 8.40 (1H, s), 8.63 (1H, d, J = 6.16 Hz), 8.85 (1H, s), 10.48 (1H, s). | 330 | 0.77 | B |
| I-34 | 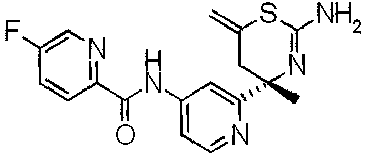 | | 358 | 1.03 | B |
| I-35 | 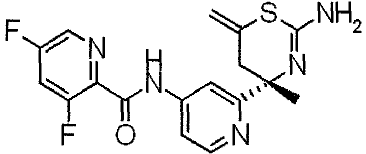 | 1H-NMR (DMSO-d6) δ: 1.36 (3H, s), 2.51-2.60 (2H, m), 4.97 (1H, s), 5.07 (1H, s), 6.14 (2H, br s), 7.67 (1H, d, J = 5.6 Hz), 7.94 (1H, s). 8.18 (1H, t, J = 9.9Hz), 8.44 (1H, d, J = 5.6 Hz). 8.66 (1H, s), 10.87 (1H, s). | 376 | 0.97 | B |
| I-36 | 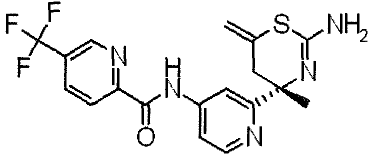 | 1H-NMR (DMSO-d6) δ: 1.37 (3H, s), 2.52-2.62 (2H, m), 4.98 (1H, s), 5.08 (1H, s), 6.16 (2H, brs), 7.75 (1H, d, J =5.0 Hz). 8.10 (1H, s). 8.34 (1H, d, J = 8.4 Hz), 8.46 (1H, d, J = 5.0Hz), 8.51 (1H, d, J = 8.4 Hz), 9.13 (1H, s), 11.03 (1H, s). | 408 | 1.31 | B |
| I-37 | 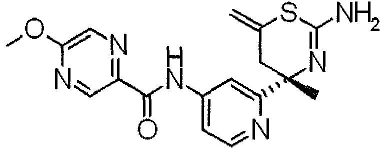 | 1H-NMR (DMSO-d6) δ: 1.36 (3H. s), 2.52-2.60 (2H, m), 4.02 (3H, s), 4.97 (1H, s), 5.07 (1H, s), 6.12 (2H, br s), 7.69 (1H, dd, J = 1.2. 5.6 Hz). 8.09 (1H, s), 8.41 (1H, s), 8.90 (1H, d. J = 1.2Hz). 10.71 (1H, s). | 371 | 1.09 | B |

**[Table 1-7]**

| No. | Structure | NMR(solvent:shift value;ascending order) | MS [M+1] | LC/MS RT | LC/MS Method |
|---|---|---|---|---|---|
| I-38 | 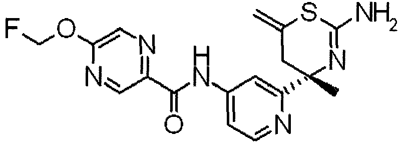 | 1H-NMR (DMSO-d6) δ: 1.36 (3H. s), 2.54-2.61 (2H, m), 4.98 (1H, s), 5.07 (1H, s), 6.14 (2H, br s), 6.21 (2H, d, J = 51 Hz), 7.71 (1H, d, J = 5.0 Hz), 8.10 (1H, s), 8.44 (1H, d, J = 5.0 Hz). 8.59 (1H, s), 10.83 (1H, s). | 389 | 1.08 | B |
| I-39 | 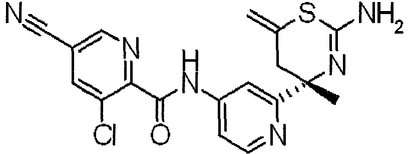 | 1H-NMR (DMSO-d6) δ: 1.36 (3H. s), 2.50-2.55 (2H, m), 4.97 (1H, s), 5.07 (1H, s), 6.15 (2H, br s), 7.63 (1H, d. J = 4.0 Hz), 7.76 (1H, d, J = 2.2 Hz), 8.46 (1H, d, J = 4.0 Hz). 8.82 (1H, s), 9.11 (1H, d, J = 2.2 Hz), 11.20(1H, s). | 399 | 1.07 | B |
| I-40 | 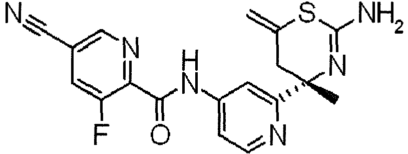 | 1H-NMR (DMSO-d6) δ: 1.36 (3H, s), 2.53-2.60 (2H, m), 4.98 (1H, s), 5.07 (1H, s), 6.18 (2H, br s), 7.66 (1H, d, J = 5.0 Hz), 7.90 (1H, s), 8.46 (1H, d, J = 5.0 Hz), 8.67 (1H, d. J = 9.6 Hz), 9.04 (1H, s), 11.11 (1H, s). | 383 | 0.97 | B |
| I-41 | 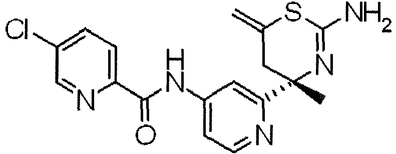 | 1H-NMR (DMSO-d6) δ: 1.36 (3H. s), 2.50-2.60 (2H, m), 4.98 (1H, s), 5.07 (1H, s), 6.13 (2H, br s), 7.72 (1H, d, J = 5.4 Hz). 8.09 (1H, s). 8.19 (2H. dd. J = 7.5, 21.8 Hz), 8.43 (1H, d, J = 5.0 Hz), 8.80 (1H, s), 10.88 (1H, s). | 374 | 1.15 | B |
| I-42 | 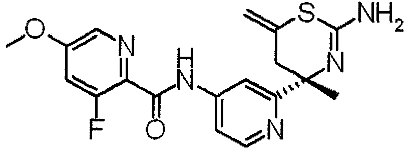 | 1H-NMR (DMSO-d6) δ: 1.36 (3H, s), 2.50-2.60 (2H, m), 3.95 (3H. s), 4.98 (1H, s), 5.08 (1H, s), 6.13 (2H, br s), 7.63 (1H, d, J = 15.2Hz). 7.66 (1H, d, J = 5.6 Hz), 7.97 (1H, s), 8.29 (1H, s), 8.41 (1H, d, J = 5.6 Hz), 10.90 (1H, s). | | | |
| I-43 | 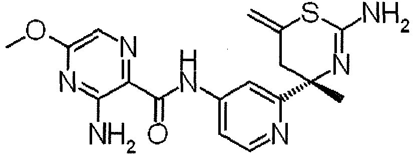 | 1H-NMR (DMSO-d6) δ: 1.34 (3H, s), 2.44 (1H, d, J = 12.8 Hz), 2.61 (1H, d, J =12.8 Hz), 4.99 (1H, s), 5.09 (1H, s). 6.12 (2H, br s), 7.54 (1H, s), 7.65 (1H, d, J = 4.0 Hz), 7.73 (2H. br s), 8.01 (1H, s). 8.38 (1H, d, J = 4.0 Hz), 10.34 (1H, s), | 386 | 1.05 | A |
| I-44 | 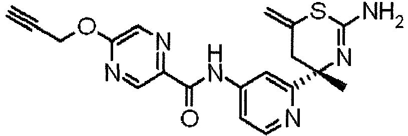 | 1H-NMR (DMSO-d6) δ: 1.36 (3H, s), 2.50-2.60 (2H, m), 3.64 (1H. s), 4.98 (1H, s), 5.07 (1H, s), 5.15 (2H, s), 6.13 (2H, br s), 7.70 (1H, d. J = 4.8 Hz), 8.10 (1H, s), 8.43 (1H, d, J = 4.8 Hz), 8.49 (1H, s), 8.92 (1H, s). 10.75 (1H, s), | 395 | 1.16 | B |

**[Table 1-8]**

| No. | Structure | NMR(solvent:shift value;ascending order) | MS [M+1] | LC/MS RT | LC/MS Method |
|---|---|---|---|---|---|
| I-45 | 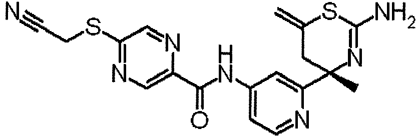 | 1H-NMR (CDCl3) δ: 1.53 (3H, s), 2.72 (1H, d, J = 14.0 Hz), 2.76 (1H, d. J = 14.0 Hz), 4.08 (2H, s), 5.08 (1H, s), 5.18 (1H, s), 7.64 (1H, d, J = 2.2 Hz), 7.88 (1H, dd, J = 5.5, 2.2 Hz), 8.52 (1H, d, J = 1.5 Hz), 8.56 (1H, d. J = 5.5 Hz), 9.34 (1H, d, J = 1.5 Hz), 9.63 (1H, s) | 412 | 1.2 | A |
| I-46 | 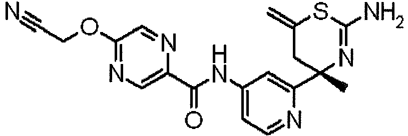 | 1H-NMR (CDCl3) δ: 1.52 (3H, s). 2.68 (1H, d. J = 13.9 Hz). 2.77 (1H, d, J = 13.9Hz), 5.08 (1H, s), 5.15 (2H, s), 5.18 (1H, s), 7.65 (1H, d. J = 2.2 Hz), 7.87 (1H, dd, J = 5.5, 2.2 Hz), 8.32 (1H, d, J = 1.2 Hz), 8.55 (1H, d. J = 5.5 Hz), 9.09 (1H, d, J = 1.2 Hz), 9.59 (1H, s) | 396 | 0.99 | A |
| I-47 | 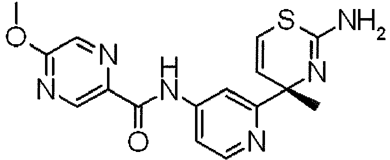 | | 357 | 1.01 | A |
| I-48 | 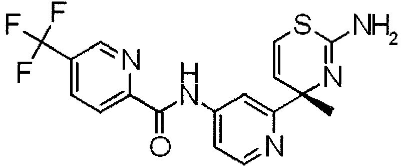 | 1H-NMR (CDCl3) δ: 1.68 (3H, s), 3.78 (2H, brs), 6.30 (1H, d. J = 9.6 Hz), 6.38 (1H, d, J = 9.6 Hz). 7.72 (1H, d, J = 2.1 Hz), 7.84 (1H, dd, J = 5.5, 2.1 Hz), 8.18 (1H, dd, J = 8.2, 2.2 Hz), 8.42 (1H, d, J = 8.2 Hz), 8.57 (1H, d, J = 5.5 Hz), 8.89 (1H, brs), 10.05 (1H, s) | 394 | 1.18 | A |
| I-49 | 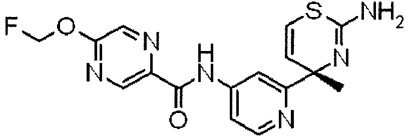 | 1H-NMR (CDCl3) δ: 1.65 (3H, s), 4.55 (2H, brs). 6.15 (2H, d, J = 50.9 Hz). 6.30 (1H, d, J = 9.6 Hz), 6.37 (1H, d, J = 9.6 Hz), 7.69 (1H, d, J = 2.2 Hz), 7.81 (1H, dd, J = 5.6, 2.2 Hz), 8.29 (1H, d, J = 1.3 Hz), 8.56 (1H, d, J = 5.6 Hz), 9.08 (1H, d, J = 1.3 Hz). 9.61 (1H, s) | 375 | 0.87 | A |
| I-50 | 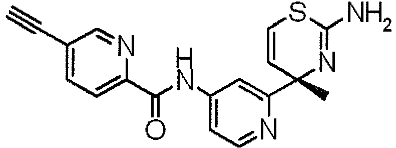 | 1H-NMR (CDCl3) δ: 1.65 (3H, s), 3.40 (1H, s), 4.54 (2H, brs), 6.30 (1H, d, J = 9.6 Hz), 6.38 (1H, d. J = 9.6 Hz), 7.71 (1H, d, J = 2.0 Hz), 7.83 (1H, dd, J = 5.5, 2.0 Hz). 7.99 (1H, dd, J = 8.1, 2.0 Hz), 8.25 (1H, d, J = 8.1 Hz), 8.56 (1H, d, J = 5.5 Hz), 8.70 (1H, m), 10.04 (1H, s) | 350 | 1.05 | A |
| I-51 | 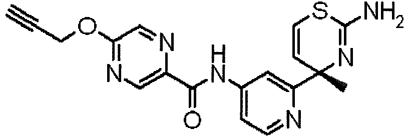 | 1H-NMR (CDCl3) δ: 1.65 (3H, s). 2.55 (1H, t, J = 2.3 Hz), 5.09 (2H, d, J = 2.3 Hz). 6.30 (1H, d, J = 9.6 Hz), 6.36 (1H, d, J = 9.6 Hz). 7.68 (1H, d, J = 2.1 Hz), 7.81 (1H, dd, J = 5.5, 2.1 Hz), 8.23 (1H, d, J = 1.2 Hz). 8.55 (1H, d, J = 5.5 Hz), 9.04 (1H, d, J = 1.2 Hz), 9.61 (1H, s) | 381 | 1.04 | A |

**[Table 1-9]**

| No. | Structure | NMR(solvent:shift value;ascending order) | MS [M+1] | LC/MS RT | LC/MS Method |
|---|---|---|---|---|---|
| I-52 | 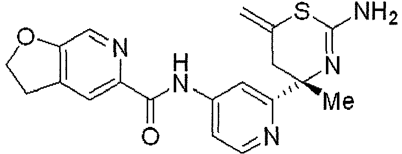 | 1H NMR (CDCl3) d: 1.51 (3H, s), 2.66 (1H, d, J = 13.8 Hz), 2.76 (1H, d, J = 13.8 Hz), 3.33 (2H, t, J = 9.0 Hz), 4.73 (2H, t, J = 9.0 Hz), 5.07 (1H, s), 5.19 (1H, s), 7.66 (1H, d, J = 1.5 Hz), 7.85 (1H, dd, J = 1.5, 8.0 Hz), 8.08 (1H, s), 8.15 (1H, s), 8.51 (1H, d, J = 8.0 Hz), 9.99 (1H, s). | 382 | 1.12 | B |
| I-53 | 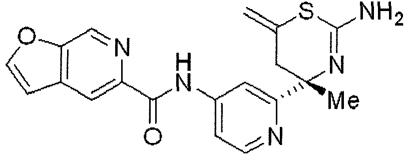 | 1H NMR (CDCl3) d: 1.81 (3H, s), 2.75 (1H, d, J = 14.1 Hz), 3.55 (1H, d, J = 14.1 Hz), 5.12 (1H, s), 5.23 (1H, s), 6.97 (1H, s) 7.54 (1H, s), 7.86 (1H, d, J = 1.5 Hz), 7.96 (1H, dd, J = 1.5, 8.0 Hz), 8.51 (1H, d, J = 8.0Hz), 8.56 (1H, s), 8.86 (1H, s), 10.39 (1H, s). | 380 | 1.17 | B |
| I-54 | 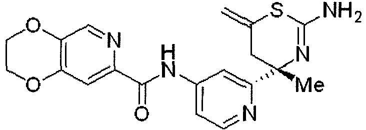 | 1H NMR (DMSO-d6) d: 1.36 (3H, s), 2.56 (2H, dd, J = 8.4, 13.8 Hz), 4.43 (4H, brs), 4.99 (1H, s), 5.08 (1H, s), 7.60 (1H, s), 7.68 (1H, dd, J = 2.1, 5.4 Hz), 8.06 (1H, d, J = 2.1 Hz), 8.26 (1H, s), 8.40 (1H, d, J = 5.4 Hz), 10.7 (1H, s) | 1.07 | 398 | B |
| I-55 | 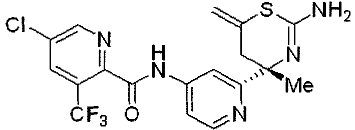 | 1H NMR (DMSO-d6) d: 1.29 (3H, s), 2.56 (2H, m), 4.98 (1H, s), 5.07 (1H, s), 7.63 (1H, dd, J = 2.1, 5.1 Hz), 7.75 (1H, d, J = 1.8 Hz), 8.46 (1H, d, J = 5.1 Hz), 8.65 (1H, d, J = 2.1 Hz), 9.05 (1H, d, J = 1.8 Hz), 11.1 (1H, s) | 1.22 | 442 | B |
| I-56 | 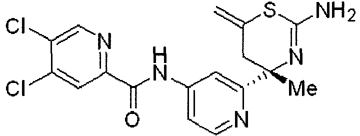 | 1H NMR (DMSO-d6) d: 1.41 (3H, s), 2.64 (2H, s), 5.02 (1H, s), 5.10 (1H, s), 7.74 (1H, dd, J = 2.1, 5.1 Hz), 8.07 (1H, d, J = 2.1 Hz), 8.32 (1H, s), 8.41 (1H, d, J = 5.1 Hz), 8.93 (1H, s), 11.0 (1H, s) | 1.3 | 408 | B |
| I-57 | 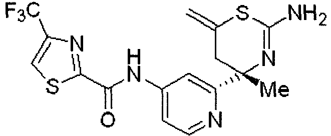 | 1H-NMR (DMSO-d6) δ: 1.36 (3H, s), 2.51-2.60 (2H, m), 4.98 (1H, s), 5.07 (1H, s), 6.13 (2H, brs), 7.69 (1H, d, J = 6.0 Hz), 8.09 (1H, s), 8.46 (1H, d J = 6.0 Hz), 8.87 (1H, s), 11.21 (1H, brs). | 414 | 1.26 | B |
| I-58 | 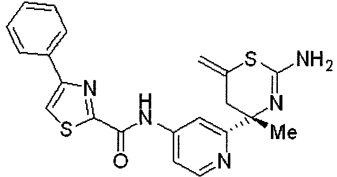 | 1H-NMR (DMSO-d6) δ: 1.36 (3H, s), 2.52 (1H, d, J = 13.5 Hz), 2.63 (1H, d, J = 13.5Hz), 5.00 (1H, s), 5.10 (1H, s), 6.13 (2H, brs), 7.42 (1H, t, J = 7.5 Hz), 7.51 (1H, dd, J = 7.5Hz), 7.75 (1H, dd, J = 5.4, 1.2 Hz), 8.08 (1H, d, J = 1.2 Hz), 8.17 (1H, d, J =7.5 Hz), 8.46 (1H, d J = 5.4 Hz), 8.54 (1H, s), 10.94 (1H, brs). | 422 | 1.5 | B |

**[Table 1-10]**

| No. | Structure | NMR(solvent:shift value;ascending order) | MS [M+1] | LC/MS RT | LC/MS Method |
|---|---|---|---|---|---|
| I-59 | 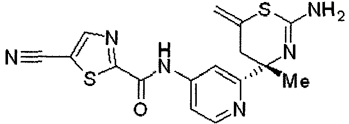 | 1H-NMR (DMSO-d6) δ: 1.36 (3H, s), 2.51-2.60 (2H, m),4.97 (1H, s), 5.06 (1H, s), 6.17 (2H, br s), 7.65 (1H, d J = 4.0 Hz), 8.10 (1H, s), 8.46 (1H, d J = 4.0 Hz), 8.93 (1H, s), 11.40 (1H, brs). | 371 | 1.05 | B |
| I-60 | 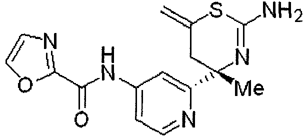 | 1H-NMR (DMSO-d6) δ: 1.38 (3H, s), 2.51-2.60 (2H, m), 4.97 (1H, s), 5.07 (1H, s), 6.16 (2H, brs). 7.57 (1H, s), 7.65 (1H, d J = 6.4 Hz), 8.02 (1H, s), 8.44 (1H, d J = 6.4 Hz), 11.18 (1H, brs). | 320 | 0.8 | B |
| I-61 | 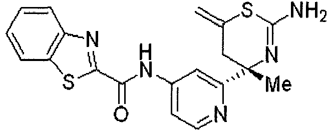 | 1H-NM R (DMSO-d6) δ: 1.38 (3H, s). 2.51-2.62 (2H, m), 4.98 (1H, s), 5.06 (1H, s), 6.16 (2H, brs). 7.62-7.72 (3H, m). 8.18 (1 H, s), 8.26 (2H, dd, J = 16.4. 8.0 Hz) 8.47 (1H, d, J = 5.2 Hz), 11.43 (1H, brs). | | | |
| I-62 | 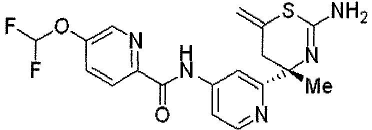 | 1H-NMR (DMSO-d6) δ: 1.36 (3H, s), 2.50-2.61 (2H, m), 4.98 (1H, s), 5.08 (1H, s), 6.17 (2H, brs). 7.49 (1H, t, J = 73.6 Hz), 7.72 (1H, d J = 4.0 Hz), 7.91 (1H, d J = 8.4 Hz), 8.09 (1H, s). 8.23 (1H, d J = 8.4 Hz), 8.43 (1H, d J = 4.0 Hz), 8.63 (1H, s), 10.84 (1H, brs). | 406 | 1.22 | B |
| I-63 | 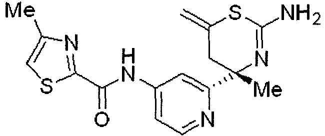 | 1H-NMR (DMSO-d6) δ: 1.36 (3H, s). 2.51 (3H, s), 2.51-2.60 (2H, m), 4.97 (1H, s), 5.06 (1H, s), 6.12 (2H. brs). 7.65 (1H, d, J = 5.2 Hz), 7.74 (1H, s), 8.11 (1H, s), 8.42 (1H, d J = 5.2 Hz), 11.00 (1H, s). | 360 | 1.12 | B |
| I-64 | 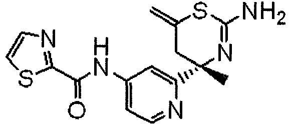 | 1H-NMR (DMSO-d6) δ: 1.37 (3H, s). 2.56 (5H, brs), 4.97 (1H, s), 5.06 (1H, s), 6.12 (2H, brs), 7.65 (1H, d, J = 6.2 Hz), 7.74 (1H, s). 8.11 (1H, s), 8.42 (1H, d, J = 5.2 Hz), 11.00 (1H, brs). | 346 | 0.94 | B |
| I-65 | 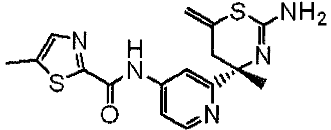 | 1H-NMR (DMSO-d6) δ: 1.35 (3H, s), 2.51 (3H, s), 2.51-2.60 (2H, m), 4.97 (1H, s), 5.06 (1H, s), 6.12 (2H, brs). 7.63 (1H, d, J = 5.2 Hz), 7.82 (1H, s), 8.11 (1H, s), 8.41 (1H, d, J = 5.2 Hz). 10.95 (1H, brs), | 360 | 1.08 | B |

**[Table 1-11]**

| No. | Structure | NMR(solvent:shift value;ascending order) | MS [M+1] | LC/MS RT | LC/MS Method |
|---|---|---|---|---|---|
| I-66 | 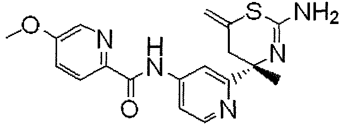 | 1H-NMR (DMSO-d6) δ: 1.35 (3H, s), 2.53-2.61 (2H, m). 3.94 (3H, s), 4.98 (1H, s), 5.08 (1H, s), 6.13 (2H, brs), 7.63 (1H, dd, J = 8.0, 2.4 Hz), 7.71 (1H, d J = 3.6 Hz), 8.07 (1H, s), 8.14 (1H, d, J = 8.0 Hz), 8.40 (1H, d J = 24 Hz), 8.41 (1H, d J = 3.6 Hz), 10.66 (1H, brs). | 370 | 1.1 | B |
| I-67 | 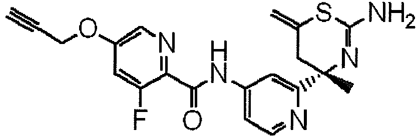 | 1H-NMR (DMSO-d6) δ: 1.36 (3H, s), 251-2.61 (2H, m), 3.74 (1H, s), 4.98 (1H, s), 5.06 (3H, s), 6.14 (2H, brs), 7.66 (1H, d, J =4.4 Hz), 7.67 (1H, d, J = 13.2 Hz), 7.97 (1H, s), 8.34 (1H, s), 8.42 (1H, d J =4.4 Hz), 10.77 (1H, brs). | 412 | 1.15 | B |
| I-68 | 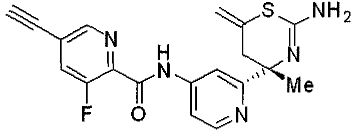 | 1H-NMR (DMSO-d6) δ: 1.36 (3H, s), 257 (2H, s), 4.81 (1H, s). 4.98 (1H, s), 5.07 (1H, s), 6.18 (1H, s), 7.66 (1H, dd. J = 5.5,2.0 Hz). 7.93 (1H, d. J = 1.5 Hz), 8.17 (1H, dd, J = 11.1, 1.5 Hz), 8.43 (1H, d, J = 5.5 Hz), 8.65-8.66 (1H. m), 10.94 (1H, s). | 382 | 1.2 | A |
| I-69 | 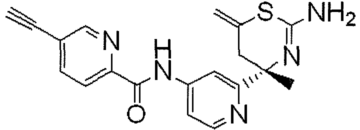 | 1H-NMR (DMSO-d6) δ: 1.36 (3H, s), 4.73 (1H, s), 4.98 (1H, s), 5.07 (1H, s), 6.15 (1H, s), 7.73 (1H, dd, J = 5.5. 2.0 Hz), 8.09-8.20 (3H, m), 8.43 (1H, d, J = 5.5 Hz), 8.83 (1H, dd, J = 1.8, 0.8 Hz), 10.90 (1H, s). | 364 | 1.19 | A |
| I-70 | 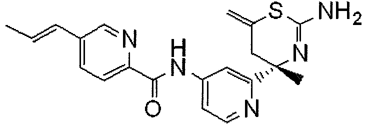 | 1H-NMR (CDCl3) δ: 1.58 (3H, s), 1.97 (3H, d J = 4.5 Hz), 2.75 (1H, d J = 13.9 Hz), 2.86 (1H, d, J = 14.1 Hz), 5.09 (1H, s), 5.20 (1H, s), 648-6.50 (2H, m), 7.64 (1H, d, J =2.2 Hz), 7.84 (1H, dd, J = 8.1, 2.2 Hz), 7.91 (1H, dd, J = 5.5, 1.9 Hz), 8.19 (1H, d, J = 8.1 Hz), 8.53 (2H, d, J = 5.5Hz), 10.12 (1H, s). | 380 | 1.55 | A |
| I- 71 | 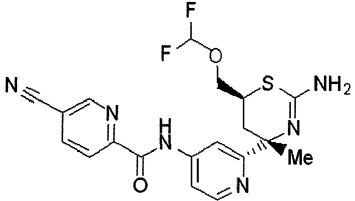 | 1H-NMR (CDCl3) δ: 147 (1H, t. J = 13.3 Hz), 1.65 (3H, s), 3.00 (1H, dd, J = 13.3, 3.5 Hz), 3.20-3.29 (1H, m), 3.85 (1H, dd, J = 9.9, 7.8 Hz), 4.00 (1H, dd, J = 10.2, 4.7 Hz), 6.18 (1H, t, J = 73.4 Hz), 7.38 (1H, d, J = 1.7 Hz), 7.95 (1H, dq. J = 5.4, 1.0 Hz), 8.22 (1H. dq. J = 8.2, 0.9 Hz), 8.42 (1H, d, J = 8.1 Hz), 8.56 (1H, d, J = 5.5 Hz), 8.92 (1H, t, J = 1.0 Hz), 9.97 (1H, br s), | 433 | 1.24 | A |
| I-72 | 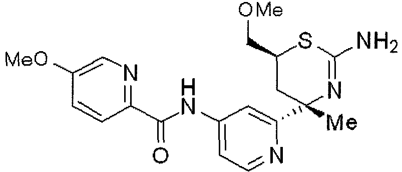 | 1H-NMR (CDCl3) δ: 146 (1H, t, J = 12.9 Hz), 1.65 (3H, s), 2.91 (1H, dd, J = 13.3, 3.4 Hz), 3.15-3.25 (1H, m), 3.31 (3H, s), 3.38 (1H, t. J = 9.0 Hz). 3.59 (1H, dd, J = 9.5, 4.3 Hz), 4.07 (3H, s). 7.28 (1H, d J = 2.0 Hz), 7.99 (1H, dd, J = 5.5, 2.1 Hz), 8.17 (1H, d, J =1.1 Hz), 8.52 (1H, d J = 5.6 Hz), 9.01 (1H, d J = 1.1 Hz), 9.64 (1H, br s). | 403 | 1.15 | A |

**[Table 1-12]**

| No. | Structure | NMR(sotvent:shift value;ascending order) | MS [M+1] | LC/MS RT | LC/MS Method |
|---|---|---|---|---|---|
| I-73 | 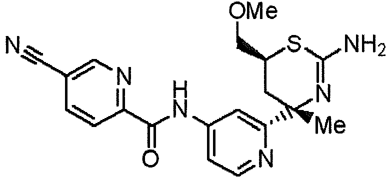 | 1H-NMR (CDCl₃) δ: 1.52 (1H, t, J = 13.0 Hz), 1.66 (3H, s), 2.95 (1H, dd, J = 13.0, 3.4 Hz), 3.16-3.25 (1H, m), 3.31 (3H, s), 3.38 (1H, t, J = 9.0 Hz), 3.59 (1H, dd, J = 9.5, 4.3 Hz), 5.13 (2H, br s). 7.40 (1H, d, J = 1.8 Hz), 7.97 (1H, dd, J = 5.5, 1.8Hz), 8.22 (1H, dd, J = 8.1, 1.8 Hz), 8.41 (1H, d, J = 8.1 Hz), 8.55 (1H, d, J = 5.4 Hz), 8.92 (1H, t, J = 0.9 Hz). | 397 | 1.07 | A |
| I-74 | 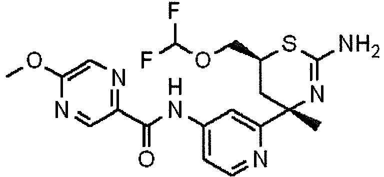 | 1H-NMR (CDCl3) δ: 1.38 (1H, t, J = 12.7 Hz), 1.61 (3H, s), 2.93 (1H, dd, J = 13.3, 3.4 Hz), 3.14-3.23 (1H, m), 3.83 (1H, dd, J = 9.8, 7.8 Hz), 3.97 (1H, dd, J = 10.0, 5.1 Hz), 4.07 (3H, d J = 0.9 Hz), 6.17 (3H, t J = 75 Hz), 7.30 (1H, s). 7.91 (1H, dd, J =5.4, 1.3 Hz). 8.16 (1H, s), 8.52 (1H, d, J = 5.5 Hz), 9.01 (1H, s), 9.60 (1H, br s). | 439 | 1.26 | A |
| I-75 | 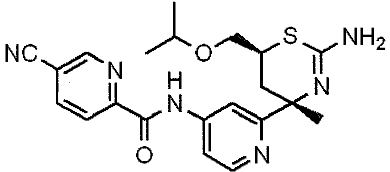 | 1H-NMR (CDCl3) δ: 1.11 (6H, t, J = 5.5 Hz), 1.40 (1H, t, J = 12.8 Hz), 1.63 (3H, s), 285 (1H, dd J = 13.3, 3.4 Hz), 3.06-3.16 (1H, m), 3.35 (1H, t, J = 9.2 Hz), 3.46-3.58 (1H, m), 3.68 (1H, dd J = 9.5, 4.3 Hz), 7.31 (1H, d J = 2.0 Hz), 7.95 (1H, dd, J = 5.5, 1.9 Hz), 8.22 (1H, dd, J = 8.2, 2.0 Hz), 8.43 (1H, d J = 8.2 Hz), 8.56 (1H, d J = 5.4 Hz), 8.92-8.93 (1H, m), 9.93 (1H, br s). | 425 | 1.33 | A |
| I-76 | 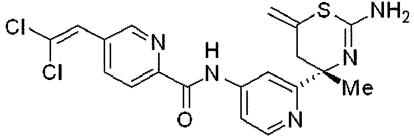 | 1H-NMR (CDCl3) δ: 1.54 (3H, s), 274 (2H, s). 5.07 (1H, s), 5.18 (1H, s), 6.93 (1H, s). 7.67 (1H, d, J = 2.0 Hz), 7.88 (1H, dd, J = 5.5, 2.1 Hz), 8.13-8.19 (1H. m), 8.28 (1H, d J = 8.2 Hz), 8.54 (1H, d, J = 5.5 Hz), 8.69 (1H, d, J = 2.0 Hz), 10.08 (1H, s). | 434 | 1.53 | A |
| I-77 | 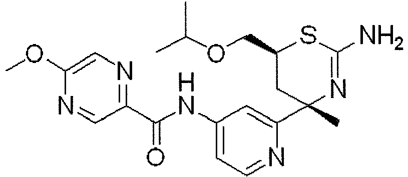 | 1H-NMR (CDCl3) δ: 1.11 (6H, dd. J = 5.6, 4.9 Hz), 1.47 (1H, t, J =12.8 Hz), 1.67 (3H, s), 2.92 (1H, dd, J = 13.5, 3.3 Hz), 3.13-3.23 (1H, m), 3.36 (1H, t. J = 9.3 Hz), 3.48-3.56 (1H, m), 3.69 (1H, dd, J = 9.3, 4.1 Hz), 4.07 (3H, s), 7.26-7.27 (1H, m), 8.01 (1H, dd J = 5.3, 1.8 Hz), 8.17-8.18 (1H, m), 8.52 (1H, d J = 5.5 Hz), 9.00-9.01 (1H, m), 9.65 (1H, br s). | 431 | 1.35 | A |

**[Table 1-13]**

| No. | Structure | NMR(solvent:shift value;ascending order) | MS [M+1] | LC/MS RT | LC/MS Method |
|---|---|---|---|---|---|
| II-1 | 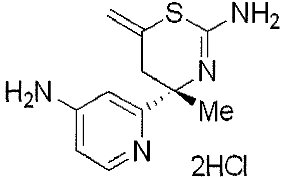 | 1H-NMR (DMSO-d6) δ: 1.78 (3H. s), 2.97 (1H, d, J = 14.6Hz), 3.74 (1H, brd, J = 14.6 Hz), 5.30 (1H, s), 5.40 (1H, s), 6.67 (1H, d, J = 2.4 Hz). 6.75 (1H, dd, J = 6.9, 24 Hz), 8.07 (1H, d, J = 6.9 Hz), 8.28 (2H, brs), 9.01 (1H, brs), 10.04 (1H, brs), 11.53 (1H, brs), 13.96 (1H, brs). | 235 | 0.24 | A |
| II-2 | 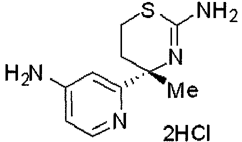 | | 223 | 0.34 | B |
| II-3 | 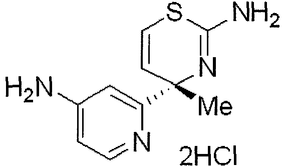 | 1H NMR (DMSO-d6) + 1.87 (3H, s). 6.50 (1H, d, J = 9.6 Hz), 6.75-6.80 (3H, m), 8.08 (1H, d, J = 7.5 Hz) | 221 | 0.35 | B |
| II-4 | 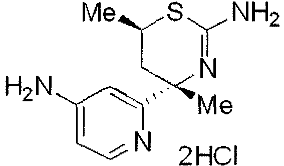 | 1H-NMR (DMSD-d6) δ: 1.26 (3H. d, J = 6.4 Hz), 1.80 (3H, s), 2.00 (1H, dd, J = 14.5, 12.8 Hz), 2.95-3.07 (1H, m), 3.17 (1H, dd, J = 14.6. 2.4 Hz), 6.65 (1H, d, J = 2.1 Hz), 6.79 (1H, dd, J = 6.9, 23 Hz), 8.04 (1H, d, J = 6.9 Hz), 8.36 (2H, s), 8.95 (1H, br s), 9.84 (1H, br s), 11.41 (1H, s), 14.16 (1H, br s). | 237 | 0.26 | A |

**[Table 1-14]**

| | | | |
|---|---|---|---|
| I-78 | 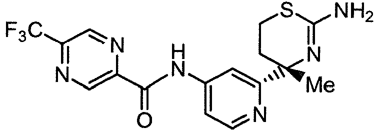 | I-85 | 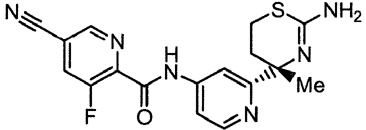 |
| I-79 | 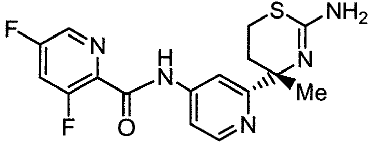 | I-86 | 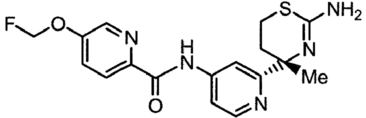 |
| I-80 | 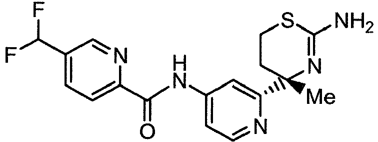 | I-87 | 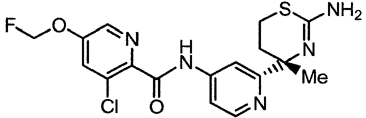 |
| I-81 | 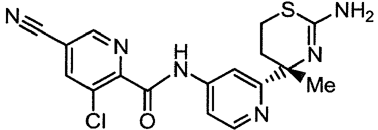 | I-88 | 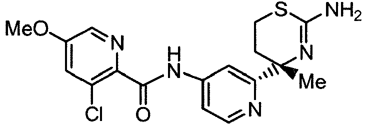 |
| I-82 | 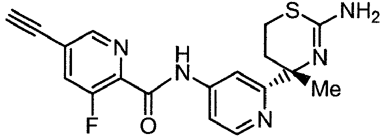 | I-89 | 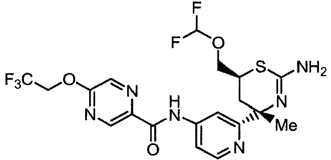 |
| I-83 | 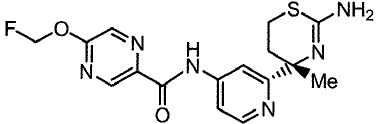 | I-90 | 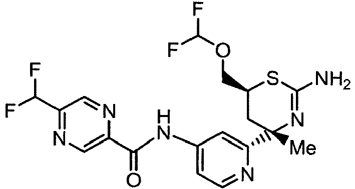 |
| I-84 | 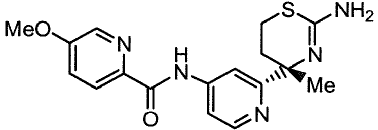 | I-91 | 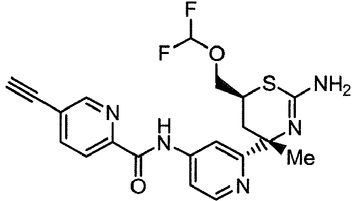 |

**[Table 1-15]**

| | | | |
|---|---|---|---|
| I-92 | 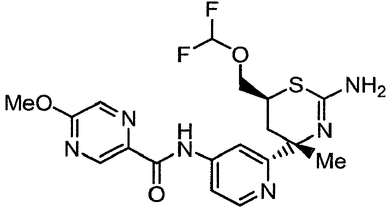 | I-98 | 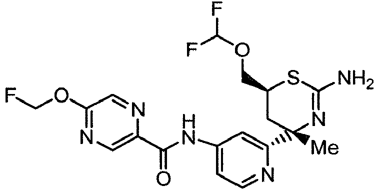 |
| I-93 | 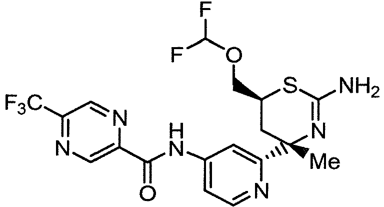 | I-99 | 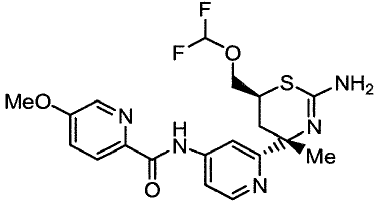 |
| I-94 | 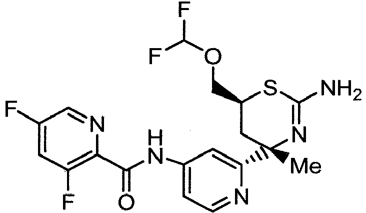 | I-100 | 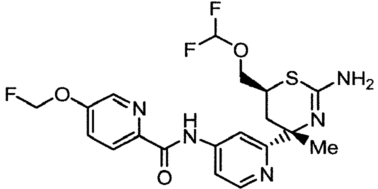 |
| I-95 | 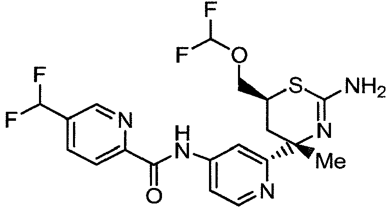 | I-101 | 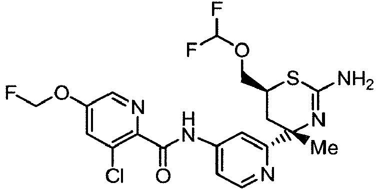 |
| I-96 | 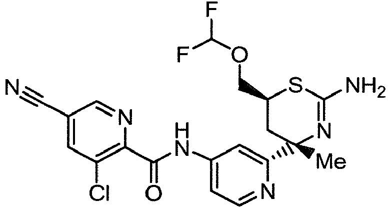 | I-102 | 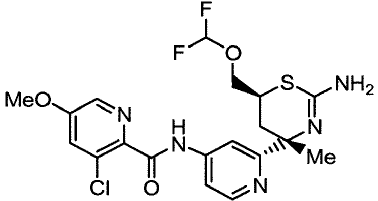 |
| I-97 | 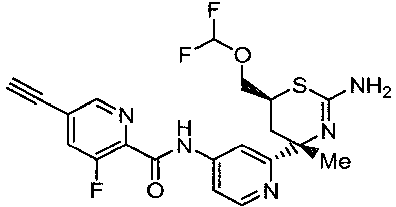 | I-103 | 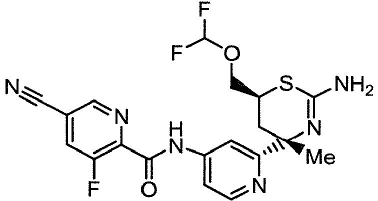 |

**[Table 1-16]**

| | | | |
|---|---|---|---|
| I-104 | 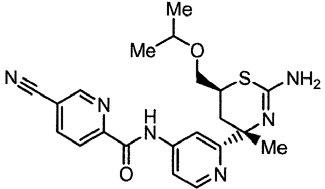 | I-111 | 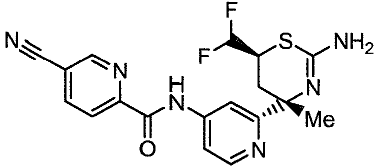 |
| I-105 | 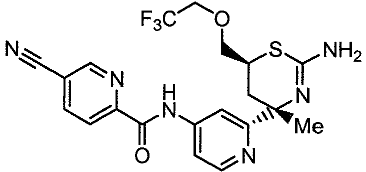 | I-112 | 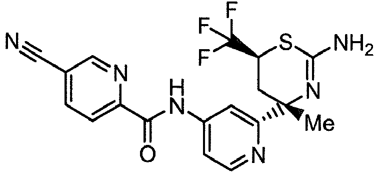 |
| I-106 | 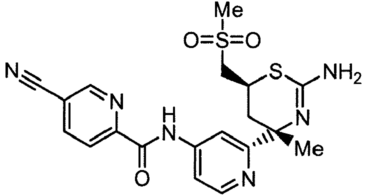 | I-113 | 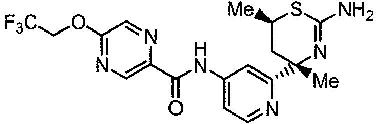 |
| I-107 | 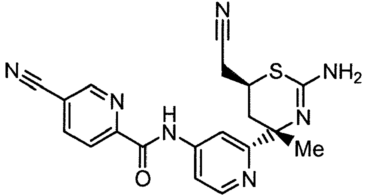 | I-114 | 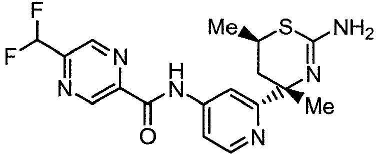 |
| I-108 | 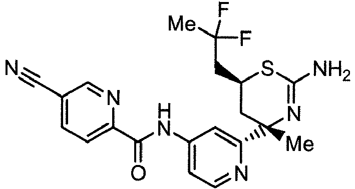 | I-115 | 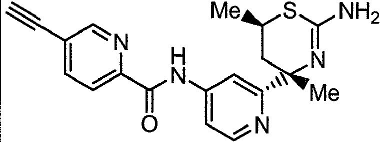 |
| I-109 | 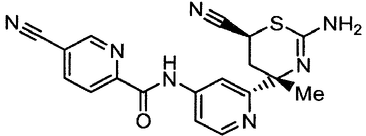 | I-116 | 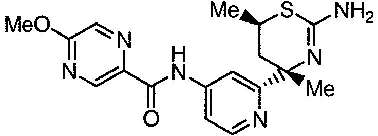 |
| I-110 | 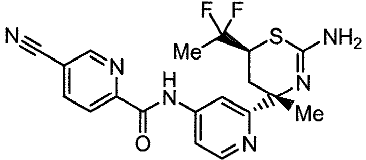 | I-117 | 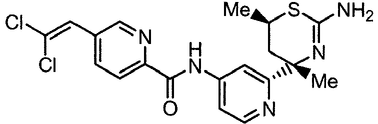 |

**[Table 1-17]**

| | | | |
|---|---|---|---|
| I-118 | 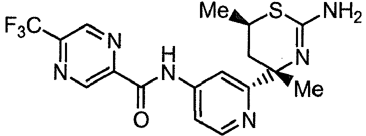 | I-125 | 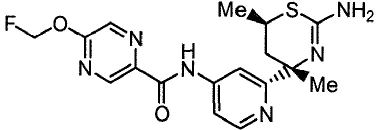 |
| I-119 | 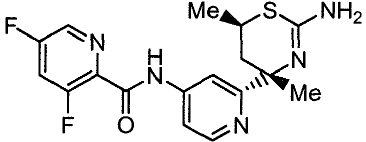 | I-126 | 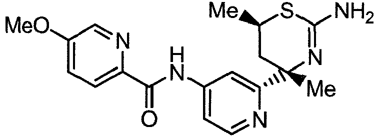 |
| I-120 | 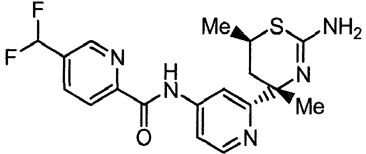 | I-127 | 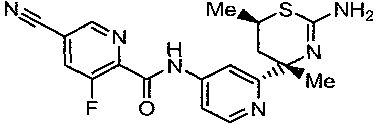 |
| I-121 | 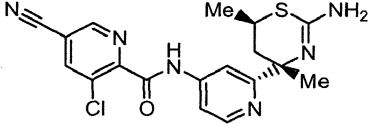 | I-128 | 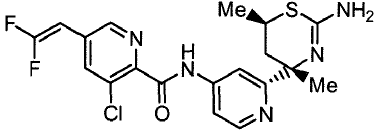 |
| I-122 | 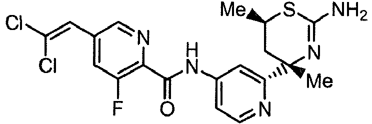 | I-129 | 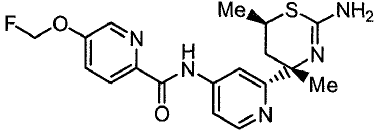 |
| I-123 | 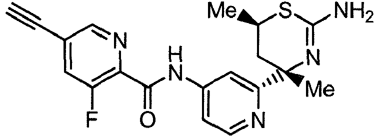 | I-130 | 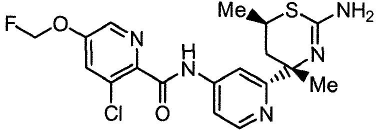 |
| I-124 | 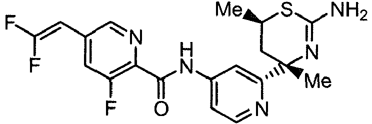 | I-131 | 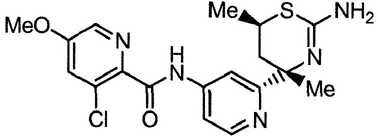 |

**[Table 1-18]**

| | | | |
|---|---|---|---|
| I-132 | 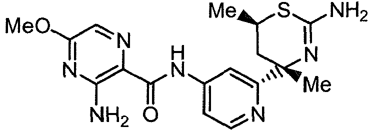 | I-139 | 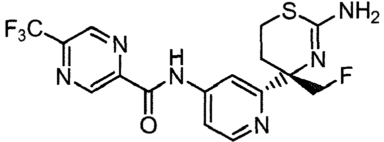 |
| I-133 | 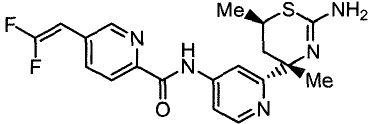 | I-140 | 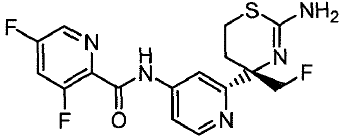 |
| I-134 | 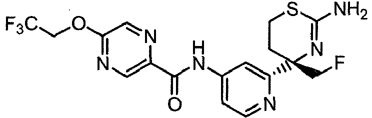 | I-141 | 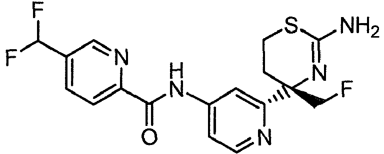 |
| I-135 | 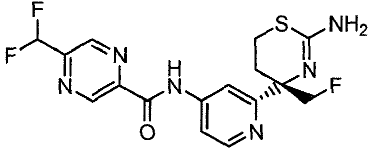 | I-142 | 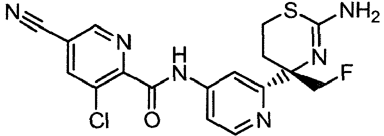 |
| I-136 | 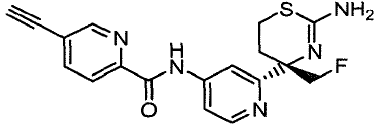 | I-143 | 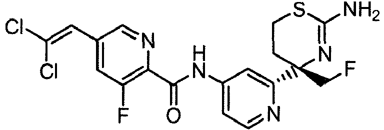 |
| I-137 | 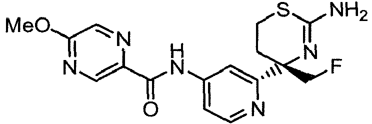 | I-144 | 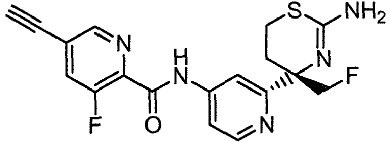 |
| I-138 | 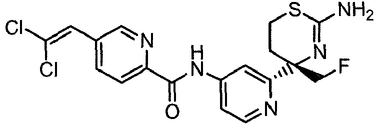 | I-145 | 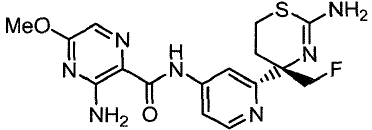 |

**[Table 1-19]**

| | | | |
|---|---|---|---|
| I-146 | 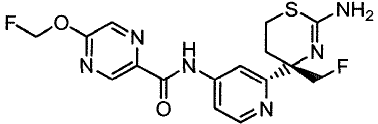 | I-153 | 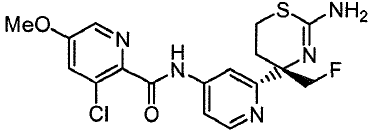 |
| I-147 | 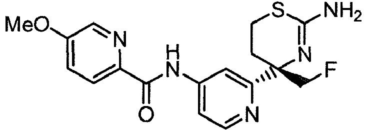 | I-154 | 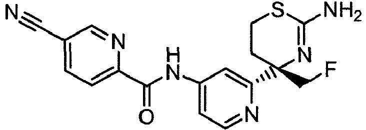 |
| I-148 | 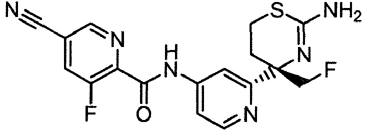 | I-155 | 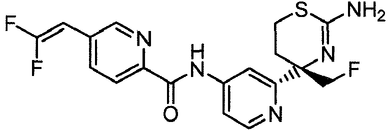 |
| I-149 | 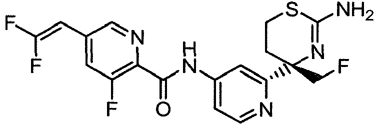 | I-156 | 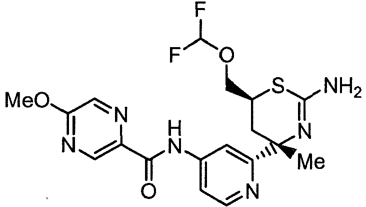 |
| I-150 | 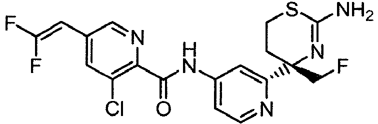 | I-157 | 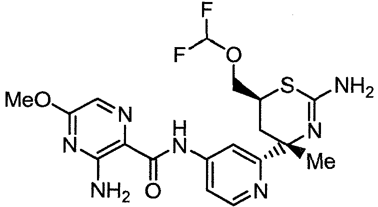 |
| I-151 | 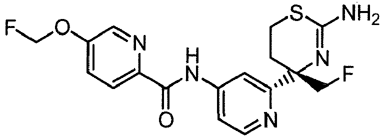 | I-158 | 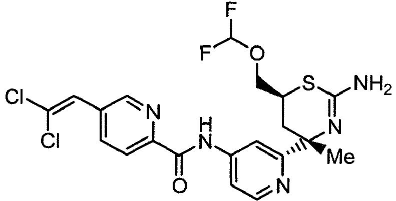 |
| I-152 | 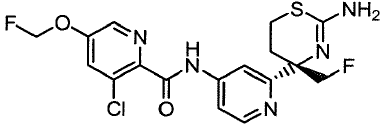 | I-159 | 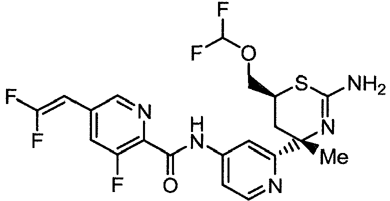 |

**[Table 1-20]**

| | | | |
|---|---|---|---|
| I-160 | 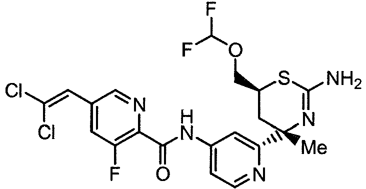 | I-166 | 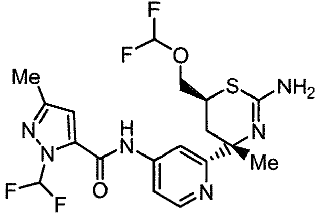 |
| I-161 | 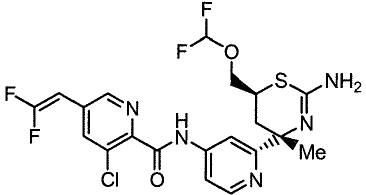 | I-167 | 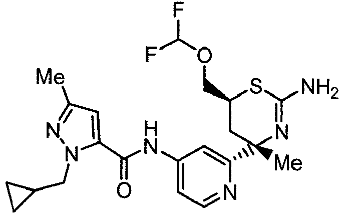 |
| I-162 | 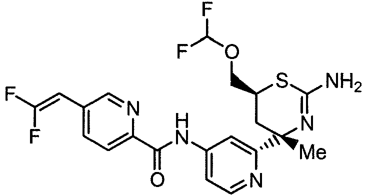 | I-168 | 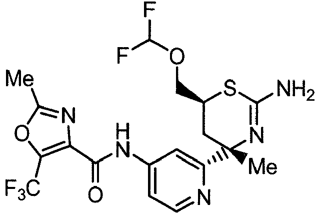 |
| I-163 | 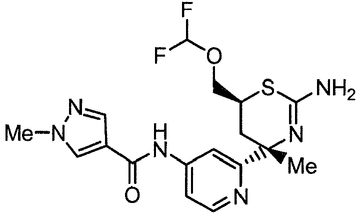 | I-169 | 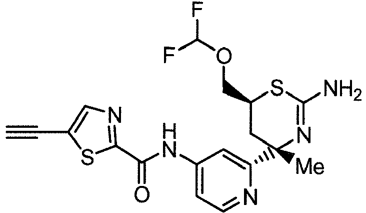 |
| I-164 | 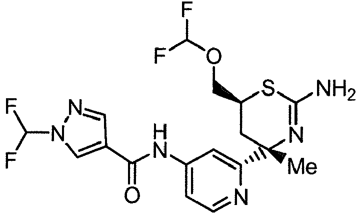 | I-170 | 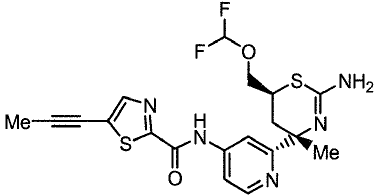 |
| I-165 | 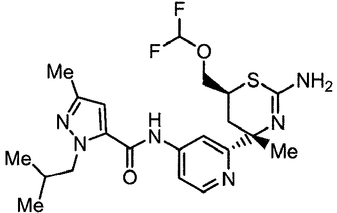 | I-171 | 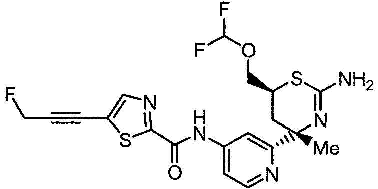 |

**[Table 1-21]**

| | | | |
|---|---|---|---|
| I-172 | 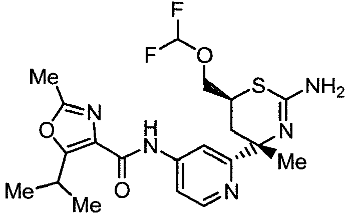 | I-178 | 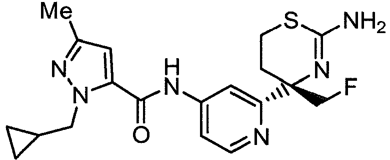 |
| I-173 | 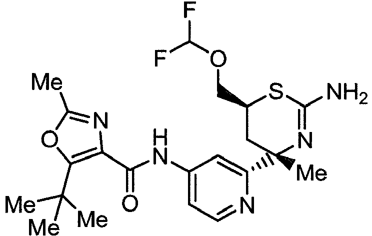 | I-179 | 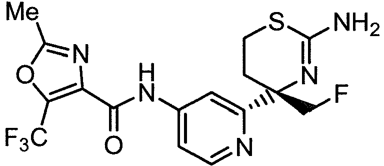 |
| I-174 | 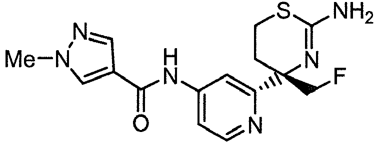 | I-180 | 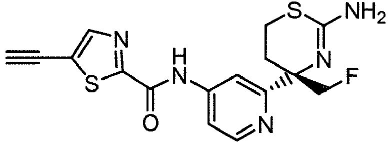 |
| I-175 | 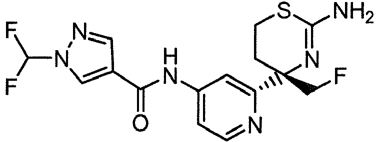 | I-181 | 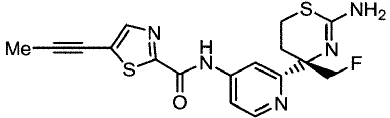 |
| I-176 | 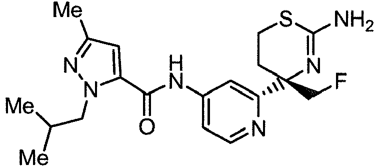 | I-182 | 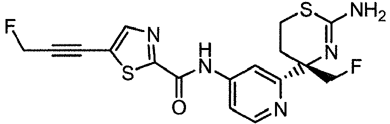 |
| I-177 | 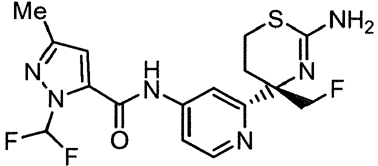 | I-183 | 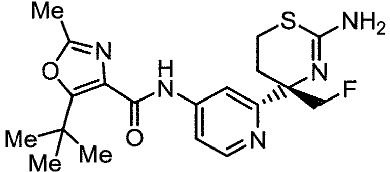 |

**[Table 1-22]**

| | | | |
|---|---|---|---|
| I-184 | 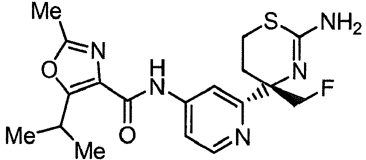 | I-190 | 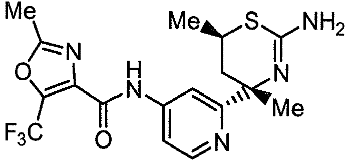 |
| I-185 | 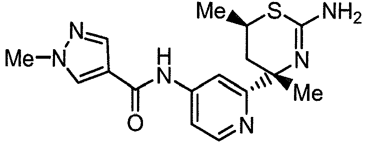 | I-191 | 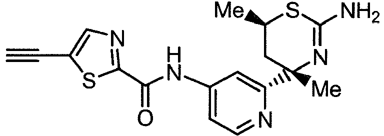 |
| I-186 | 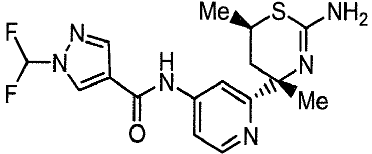 | I-192 | 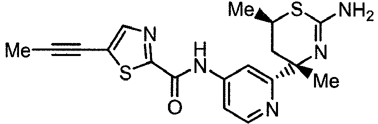 |
| I-187 | 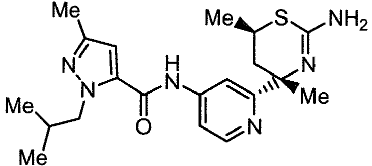 | I-193 | 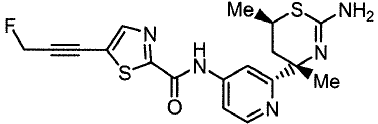 |
| I-188 | 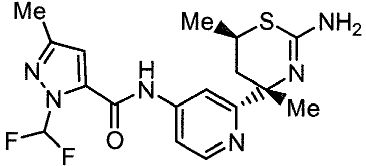 | I-194 | 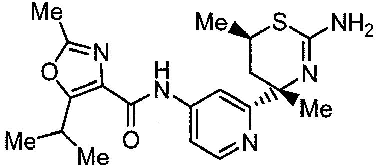 |
| I-189 | 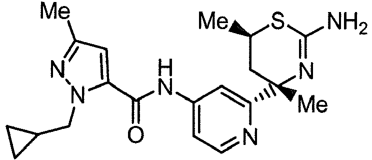 | I-195 | 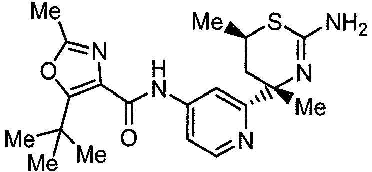 |

**[Table 1-23]**

| No. | Structure | NMR(soLvent:shift value;ascending order) | MS [M+1] | LC/MS RT | LC/MS Method |
|---|---|---|---|---|---|
| I-196 | 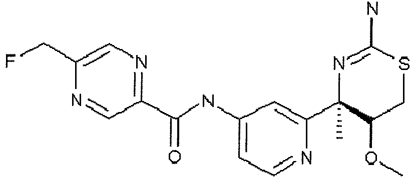 | 1H-NMR (CDCl3) δ: 1.66 (3H, s), 2.77 (1H, dd, J = 12.9, 2.4 Hz), 3.08 (1H, dd, J =12.9, 4.5 Hz), 3.51 (3H, s), 4.23 (1H, dd, J = 4.5, 2.4 Hz), 5.67 (1H, d, J = 46.2 Hz), 7.37 (1H, d, J = 2.1 Hz), 7.96 (1H, dd, J = 5.4. 2.1 Hz), 8.55 (1H. d, J = 5.4 Hz), 8.77 (1H, s), 9.44 (1H, s), 9.76 (1H, br). | 391 | 0.84 | B |
| I-197 | 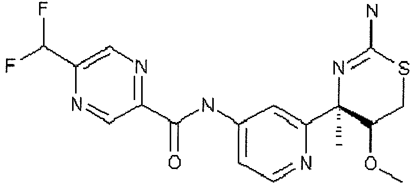 | 1H-NMR (CDCl3) δ: 1.66 (3H, s), 2.76 (1H. dd. J =12.9 . 2.4 Hz), 3.08 (1H, dd, J = 12.9, 4.5 Hz), 3.51 (3H. s), 4.23 (1H, dd, J = 4.5, 24 Hz), 6.80 (1H, t, J = 54.0 Hz), 7.38 (1H, d, J = 2.1 Hz), 7.96 (1H, dd, J = 5.4, 2.1 Hz), 8.56 (1H, d, J = 5.4 Hz), 8.94 (1H, s). 9.52 (1H, s). | 409 | 0.92 | B |
| I-198 | 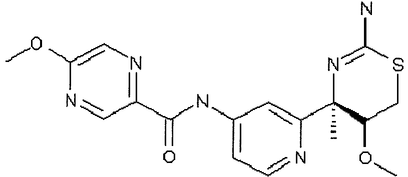 | 1H-NMR (CDCl3) δ: 1.66 (3H, s), 2.76 (1H, dd, J = 12.9, 2.4 Hz), 3.07 (1H, dd, J = 12.9, 4.5 Hz), 3.51 (3H, s). 4.08 (3H, s). 4.22 (1H, dd, J = 4.2, 2.4 Hz), 7.33 (1H, d, J = 2.1 Hz), 7.96 (1H, dd, J =5.4, 2.1 Hz), 8.16 (1H, d, J = 1.2 Hz), 8.52 (1H, d, J = 5.4 Hz), 9.01 (1H, d, J = 1.2 Hz), 9.63 (1H, br). | 389 | 0.92 | B |
| I-199 | 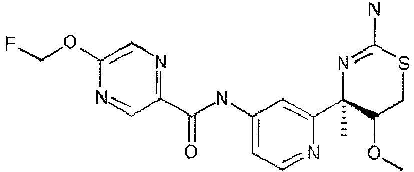 | 1H-NMR (CDCl3) δ: 1.66 (3H, s), 2.76 (1H, dd, J = 12.9, 2.1 Hz), 3.07 (1H, dd, J =12.9, 4.2 Hz), 3.51 (3H, s), 4.22 (1H, dd, J = 4.2, 2.1 Hz), 6.15 (2H, ddd, J = 51.0, 3.3, 2.1 Hz), 7.34 (1H, d, J = 2.1 Hz), 7.95 (1H, dd, J =5.7, 2.1 Hz), 8.30 (1H, d, J = 1.2 Hz), 8.53 (1H, d. J = 5.7 Hz), 9.08 (1H, d, J = 1.2 Hz), 9.66 (1H, br). | 407 | 0.92 | B |
| I-200 | 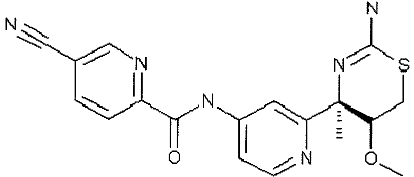 | 1H-NMR (CDCl3) δ: 1.66 (3H, s), 2.76 (1H, dd, J =12.9, 2.1 Hz), 3.07 (1H, dd, J =12.9, 4.2 Hz), 3.51 (3H, s). 4.22 (1H, dd, J = 4.2, 2.1 Hz), 7.37 (1H, d, J = 2.1 Hz), 7.95 (1H, dd, J = 5.7, 2.1 Hz), 8.22 (1H, dd, J = 8.4, 2.1 Hz), 8.43 (1H, dd, J = 8.4, 0.9 Hz), 8.55 (1H, d, J = 5.4 Hz), 8.92 (1H, dd, J = 2.1, 0.9 Hz), 9.83 (1H, br). | 383 | 0.87 | B |

**[Table 1-24]**

| No. | Structure | NMR(solvent:shift value;ascending order) | MS [M+1] | LC/M S RT | LC/MS Method |
|---|---|---|---|---|---|
| I-201 | 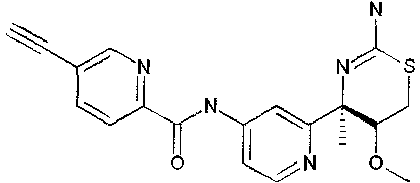 | 1H-NMR (CDCl3) δ: 1.66 (3H, s), 2.77 (1H, dd, J = 12.9, 2.4 Hz), 3.07 (1H, dd, J = 12.9, 4.2 Hz), 3.41 (1H, s), 3.51 (3H, s), 4.22 (1H, dd, J = 4.2, 2.4 Hz), 7.96-8.01 (2H, m), 8.25 (1H, dd. J=8.1, 0.9 Hz), 8.53 (1H, d, J = 5.7 Hz), 8.70 (1H, dd, J = 2.1, 0.9 Hz), 10.04 (1H, br). | 382 | 1.04 | B |
| I-202 | 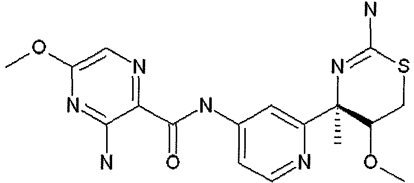 | 1H-NMR (CDCl3) δ: 1.65 (3H, s), 2.77 (1H, dd, J= 12.9, 2.4 Hz). 3.06 (1H, dd, J = 12.9. 4.2 Hz), 3.50 (3H, s), 3.94 (3H, s), 4.21 (1H, dd, J = 4.2, 2.4 Hz), 7.27 (1H, d, J = 2.1 Hz), 7.46 (1H, s), 7.88 (1H, dd, J = 5.4, 2.1 Hz). 8.47 (1H, d, J = 5.4 Hz), 9.66 (1H, br). | 404 | 1.01 | B |
| I-203 | 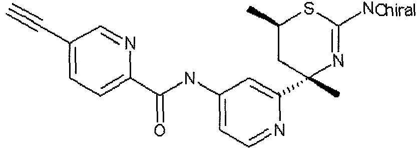 | 1H-NMR (DMSO-d6) δ: 1.20 (3H, d, J = 6.3 Hz), 1.60 (3H, s), 2.75 (2H, d, J = 13.0 Hz), 4.75 (1H, s), 7.89 (1H, d, J = 6.1 Hz), 7.95 (1H, s), 8.14-8.21 (2H, m), 8.50 (1H, d, J = 5.5 Hz), 8.84 (1H, s), 11.14 (1H, s). | 366 | 1.21 | A |
| 1-204 | 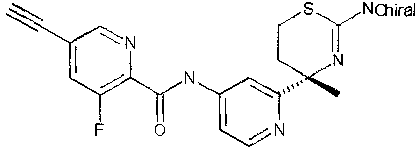 | 1H-NMR (DMSO-d6) δ: 1.45 (3H, s), 1.76 (1H, t, J = 10.2 Hz). 2.26-2.33 (1H, m), 2.56-2.62 (2H, m), 2.92-2.99 (1H, m), 4.83 (1H, s), 6.08 (1H, br s), 7.73 (1H, d, J = 5.4 Hz), 7.84 (1H, s), 8.20 (1H, d, J = 10.9 Hz), 8.48 (1H, d, J = 5.4 Hz), 8.69 (1H, s), 11.01 (1H, br s). | 370 | 1.05 | A |
| 1-205 | 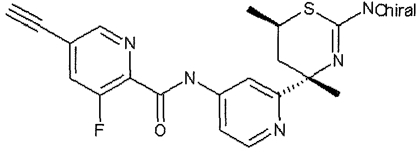 | 1H-NMR (DMSO-d6) δ: 1.14 (3H. d, J = 6.4 Hz), 1.21 (1H, t, J = 12.6 Hz), 1.49 (3H, s), 2.59-2.65 (3H, br m), 2.71 (1H, d, J = 12.0 Hz). 4.83 (1H, s), 7.70 (1H, s), 7.73 (1H, d, J = 5.4 Hz), 8.20 (1H, d. J = 10.9 Hz). 8.49 (1H, d, J = 5.4 Hz), 8.69 (1H, s), 11.03 (1H, s). | 384 | 1.16 | A |

**[Table 1-25]**

| No. | Structure | NMR(solvent:shift value;ascending order) | MS [M+1] | LC/M S RT | LC/MS Method |
|---|---|---|---|---|---|
| I-206 | 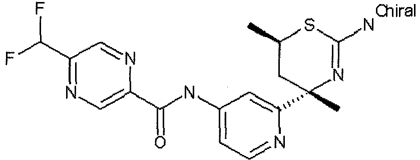 | 1H-NMR (DMSO-d6) δ: 1.10 (4H, d, J = 6.5 Hz), 1.17-1.21 (1H, m), 1.46 (4H, s), 2.55-2.62 (1H, m). 2.68 (2H, d, J = 12.0 Hz), 6.05 (2H, br s), 7.26 (1H, t, J=53.9Hz), 7.75(1H, d, J=5.4 Hz), 7.88 (1H, s), 8.48 (1H, d, J = 5.4 Hz), 9.10 (1H, s), 9.39 (1H, s), 11.18 (1H, br s), | 393 | 1.11 | A |
| I-207 | 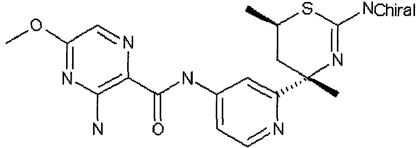 | 1H-NMR (DMSO-d6) δ: 1.10 (3H, d, J = 6.5 Hz), 1.16-1.23 (1H, m), 1.45 (3H, s), 2.58-2.71 (3H, m), 3.91 (3H, s), 7.55 (1H, d, J = 2.4 Hz), 7.66 (1H, dd, J = 5.5, 2.2 Hz), 7.74 (1H, d, J = 2.2 Hz), 8.39 (1H, d, J = 5.5 Hz), 10.40 (1H, s). | 388 | 1.1 | A |
| I-208 | 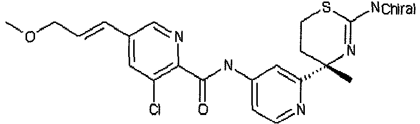 | 1H-NMR (DMSO-d6) δ: 1.43 (3H, s), 1.74 (1H, t, J = 10.1 Hz), 2.28-2.32 (1H, m), 2.57-2.60 (1H, m), 2.92-2.94 (1H, m), 3.33 (3H, s), 4.11 (2H, d, J = 4.2 Hz). 6.67-6.81 (2H, m), 7.70 (2H, d, J = 7.4 Hz), 8.26 (1H, s), 8.45 (1H, d, J = 5.4 Hz). 8.71 (1H, s), 11.00 (1H, s). | 432 | 1.22 | A |
| 1-209 | 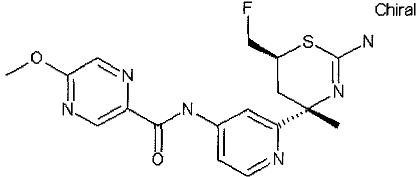 | 1H-NMR (CDCl3) δ: 1.65 (3H, s), 2.95 (1H, dd, J = 13.3, 3.7 Hz), 3.26-3.37 (1H, m), 4.08 (3H, s), 4.31 (1H, dd, J = 9.5, 7.0 Hz), 4.40-4.50 (1H, m), 4.58 (1H, dd, J = 9.5, 4.4 Hz), 7.35 (1H, d, J = 2.0 Hz), 7.92 (1H, dd, J = 5.5, 1.8 Hz), 8.16 (1H, d, J = 0.7 Hz), 8.52 (1H, d, J = 5.5 Hz), 9.01 (1H, d, J = 0.7 Hz), 9.64 (1H, s). | 391 | 1.09 | A |
| I-210 | 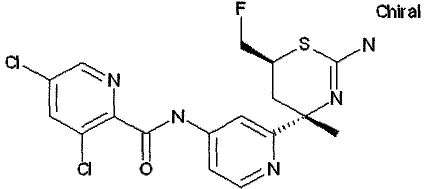 | 1H-NMR (CDCl3) δ: 1.63 (3H, s), 2.95 (1H, dd, J = 13.2, 3.8 Hz), 3.24-3.37 (1H, m), 4.31 (1H, dd, J = 9.5, 7.0 Hz), 4.40-4.49 (1H, m), 4.58 (1H, dd, J = 9.5, 4.5 Hz), 7.24 (1H, d, J = 2.2 Hz), 7.92 (1H, dd, J = 2.1, 0.6 Hz). 8.01 (1H, dd, J = 5.5, 2.1 Hz). 8.50 (2H, d, J = 2.1 Hz), 8.52 (2H, d, J = 5.5 Hz). | 428 | 1.21 | A |

**[Table 1-26]**

| No. | Structure | NMR(solvent:shift value;ascending order) | MS [M+1] | LC/M S RT | LC/MS Method |
|---|---|---|---|---|---|
| I-211 | 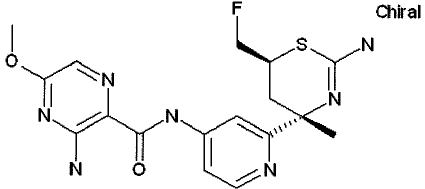 | 1H-NMR (CDCl3) δ: 1.63 (3H, s). 2.93 (1H, dd, J = 13.3, 3.7 Hz), 3.23-3.38 (1H, m), 3.94 (3H, s), 4.30 (1H, dd, J = 9.5, 7.0 Hz), 4.40-4.49 (1H, m), 4.58 (1H, dd, J = 9.5, 4.7 Hz). 7.30 (1H, d. J = 2.0 Hz), 7.47 (1H, s), 7.84 (1H, dd, J = 5.5, 2.0 Hz), 8.48 (1H, d. J = 5.5 Hz), 9.67 (1H, s). | 406 | 1.14 | A |
| I-212 | 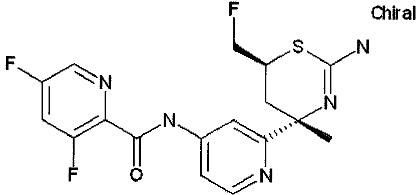 | 1H-NMR (CDCl3) δ: 1.62 (3H, s), 2.92 (1H, dd, J = 13.3, 3.7 Hz), 3.20-3.33 (1H, m), 4.30 (1H, dd, J = 9.3, 7.0 Hz), 4.39-4.48 (1H, m), 4.57 (1H, dd, J = 9.3. 4.6 Hz), 7.28 (1H, d, J = 2.0 Hz), 7.38-7.45 (1H, m), 7.95 (1H, dd. J = 5.5, 2.2 Hz), 8.38 (1H, d, J = 2.4 Hz). 8.53 (1H, d, J = 5.5 Hz), 9.74 (1H, s). | 396 | 1.09 | A |
| I-213 | 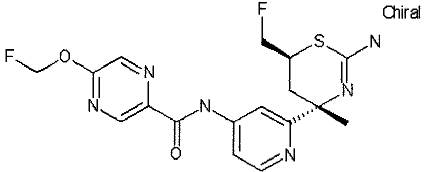 | 1H-NMR (CDCl3) δ: 1.62 (3H, s), 2.91 (1H, dd, J = 13.1, 3.7 Hz), 3.19-3.32 (1H, m), 4.30 (1H, dd, J = 9.3, 7.0 Hz), 4.39-4.48 (1H, m), 4.57 (1H, dd, J = 9.3, 4.7 Hz), 6.07 (1H, s), 6.24 (1H, s), 7.35 (1H, d, J = 2.1 Hz), 7.89 (1H, dd, J = 5.5, 2.1 Hz), 8.29 (1H, d, J = 1.3 Hz), 8.54 (1H, d, J = 5.5 Hz), 9.08 (1H, d, J = 1.2 Hz). 9.60 (1H, s). | 409 | 1.15 | A |
| I-214 | 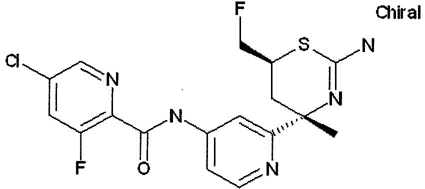 | 1H-NMR (CDCl3) δ: 1.61 (3H, s), 2.92 (1H, dd, J = 13.2, 3.8 Hz), 3.20-3.35 (1H, m), 4.30 (1H, dd, J = 9.4, 6.9 Hz), 4.39-4.48 (1H, m), 4.57 (1H, dd, J = 9.4, 4.7 Hz), 7.29 (1H, d, J = 1.9 Hz), 7.68 (1H, dd, J = 10.0, 1.9 Hz), 7.94 (1H, dd, J = 5.5, 2.0 Hz), 8.43 (1H, t, J = 0.9 Hz), 8.53 (1H, d, J = 5.5 Hz). 9.77 (1H, s). | 412 | 1.18 | A |
| I-215 | 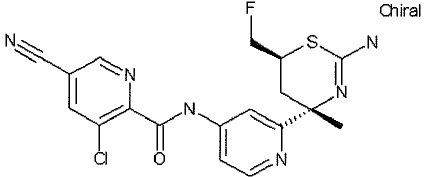 | 1H-NMR (CDCl3) δ: 1.61 (3H, s), 2.92 (1H, dd, J = 13.2, 3.8 Hz), 3.20-3.35 (1H, m), 4.30 (1H, dd, J = 9.4. 6.9 Hz), 4.39-4.48 (1H, m), 4.57 (1H, dd, J = 9.4, 4.7Hz), 7.29 (1H, d, J=1.9Hz), 7.68 (1H, dd, J = 10.0, 1.9 Hz), 7.94 (1H, dd, J = 5.5, 2.0 Hz). 8.43 (1H, t, J = 0.9 Hz), 8.53 (1H, d, J = 5.5 Hz), 9.77 (1H, s). | 419 | 1.15 | A |

**[Table 1-27]**

| No. | Structure | NMR(solvent:shift value;ascending order) | MS [M+1] | LC/M S RT | LC/MS Method |
|---|---|---|---|---|---|
| I-216 | 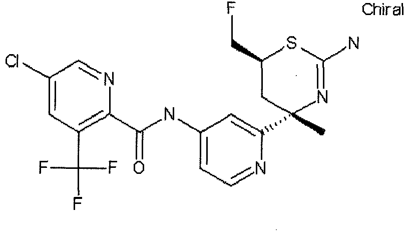 | 1H-NMR (CDCl3) δ: 1.60 (3H. s), 2.92 (1H, dd, J= 13.2, 3.6 Hz), 3.20-3.33 (1H, m), 4.30 (1H, dd, J = 9.4, 6.9 Hz), 4.39-4.48 (1H, m), 4.57 (1H, dd, J = 9.4, 4.7 Hz), 7.18 (1H, d. J = 2.2 Hz), 8.06 (1H, dd. J = 5.5, 2.0 Hz), 8.24 (1H, d, J = 2.2 Hz), 8.53 (1H, d, J = 5.5 Hz), 8.76 (1H, d, J=2.2Hz). | 462 | 1.38 | A |
| I-217 | 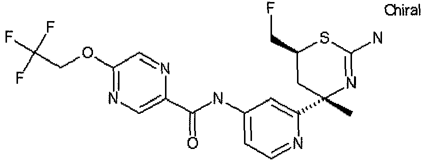 | 1H-NMR (CDCl3) δ: 1.62 (3H, s), 2.92 (1H, dd, J =13.2, 3.8 Hz), 3.20-3.34 (1H, m), 4.30 (1H, dd. J = 9.5, 7.0 Hz), 4.39-4.48 (1H, m), 4.57 (1H, dd, J= 9.4, 4.7 Hz), 4.88 (2H, q, J = 9.0 Hz), 7.35 (1H, d, J = 2.1 Hz), 7.91 (1H, dd, J = 5.5, 2.1 Hz), 8.33 (1H, d, J = 1.3 Hz), 8.54 (1H, d, J = 5.5 Hz), 9.03 (1H, t, J = 0.7 Hz), 9.60 (1H, s). | 459 | 1.41 | A |
| I-218 | 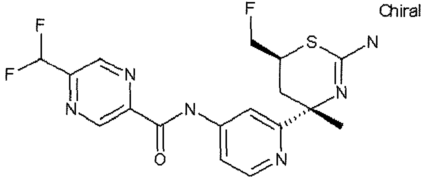 | 1H-NMR (CDCl3) δ: 1.63 (3H. s), 2.93 (1H, dd, J= 13.3. 3.7 Hz). 3.20-3.35 (1H, m), 4.30 (1H, dd, J = 9.4, 6.9 Hz), 4.40-4.49 (1H, m), 4.58 (1H, dd, J = 9.4. 4.7 Hz), 6.80 (1H, t. J = 54.3 Hz), 7.40 (1H, d, J = 1.7 Hz), 7.91 (1H. dd, J = 5.5, 2.2 Hz), 8.57 (1H, d, J = 5.5 Hz), 8.95 (1H, s), 9.53 (1H, d, J = 0.8 Hz), 9.74 (1H, s). | 411 | 1.09 | A |
| I-219 | 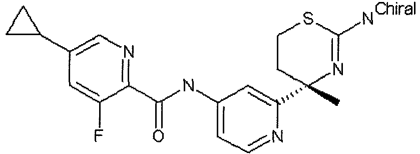 | 1H-NMR (CDCl3) δ: 0.80-0.90 (2H, m), 1.15-1.30 (2H, m), 1.59 (3H, s), 1.85-2.08 (2H, m), 2.50-2.60 (1H, m), 2.71-2.81 (1H, m), 2.91-3.00 (1H, m), 7.14 (1H, dd, J = 12.0, 1.5 Hz), 7.40 (1H, d, J = 1.5 Hz), 7.99 (1H, dd, J = 5.6, 2.1 Hz), 8.24 (1H, s), 8.52 (1H, d, J = 5.6 Hz), 9.97 (1H, s). | 386 | 1.02 | B |
| I-220 | 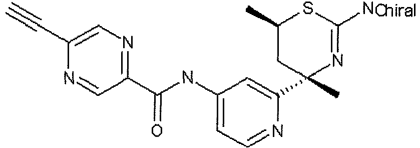 | 1H-NMR (CDCl3) δ: 1.23 (3H, d. J = 6.6 Hz), 1.36 (1H, dd, J = 12.6, 13.0 Hz), 1.61 (3H, s), 2.70-2.87 (1H, m), 2.89 (1H, dd, J = 13.2. 3.0 Hz), 3.55 (1H, m), 7.30 (1H, d, J = 1.9 Hz), 7.93 (1H, dd, J = 5.5, 1.9 Hz). 8.56 (1H, d, J = 5.5 Hz), 8.70 (1H, d, J = 1.3 Hz), 9.45 (1H, d, J = 1.3 Hz). | 367 | 1.01 | B |

**[Table 1-28]**

| No. | Structure | NMR(solvent:shift value;ascending order) | MS [M+1] | LC/M S RT | LC/MS Method |
|---|---|---|---|---|---|
| I-221 | 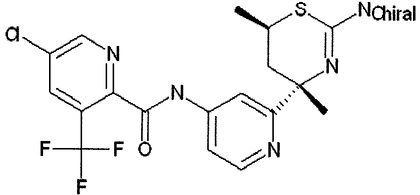 | 1H-NMR (CDCl3) δ: 1.23 (3H, d. J = 6.6 Hz), 1.35 (1H, t, J = 12.6 Hz), 1.59 (3H, s), 2.62-2.87 (1H. m). 2.90 (1H, dd, J = 3.0, 13.5 Hz), 4.64 (2H, brs), 7.14 (1H, s). 8.08 (1H, dd, J = 1.5, 5.7 Hz). 8.23 (1H, s), 8.53 (1H, d, J = 5.7 Hz), 8.76 (1H, s). | 444 | 1.16 | B |
| I-222 | 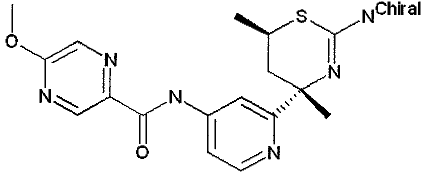 | 1H-NMR (CDCl3) δ: 1.22 (3H, d, J=6.3Hz), 1.34(1H, t, J=12.6 Hz), 1.61 (3H, s), 2.76-2.80 (1H, m), 2.86 (1H, dd, J= 3.0, 16.2 Hz), 3.32 (2H, brs), 4.07 (3H, s), 7.28 (1H, d, J = 1.8 Hz), 7.91 (1H, dd. J = 1.8, 5.4 Hz), 8.16 (1H, s), 8.53 (1H, d, J = 5.4 Hz), 9.01 (1H, s). 9.61 (1H, brs). | 373 | 1 | B |
| I-223 | 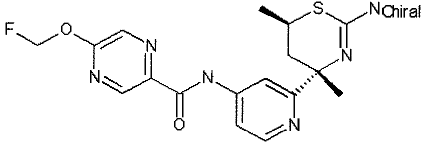 | 1H-NMR (CDCl3) δ: 1.22 (3H, d, J = 6.3 Hz), 1.34 (1H, t, J = 12.6 Hz), 1.60 (3H, s), 2.75-2.80 (1H, m), 2.86 (1H, dd, J = 3.0, 15.9 Hz), 6.15 (1H, d, J= 49.8 Hz), 7.28 (1H, d, J = 1.8 Hz), 7.90 (1H, dd. J = 2.1. 5.4 Hz), 8.29 (1H, d, J = 1.2 Hz), 8.54 (1H, d, J = 5.4 Hz), 9.07 (1H, d, J = 1.2 Hz), 9.58 (1H, brs). | 391 | 0.95 | B |
| I-224 | 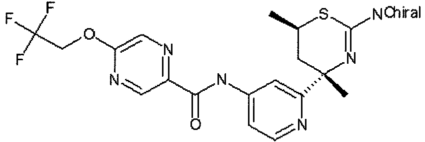 | 1H-NMR (CDCl3) δ: 1.21 (3H, d, J = 6.0 Hz), 1.35 (1H, t, J = 12.6 Hz), 1.61 (3H, s), 2.79-2.89 (1H, m), 4.42 (2H, brs), 4.86 (2H, dq, J = 7.8, 15.9 Hz). 7.33 (1H, s). 7.88 (1H, d, J = 5.4 Hz), 8.29 (1H, s). 8.53 (1H, d. J = 5.4 Hz), 9.00 (1H, brs). | 441 | 1.27 | B |
| -225 | 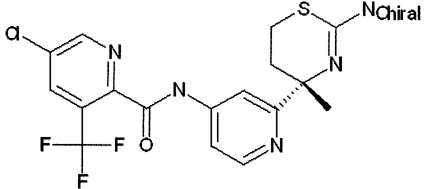 | 1H-NMR (CDCl3) δ: 1.56 (3H, s), 1.86-2.05 (1H, m), 2.46-2.53 (1H, m), 2.71-2.79 (1H, m), 2.93-3.01 (1H, m), 7.32 (1H, s), 8.05 (1H, d, J = 5.4 Hz). 8.24 (1H, s), 8.54 (1H, d, J = 4.8 Hz), 8.76 (1H, s). | 430 | 1.08 | B |

**[Table 1-29]**

| No. | Structure | NMR(solvent:shift value;ascending order) | MS [M+1] | LC/M S RT | LC/MS Method |
|---|---|---|---|---|---|
| I-226 | 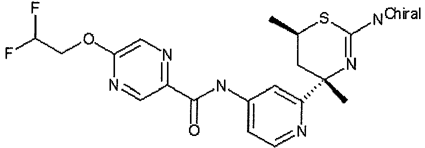 | 1H-NMR (CDCl3) δ: 1.23 (3H, d, J = 6.6 Hz), 1.36 (1H, t, J = 12.6 Hz), 1.61 (3H, s), 2.80-2.86 (1H, m), 2.98 (1H, dd, J = 3.0, 15.9 Hz), 3.16 (2H, brs), 4.66 2H, dt, J=3.6, 13.2Hz), 6.16(1H, tt, J = 3.6, 54.9 Hz), 7.29 (1H, d, J = 1.8 Hz). 7.92 (1H, ddd, J = 0.6, 2.1. 5.4 Hz), 8.27 (1H, dd, J = 0.6, 1.2 Hz), 8.54 (1H, d, J = 5.4 Hz). 9.01 (1H, dd, J = 0.6, 1.2 Hz), 9.62 (1H, brs). | 423 | 1.09 | B |
| I-227 | 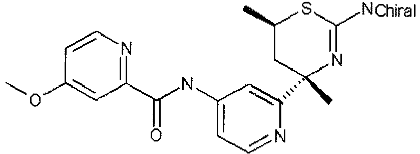 | 1H-NMR (CDCl3) δ: 1.24 (3H, d, J=6.6Hz), 1.43 (1H, t, J=12.6 Hz), 1.65 (3H, s), 2.83-2.88 (1H, m), 2.93 (1H, dd, J = 3.0, 16.2 Hz). 3.70 (2H, brs), 3.94 (3H, s), 6.99 (1H, dd. J = 2.4. 5.4 Hz), 7.36 (1H, d. J = 1.8 Hz). 7.80 (1H, d, J = 2.4 Hz), 7.95 (1H, dd, J = 2.1, 5.4 Hz), 8.42 (1H, d. J = 5.7 Hz), 8.53 (1H, d, J = 5.7 Hz), 10.23 (1H, brs). | 372 | 1 | B |
| I-228 | 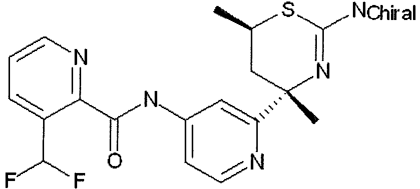 | 1H-NMR (CDCl3) δ: 1.23 (3H, d, J=6.3Hz), 1.32 (1H, t, J= 12.6 Hz), 1.61 (3H, s), 2.78-2.87 (1H, m), 2.90 (1H, dd, J = 2.7, 13.2 Hz), 4.11 (2H, brs), 7.25 (1H, d, J = 1.8Hz), 7.65 (1H, dd, J = 4.8, 7.8 Hz). 7.95 (1H, dd, J = 1.8, 5.4 Hz), 7.97 (1H, t, J = 54.9 Hz), 8.28 (1H, d, J = 7.8 Hz), 8.52 (1H, d, J = 5.4 Hz), 8.71 (1H, d, J=4.8Hz). | 392 | 1.06 | B |
| I-229 | 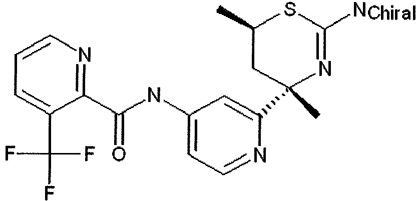 | 1H-NMR (CDCl3) δ: 1.23 (3H, d, J=6.3Hz), 1.36 (1H, t, J=12.6 Hz), 1.59 (3H, s), 2.79-2.86 (1H, m), 2.91 (1H, dd, J = 2.7, 12.9 Hz), 7.15 (1H, d, J=1.8Hz), 7.66 (1H, dd. J = 4.5, 7.8 Hz), 8.12 (1H, dd, J = 1.8. 5.4 Hz), 8.25 (1H, d. J = 8.1 Hz). 8.53 (1H, d, J = 5.4 Hz), 8.82 (1H, d, J = 4.5 Hz). | 410 | 1.02 | B |
| I-230 | 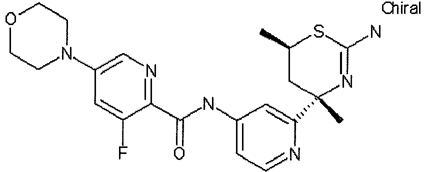 | 1H-NMR (CDCl3) δ: 1.22 (3H, d, J=6.3Hz), 1.35 (1H, t, J = 12.6 Hz), 1.60 (3H, s), 2.80-2.89 (1H, n). 3.15 (4H, brs), 3.94 (4H, brs), 4.18 (2H, brs), 7.25 (1H, s), 7.28 (1H, s), 7.97 (1H, d, J= 5.4 Hz), 8.09 (1H, s), 8.51 (1H, d, J = 5.4 Hz). | 445 | 1.03 | B |

**[Table 1-30]**

| No. | Structure | NMR(solvent:shiftvalue;ascending order) | MS [M+1] | LC/M S RT | LC/MS Method |
|---|---|---|---|---|---|
| I-231 | 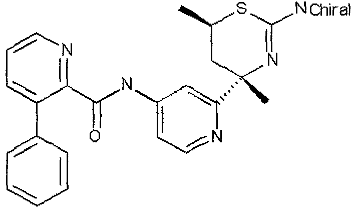 | 1H-NMR (CDCl3) δ: 1.20 (3H, d, J=6.3 Hz), 1.33 (1H, t, J = 12.6 Hz), 1.58 (3H, s), 2.79-2.88 (1H, H. m), 4.13 (2H, brs). 7.10 (1H, s), 7.34-7.41 (5H, m), 7.72 (1H, d, J = 7.8 Hz), 7.96 (1H, d, J = 5.4 Hz). 8.41 (1H, d, J = 5.4 Hz), 8.61 (1H, d, J = 4.2 Hz). | 418 | 1.19 | B |
| I-232 | 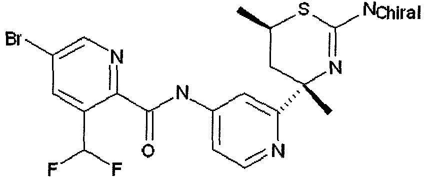 | 1H-NMR (CDCl3) δ: 1.13 (3H. d, J = 6.0 Hz). 1.45 (1H, t, J= 12.6 Hz), 1.65 (3H, s), 2.85-2.91 (1H, m), 2.97 (1H, dd, J = 2.7, 13.2 Hz), 4.40 (2H, brs), 6.68 (1H, s), 7.92 (1H, t, J = 54.9 Hz), 7.98 (1H, d, J = 5.7 Hz), 8.41 (1H, s), 8.54 (1H, d, J = 5.7 Hz), 8.79 (1H, s). | 470 | 1.39 | B |
| I-233 | 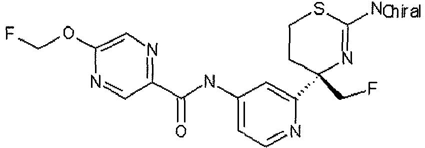 | 1H-NMR (CDCl3) δ: 1.92-2.05 (1H, m), 2.65-2.78 (2H, m), 2.88-294 (1H, m), 3.88 (2H, brs), 4.58 (1H, dd, J = 8.7, 10.8 Hz), 4.74 (1H, dd, J = 8.7, 10.8 Hz), 6.15 (2H, dd, J =1.8, 51.0 Hz), 7.26 (1H, d. J = 0.9 Hz), 7.48 (1H, s), 7.96 (1H, dd, J = 0.9, 5.4 Hz), 8.28 (1H, s), 8.55 (1H, d, J = 5.4 Hz), 9.07 (1H, s), 9.64 (1H, brs). | 395 | 0.87 | B |
| I-234 | 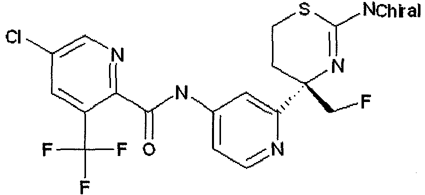 | 1H-NMR (CDCl3) δ: 1.89-2.04 (1H, m), 2.62-2.78 (2H, m), 2.87-295 (1H, m), 3.88 (2H, brs). 4.55 (1H, dd, J = 8.4, 12.9 Hz), 4.70 (1H, dd, J = 8.4, 12.9 Hz), 7.31 (1H, d, J = 2.1 Hz), 8.10 (1H, dd, J = 2.1, 5.4 Hz), 8.23 (1H, d, J = 2.1 Hz), 8.54 (1H, d, J = 5.4Hz), 8.74 (1H, d, J = 2.1 Hz), 9.95 (1H, brs). | 448 | 1.11 | B |
| I-235 | 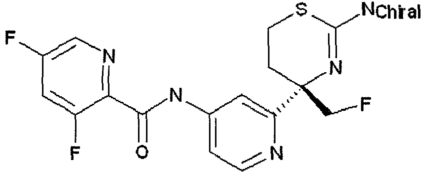 | 1H-NMR (CDCl3) δ: 1.91-2.00 (1H, m), 2.68-2.78 (2H, m), 2.88-295 (1H, m), 4.56 (1H, dd, J = 8.7, 12.9 Hz), 4.72 (1H, dd, J = 8.7, 12.9 Hz), 7.40 (1H, ddd, J = 2.4, 9.3, 10.8 Hz). 7.99 (1H, dd, J = 2.1, 5.7 Hz), 8.35 (1H, d, J = 2.1 Hz), 8.53 (1H, d, J = 5.7 Hz), 9.76 (1H, brs), | 382 | 0.79 | B |

**[Table 1-31]**

| No. | Structure | NMR(solvent:shift value;ascending order) | MS [M+1] | LC/M S RT | LC/MS Method |
|---|---|---|---|---|---|
| I-236 | 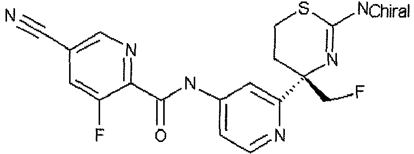 | 1H-NMR (CDCl3) δ: 1.92-2.00 (1H, m), 2.63-2.79 (2H, m), 2.91-2.98 (1H, m), 4.56 (1H, dd, J = 3.4, 12.3 Hz), 4.72 (1H, dd, J = 3.4, 12.3 Hz), 7.43 (1H, d, J = 1.8 Hz), 7.94 (1H, dd, J = 1.8, 9.6 Hz), 8.00 (1H, dd, J = 1.8, 5.4 Hz), 8.57 (1H, d, J = 5.4 Hz), 3.74 (1H, s), 9.80 (1H, brs). | 389 | 0.79 | B |
| I-237 | 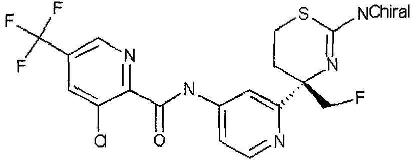 | 1H-NMR (CDCl3) δ: 1.90-2.00 (1H, m), 2.63-2.79 (2H, m), 2.89-2.96 (1H, m), 4.55 (1H, dd, J = 8.7, 11.7 Hz), 4.71 (1H, dd, J = 8.7, 11.7 Hz), 7.41 (1H, d, J = 2.1 Hz), 8.04 (1H, dd, J = 2.1, 5.7 Hz), 8.15 (1H, d, J = 1.8 Hz), 8.56 (1H, d, J = 5.7 Hz), 8.79 (1H, s), | 448 | 1.09 | B |
| I-238 | 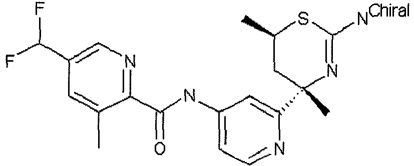 | 1H-NMR (CDCl3) δ: 1.22 (3H, d, J = 6.0 Hz), 1.35 (1H, t, J = 12.6 Hz), 1.61 (3H, s), 2.84 (3H, s), 2.79-2.91 (2H, m), 4.11 (2H, brs), 6.76 (1H, t, J = 55.5 Hz), 7.27 (1H, s), 7.79 (1H, s), 7.96 (1H, d, J = 5.4 Hz), 8.52 (1H, d, J = 5.4 Hz), 8.58 (1H, s), | 406 | 1.1 | B |
| I-239 | 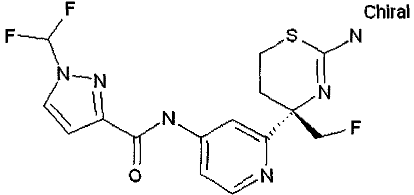 | 1H-NMR (CDCl3) δ: 1.91-2.01 (1H, m), 2.62-2.78 (2H, m), 2.88-2.95 (1H, m), 4.56 (1H, dd, J = 8.4, 12.6 Hz), 4.72 (1H, dd, J = 8.4, 12.6 Hz), 7.05 (1H, d, J = 2.7 Hz), 7.22 (1H, t, J = 60.0 Hz), 7.42 (1H, d, J = 2.4 Hz), 7.89 (1H, d, J =2.4 Hz), 7.90 (1H, dd, J = 2.7, 5.4 Hz), 8.52 (1H, d, J = 5.4 Hz), 8.79 (1H, brs). | 385 | 0.82 | B |
| I-240 | 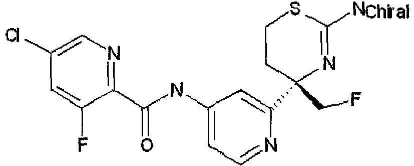 | 1H-NMR (CDCl3) δ: 1.91-2.01 (1H, m), 2.62-2.79 (2H, m). 2.88-2.95 (1H, m), 4.56 (1H, dd, J = 8.7, 13.8 Hz), 4.72 (1H, dd, J = 8.7. 13.8 Hz), 7.41 (1H, d, J = 1.5 Hz), 7.68 (1H, dd, J = 2.1, 10.2 Hz), 7.99 (1H, dd, J = 2.1, 5.4 Hz), 8.42 (1H, d, J = 1.5 Hz), 8.54 (1H, d, J = 5.4 Hz), 9.80 (1H, brs). | 398 | 0.93 | B |

**[Table 1-32]**

| No. | Structure | NMR(solvent:shiftvalue;ascending order) | MS [M+1] | LC/MS RT | LC/MS Method |
|---|---|---|---|---|---|
| I-241 | 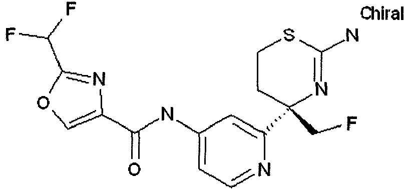 | 1H-NMR (CDCl3) δ: 1.90-2.00 (1H. m). 2.60-2.77 (2H, m), 2.88-2.95 (1H, m), 4.58 (1H, dd, J = 8.4, 12.3 Hz), 4.71 (1H, dd, J = 8.4, 12.3 Hz), 6.70 (1H, t. J = 52.2 Hz). 7.45 (1H , d. J = 2.1 Hz), 7.87 (1H, dd, J = 2.1, 5.4 Hz), 8.42 (1H, s), 8.54 (1H, d, J = 5.4 Hz), 8.90 (1H, brs). | 386 | 0.83 | B |
| I-242 | 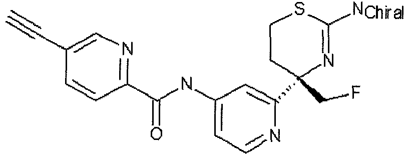 | 1H-NMR (DMSO-d6) δ: 1.72-1.79 (1H, m), 2.50-2.57 (2H, m), 2.85-2.89 (1H, m), 4.45 (1H, dd. J = 8.1, 27.3 Hz). 4.61 (1H, dd, J = 8.1, 27.3 Hz), 4.72 (1H, s), 6.08 (2H, s), 7.80 (1H, dd, J = 2.1.5.4 Hz), 8.01 (1H, s), 8.17 (1H, dt, J = 1.5, 9.9 Hz), 8.46 (1H, d, J = 5.4 Hz). 8.83 (1H, s), 11.03 (1H, brs), | 370 | 1.14 | B |
| I-243 | 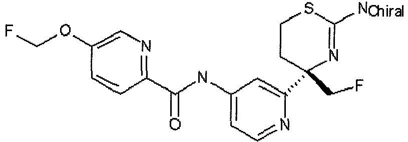 | 1H-NMR (CDCl3) δ: 1.92-2.04 (1H, m), 2.63-2.78 (2H, m), 2.89-2.93 (1H, m), 4.57 (1H, dd, J = 8.4, 15.3 Hz). 4.70 (1H, dd, J = 8.4, 15.3 Hz), 5.81 (2H, dt, J = 0.6, 53.1 Hz). 7.48 (1 H. s), 7.56 (1H, dd. J = 2.1, 6.3 Hz), 7.96 (1H, dd, J = 2.1, 3.6 Hz), 8.26 (1H, d, J = 8.4 Hz). 8.40 (1H, s), 8.53 (1H, d, J = 5.7 Hz), 9.98 (1H, s), | 394 | 1.08 | B |
| I-244 | 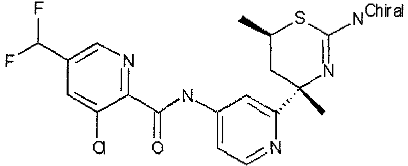 | 1H-NMR (CDCl3) δ: 1.22 (3H, d, J = 6.3 Hz), 1.32 (1H,t, J = 12.6 Hz), 1.58 (3H, s), 2.75-2.85 (1H, m), 6.79 (1H, t, J = 55.2 Hz), 7.23 (1H, d, J = 0.6 Hz), 8.00 (1H, dd, J = 0.6, 2.1 Hz), 8.02 (1H, s), 8.53 (1H, d, J = 5.4 Hz), 8.65 (1H, s), | 426 | 1.48 | B |
| I-245 | 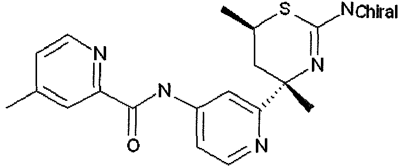 | 1H-NMR (CDCl3) δ: 1.23 (3H, d, J = 6.6 Hz), 1.35 (1H, t, J = 12.6 Hz), 1.61 (3H, s), 1.89 (3H, s), 2.74-2.85 (2H, m), 2.87 (1H, dd, J = 3.0, 13.2 Hz), 7.34 (1H, d, J = 2.1 Hz), 7.75 (1H, dd, J = 1.2, 4.8 Hz), 7.92 (1H, dd. J = 2.1, 5.4 Hz), 8.53 (1H, d, J = 0.6 Hz), 8.56 (1H, d, J = 5.4 Hz), 8.84 (1H, d, J = 4.8 Hz), 9.85 (1H, s). | 356 | 1.01 | B |

**[Table 1-33]**

| No. | Structure | NMR(solvent:shift value;ascending order) | MS [M+1] | LC/M S RT | LC/MS Method |
|---|---|---|---|---|---|
| I-246 | 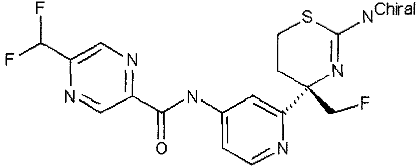 | 1H-NMR (CDCl3) δ: 1.91-2.08 (1H, m), 2.63-2.78 (2H, m), 2.90-2.96 (1H, m), 4.66 (2H, d, J = 47.4 Hz), 6.79 (1H, t, J = 54.3 Hz), 7.55 (1H, d, J = 1.2 Hz), 7.95 (1H, dd. J = 1.2, 5.4 Hz), 8.57 (1H, d, J = 5.4 Hz), 8.90 (1H, s), 9.50 (1H, s). | 397 | 0.86 | B |
| I-247 | 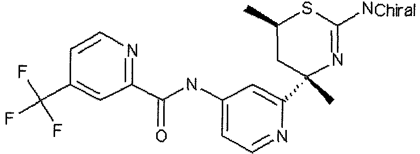 | 1H-NMR (CDCl3) δ: 1.22 (3H, d, J = 6.6 Hz), 1.35 (1H, t, J = 12.6 Hz), 1.61 (3H, s), 2.74-2.86 (2H, m), 2.88 (1H, dd, J = 3.0, 13.2 Hz), 7.34 (1H, d, J = 2.1 Hz), 7.75 (1H, dd, J = 1.2, 4.8 Hz), 7.92 (1H, dd, J = 2.1, 5.4 Hz), 8.53 (1H, d, J = 0.6 Hz), 8.56 (1H, d, J = 5.4 Hz), 8.84 (1H, d, J = 4.8 Hz), 10.02 (1H, s), | 410 | 1.23 | B |
| I-248 | 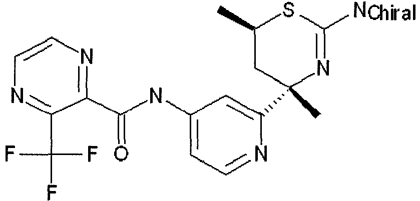 | 1H-NMR (CDCl3) δ: 1.20 (3H, d, J = 6.3 Hz), 1.21-1.26 (1H, m), 1.45 (3H, s), 2.72-2.82 (2H, m), 5.35 (2H, brs), 7.18 (1H, d, J = 1.5 Hz), 8.08 (1H, dd, J = 1.5. 5.4 Hz), 8.54 (1H, d, J = 5.4 Hz), 8.81 (1H, d, J = 24 Hz), 8.90 (1H, d, J = 2.4 Hz). | 411 | 1 | B |
| I-249 | 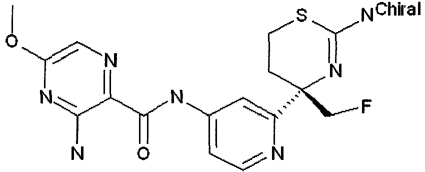 | 1H-NMR (DMSO-d6) δ: 2.40-2.58 (2H, m), 2.85-2.89 (2H, m), 3.91 (3H, s), 4.43 (1H, dd, J = 8.4, 30.9 Hz), 4.60 (1H, dd, J = 8.4. 30.9 Hz), 6.04 (2H, s), 7.55 (1H, s), 7.71 (1H, d, J = 5.7 Hz), 7.90 (1H, d, J = 5.7 Hz), 7.90 (1H, s), 8.40 (1H, d, J = 5.1 Hz), 10.47 (1H, s). | 392 | 0.96 | B |
| I-250 | 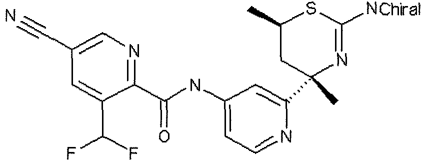 | 1H-NMR (CDCl3) δ: 1.23 (3H, d, J = 6.6 Hz), 1.34 (1H, t, J = 12.6 Hz), 1.61 (3H, s), 2.77-2.88 (1H, m), 2.91 (1H, dd, J = 3.3, 13.5 Hz), 7.26 (1H, s), 7.94 (1H, dd, J = 2.1, 5.4 Hz), 7.97 (1H, t, J = 54.6 Hz), 8.56 (1H, d, J = 5.4 Hz), 8.58 (1H, s), 9.00 (1H, d, J = 0.9 Hz). | 417 | 1.11 | B |

**[Table 1-34]**

| No. | Structure | NMR(solvent:shiftvalue;ascending order) | MS [M+1] | LC/M S RT | LC/MS Method |
|---|---|---|---|---|---|
| I-251 | 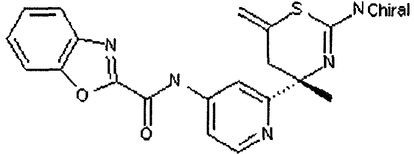 | 1H-NMR (DMSO-d6) δ: 1.38 (3H, s), 2.51 (2H, s), 4.97 (1H, s), 5.06 (1 H, s), 6.15 (2H, brs), 7.54 (1H, t, J = 7.7 Hz), 7.62 (1H, t, J = 7.7 Hz), 7.71 (1H, d, J = 5.3 Hz), 7.91 (1H, d, J = 7.7 Hz), 7.96 (1H, d, J = 7.7 Hz), 8.09 (1H, s), 8.47 (1H, d, J = 5.3 Hz), 11.47 (1H, brs). | 380 | 1.18 | B |
| I-252 | 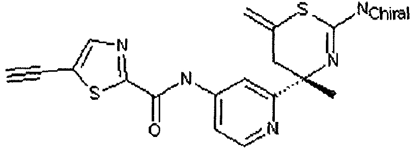 | 1H-NMR (DMSO-d6) δ: 1.36 (3H, s), 2.51 (2H, s), 4.96 (1H, s), 5.04 (1H, s), 5.06 (1H, s), 6.13 (2H, brs), 7.63 (1H, d, J = 5.3 Hz), 8.10 (1H, s), 8.36 (1H, s), 8.44 (1H, d, J =5.3 Hz), 11.20 (1H, brs). | 370 | 1.22 | B |
| I-253 | 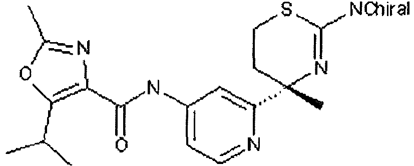 | 1H-NMR (DMSO-d6) δ: 1.23 (3H, s), 1.25 (3H, s), 1.38 (3H, s), 1.65-1.74 (1H, m), 2.20-2.26 (1H, m), 2.47 (3H, s), 2.52-2.62 (1H, m), 2.87-2.92 (1H, m), 3.74-3.83 (1H, m), 5.80 (2H, brs), 7.62 (1H , dd , J = 5.4, 2.1 Hz), 7.92 (1H, d, J = 2.1 Hz), 8.36 (1H, d, J =5.4 Hz), 10.22 (1H, s), | 374 | 1.16 | B |
| I-254 | 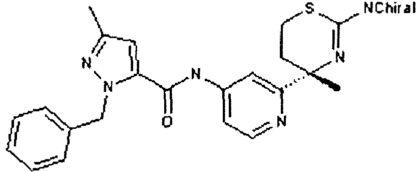 | 1H-NMR (DMSO-d6) δ: 1.38 (3H, s), 1.65-1.74 (1H, m), 2.23-2.29 (1H. m), 2.23 (3H, s), 2.49-2.60 (1H, m), 2.86-2.94 (1H, m), 5.65 (2H, s), 5.82 (2H, brs), 6.96 (1H, s), 7.15 (2H, dd, J = 8.1, 1.4 Hz), 7.21-7.33 (1H, m), 7.64 (1H, dd, J = 5.4, 2.1 Hz), 7.68 (1H, d, J = 1.5 Hz), 8.39 (1H, d, J = 5.4 Hz), 10.22 (1H, s), | 425 | 1.18 | B |
| I-255 | 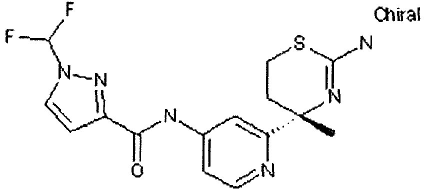 | 1H-NMR (DMSO-d6) δ: 1.43 (3H, s), 1.69-1.78 (1H, m), 2.23-2.31 (1H, m), 2.53-2.63 (1H, m), 2.90-2.96 (1H, m), 5.81 (2H, s), 6.09 (1H, d, J = 2.5 Hz), 7.73 (1H, dd, J = 5.4, 1.8 Hz), 7.91 (1H, d, J = 1.8 Hz), 7.95 (1H, t, 59 Hz), 8.44 (1H, d, J = 5.4 Hz), 8.45 (1H, d, J = 2.1 Hz), 10.72 (1H, s), | 367 | 0.84 | B |

**[Table 1-35]**

| No. | Structure | NMR(solvent:shift value;ascending order) | MS [M+1] | LC/M S RT | LC/MS Method |
|---|---|---|---|---|---|
| I-256 | 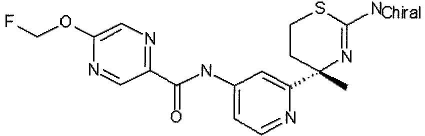 | 1H-NMR (DMSO-d6) δ: 1.44 (3H, s), 1.70-1.80 (1H, m), 2.25-232 (1H, m), 2.54-2.65 (1H, m), 2.89-2.97 (1H, m), 5.84 (2H, s), 6.24 (1H, d. J = 52 Hz), 7.75 (1H, dd, J = 5.5, 2.1 Hz), 8.03 (1H, d, J = 2.1Hz), 8.47 (1H, d. J = 5.5 Hz), 8.63 (1H, d, J = 1.3 Hz), 9.01 (1 H. d, J = 1.3 Hz), 10.90 (1H, s), | 377 | 0.89 | B |
| I-257 | 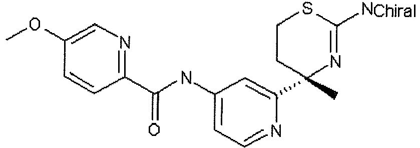 | 1H-NMR (DMSO-d6) δ: 1.40 (3H, s), 1.67-1.76 (1H, m), 2.19-226 (1H. m). 2.53-2.64 (1H, m), 2.87-2.94 (1H, m), 3.94 (3H, s), 5.79 (2H, s), 7.63 (1H, dd, J = 8.7, 2.9 Hz), 7.72 (1H, dd. J = 5.5, 2.0 Hz), 7.95 (1H, d, J = 2.0Hz), 8.14 (1H, d, J = 8.7Hz), 8.40 (1H, d, J = 29 Hz), 8.41 (1H, d, J = 5.5 Hz), 10.68 (1H, s), | 358 | 0.92 | B |
| I-258 | 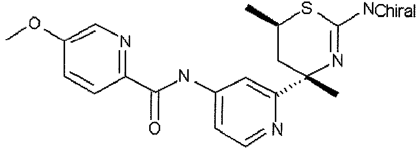 | 1H-NMR (DMSO-d6) δ:1.09 (3H, d, J = 6.6 Hz), 1.44 (3H, s), 253-260 (1H, m), 2.67 (1H, dd, J = 10.2. 3.0 Hz). 3.93 (3H, s), 5.89 (2H, s), 7.62 (1H, dd, J = 8.7, 2.9 Hz), 7.69 (1H, dd, J = 5.6, 1.9 Hz), 7.81 (1H, d, J = 29 Hz), 8.13 (1H, d, J = 8.7Hz), 8.40 (1H, d, J = 1.9 Hz), 8.41 (1H, d, J = 5.6 Hz), 10.70 (1H, s), | 372 | 1.03 | B |
| I-259 | 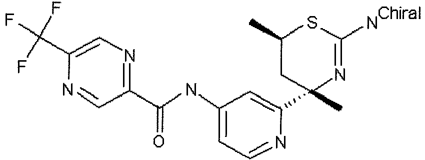 | 1H-NMR (DMSO-d6) δ:1.10 (3H, d, J = 6.6 Hz), 1.45 (3H, s), 249-260 (1H, m), 2.68 (1H, dd, J = 13.1, 2.7 Hz), 5.91 (2H, s), 7.73 (1H, dd, J = 5.3, 1.9 Hz), 7.87 (1H, d, J = 1.9 Hz), 8.47 (1H, d, J = 5.5 Hz), 9.31 (1H, s), 9.45 (1H, s), 11.23 (1H, s), | 411 | 1.03 | B |
| I-260 | 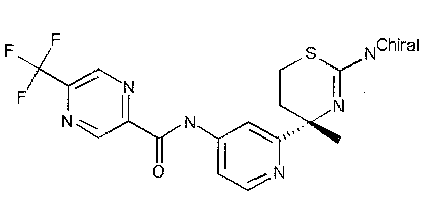 | 1H-NMR (DMSO-d6) δ: 1.41 (3H, s), 1.65-1.75 (1H, m), 2.23-2.28 (1H, m), 2.53-2.60 (1H, m), 2.87-2.92 (1H, m), 5.82 (2H, s), 7.74 (1H, dd. J = 5.3, 1.9 Hz), 8.00 (1H, d, J = 1.9 Hz), 8.46 (1H, d, J = 5.5 Hz), 9.32(1H, s). 9.45 (1H, s), 11.24 (1H, s). | 397 | 1.05 | B |

**[Table 1-36]**

| No. | Structure | NMR(solvent:shift value;ascending order) | MS [M+1] | LC/M S RT | LC/MS Method |
|---|---|---|---|---|---|
| I-261 | 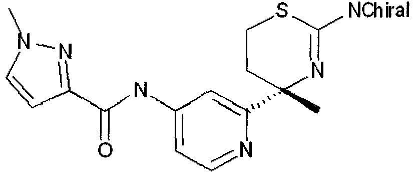 | 1H-NMR (DMSO-d6) δ: 1.39 (3H, s), 1.64-1.73 (1H, m), 2.20-2.28 (1H, m), 2.52-2.61 (1H, m), 2.86-293 (1H, m), 3.97 (3H, s), 5.77 (2H, s), 6.79 (1H, dd, J = 2.1, 1.2 Hz), 7.67 (1H, dd, J = 5.2, 1.5 Hz), 7.85(1H, d, J=2.1 Hz), 7.85 (1H, d, J = 1.2 Hz), 8.37 (1H, d, J = 5.5 Hz), 10.35 (1H, s). | 331 | 0.71 | B |
| I-262 | 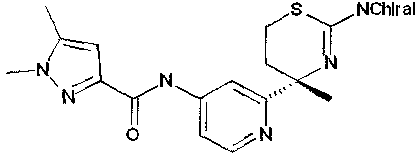 | 1H-NMR (DMSO-d6) δ: 1.39 (3H, s). 1.67-1.75 (1H, m), 2.23-2.31 (1H, m), 2.31 (3H.s), 2.53-2.60 (1H, m), 2.86-2.92 (1H, m), 3.84 (3H, s), 5.84 (2H, s), 6.59 (1H, dd, J = 2.1, 1.2 Hz), 7.67 (1H, d, J = 5.2 Hz), 7.87 (1H, s). 8.37 (1H, d, J= 5.1 Hz), 10.25 (1H, s). | 345 | 0.8 | B |
| I-263 | 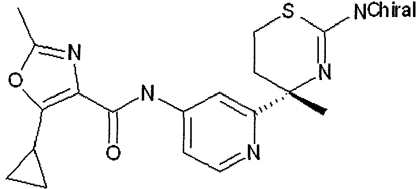 | 1H-NMR (DMSO-d6) δ: 0.97-0.99 (2H, m), 1.11-1.18 (2H, m), 1.39 (3H, s), 1.65-1.74 (1H, m), 2.19-226 (1H, m), 2.41 (3H,s), 2.53-2.62 (1H, m), 2.74-2.83 (1H. m), 2.86-2.92(1H, m), 5.77 (2H, s), 7.64 (1H, dd, J = 5.4, 2.0 Hz), 7.92 (1H, d, J = 2.0 Hz), 8.37 (1H, d, J = 5.4 Hz). 10.16 (1H, s). | 372 | 1.07 | B |
| I-264 | 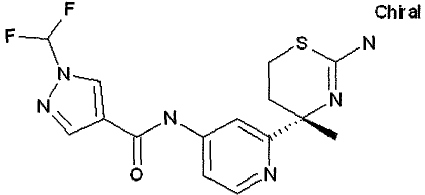 | 1H-NMR (DMSO-d6) δ: 1.39 (3H, s), 1.65-1.74 (1H, m), 2.22-2.28 (1H. m), 2.52-2.61 (1H, m), 2.86-2.94 (1H, m), 5.79 (21, s), 7.70 (1H, d, J = 5.4 Hz). 7.91 (1H, t, J = 59 Hz), 8.33 (1H, s) 8.41 (1H, d, J = 5.4 Hz), 8.91 (1H, s), 10.44 (1H, s). | | | |
| I-265 | 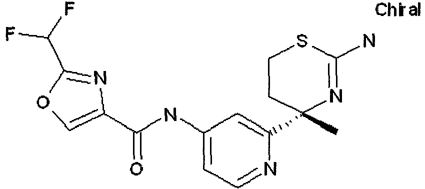 | 1H-NMR (DMSO-d6) δ: 1.40 (3H, s), 1.65-1.74 (1H, m), 2.21-2.27 (1H, m), 2.52-2.60 (1H, m), 2.86-2.93 (1H, m), 5.78 (2H, s). 7.24 (1H, t, J = 51.8 Hz), 7.69 (1H, dd, J = 5.5. 2.1 Hz), 7.84 (1H, d, J = 2.0 Hz), 8.42 (1H, d, J= 5.5Hz), 9.07 (1H, s). 10.70 (1H, s). | | | |

**[Table 1-37]**

| No. | Structure | NMR(solvent:shift value;ascending order) | MS [M+1 ] | LC/M S RT | LC/MS Method |
|---|---|---|---|---|---|
| I-266 | 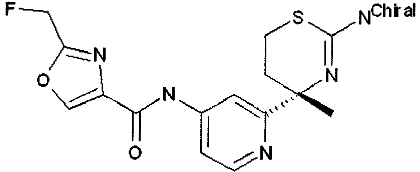 | 1H-NMR (DMSO-d6) δ: 1.40 (3H, s), 1.64-1.73 (1H, m), 2.212-2.27 (1H, m), 2.54-2.60 (1H, m), 2.86-2.93 (1H, m), 5.59 (2H, d, J = 46.8 Hz), 5.78 (2H, s), 7.68 (1H, dd. J = 5.3, 1.5 Hz), 7.86 (1H, d, J = 2.0 Hz), 8.40 (1H, d, J=5.6 Hz), 8.94 (1H, s), 10.61 (1H, s). | 350 | 0.72 | B |
| I-267 | 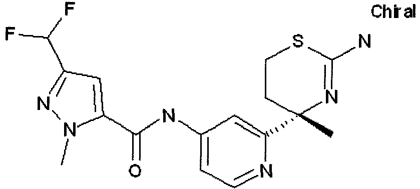 | 1H-NMR (DMSO-d6) δ:1.39 (3H, s), 1.65-1.75(1H, m), 2.21-2.30 (1H, m), 2.52-2.61 (1H, m), 2.87-2.94 (1H, m), 4.13 (3H, s), 5.80 (2H, s), 7.07 (1H, t, d = 54.4 Hz), 7.38 (1H, s), 7.67 (1H, dd, J = 5.4, 2.1 Hz), 7.72 (1H, d. J=2.0Hz), 8.43 (1H, d, J = 5.4 Hz), 10.67 (1H, s). | 381 | 0.91 | B |
| I-268 | 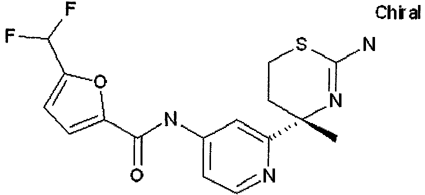 | 1H-NMR (DMSO-d6) δ: 1.40 (3H. s), 1.66-1.75 (1H, m), 2.20-2.27 (1H. m), 2.52-2.61 (1H, m), 2.87-2.94(1H, m), 5.78 (2H, s), 7.10 (1H, d, J=3.6Hz), 7.19 (1H, t, J = 53.1 Ha), 7.52 (1H, d, J = 3.4Hz), 7.68-7.70 (2H, m), 8.40-8.43(1H, m), 10.70 (1H, s). | 367 | 0.89 | B |
| I-269 | 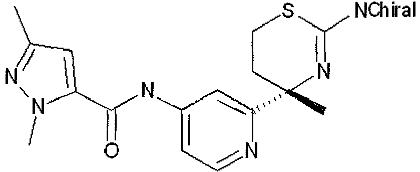 | 1H-NMR (DMSO-d6) δ: 1.39 (3H, s). 1.64-1.73 (1H, m), 2.22 (3H, s), 2.21-2.30 (1H, m), 2.49-260 (1H, m), 2.86-2.94 (1H, m), 3.99 (3H, s), 5.79 (2H, s), 6.88 (1H, s), 7.64 (1H, dd, J = 5.3, 2.0 Hz), 7.72 (1H, d, J = 2.0 Hz), 8.40 (1H, d, J = 5.5 Hz), 10.45 (1 H. s). | 345 | 0.79 | B |
| I-270 | 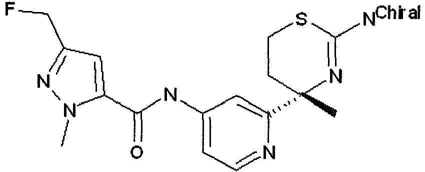 | 1H-NMR (DMSO-d6) δ: 1.40 (3H, s), 1.66-1.75 (1H, m), 2.22-2.30 (1H, m), 2.53-2.60 (1H, m), 2.87-296 (1H, m), 4.09 (3H, s), 5.37 (2H, d, J = 48.3 Hz), 5.85 (2H, s), 7.25 (1H, s), 7.66 (1H. dd, J = 5.3, 1.5 Hz), 7.73 (1H, s), 8.42 (1H, d, J = 5.3 Hz), 10.62 (1H, s), | 363 | 0.82 | B |

**[Table 1-38]**

| No. | Structure | NMR(solvent:shift value;ascending order) | MS [M+1] | LC/M S RT | LC/MS Method |
|---|---|---|---|---|---|
| I-271 | 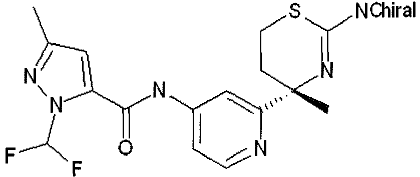 | 1H-NMR (CDCl3) δ: 1.52 (3H. s), 1.81-1.90 (1H, m), 2.40 (3H. s). 2.52-2.60 (1H, m), 2.69-2.79 (1H, m), 292-3.00 (1H, m), 6.71 (1H, s). 7.34 (1H, d. J = 2.0 Hz), 7.72 (1H, d, J = 2.0Hz), 7.87 (1H, dd, J = 5.5. 2.1 Hz), 8.04 (1H, t, 59.1 Hz), 8.52 (1H, d, J = 5.5 Hz) | 381 | 0.96 | B |
| I-272 | 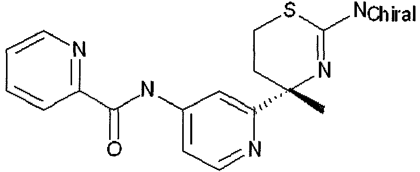 | 1H-NMR (DMSO-d6) δ: 1.41 (3H, s), 1.68-1.77 (1H, m), 2.19-2.28 (1H, m), 2.54-2.64 (1H, m), 2.87-295(1H, m), 5.80 (2H, s), 7.70 (1H, ddd, J = 7.3. 4.7. 1.1 Hz), 7.75 (1H, dd. J = 5.5, 2.1 Hz), 7.98 (1H, d = 2.0 Hz), 8.09 (1H, td, J = 7.6, 1.6 Hz). 8.17 (1H, d. J = 7.7 Hz), 8.43 (lH, d, J = 5.5 Hz), 8.76 (1H, d. J = 4.9 Hz), 10.87 (1H, s), | 328 | 0.9 | B |
| I-273 | 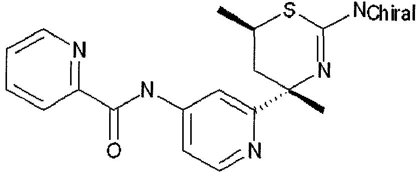 | 1H-NMR (DMSO-d6) δ: 1.10 (3H, d = 6.0 Hz). 1.14-1.20 (1H, m), 1.45 (3H, s), 2.53-2.62 (1H, m), 2.68 (1H, d, J = 13.2 Hz). 5.90 (2H, s), 7.68-7.74 (2H, m). 7.86 (1H, d = 1.4 Hz), 8.09 (1H, td, J = 7.5, 1.5 Hz), 8.16 (1H, d, J = 7.7Hz), 8.44(!H, d, J = 5.5 Hz), 8.76 (1H, d. J = 4.4 Hz), 10.91 (1H, s). | 342 | 1.01 | B |
| I-274 | 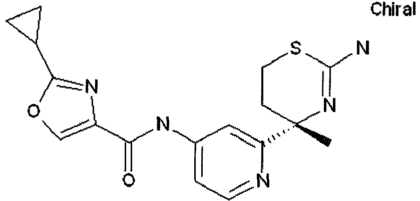 | 1H-NMR (CDCl3) δ: 1.12-1.15 (4H, m), 1.56 (3H, s), 1.86-1.95 (1H, m), 2.04-2.14 (1H, m), 2.42-2.50 (1H, m), 271-2.79 (1H, m), 2.93-3.00 (1H, m), 7.38 (1H, d, J = 1.7Hz), 7.86(1H, dd, J=5.5, 2.0 Hz), 8.12 (1H, s), 8.50 (1H, d, J = 5.4 Hz), 8.75 (1H, s) | 358 | 0.92 | B |
| I-275 | 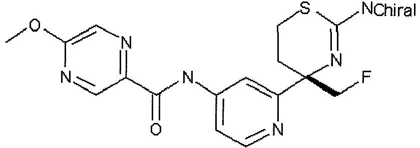 | 1H-NMR (CDCl3) δ: 1.91-2.00 (1H, m), 2.62-2.79 (2H, m), 2.88-2.95 (1H, m), 4.07 (3H, s), 4.64 (2H, ddd, J = 47.5, 16.1, 8.5 Hz), 7.44 (1H, d, J = 1.8 Hz), 7.96 (1H, dd, J = 5.5, 1.8 Hz), 8.15 (1H, d, J = 0.6 Hz), 8.53 (1H, d. J = 5.5Hz), 9.00-9.01 (1H, m), 9.63 (1H, s). | 377 | 1 | A |

**[Table 1-39]**

| No. | Structure | NMR(solvent:shift value;ascending order) | MS [M+1] | LC/M S RT | LC/MS method |
|---|---|---|---|---|---|
| I-276 | 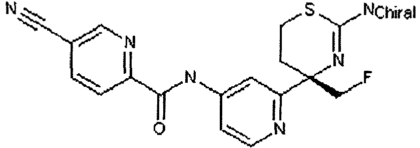 | 1H-NMR (CDCl3) δ: 1.93-2.01 (1H, m), 2.62-2.78 (2H, m), 2.89-2.96 (1H, m), 4.64 (2H, ddd, J= 47.6, 13.2,8.5 Hz), 7.49 (1H, d, J = 1.8 Hz), 7.96 (1H, dd, J = 5.5, 1.8 Hz), 8.22 (1 H, dd, J = 8.1, 2.0 Hz), 8.42 (1H, d, J =8.1 Hz), 8.56 (1H, d, J = 5.5 Hz), 8.90-8.91 (1H, m), 9.96 (1H, s). | 371 | 0.98 | A |
| I-277 | 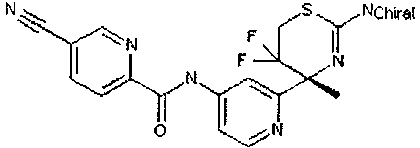 | 1H-NMR (CDCl3) δ: 1.82 (3H, s), 3.34 (2H, dd, J = 13.9, 11.7 Hz), 4.45 (2H, brs), 7.73 (1H, d, J = 2.1 Hz), 7.87 (1H, dd, J = 5.5, 2.1 Hz), 8.22 (1H, dd, J = 8.1, 2.0 Hz), 8.43 (1H, dd, J = 8.1, 0.9 Hz), 8.63 (1H, d, J = 5.5 Hz), 8.91 (1H, dd, J = 2.0, 0.9 Hz), 9.97 (1H, s). | 389 | 1.08 | A |
| I-278 | 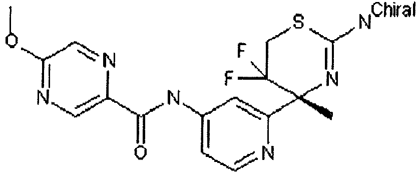 | 1H-NMR (CDCl3) δ: 1.81 (3H, s),3.33 (2H, m), 4.07 (2H, brs), 4.07 (3H , s), 7.74 (1H, d, J= 2.0 Hz), 7.84 (1H, dd, J = 5.4, 2.0 Hz), 8.16 (1H, d, J =1.2 Hz), 8.60 (1H, d, J = 5.4Hz), 9.02 (1H, d, J = 1.2 Hz), 9.66 (1H, s). | 395 | 1.14 | A |
| I-279 | 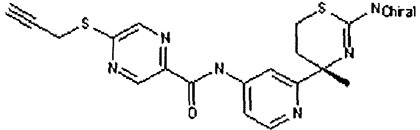 | 1H-NMR (CDCl3) δ: 1.58 (3H, s), 1.92 (1H, ddd, J = 13.6, 10.4, 4.0 Hz), 2.24 (1H, t, J = 2.7 Hz), 2.52 (1H, ddd, J = 13.6, 6.6, 3.7 Hz), 2.75 (1H, ddd, J = 12.3, 10.4, 3.7 Hz), 2.96 (1H, ddd, J = 12.3, 6.4, 4.0 Hz), 4.03 (2H, d, J =2.7 Hz),4.90 (2H, brs), 7.47 (1H, dd, J = 2.2, 0.4 Hz), 7.92 (1H, dd, J = 5.5, 2.2 Hz), 8.42 (1H, d, J = 1.5Hz), 8,53 (1H, dd, J = 5.5, 0.4 Hz), 9.25 (1H, d, J= 1.5 Hz) | 399 | 1.2 | A |
| 1-280 | 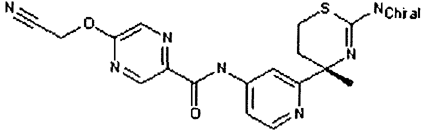 | 1H-NMR (CDCl3) δ: 1.64 (3H, s), 1.92 (1H, m), 2.75 (2H, m), 2.93 (1H, m), 5.15 (2H, s), 5.74 (2H, brs), 7.42 (1H, d, J = 2.1 Hz), 8.00 (1H, dd, J = 5.5, 2.1 Hz), 8.33 (1H, d, J =1.3 Hz), 8.54 (1H, d, J = 5.5 Hz), 9.07 (1H, d, J = 1.3 Hz), 9.68 (1H, brs). | 384 | 0.89 | B |

**[Table 1-40]**

| No. | Structure | NMR(solvent:shift value;ascending order) | MS [M+1] | LC/M S RT | LC/MS Method |
|---|---|---|---|---|---|
| I-281 | 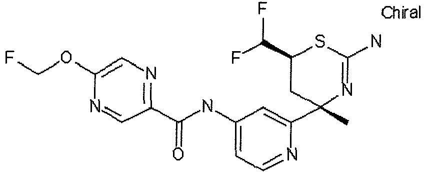 | 1H-NMR (CDCl3) δ: 1.57 (1H, dd. J = 13.0, 13.0 Hz), 1.67 (3H, s), 3.14 (1H, dd, J = 13.0, 4.6 Hz), 3.49 (1H, m), 3.85 (2H, s), 5.75 (1H, td, J = 55.7, 4.9 Hz), 6.16 (2H, d, J = 50.9 Hz), 7.36 (1H, d, J = 2.0 Hz), 7.98 (1H, dd. J = 5.5, 2.0 Hz), 8.32 (1H, d, J = 1.3 Hz), 8.54 (1H, d, J = 5.5 Hz). 9.08 (1H, d, J = 1.3 Hz). 9.68 (1H, s). | 427 | 1.17 | A |
| I-282 | 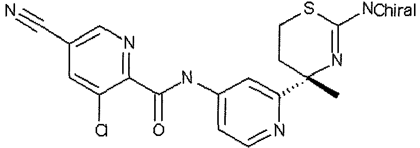 | 1H-NMR (CDCl3) d: 1.55 (3H. s), 1.87-1.95 (1H, m), 2.42-2.49 (1H, m). 2.70-2.79 (1H, m), 2.94-3.02 (1H, m), 7.40 (1H, d, J = 1.8 Hz), 7.97 (1H, dd, J = 5.5, 1.8 Hz), 8.19 (1H, d, J = 1.8 Hz), 8.55 (1H, d. J = 5.5 Hz), 8.79 (1H, d, J = 1.8Hz). | 387 | 0.91 | B |
| I-283 | 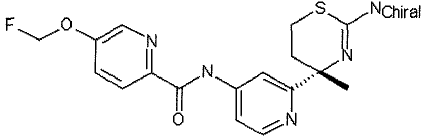 | 1H-NMR (CDCl3) d: 1.58 (3H. s), 1.91 (1H, ddd, J =13.6, 10.2, 3.9 Hz), 2.47 (1H, ddd, J = 13.6, 6.6, 3.6 Hz). 2.76 (1H, ddd, J = 12.1, 10.2, 3.6 Hz), 2.97 (1H, ddd, J = 12.1, 6.6, 3.9 Hz), 5.82 (2H, d, J = 53.4 Hz), 7.46 (1H, d, J = 25 Hz), 7.59 (1H, dd, J = 8.5, 2.5 Hz), 7.94 (1H, dd, J = 5.4, 2.5 Hz), 8.29 (1H, d, J = 8.5 Hz), 8.43 (1H, d, J = 25 Hz). 8.54 (1H, d, J = 5.4 Hz), 9.98 (1H, brs). | 376 | 0.92 | B |
| I-284 | 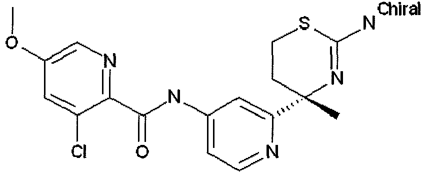 | 1H-NMR (CDCl3) d: 1.56 (3H, s), 1.91 (1H, ddd, J = 13.8, 10.2 3.9 Hz), 2.47 (1H, ddd, J = 13.8, 6.9, 3.9 Hz), 2.72-2.80 (1H, m), 2.93-3.01 (1H, m), 3.96 (3H, s), 7.32-7.34 (2H, m), 8.02 (1H, dd, J = 5.7, 2.1 Hz), 8.22 (1H, d. J = 2.7 Hz), 8.51 (1H, d. J = 5.7 Hz), 14.00 (1H, brs). | 392 | 1.01 | B |
| I-285 | 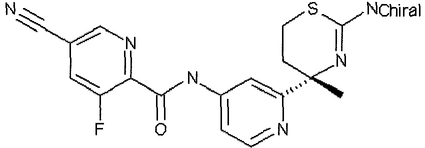 | 1H-NMR (CDCl3) d: 1.57 (3H. s), 1.93 (1H, ddd, J = 13.8, 10.1, 4.1 Hz), 2.46 (1H, ddd, J = 13.8, 6.9, 3.5 Hz), 2.75 (1H, ddd, J = 12.3, 10.1, 3.5 Hz), 2.98 (1H, ddd. J = 12.3, 6.9, 4.1 Hz), 7.42 (1H, d, J = 2.1 Hz), 7.92 (1H, dd, J= 5.1, 2.1 Hz). 7.95 (1H, d, J= 1.6 Hz). 8.55 (1H, d, J =5.1 Hz), 8.73 (1H, d, J =1.6 Hz). | 371 | 0.82 | B |

**[Table 1-41]**

| No. | Structure | NMR(solvent:shift value;ascending order) | MS [M+1] | LC/M S RT | LC/MS Method |
|---|---|---|---|---|---|
| I-286 | 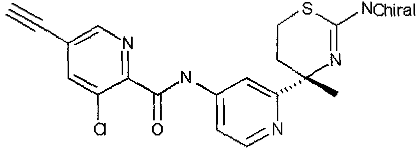 | 1H-NMR (CDCl3) d: 1.56 (3H, s), 1.92 (1H, ddd. J = 13.5, 10.1, 3.8 Hz), 2.46 (1H, ddd, J = 13.5, 6.5, 3.8 Hz), 2.76 (1H, ddd, J = 12.6, 10.1, 3.8 Hz). 2.97 (1H, ddd, J = 12.6, 6.5, 3.8 Hz), 3.45 (1H, s), 7.36 (1H, d, J = 2.0 Hz), 7.96 (1H, d, J = 2.0 Hz), Hz), 7.99 (1H, dd, J = 5.6, 2.0 Hz), 8.52 (1H, d, J = 5.6 Hz), 8.58 (1H, d, J = 2.0 Hz). | 386 | 1.04 | B |
| I-287 | 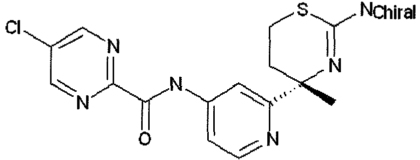 | 1H-NMR (CDCl3) d: 1.57 (3H, s), 1.92 (1H, ddd, J =13.8, 10.2, 3.7 Hz), 2.47 (1H, ddd, J = 13.8, 6.8, 3.9 Hz), 2.75 (1H, ddd, J = 12.0, 10.2, 3.9 Hz), 2.97 (1H. ddd, J = 12.0, 6.8, 3.7 Hz), 7.46 (1H, d, J = 2.4 Hz), 7.99 (1H, dd, J = 5.5, 2.4 Hz), 8.57 (1H, d, J = 5.5 Hz), 8.90 (2H, s). | 363 | 0.75 | B |
| I-288 | 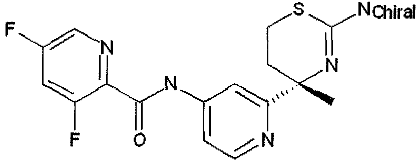 | 1H-NMR (CDCl3) d: 1.57 (3H, s), 1.91 (1H, ddd, J = 13.7, 10.4, 3.8 Hz), 2.46 (1H, ddd. J = 13.7, 6.6, 3.9 Hz), 2.75 (1H, ddd, J = 12.2, 10.4, 3.9 Hz), 2.97 (1H, ddd. J = 12.2, 6.6. 3.8 Hz), 7.37-7.44 (2H, m), 7.94 (1H, dd, J = 5.4, 2.0 Hz), 8.37 (1H, d, J = 20 Hz), 8.53 (1H, d. J = 5.4 Hz), 9.76 (1H, brs). | 364 | 0.81 | B |
| I-289 | 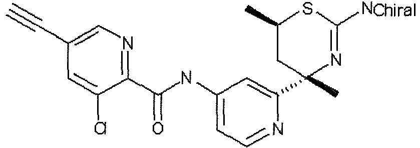 | 1H-NMR (CDCl3) d: 1.22 (3H, d. J = 6.3 Hz), 1.34 (1H, t, J = 13.2 Hz), 1.60 (3H, s), 2.76-2.84 (1H, m), 2.89 (1H, dd, J = 13.2, 3.0 Hz), 3.46 (1H, s), 7.17 (1H, d, J = 1.8Hz), 7.97 (1H, d, J= 1.8 Hz), 8.03 (1H, d, J = 5.6, 1.8 Hz). 8.53 (1H, d, J = 5.6 Hz), 8.59 (1H, d, J = 1.8 Hz). | 400 | 1.1 | B |
| I-290 | 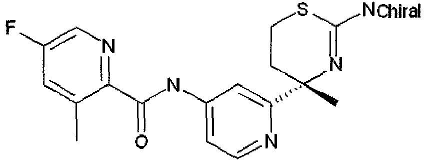 | 1H-NMR (CDCl3) d: 1.57 (3H, s). 1.91 (1H, ddd, J = 13.7, 10.1, 3.9 Hz), 2.47 (1H, ddd, J = 13.7, 6.9, 3.8 Hz), 2.76 (1H, ddd, J = 12.3, 10.1, 3.8 Hz), 2.83 (3H, s), 297 (1H, ddd, J = 12.3, 6.9, 3.9 Hz). 7.36-7.40 (2H, m), 7.95 (1H, dd, J = 5.6, 2.2 Hz), 8.32 (1H, d, J = 2.2 Hz), 8.51 (1H, d, J=5.6 Hz), 10.15 (1H, brs). | 360 | 1.01 | B |

**[Table 1-42]**

| No. | Structure | NMR(solvent:shiftvalue;ascending order) | MS [M+1] | LC/M S RT | LC/MS method |
|---|---|---|---|---|---|
| I-291 | 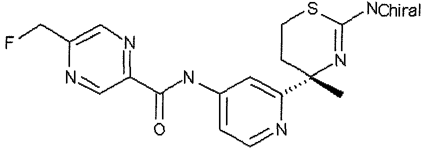 | 1H-NMR (CDCl3) d: 1.58 (3H. s), 1.93 (1H, ddd, J = 13.8, 10.4, 4.1 Hz), 2.49 (1H, ddd, J = 13.8. 6.9, 3.6 Hz), 2.76 (1H, ddd, J = 12.3, 10.4, 3.6 Hz), 2.98 (1H, ddd, J = 12.3, 6.9, 4.1 Hz), 5.68 (2H. d, J = 46.5 Hz), 7.49 (1H, d, J = 2.1 Hz), 7.93 (1H, dd, J = 5.4. 2.1 Hz), 8.57 (1H, d. J = 5.4 Hz), 8.77 (1H, s), 9.45 (1H, s), 9.79 (1H, brs), | 361 | 0.77 | B |
| I-292 | 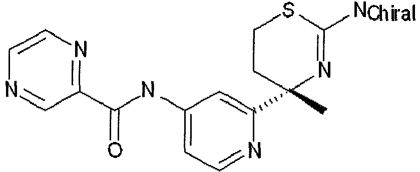 | 1H-NMR (CDCl3) d: 1.57 (3H, s), 1.92 (1H, ddd, J = 13.8, 10.2 4.1 Hz), 2.46 (1H, ddd, J = 13.8. 6.6, 3.8 Hz), 2.75 (1H, ddd, J = 12.5, 10.2, 3.8 Hz), 2.98 (1H, ddd, J = 12.5, 6.6, 4.1 Hz), 7.47 (1H, d, J = 2.1 Hz), 7.93 (1H, dd, J = 5.6. 2.1 Hz), 8.55 (1H, d, J = 5.6 Hz), 8.61 (1H, m), 8.84 (1H, m), 9.50 (1H, m), 9.80 (1H, brs). | 329 | 0.67 | B |
| I-293 | 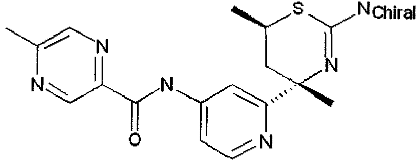 | 1H-NMR (CDCl3) d: 1.22 (3H, d. J = 6.6 Hz), 1.35 (1H, dd, J = 13.2, 12.0 Hz), 1.61 (3H, s), 2.71 (3H, s), 2.76-2.84 (1H, m), 2.88 (1H, dd, J = 13.2, 3.0 Hz). 7.31 (1H. d, J = 2.1 Hz), 7.92 (1H, dd, J = 5.6, 2.1 Hz), 8.46 (1H, s), 8.54 (1H, d, J = 5.6 Hz), 9.36 (1H, s), 9.75 (1H, brs). | 357 | 0.9 | B |
| I-294 | 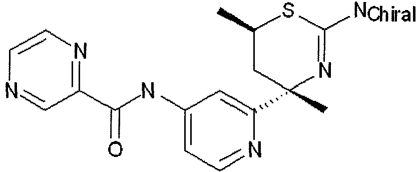 | 1H-NMR (CDCl3) d: 1.22 (3H, d. J = 6.6 Hz), 1.35 (1H, t, J = 12.9 Hz), 1.61 (3H, s), 2.76-2.84 (1H, m), 2.88 (1H, dd, J = 12.9, 3.3 Hz). 7.31 (1H, d, J = 2.1 Hz), 7.94 (1H, dd, J = 5.6, 2.1 Hz), 8.56 (1H, d, J = 5.6 Hz), 8.62 (1H, m), 8.85 (1H, d, J = 2.4 Hz). 9.51 (1H, s). 9.78 (1H, brs). | 343 | 0.82 | B |

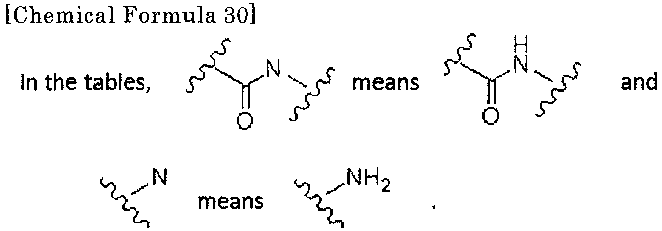

Experimental procedures for biological testing are described bellow:

### (Test Example 1: Assay of BACE-1 inhibiting activity)

48.5 µL of substrate peptide solution (Biotin-XSEVNLDAEFRHDSGC-Eu: X=ε-amino-n-capronic acid, Eu=Europium cryptate) was added to each well of 96-hole half-area plate (a black plate: Costar), and after addition of 0.5 µl of the compound of the present invention (DMSO solution) and 1 µl of Recombinant human BACE-1(R&D Systems), the reaction mixture was incubated at 30 °C for 3.5 hours. The substrate peptide was synthesized by reacting Cryptate TBPCOOH mono SMP (CIS bio international) with Biotin-XSEVNLDAEFRHDSGC (Peptide Institute, Inc.). The final concentrations of the substrate peptide and Recombinant human BACE-1 were adjusted to 18 nmol/L and 7.4 nmol/L, respectively, and the reaction was performed in sodium acetate buffer (50 mmol/L sodium acetate, pH 5.0, 0.008% Triton X-100).

After the incubation for reaction, 50 µl of 8.0 µg/ml Streptavidin-XL665 (CIS bio international) dissolved in phosphate buffer (150 mmol/L K₂ HPO₄ -KH₂ PO₄, pH 7.0, 0.008 % Triton X-100, 0.8 mol/L KF) was added to each well and left stand at 30 °C for 45 minutes. After then, fluorescence intensity was measured (excitation wavelength: 320 nm, measuring wavelength: 620 nm and 665 nm) using Wallac 1420 multilabel counter (Perkin Elmer life sciences). Enzymatic activity was determined from counting ratio of each wavelength (10,000 x Count 665/Count 620) and 50% inhibitory concentration against the enzymatic activity (IC₅₀) was calculated.
Compound I-2: IC₅₀ value 0.033 µmol/L
Compound I-8: IC₅₀ value 0.078 µmol/L
Compound I-11: IC₅₀ value 0.048 µmol/L
Compound I-30: IC₅₀ value 0.048 µmol/L
Compound I-1, 3 to 7, 9, 10, 12 to 29, 31 to 57, 59 to 77, 196 to 226, 228 to 244, 246, 248 to 252, 254 to 261, 265 to 270, 272, 273, 275 to 291, 293 and 294 also showed the IC₅₀ values of 1 µmol/L or less.

### (Test Example 2: Measurement of β-amyloid (Aβ) production inhibitory effect in cell)

Neuroblastoma SH-SY5Y cells (SH/APPwt) with human wild-type β-APP excessively expressed therein are prepared at 8 X 10⁵ cells/mL, and 150 µl portions thereof are inoculated into each well of a 96-well culture plate (Falcon). The cells are cultured for 2 hours at 37 °C in a 5% gaseous carbon dioxide incubator. Then, a solution which have been preliminarily prepared by adding and suspending the compound of the present invention (DMSO (dimethyl sulfoxide) solution) so as to be 2 µl/50 µl medium is added to the cell sap. Namely, the final DMSO concentration is 1%, and the amount of the cell culture is 200 µl. After the incubation is performed for 24 hours from the addition of the test compound, 100 µl of the culture supernatant is collected from each fraction. The amount of the Aβ in each fraction is measured.

The Aβ amount is measured as follows. 10 µl of a homogeneous time resolved fluorescence (HTRF) measurement reagent (Amyloid β 1-40 peptide; CIS bio international) and 10 µl of the culture supernatant are put into a 384-well half area microplate (black microplate, Costar) and mixed with each other, and then left standing overnight at 4 °C while the light is shielded. Then, the fluorescence intensity (excitation wavelength: 337 nm, measurement wavelength: 620 nm and 665 nm) is measured with a micro plate reader (Artemis K-101; FURUNO ELECTRIC). The Aβ amount is determined from the count rate at each measurement wavelength (10000 X Count 665/Count 620), and the amount needed to inhibit Aβ production by 50 % (IC₅₀) is calculated from at least six different dosages.

### (Test Example 3: Lowering effect on brain β amyloid in rats)

Compound of the present invention is suspended in 0.5% methylcellulose, the final concentration is adjusted to 2 mg/mL, and this is orally administered to male Crl:SD rat (7 to 9 weeks old) at 10 mg/kg. In a vehicle control group, only 0.5% methylcellulose is administered, and an administration test is performed at 3 to 8 animals per group. A brain is isolated 3 hours after administration, a cerebral hemisphere is isolated, a weight thereof is measured, the hemisphere is rapidly frozen in liquid nitrogen, and stored at -80°C until extraction date. The frozen cerebral hemisphere is transferred to a homogenizer manufactured by Teflon (registered trademark) under ice cooling, a 4-fold volume of a weight of an extraction buffer (containing 1% CHAPS ({3-[(3-chloroamidopropyl)dimethylammonio]-1-propanesulfonate}), 20 mmol/L Tris-HCl (pH 8.0), 150 mmol/L NaCl, Complete (Roche) protease inhibitor) is added, up and down movement is repeated, and this is homogenized to solubilize for 2 minutes. The suspension is transferred to a centrifugation tube, allowed to stand on an ice for 3 hours or more and, thereafter centrifuged at 100,000 x g, 4°C for 20 minutes. After centrifugation, the supernatant is transferred to an ELISA plate (product No. 294-62501, Wako Junyaku Kogyo) for measuring β amyloid 40. ELISA measurement is performed according to the attached instruction. The lowering effect is calculated as a ratio compared to the brain β amyloid 40 level of vehicle control group of each test.

### (Test Example 4: CYP3A4 fluorescent MBI test)

The CYP3A4 fluorescent MBI test is a test of investigating enhancement of CYP3A4 inhibition of a compound by a metabolism reaction. 7-benzyloxytrifluoromethylcoumarin (7-BFC) is debenzylated by the CYP3A4 enzyme (enzyme expressed in *Escherichia coli*) and 7-hydroxytrifluoromethylcoumarin (7-HFC) is produced as a fluorescing metabolite. The test is performed using 7-HFC production reaction as an index.

The reaction conditions are as follows: substrate, 5.6 µmol/L 7-BFC; pre-reaction time, 0 or 30 minutes; reaction time, 15 minutes; reaction temperature, 25°C (room temperature); CYP3A4 content (expressed in *Escherichia coli*), at pre-reaction 62.5 pmol/mL, at reaction 6.25 pmol/mL (at 10-fold dilution); concentration of the compound of the present invention, 0.625, 1.25, 2.5, 5, 10, 20 µmol/L (six points).

An enzyme in a K-Pi buffer (pH 7.4) and a compound of the present invention solution as a pre-reaction solution were added to a 96-well plate at the composition of the pre-reaction. A part of pre-reaction solution was transferred to another 96-well plate, and 1/10 diluted by a substrate in a K-Pi buffer. NADPH as a co-factor was added to initiate a reaction as an index (without preincubation). After a predetermined time of a reaction, acetonitrile/0.5 mol/L Tris (trishydroxyaminomethane) = 4/1 (v/v) was added to stop the reaction. On the other hand, NADPH was added to a remaining pre-reaction solution in order to initiate a preincubation (with preincubation). After a predetermined time of a preincubation, a part was transferred to another plate, and 1/10 diluted by a substrate in a K-Pi buffer in order to initiate a reaction as an index. After a predetermined time of a reaction, acetonitrile/0.5 mol/L Tris (trishydroxyaminomethane) = 4/1 (v/v) solution was added to stop the reaction. Fluorescent values of 7-HFC as a metabolite were measured in each index reaction plate with a fluorescent plate reader (Ex = 420 nm, Em = 535 nm).

The sample adding DMSO to a reaction system instead of compound of the present invention solution is adopted as a control (100%) because DMSO is used as a solvent to dissolve a compound of the present invention. Remaining activity (%) was calculated at each concentration of the compound of the present invention added as the solution, and IC₅₀ was calculated by reverse-presumption by a logistic model using a concentration and an inhibition rate. When a difference between IC₅₀ values with preincubation from that without IC₅₀ value was 5 µM or more, this was defined as positive (+). When the difference was 3 µM or less, this was defined as negative(-). Compound I-1: (-)

### (Test Example 5: CYP inhibition test)

The CYP inhibition test is a test to assess the inhibitory effect of a compound of the present invention towards typical substrate metabolism reactions on CYP enzymes in human liver microsomes. The marker reactions on human main five CYP enzymes (CYP1A2, 2C9, 2C19, 2D6, and 3A4) were used as follows; 7-ethoxyresorufin O-deethylation (CYP1A2), tolbutamide methyl-hydroxylation (CYP2C9), mephenytoin 4'-hydroxylation (CYP2C19), dextromethorphan O-demethylation (CYP2D6), and terfenedine hydroxylation (CYP3A4). The commercially available pooled human liver microsomes were used as an enzyme resource.

The reaction conditions were as follows: substrate, 0.5 µmol/L ethoxyresorufin (CYP1A2), 100 µmol/L tolbutamide (CYP2C9), 50 µmol/L S-mephenytoin (CYP2C19), 5 µmol/L dextromethorphan (CYP2D6), 1 µmol/L terfenedine (CYP3A4); reaction time, 15 minutes; reaction temperature, 37°C; enzyme, pooled human hepatic microsome 0.2 mg protein/mL; concentration of the compound of the present invention, 1, 5, 10, 20 µmol/L (four points).

Five kinds of substrates, human liver microsomes, and a compound solution of the present invention in 50 mmol/L Hepes buffer were added to a 96-well plate at the composition as described above as a reaction solution. NADPH as a cofactor was added to this 96-well plate in order to initiate metabolism reactions. After the incubation at 37°C for 15 minutes, a methanol/acetonitrile = 1/1 (v/v) solution was added to stop the reaction. After the centrifugation at 3000 rpm for 15 minutes, resorufin (CYP1A2 metabolite) in the supernatant was quantified by a fluorescent plate reader, and hydroxytolbutamide (CYP2C9 metabolite), 4'-hydroxymephenytoin (CYP2C19 metabolite), dextrorphan (CYP2D6 metabolite), and terfenadine alcohol metabolite (CYP3A4 metabolite) in the supernatant were quantified by LC/MS/MS.

The sample adding DMSO to a reaction system instead of compound of the present invention solution was adopted as a control (100 %) because DMSO was used as a solvent to dissolve a compound of the present invention. Remaining activity (%) was calculated at each concentration of a compound of the present invention, and IC₅₀ value was calculated by reverse presumption by a logistic model using a concentration and an inhibition rate.

### Compound I-5: five kinds >20 µM

### (Test Example 6: Fluctuation Ames Test)

The mutagenicity of the compound of the present invention is evaluated.

Each 20 µL of freeze-stored *Salmonella typhimurium* (TA98 and TA100 strain) is inoculated in 10 mL of liquid nutrient medium (2.5% Oxoid nutrient broth No.2), and the cultures are incubated at 37°C under shaking for 10 hours. 9 mL of TA98 culture is centrifuged (2000 x g, 10 minutes) to remove medium, and the bacteria is suspended in 9 mL of Micro F buffer (K₂HPO₄: 3.5 g/L, KH₂PO₄: 1 g/L, (NH₄)₂SO₄: 1 g/L, trisodium citrate dihydrate: 0.25 g/L, MgSO₄ · 7H₂O: 0.1 g/L), and the suspension is added to 110 mL of Exposure medium (Micro F buffer containing Biotin: 8 µg/mL, histidine: 0.2 µg/mL, glucose: 8 mg/mL). 3.16 mL of TA100 culture is added to 120 mL of Exposure medium to prepare the test bacterial solution. 588 µL of the test bacterial solution (or mixed solution of 498 µl of the test bacterial solution and 90 µL of the S9 mix in the case with metabolic activation system) are mixed with each 12 µL of the following solution: DMSO solution of the compound of the present invention (several stage dilution from maximum dose 50 mg/mL at 2 to 3-fold ratio); DMSO as negative control; 50 µg/mL of 4-nitroquinoline-1-oxide DMSO solution as positive control for TA98 without metabolic activation system; 0.25 µg/mL of 2-(2-furyl)-3-(5-nitro-2-furyl)acrylamide DMSO solution as positive control for TA100 without metabolic activation system; 40 µg/mL of 2-aminoanthracene DMSO solution as positive control for TA98 with metabolic activation system; or 20 µg/mL of 2-aminoanthracene DMSO solution as positive control for TA100 with metabolic activation system. A mixed solution is incubated at 37°C under shaking for 90 minutes. 460 µL of the bacterial solution exposed to the compound of the present invention is mixed with 2300 µL of Indicator medium (Micro F buffer containing biotin: 8 µg/mL, histidine: 0.2 µg/mL, glucose: 8 mg/mL, Bromo Cresol Purple: 37.5 µg/mL), each 50 µL is dispensed into 48 wells/dose in the microwell plates, and is subjected to stationary cultivation at 37°C for 3 days. A well containing the bacteria, which has obtained the ability of proliferation by mutation in the gene coding amino acid (histidine) synthetase, turns the color from purple to yellow due to pH change. The number of the yellow wells among the 48 total wells per dose is counted, and evaluate the mutagenicity by comparing with the negative control group. (-) means that mutagenicity is negative and (+) means positive.

### (Test example 7-1: Solubility test)

A 2-fold dilution series (12 points) of a 10 mM solution of a compound of the present invention in DMSO was added to a medium (JP-I, JP-II) (2%), and solubility was assessed by 3 stages (High; > 40 µM, Medium; 3-40 µM, Low; < 3 µM) from a turbidity after 4 hours.

### Compound I-47: High (JP-I, JP-II)

### (Test example 7-2 : Solubility test)

The solubility of each compound of the present invention is determined under 1% DMSO addition conditions. A 10 mmol/L solution of the compound is prepared with DMSO, and 6 µL of the compound of the present invention solution is added to 594 µL of an artificial intestinal juice (water and 118 mL of 0.2 mol/L NaOH reagent are added to 250 mL of 0.2 mol/L potassium dihydrogen phosphate reagent to reach 1000 mL) with a pH of 6.8. The mixture is left standing for 16 hours at 25 °C, and the mixture is vacuum-filtered. The filtrate is two-fold diluted with methanol/water = 1/1 (v/v), and the compound concentration in the filtrate is measured with HPLC or LC/MS/MS by the absolute calibration method.

### (Test Example 8: Metabolism Stability Test)

Using a commercially available pooled human liver microsomes, a compound of the present invention is reacted for a constant time, a remaining rate is calculated by comparing a reacted sample and an unreacted sample, thereby, a degree of metabolism in liver was assessed.

A reaction was performed (oxidative reaction) at 37°C for 0 minute or 30 minutes in the presence of 1 mmol/L NADPH in 0.2 mL of a buffer (50 mmol/L Tris-HCl pH 7.4, 150 mmol/L potassium chloride, 10 mmol/L magnesium chloride) containing 0.5 mg protein/mL of human liver microsomes. After the reaction, 50 µL of the reaction solution was added to 100 µL of a methanol/acetonitrile = 1/1 (v/v), mixed and centrifuged at 3000 rpm for 15 minutes. The compound of the present invention in the supernatant was quantified by LC/MS/MS, and a remaining amount of the compound of the present invention after the reaction was calculated, letting a compound amount at 0 minute reaction time to be 100%.

### Compound I-24: 98%

### (Test Example 9: hERG Test)

For the purpose of assessing risk of an electrocardiogram QT interval prolongation, effects on delayed rectifier K+ current (IKr), which plays an important role in the ventricular repolarization process of the compound of the present invention, is studied using HEK293 cells expressing human ether-a-go-go related gene (hERG) channel.

A cell is retained at a membrane potential of -80 mV by whole cell patch clamp method using an automated patch clamp system (PatchXpress 7000A, Axon Instruments Inc.). I_{Kr} induced by depolarization pulse stimulation at +40 mV for 2 seconds and, further, repolarization pulse stimulation at -50 mV for 2 seconds is recorded.

After the generated current is stabilized, extracellular solution (NaCl: 135 mmol/L, KCl: 5.4 mmol/L, NaH₂PO₄: 0.3 mmol/L, CaCl_{2·}2H₂O: 1.8 mmol/L, MgCl_{2·}6H₂O: 1 mmol/L, glucose: 10 mmol/L, HEPES (4-(2-hydroxyethyl)-1-piperazine ethanesulfonic acid): 10 mmol/L, pH=7.4) in which the compound of the present invention have been dissolved at an objective concentration is applied to the cell under the room temperature condition for 10 minutes. From the recording I_{Kr}, an absolute value of the tail peak current is measured based on the current value at the resting membrane potential using an analysis software (DataXpress ver.1, Molecular Devices Corporation). Further, the % inhibition relative to the tail peak current before application of the compound of the present invention is calculated, and compared with the vehicle-applied group (0.1% dimethyl sulfoxide solution) to assess influence of the compound of the present invention on I_{Kr}.

### (Test Example 10: Powder solubility test)

Appropriate amounts of the compound of the present invention are put into appropriate containers. 200 µL of JP 1st fluid (Dissolve 2.0 g of sodium chloride in 7.0 mL of hydrochloric acid and water to make 1000 mL), 200 µL of JP 2nd fluid (A mixture of phosphate buffer (pH 6.8) and water (1:1)), and 200 µL of JP 2nd fluid containing 20 mmol/L of sodium taurocholate (TCA) (TCA 1.08 g and JP 2nd fluid to make 100 mL) were added to the respective containers. When the compound of the present invention is dissolved after the addition of the test fluid, the bulk powder is added as appropriate. The containers are sealed, and shaken for 1 hour at 37°C. The mixtures are filtered, and 100 µL of methanol is added to each of the filtrate (100 µL) so that the filtrates are two-fold diluted. The dilution ratio may be changed if necessary. After confirming that there is no bubbles and precipitates in the diluted solution, the containers are sealed and shaken. Quantification is performed by HPLC with an absolute calibration method.

### (Test Example 11: BA test)

Materials and methods for studies on oral absorption
(1) Animal: mouse or SD rat
(2) Breeding conditions: mouse or SD rat is allowed free access to the sterilized tap water and the solid food.
(3) Dose and grouping: orally or intravenously administered at a predetermined dose; grouping was as follows (Dose depends on the compound)
   Oral administration: 1 to 30 mg/kg (n=2 to 3)
   Intravenous administration: 0.5 to 10 mg/kg (n=2 to 3)
(4) Preparation of dosing formulation: for oral administration, in a solution or a suspension state; for intravenous administration, in a solubilized state
(5) Dosing method: in oral administration, forcedly and intragastrically administer using a syringe attached a flexible feeding tube; in intravenous administration, administer from caudal vein using a syringe attached with a needle.
(6) Evaluation items: blood was collected at the scheduled time, and the plasma concentration of the compound of the present invention was measured by LC/MS/MS
(7) Statistical analysis: regarding the transition of the plasma concentration of the compound of the present invention, the area under the plasma concentration-time curve (AUC) was calculated by non-linear least squares program WinNonlin (Registered trademark), and the bioavailability (BA) of the compound of the present invention was calculated from the AUCs of the oral administration group and intravenous administration group

### Compound I-10: 96.7%

### (Test Example 12: Brain distribution studies)

Compound of the present invention is intravenously administered to a rat at 0.5 mg/mL/kg dosage. 30 minutes later, all blood is drawn from the abdominal aorta under isoflurane anesthesia for death from exsanguination.

The brain is enucleated and 20 to 25% of homogenate thereof is prepared with distilled water.

The obtained blood is used as plasma after centrifuging. The control plasma is added to the brain sample at 1:1. The control brain homogenate is added to the plasma sample at 1:1. Each sample is measured using LC/MS/MS. The obtained area ratio (a brain/plasma) is used for the brain Kp value.

### (Test Example 13: Ames test)

Ames test is performed by using Salmonellas (*Salmonella typhimurium*) TA 98, TA100, TA1535 and TA1537 and *Escherichia coli* WP2uvrA as test strains with or without metabolic activation in the pre-incubation method to check the presence or absence of gene mutagenicity of compounds of the present invention.

### (Test Example14: P-gp Substrate Test)

Compound of the present invention is added in the culture insert of the Transwell wherein human MDR1 expressing cells or parent cells are monolayer cultivated, and reacted for a predetermined period of times. The compound of the present invention is investigated whether a P-gp substrate or not by comparing Efflux Ratio (ER) values of MDR1 expressing cells and parent cells. Here, ER is calculated from the membrane permeability coefficients of the direction from Basolateral side to Apical side (B to A) and the direction from Apical side to Basolateral side (A to B)) of MDR1 expressing cells and parent cells.

### Formulation Examples

The following Formulation Examples are only exemplified and not intended to limit the scope of the present invention.

### Formulation Example 1 Tablet

| | |
|---|---|
| Compound of the present invention | 15 mg |
| Lactose | 15 mg |
| Calcium stearate | 3 mg |

All of the above ingredients except for calcium stearate are uniformly mixed. Then the mixture is crushed, granulated and dried to obtain a suitable size of granules. Then, calcium stearate is added to the granules. Finally, tableting is performed under a compression force.

### Formulation Example 2 Capsules

| | |
|---|---|
| Compound of the present invention | 10 mg |
| Magnesium stearate | 10 mg |
| Lactose | 80 mg |

The above ingredients are mixed uniformly to obtain powders or fine granules, and then the obtained mixture is filled in capsules.

### Formulation Example 3 Granules

| | |
|---|---|
| Compound of the present invention | 30 g |
| Lactose | 265 g |
| Magnesium stearate | 5 g |

After the above ingredients are mixed uniformly, the mixture is compressed. The compressed matters are crushed, granulated and sieved to obtain suitable size of granules.

### [Industrial Applicability]

The compounds of the present invention can be a useful therapeutic or prophylactic agent for diseases induced by production, secretion and/or deposition of amyloid β proteins.

## Claims

1. A compound of formula (I): 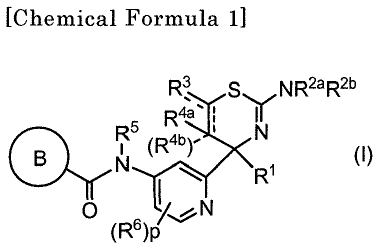 wherein ring B is a substituted or unsubstituted carbocycle or a substituted or unsubstituted heterocycle,
R¹ is substituted or unsubstituted alkyl, substituted or unsubstituted alkenyl, substituted or unsubstituted alkynyl, substituted or unsubstituted acyl, cyano, carboxy, substituted or unsubstituted alkoxycarbonyl, substituted or unsubstituted alkenyloxycarbonyl, substituted or unsubstituted alkynyloxycarbonyl, substituted or unsubstituted carbamoyl, substituted or unsubstituted thiocarbamoyl, a substituted or unsubstituted carbocyclic group or a substituted or unsubstituted heterocyclic group, R^{2a} and R^{2b} are each independently hydrogen, substituted or unsubstituted alkyl, substituted or unsubstituted acyl, substituted or unsubstituted alkoxycarbonyl or substituted or unsubstituted carbamoyl, 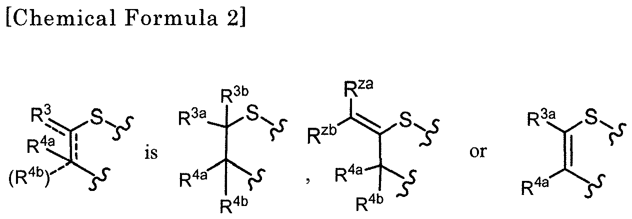 wherein R^{3a}, R^{3b}, R^{4a} and R^{4b} are each independently
hydrogen, halogen, hydroxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted alkylthio, substituted or unsubstituted alkenylthio, substituted or unsubstituted alkynylthio, substituted or unsubstituted acyl, substituted or unsubstituted acyloxy, cyano, nitro, carboxy, substituted or unsubstituted alkoxycarbonyl, substituted or unsubstituted alkenyloxycarbonyl, substituted or unsubstituted alkynyloxycarbonyl, substituted or unsubstituted amino, substituted or unsubstituted carbamoyl, substituted or unsubstituted thiocarbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl, substituted or unsubstituted alkynylsulfonyl, substituted or unsubstituted carbocyclyl, substituted or unsubstituted carbocyclyloxy, substituted or unsubstituted carbocyclylthio, substituted or unsubstituted carbocyclylalkyl, substituted or unsubstituted carbocyclylalkoxy, substituted or unsubstituted carbocyclyloxycarbonyl, substituted or unsubstituted carbocyclylsulfinyl, substituted or unsubstituted carbocyclylsulfonyl, substituted or unsubstituted heterocyclyl, substituted or unsubstituted heterocyclyloxy, substituted or unsubstituted heterocyclylthio, substituted or unsubstituted heterocyclylalkyl, substituted or unsubstituted heterocyclylalkoxy, substituted or unsubstituted heterocyclyloxycarbonyl, substituted or unsubstituted heterocyclylsulfinyl or substituted or unsubstituted heterocyclylsulfonyl,
R^{3a} and R^{3b} together with the carbon atom to which they are attached may form a substituted or unsubstituted carbocycle or a substituted or unsubstituted heterocycle,
R^{4a} and R^{4b} together with the carbon atom to which they are attached may form a substituted or unsubstituted carbocycle or a substituted or unsubstituted heterocycle,
R^{za} and R^{zb} are each independently hydrogen, halogen, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted alkylthio, substituted or unsubstituted alkenylthio, substituted or unsubstituted alkynylthio, substituted or unsubstituted acyl, carboxy, substituted or unsubstituted alkoxycarbonyl, substituted or unsubstituted alkenyloxycarbonyl, substituted or unsubstituted alkynyloxycarbonyl, substituted or unsubstituted amino, substituted or unsubstituted carbamoyl, substituted or unsubstituted thiocarbamoyl, substituted or unsubstituted carbocyclyl, substituted or unsubstituted carbocyclyloxy, substituted or unsubstituted carbocyclylthio, substituted or unsubstituted carbocyclylalkyl, substituted or unsubstituted carbocyclylalkoxy, substituted or unsubstituted carbocyclyloxycarbonyl, substituted or unsubstituted heterocyclyl, substituted or unsubstituted heterocyclyloxy, substituted or unsubstituted heterocyclylthio, substituted or unsubstituted heterocyclylalkyl, substituted or unsubstituted heterocyclylalkoxy or substituted or unsubstituted heterocyclyloxycarbonyl, or
R^{za} and R^{zb} together with the carbon atom to which they are attached may form a substituted or unsubstituted non-aromatic carbocycle or a substituted or unsubstituted non-aromatic heterocycle,
R⁵ is hydrogen, substituted or unsubstituted alkyl, substituted or unsubstituted alkenyl, substituted or unsubstituted alkynyl or substituted or unsubstituted acyl,
R⁶ is each independently halogen, hydroxy, substituted or unsubstituted alkyl, substituted or unsubstituted alkenyl, substituted or unsubstituted alkynyl, substituted or unsubstituted alkoxy, substituted or unsubstituted alkenyloxy, substituted or
unsubstituted alkynyloxy, substituted or unsubstituted alkylthio, substituted or unsubstituted alkenylthio, substituted or unsubstituted alkynylthio, substituted or unsubstituted acyl, substituted or unsubstituted acyloxy, cyano, nitro, carboxy, substituted or unsubstituted alkoxycarbonyl, substituted or unsubstituted alkenyloxycarbonyl, substituted or unsubstituted alkynyloxycarbonyl, substituted or unsubstituted amino, substituted or unsubstituted carbamoyl, substituted or unsubstituted thiocarbamoyl, substituted or unsubstituted sulfamoyl, substituted or unsubstituted alkylsulfinyl, substituted or unsubstituted alkenylsulfinyl, substituted or unsubstituted alkynylsulfinyl, substituted or unsubstituted alkylsulfonyl, substituted or unsubstituted alkenylsulfonyl or substituted or unsubstituted alkynylsulfonyl,
p is an integer of 0 to 3,
provided that the following compounds are excluded: 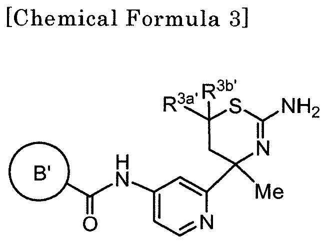 wherein R^{3a'} and R^{3b'} are both hydrogen or both methyl,
ring B' is 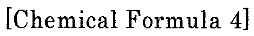 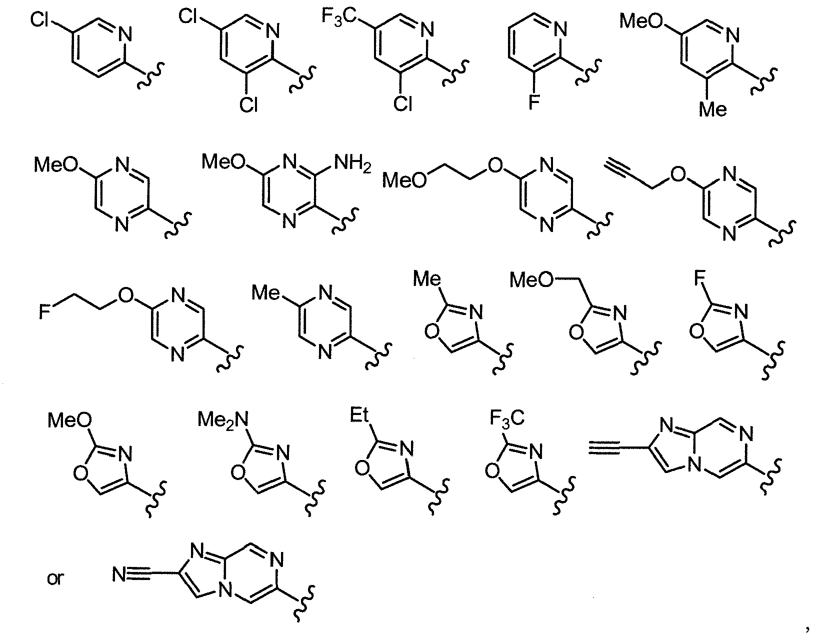 or a pharmaceutically acceptable salt thereof.

2. The compound according to claim 1wherein R¹ is C1 to C3 halogenoalkyl, or a pharmaceutically acceptable salt thereof.

3. The compound according to claim 1 wherein R¹ is C1 to C3 unsubstituted alkyl, or a pharmaceutically acceptable salt thereof.

4. The compound according to any one of claims 1 to 3 wherein 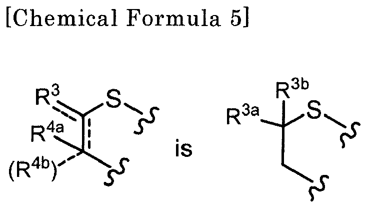 wherein R^{3a} and R^{3b} are each independently hydrogen, substituted or unsubstituted alkyl, or a pharmaceutically acceptable salt thereof.

5. The compound according to claim 4 wherein one of R^{3a} and R^{3b} is hydrogen and the other is substituted or unsubstituted alkyl, or a pharmaceutically acceptable salt thereof.

6. The compound according to claim 4 wherein one of R^{3a} and R^{3b} is hydrogen and the other is halogenoalkyl, or a pharmaceutically acceptable salt thereof.

7. The compound according to claim 4 wherein R^{3a} and R^{3b} are both hydrogen or both alkyl, and ring B is any one of the followings:
1) pyridine which has at least one substituent selected from the group of dihalogenoalkyl, halogenoalkoxy, alkoxyalkoxy, cyanoalkoxy, alkenyl, alkoxyalkenyl, alkynyl, halogenoalkynyl, alkynyloxy, alkylthio, cyanoalkylthio, cyano, amino and cycloalkyl and which may have additional substituents,
2) pyrazine optionally substituted with one or more selected from the group of halogen, halogenoalkyl, monohalogenomethoxy, monohalogenopropyloxy, dihalogenoalkoxy, trihalogenoalkoxy, ethoxyethoxy, cyanoalkoxy, alkenyl, alkynyl, halogenoalkynyl, alkylthio, cyanoalkylthio, cyano and amino, or
3) substituted or unsubstituted benzene,
or a pharmaceutically acceptable salt thereof.

8. The compound according to any one of claims 1 to 3wherein 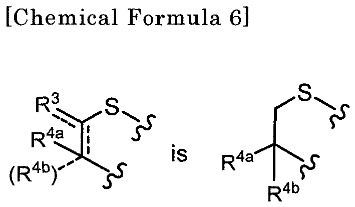 wherein R^{3a} and R^{3b} are each independently hydrogen, substituted or unsubstituted alkyl, or a pharmaceutically acceptable salt thereof.

9. The compound according to any one of claims 1 to 3 wherein 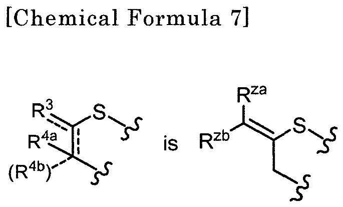 wherein R^{za} and R^{zb} are each independently hydrogen, halogen or substituted or unsubstituted alkyl,
or a pharmaceutically acceptable salt thereof.

10. The compound according to any one of claims 1 to 3 wherein 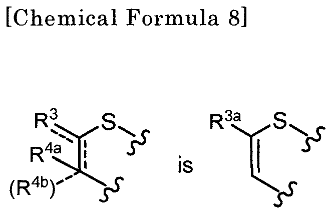 wherein R^{3a} is hydrogen or substituted or unsubstituted alkyl,
or a pharmaceutically acceptable salt thereof.

11. The compound according to any one of claims 1 to 6 and 8 to 10 wherien ring B is substituted or unsubstituted pyridine, substituted or unsubstituted pyrimidine, substituted or unsubstituted pyrazine, substituted or unsubstituted furan, substituted or unsubstituted oxazole, substituted or unsubstituted thiazole, substituted or unsubstituted pyrazole, substituted or unsubstituted benzene, substituted or unsubstituted benzoxazole, substituted or unsubstituted benzothiazole, substituted or unsubstituted dihydrofuropyridine, substituted or unsubstituted dihydrodioxinopyridine or substituted or unsubstituted furopyridine, or a pharmaceutically acceptable salt thereof.

12. The compound according to any one of claims 1 to 6 and 8 to 11 wherein ring B is optionally substituted with one or more selected from the group of halogen, hydroxy, substituted or unsubstituted alkyl, substituted or unsubstituted alkenyl, substituted or unsubstituted alkynyl, substituted or unsubstituted alkoxy, substituted or unsubstituted alkenyloxy, substituted or unsubstituted alkynyloxy, substituted or unsubstituted alkylthio, substituted or unsubstituted alkenylthio, substituted or unsubstituted alkynylthio, cyano, nitro, substituted or unsubstituted amino and a substituted or unsubstituted carbocyclic group,
or a pharmaceutically acceptable salt thereof.

13. The compound according to any one of claims 1 to 12 wherein R^{2a} and R^{2b} are both hydrogen, or a pharmaceutically acceptable salt thereof.

14. A pharmaceutical composition comprising the compound according to any one of claims 1 to 13, or a pharmaceutically acceptable salt thereof.

15. A pharmaceutical composition having BACE1 inhibitory activity comprising the compound according to any one of claims 1 to 10, or a pharmaceutically acceptable salt thereof.

16. A method for inhibiting BACE1 activity comprising administering the compound according to any one of claims 1 to 13, or a pharmaceutically acceptable salt thereof.

17. A compound according to any one of claims 1 to 13, or a pharmaceutically acceptable salt thereof for use in a method for inhibiting BACE1 activity.
